# EUROPEAN PATENT APPLICATION

(11) **EP 4 365 176 A1**
(43) Date of publication of application: **08.05.2024**
(21) Application number: 22832195.6
(22) Date of filing: 30.06.2022
(51) Int. Cl.: C07D 498/22, C07D 519/00, A61P 35/00, A61K 31/553

(54) **KRAS G12D INHIBITOR AND USE THEREOF**

(30) Priority: 02.07.2021 CN 202110752493; 30.07.2021 CN 202110873254; 10.09.2021 CN 202111062878; 19.11.2021 CN 202111398357; 14.01.2022 CN 202210042057; 15.06.2022 CN 202210675157
(71) Applicant: Shanghai de Novo Pharmatech Co., Ltd., Shanghai 201203 (CN)
(72) Inventor: WANG, Feng, Shanghai 201203 (CN); WANG, Jinglu, Shanghai 201203 (CN); ZHANG, Chuanxiu, Shanghai 201203 (CN); ZHOU, Feng, Shanghai 201203 (CN); ZHANG, Man, Shanghai 201203 (CN); GAO, Daxin, Shanghai 201203 (CN)
(74) Representative: Cabinet Becker et Associés
(86) International application number: PCT/CN2022/103056
(87) International publication number: WO 2023/274383

(57) **Abstract**

A KRAS inhibitor compound represented by formula (I), and an isomer or a pharmaceutically acceptable salt thereof. The compound represented by formula (I) and the composition thereof can effectively treat diseases related to KRAS mutation.

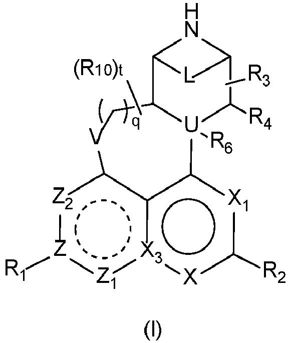

## Description

The present application claims the right of the priorities of Chinese patent application CN202110752493.4 filed on July 2, 2021, Chinese patent application CN202110873254.4 filed on July 30, 2021, Chinese patent application CN202111062878.4 filed on September 10, 2021, Chinese patent application CN202111398357.6 filed on November 19, 2021, Chinese patent application CN202210042057.2 filed on January 14, 2022, and Chinese patent application CN202210675157.9 filed on June 15, 2022. The contents of the above Chinese patent applications are incorporated herein by reference in its entirety.

### TECHNICAL FIELD

The present disclosure relates to a novel compound that inhibits KRAS G12D activity. In particular, the present disclosure relates to a compound that irreversibly inhibits KRAS G12D activity, a pharmaceutical composition comprising the same, and a use thereof as a therapeutic agent for cancer.

### BACKGROUND

The RAS gene family mainly includes three genes: KRAS, NRAS, and HRAS. RAS gene is a proto-oncogene, which becomes an oncogene with carcinogenic activity when activated. RAS encodes a group of monomeric globular proteins (21 kD) consisting of 188 to 189 amino acids, known as p21 proteins or RAS proteins. RAS protein is a GDP/GTP-binding protein that can cycle between an inactive GDP-bound state and an active GTP-bound state, acting as a "molecular switch". The GDP/GTP cycle of RAS is subjected to activation by guanine nucleotide exchange factors (e.g., SOS or RASGRP) and inactivation by GTPase-activating proteins (GAPs, e.g., p120GAP or neurofibromin). The interaction between GAPs and RAS can greatly accelerate the conversion of GTP to GDP. The off switch inactivates RAS. Any mutation affecting the interaction between RAS and GAP or the ability to convert GTP back to GDP will allow for the continued activation of RAS and consequent prolonged cell signaling. Since RAS can regulate cell proliferation, differentiation, and senescence through a variety of important signaling pathways, prolonged activation of downstream signaling pathways (e.g., PI3K-AKT-mTOR, RAF-MEK-ERK, and RALGDS-RAL) will eventually lead to cancer development.

RAS proteins include a highly conserved N-terminal G domain that also contains p-loop, switch I (residues 30 to 40), and switch II (residues 60 to 76) binding nucleotides. Among them, p-loop is a rigid part of the domain with conserved amino acid residues (glycine 12, threonine 26, and lysine 16), which is necessary for amino acid binding and hydrolysis. Threonine 35 and glycine 60 in switch I and switch II can form hydrogen bonds with the γ-phosphate/phosphoric ester of GTP to maintain the active conformation of the switch I and II regions, respectively. After GTP hydrolysis and phosphate/phosphoric ester release, both can relax into an inactive GDP conformation. In addition, RAS proteins also include a C-terminal extension called CAAX box, which can undergo post-translational modifications and is responsible for targeting the protein to the cell membrane (Jonathan et al. Nature Reviews, 2016, 15: 771-785).

RAS genes can keep RAS in a persistently activated state through point mutation, overexpression, gene insertion, and translocation, the most common of which is point mutation. Point mutations in RAS genes are present in approximately 30% of human malignant tumors. The most common are point mutations in KRAS, and the common mutation sites are codons 12, 13, and 61, with codon 12 mutations being the most common. These activating mutations increase the content of RAS protein in the GTP-bound state by inactivating endogenous GTPase activity and causing GTPase-activating protein resistance (Zenker et al. J Med Genet, 2007, 44: 131-135).

Abnormal expression of KRAS accounts for 20% of all malignant tumors, among which G12D mutation is one of the most common mutations. The incidence of G12D mutations is about 25% in patients with pancreatic ductal adenocarcinoma, about 13.3% in patients with colorectal cancer, about 10.1% in patients with rectal cancer, about 4.1% in patients with non-small cell lung cancer, and about 1.7% in patients with small cell lung cancer (Cancer Discovery, 2017, 7(8): 818-831).

More and more studies have demonstrated the critical role of KRAS, especially mutants including KRAS G12D, in malignant tumors, making KRAS an important target in the pharmaceutical industry. However, there are currently no relevant drugs on the market. Therefore, there is an urgent need to develop novel inhibitors of KRAS mutants including KRAS G12D.

### CONTENT OF THE PRESENT INVENTION

The technical problem to be solved by the present disclosure is to provide a novel compound with KRAS G12D inhibitory activity, which can effectively treat various diseases related to KRAS G12D, such as cancer.

The present disclosure provides a compound of formula (I), a stereoisomer thereof, a stable deuterated derivative thereof, or a pharmaceutically acceptable salt thereof; 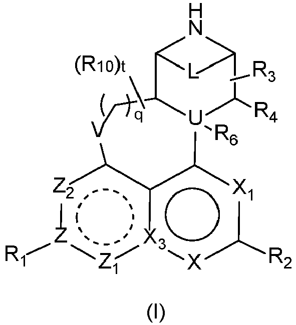
wherein V is -CH₂-, -O-, -S-, or -NR₁₀ₐ-;
Z, Z₁, and Z₂ are any combination of the following:
   1) Z is C; Z₁ and Z₂ are each independently CR₈ or N; or
   2) Z is N or CH; Z₁ and Z₂ are each independently NR₈, C(R₈)₂, or C(O);
Xis N or CH;
X₁ is N or CR₇;
X₃ is N or C;
R₁ is C₆₋₁₂ aryl or 5- to 12-membered heteroaryl; the R₁ is unsubstituted or selectively substituted at any position by one or more than one R₉;
R₂ is hydrogen, -R_{A}, -NR_{A}R_{B}, -OR_{A}, or -SR_{A};
R₃ and R₄ are each independently hydrogen, hydroxyl, cyano, amino, C₁₋₆ alkyl, C₁₋₆ alkylamino, C₁₋₆ alkoxy, halo-C₁₋₆ alkoxy, 5- to 6-membered heteroaryl, -C(O)R", -C(O)OR", or -C(O)N(R")₂; in R₃ or R₄, the C₁₋₆ alkyl is unsubstituted or selectively substituted at any position by one or more than one substituent selected from halogen, hydroxyl, amino, carboxyl, cyano, C₁₋₆ alkoxy, halo-C₁₋₆ alkoxy, and C₁₋₆ alkylamino;
U is N or C; when U is N, L is -(CH₂)ₘW(CH₂)ₘ-, and R₆ is absent; or when U is C, L is absent or -(CH₂)ₘW(CH₂)ₘ-, and R₆ is hydrogen, halogen, or C₁₋₆ alkyl; in R₆, the C₁₋₆ alkyl is unsubstituted or selectively substituted at any position by one or more than one substituent selected from halogen, hydroxyl, amino, carboxyl, cyano, C₁₋₆ alkoxy, halo-C₁₋₆ alkoxy, and C₁₋₆ alkylamino;
the R₄ and R₆ are each independent substituents, or R₄ and R₆ are attached to each other to form a double bond, a C₃₋₈ cycloalkyl group, or a 3- to 8-membered heterocycloalkyl group; the C₃₋₈ cycloalkyl or 3- to 8-membered heterocycloalkyl is unsubstituted or selectively substituted at any position by one or more than one R₁₀;
W is a linkage bond, -CH=CH-, 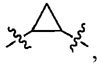 -NH-, -O-, -S-, or -S(O)₁₋₂-;
R₇ is hydrogen, halogen, or cyano;
R₈ is hydrogen, halogen, cyano, amino, hydroxyl, C₁₋₆ alkyl, C₁₋₆ alkoxy, halo-C₁₋₆ alkyl, or halo-C₁₋₆ alkoxy;
each R₉ is independently hydrogen, hydroxyl, halogen, cyano, amino, C₁₋₆ alkyl, C₂₋₆ alkenyl, C₂₋₆ alkynyl, C₃₋₈ cycloalkyl, 3- to 8-membered heterocycloalkyl, C₆₋₁₀ aryl, 5- to 10-membered heteroaryl, -N(R')₂, -OR', -SR', -NHC(O)R", -NHC(O)N(R")₂, -C(O)R", - C(O)N(R")₂, -OC(O)R", -OC(O)N(R")₂, or -B(OR")₂; in R₉, the C₁₋₆ alkyl, C₂₋₆ alkenyl, C₂₋₆ alkynyl, or C₃₋₈ cycloalkyl is unsubstituted or selectively substituted at any position by one or more than one substituent selected from deuterium, halogen, cyano, amino, C₁₋₆ alkyl, C₃₋₈ cycloalkyl, 3- to 8-membered heterocycloalkyl, C₆₋₁₀ aryl, 5- to 10-membered heteroaryl, - N(R')₂, -OR', -SR', -NHC(O)R", -C(O)R", -C(O)N(R")₂, -OC(O)R", -OC(O)N(R")₂, and - B(OR")₂;
each R₁₀ is independently hydrogen, halogen, hydroxyl, cyano, amino, C₁₋₆ alkyl, oxo, C₁₋₆ alkylene, C₂₋₆ alkenyl, C₂₋₆ alkynyl, -N(R')₂, -OR', -C(O)R", -C(O)OR", or -C(O)N(R")₂; in R₁₀, the C₁₋₆ alkyl, C₁₋₆ alkylene, C₂₋₆ alkenyl, or C₂₋₆ alkynyl is unsubstituted or selectively substituted at any position by one or more than one substituent selected from deuterium, halogen, hydroxyl, amino, cyano, -N(R')₂, and -OR';
R₁₀ₐ is hydrogen or C₁₋₆ alkyl; the C₁₋₆ alkyl is unsubstituted or selectively substituted at any position by one or more than one substituent selected from halogen, hydroxyl, amino, cyano, -N(R')₂, and -OR';
R_{A} is hydrogen, C₁₋₆ alkyl, C₂₋₆ alkenyl, C₂₋₆ alkynyl, C₆₋₁₀ aryl, 5- to 10-membered heteroaryl, C₃₋₁₀ cycloalkyl, 3- to 10-membered heterocycloalkyl, C₃₋₁₀ cycloalkyl-C₁₋₆ alkyl, 3- to 10-membered heterocycloalkyl-C₁₋₆ alkyl, C₆₋₁₀ aryl-C₁₋₆ alkyl, or 5- to 10-membered heteroaryl-C₁₋₆ alkyl; the R_{A} is unsubstituted or selectively substituted at any position by one or more than one R_{c};
R_{B} is hydrogen, cyano, hydroxyl, or C₁₋₆ alkyl;
R_{A} and R_{B} are independent substituents, or R_{A} and R_{B} are attached to each other to form a 4- to 8-membered heterocycloalkyl group; the heterocycloalkyl is unsubstituted or selectively substituted at any position by one or more than one substituent selected from hydroxyl, cyano, amino, oxo, halogen, C₁₋₆ alkyl, halo-C₁₋₆ alkyl, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, 5- to 10-membered heteroaryl-C₁₋₄ alkyl, -C(O)R", -(CH₂)ₙOR", and -(CH₂)ₙN(R")₂;
R_{c} is hydroxyl, cyano, amino, oxo, halogen, C₁₋₆ alkyl, C₁₋₆ alkylene, halo-C₁₋₆ alkyl, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, 5- to 10-membered heteroaryl-C₁₋₄ alkyl, -(CH₂)ₙOR', -(CH₂)ₙN(R')₂, -(CH₂)ₙ-N(CN)R', - (CH₂)ₙ-C(O)R', -(CH₂)ₙ-C(O)N(R')₂, -(CH₂)ₙ-S(O)₂N(R')₂, -(CH₂)ₙ-NR"C(O)R', -(CH₂)ₙ-NR"S(O)₂R', -(CH₂)ₙ-N(NH)N(R')₂, -(CH₂)ₙ-NR"C(O)N(R')₂, -(CH₂)ₙ-NR"C(O)OR', -(CH₂)ₙ-OC(O)R', -(CH₂)ₙ-OC(O)N(R')₂, or -(CH₂)ₙ-B(OR")₂; in R_{c}, the C₁₋₆ alkylene, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, or 5- to 10-membered heteroaryl-C₁₋₄ alkyl is unsubstituted or selectively substituted at any position by one or more than one substituent selected from halogen, hydroxyl, amino, cyano, C₁₋₆ alkoxy, C₁₋₆ alkylamino, and C₁₋₆ alkyl;
each R' is independently hydrogen, C₁₋₄ alkyl, halo-C₁₋₄ alkyl, hydroxy-C₁₋₄ alkyl, cyano-C₁₋₄ alkyl, C₁₋₄ alkoxy-C₁₋₄ alkyl, amino-C₁₋₄ alkyl, C₁₋₆ alkylamino-C₁₋₄ alkyl, C₃₋₈ cycloalkyl, 3- to 8-membered heterocycloalkyl, C₆₋₁₀ aryl, 6- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, or 6-to 10-membered heteroaryl-C₁₋₄ alkyl;
each R" is independently hydrogen or C₁₋₆ alkyl;
each m is independently 0, 1, 2, or 3;
n is 0, 1, 2, 3, or 4;
q is 0, 1, 2, or 3;
t is 0, 1, 2, or 3.

All embodiments described below and combinations of variables of formula (I) are included in the scope of the structural formula of formula (I) of the present disclosure.

In some embodiments, in the compound of formula (I), the stereoisomer thereof, the stable deuterated derivative thereof, or the pharmaceutically acceptable salt thereof, some of the groups are defined as follows, and the rest of the groups are defined as described in any other embodiment (hereinafter referred to as "in some embodiments"): 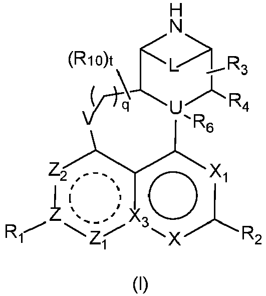
wherein V is -CH₂-, -O-, -S-, or -NR₁₀ₐ-;
Z, Z₁, and Z₂ are any combination of the following:
   1) Z is C; Z₁ and Z₂ are each independently CR₈ or N; or
   2) Z is N or CH; Z₁ and Z₂ are each independently NR₈, C(R₈)₂, or C(O);
Xis N or CH;
X₁ is N or CR₇;
X₃ is N or C;
R₁ is C₆₋₁₂ aryl or 5- to 12-membered heteroaryl; the R₁ is unsubstituted or selectively substituted at any position by one or more than one R₉;
R₂ is hydrogen, -R_{A}, -NR_{A}R_{B}, -OR_{A}, or -SR_{A};
R₃ and R₄ are each independently hydrogen, hydroxyl, cyano, amino, C₁₋₆ alkyl, C₁₋₆ alkylamino, C₁₋₆ alkoxy, halo-C₁₋₆ alkoxy, 5- to 6-membered heteroaryl, -C(O)R", -C(O)OR", or -C(O)N(R")₂; in R₃ or R₄, the C₁₋₆ alkyl is unsubstituted or selectively substituted at any position by one or more than one substituent selected from halogen, hydroxyl, amino, carboxyl, cyano, C₁₋₆ alkoxy, halo-C₁₋₆ alkoxy, and C₁₋₆ alkylamino;
U is N or C; when U is N, L is -(CH₂)ₘW(CH₂)ₘ-, and R₆ is absent; or when U is C, L is absent or -(CH₂)ₘW(CH₂)ₘ-, and R₆ is hydrogen, halogen, or C₁₋₆ alkyl; in R₆, the C₁₋₆ alkyl is unsubstituted or selectively substituted at any position by one or more than one substituent selected from halogen, hydroxyl, amino, carboxyl, cyano, C₁₋₆ alkoxy, halo-C₁₋₆ alkoxy, and C₁₋₆ alkylamino;
the R₄ and R₆ are each independent substituents, or R₄ and R₆ are attached to each other to form a double bond, a C₃₋₈ cycloalkyl group, or a 3- to 8-membered heterocycloalkyl group; the C₃₋₈ cycloalkyl or 3- to 8-membered heterocycloalkyl is unsubstituted or selectively substituted at any position by one or more than one R₁₀;
W is a linkage bond, -CH=CH-, 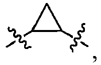 -NH-, -O-, -S-, or -S(O)₁₋₂-;
R₇ is hydrogen, halogen, or cyano;
R₈ is hydrogen, halogen, cyano, amino, hydroxyl, C₁₋₆ alkyl, C₁₋₆ alkoxy, halo-C₁₋₆ alkyl, or halo-C₁₋₆ alkoxy;
each R₉ is independently hydrogen, hydroxyl, halogen, cyano, amino, C₁₋₆ alkyl, C₂₋₆ alkenyl, C₂₋₆ alkynyl, C₃₋₈ cycloalkyl, 3- to 8-membered heterocycloalkyl, C₆₋₁₀ aryl, 5- to 10-membered heteroaryl, -N(R')₂, -OR', -SR', -NHC(O)R", -C(O)R", -C(O)N(R")₂, -OC(O)R", - OC(O)N(R")₂, or -B(OR")z; in R₉, the C₁₋₆ alkyl, C₂₋₆ alkenyl, C₂₋₆ alkynyl, or C₃₋₈ cycloalkyl is unsubstituted or selectively substituted at any position by one or more than one substituent selected from deuterium, halogen, cyano, amino, C₁₋₆ alkyl, C₃₋₈ cycloalkyl, 3- to 8-membered heterocycloalkyl, C₆₋₁₀ aryl, 5- to 10-membered heteroaryl, -N(R')₂, -OR', -SR', -NHC(O)R", - C(O)R", -C(O)N(R")₂, -OC(O)R", -OC(O)N(R")_{z}, and -B(OR")₂;
each R₁₀ is independently hydrogen, halogen, hydroxyl, cyano, amino, C₁₋₆ alkyl, oxo, C₁₋₆ alkylene, C₂₋₆ alkenyl, C₂₋₆ alkynyl, -N(R')₂, -OR', -C(O)R", -C(O)OR", or -C(O)N(R")₂; in R₁₀, the C₁₋₆ alkyl, C₁₋₆ alkylene, C₂₋₆ alkenyl, or C₂₋₆ alkynyl is unsubstituted or selectively substituted at any position by one or more than one substituent selected from deuterium, halogen, hydroxyl, amino, cyano, -N(R')₂, and -OR';
R₁₀ₐ is hydrogen or C₁₋₆ alkyl; the C₁₋₆ alkyl is unsubstituted or selectively substituted at any position by one or more than one substituent selected from halogen, hydroxyl, amino, cyano, -N(R')₂, and -OR';
R_{A} is hydrogen, C₁₋₆ alkyl, C₂₋₆ alkenyl, C₂₋₆ alkynyl, C₆₋₁₀ aryl, 5- to 10-membered heteroaryl, C₃₋₁₀ cycloalkyl, 3- to 10-membered heterocycloalkyl, C₃₋₁₀ cycloalkyl-C₁₋₆ alkyl, 3- to 10-membered heterocycloalkyl-C₁₋₆ alkyl, C₆₋₁₀ aryl-C₁₋₆ alkyl, or 5- to 10-membered heteroaryl-C₁₋₆ alkyl; the R_{A} is unsubstituted or selectively substituted at any position by one or more than one R_{c};
R_{B} is hydrogen, cyano, hydroxyl, or C₁₋₆ alkyl;
R_{A} and R_{B} are independent substituents, or R_{A} and R_{B} are attached to each other to form a 4- to 8-membered heterocycloalkyl group; the heterocycloalkyl is unsubstituted or selectively substituted at any position by one or more than one substituent selected from hydroxyl, cyano, amino, oxo, halogen, C₁₋₆ alkyl, halo-C₁₋₆ alkyl, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, 5- to 10-membered heteroaryl-C₁₋₄ alkyl, -C(O)R", -(CH₂)ₙOR", and -(CH₂)ₙN(R")₂;
R_{c} is hydroxyl, cyano, amino, oxo, halogen, C₁₋₆ alkyl, C₁₋₆ alkylene, halo-C₁₋₆ alkyl, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, 5- to 10-membered heteroaryl-C₁₋₄ alkyl, -(CH₂)ₙOR', -(CH₂)ₙN(R')₂, -(CH₂)ₙ-N(CN)R', - (CH₂)ₙ-C(O)R', -(CH₂)ₙ-C(O)N(R')₂, -(CH₂)ₙ-S(O)₂N(R')₂, -(CH₂)ₙ-NR"C(O)R', -(CH₂)ₙ-NR"S(O)₂R', -(CH₂)ₙ-N(NH)N(R')₂, -(CH₂)ₙ-NR"C(O)N(R')₂, -(CH₂)ₙ-NR"C(O)OR', -(CH₂)ₙ-OC(O)R', -(CH₂)ₙ-OC(O)N(R')₂, or -(CH₂)ₙ-B(OR")₂; in R_{c}, the C₁₋₆ alkylene, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, or 5- to 10-membered heteroaryl-C₁₋₄ alkyl is unsubstituted or selectively substituted at any position by one or more than one substituent selected from halogen, hydroxyl, amino, cyano, C₁₋₆ alkoxy, C₁₋₆ alkylamino, and C₁₋₆ alkyl;
each R' is independently hydrogen, C₁₋₄ alkyl, halo-C₁₋₄ alkyl, hydroxy-C₁₋₄ alkyl, cyano-C₁₋₄ alkyl, C₁₋₄ alkoxy-C₁₋₄ alkyl, amino-C₁₋₄ alkyl, C₁₋₆ alkylamino-C₁₋₄ alkyl, C₃₋₈ cycloalkyl, 3- to 8-membered heterocycloalkyl, C₆₋₁₀ aryl, 6- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, or 6-to 10-membered heteroaryl-C₁₋₄ alkyl;
each R" is independently hydrogen or C₁₋₆ alkyl;
each m is independently 0, 1, 2, or 3;
n is 0, 1, 2, 3, or 4;
q is 0, 1, 2, or 3;
t is 0, 1, 2, or 3.

In some embodiments, 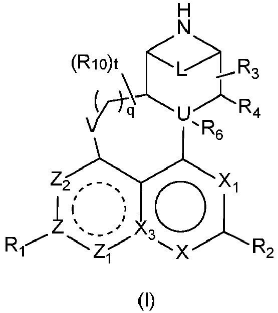
wherein V is -CH₂-, -O-, -S-, or -NR₁₀ₐ-;
Z, Z₁, and Z₂ are any combination of the following:
   1) Z is C; Z₁ and Z₂ are each independently CR₈ or N; or
   2) Z is N or CH; Z₁ and Z₂ are each independently NR₈, C(R₈)₂, or C(O);
Xis N or CH;
X₁ is N or CR₇;
X₃ is N or C;
R₁ is C₆₋₁₂ aryl or 5- to 12-membered heteroaryl; the R₁ is unsubstituted or selectively substituted at any position by one or more than one R₉;
R₂ is hydrogen, -R_{A}, -NR_{A}R_{B}, -OR_{A}, or -SR_{A};
R₃ and R₄ are each independently hydrogen, hydroxyl, cyano, amino, C₁₋₆ alkyl, C₁₋₆ alkylamino, C₁₋₆ alkoxy, halo-C₁₋₆ alkoxy, 5- to 6-membered heteroaryl, -C(O)R", -C(O)OR", or -C(O)N(R")₂; in R₃ or R₄, the C₁₋₆ alkyl is unsubstituted or selectively substituted at any position by one or more than one substituent selected from halogen, hydroxyl, amino, carboxyl, cyano, C₁₋₆ alkoxy, halo-C₁₋₆ alkoxy, and C₁₋₆ alkylamino;
U is N or C; when U is N, L is -(CH₂)ₘW(CH₂)ₘ-, and R₆ is absent; or when U is C, L is absent or -(CH₂)ₘW(CH₂)ₘ-, and R₆ is hydrogen, halogen, or C₁₋₆ alkyl; in R₆, the C₁₋₆ alkyl is unsubstituted or selectively substituted at any position by one or more than one substituent selected from halogen, hydroxyl, amino, carboxyl, cyano, C₁₋₆ alkoxy, halo-C₁₋₆ alkoxy, and C₁₋₆ alkylamino;
the R₄ and R₆ are each independent substituents, or R₄ and R₆ are attached to each other to form a double bond, a C₃₋₈ cycloalkyl group, or a 3- to 8-membered heterocycloalkyl group; the C₃₋₈ cycloalkyl or 3- to 8-membered heterocycloalkyl is unsubstituted or selectively substituted at any position by one or more than one R₁₀;
W is a linkage bond, -CH=CH-, -NH-, -O-, -S-, or -S(O)₁₋₂-;
R₇ is hydrogen, halogen, or cyano;
R₈ is hydrogen, halogen, cyano, amino, hydroxyl, C₁₋₆ alkyl, C₁₋₆ alkoxy, halo-C₁₋₆ alkyl, or halo-C₁₋₆ alkoxy;
each R₉ is independently hydrogen, hydroxyl, halogen, cyano, amino, C₁₋₆ alkyl, C₂₋₆ alkenyl, C₂₋₆ alkynyl, C₃₋₈ cycloalkyl, 3- to 8-membered heterocycloalkyl, C₆₋₁₀ aryl, 5- to 10-membered heteroaryl, -N(R')₂, -OR', -SR', -NHC(O)R", -C(O)R", -C(O)N(R")₂, -OC(O)R", - OC(O)N(R")₂, or -B(OR")z; in R₉, the C₁₋₆ alkyl, C₂₋₆ alkenyl, C₂₋₆ alkynyl, or C₃₋₈ cycloalkyl is unsubstituted or selectively substituted at any position by one or more than one substituent selected from deuterium, halogen, cyano, amino, C₁₋₆ alkyl, C₃₋₈ cycloalkyl, 3- to 8-membered heterocycloalkyl, C₆₋₁₀ aryl, 5- to 10-membered heteroaryl, -N(R')₂, -OR', -SR', -NHC(O)R", - C(O)R", -C(O)N(R")₂, -OC(O)R", -OC(O)N(R")_{z}, and -B(OR")₂;
each R₁₀ is independently hydrogen, halogen, hydroxyl, cyano, amino, C₁₋₆ alkyl, oxo, C₁₋₆ alkylene, C₂₋₆ alkenyl, C₂₋₆ alkynyl, -N(R')₂, -OR', -C(O)R", -C(O)OR", or -C(O)N(R")₂; in R₁₀, the C₁₋₆ alkyl, C₁₋₆ alkylene, C₂₋₆ alkenyl, or C₂₋₆ alkynyl is unsubstituted or selectively substituted at any position by one or more than one substituent selected from deuterium, halogen, hydroxyl, amino, cyano, -N(R')₂, and -OR';
R₁₀ₐ is hydrogen or C₁₋₆ alkyl; the C₁₋₆ alkyl is unsubstituted or selectively substituted at any position by one or more than one substituent selected from halogen, hydroxyl, amino, cyano, -N(R')₂, and -OR';
R_{A} is hydrogen, C₁₋₆ alkyl, C₂₋₆ alkenyl, C₂₋₆ alkynyl, C₆₋₁₀ aryl, 5- to 10-membered heteroaryl, C₃₋₁₀ cycloalkyl, 3- to 10-membered heterocycloalkyl, C₃₋₁₀ cycloalkyl-C₁₋₆ alkyl, 3- to 10-membered heterocycloalkyl-C₁₋₆ alkyl, C₆₋₁₀ aryl-C₁₋₆ alkyl, or 5- to 10-membered heteroaryl-C₁₋₆ alkyl; the R_{A} is unsubstituted or selectively substituted at any position by one or more than one R_{c};
R_{B} is hydrogen, cyano, hydroxyl, or C₁₋₆ alkyl;
R_{A} and R_{B} are independent substituents, or R_{A} and R_{B} are attached to each other to form a 4- to 8-membered heterocycloalkyl group; the heterocycloalkyl is unsubstituted or selectively substituted at any position by one or more than one substituent selected from hydroxyl, cyano, amino, oxo, halogen, C₁₋₆ alkyl, halo-C₁₋₆ alkyl, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, 5- to 10-membered heteroaryl-C₁₋₄ alkyl, -C(O)R", -(CH₂)ₙOR", and -(CH₂)ₙN(R")₂;
R_{c} is hydroxyl, cyano, amino, oxo, halogen, C₁₋₆ alkyl, C₁₋₆ alkylene, halo-C₁₋₆ alkyl, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, 5- to 10-membered heteroaryl-C₁₋₄ alkyl, -(CH₂)ₙOR', -(CH₂)ₙN(R')₂, -(CH₂)ₙ-N(CN)R', -
(CH₂)ₙ-C(O)R', -(CH₂)ₙ-C(O)N(R')₂, -(CH₂)ₙ-S(O)₂N(R')₂, -(CH₂)ₙ-NR"C(O)R', -(CH₂)ₙ-NR"S(O)₂R', -(CH₂)ₙ-N(NH)N(R')₂, -(CH₂)ₙ-NR"C(O)N(R')₂, -(CH₂)ₙ-NR"C(O)OR', -(CH₂)ₙ-OC(O)R', -(CH₂)ₙ-OC(O)N(R')₂, or -(CH₂)ₙ-B(OR")₂; in R_{c}, the C₁₋₆ alkylene, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, or 5- to 10-membered heteroaryl-C₁₋₄ alkyl is unsubstituted or selectively substituted at any position by one or more than one substituent selected from halogen, hydroxyl, amino, cyano, C₁₋₆ alkoxy, C₁₋₆ alkylamino, and C₁₋₆ alkyl;
each R' is independently hydrogen, C₁₋₄ alkyl, halo-C₁₋₄ alkyl, hydroxy-C₁₋₄ alkyl, cyano-C₁₋₄ alkyl, C₁₋₄ alkoxy-C₁₋₄ alkyl, amino-C₁₋₄ alkyl, C₁₋₆ alkylamino-C₁₋₄ alkyl, C₃₋₈ cycloalkyl, 3- to 8-membered heterocycloalkyl, C₆₋₁₀ aryl, 6- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, or 6-to 10-membered heteroaryl-C₁₋₄ alkyl;
each R" is independently hydrogen or C₁₋₆ alkyl;
each m is independently 0, 1, 2, or 3;
n is 0, 1, 2, 3, or 4;
q is 0, 1, 2, or 3;
t is 0, 1, 2, or 3.

In some embodiments, 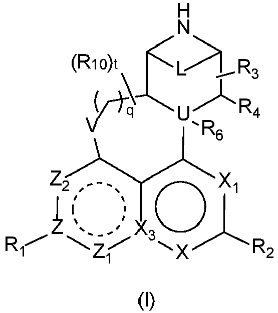
wherein V is -O-;
Z is C; Z₁ and Z₂ are each independently CR₈ or N;
X is N; X₁ is N; X₃ is C;
R₁ is C₆₋₁₂ aryl or 5- to 12-membered heteroaryl; the R₁ is unsubstituted or selectively substituted at any position by one or more than one R₉;
R₂ is hydrogen, -R_{A}, -NR_{A}R_{B}, -OR_{A}, or -SR_{A};
R₃ and R₄ are each independently hydrogen;
U is N and R₆ is absent;
Lis -CH₂CH₂-;
each R₈ is independently hydrogen, halogen, cyano, amino, hydroxyl, C₁₋₆ alkyl, C₁₋₆ alkoxy, halo-C₁₋₆ alkyl, or halo-C₁₋₆ alkoxy;
each R₉ is independently hydrogen, hydroxyl, halogen, cyano, amino, C₁₋₆ alkyl, C₂₋₆ alkenyl, C₂₋₆ alkynyl, C₃₋₈ cycloalkyl, 3- to 8-membered heterocycloalkyl, C₆₋₁₀ aryl, 5- to 10-membered heteroaryl, -N(R')₂, -OR', -SR', -NHC(O)R", -C(O)R", -C(O)N(R")₂, -OC(O)R", - OC(O)N(R")₂, or -B(OR")z; in R₉, the C₁₋₆ alkyl, C₂₋₆ alkenyl, C₂₋₆ alkynyl, or C₃₋₈ cycloalkyl is unsubstituted or selectively substituted at any position by one or more than one substituent selected from halogen, cyano, amino, C₁₋₆ alkyl, C₃₋₈ cycloalkyl, 3- to 8-membered heterocycloalkyl, C₆₋₁₀ aryl, 5- to 10-membered heteroaryl, -N(R')₂, -OR', -SR', -NHC(O)R", - C(O)R", -C(O)N(R")₂, -OC(O)R", -OC(O)N(R")₂, and -B(OR")₂;
each R₁₀ is independently hydrogen, halogen, C₁₋₆ alkyl, or oxo;
R_{A} is hydrogen, C₁₋₆ alkyl, C₂₋₆ alkenyl, C₂₋₆ alkynyl, C₆₋₁₀ aryl, 5- to 10-membered heteroaryl, C₃₋₁₀ cycloalkyl, 3- to 10-membered heterocycloalkyl, C₃₋₁₀ cycloalkyl-C₁₋₆ alkyl, 3- to 10-membered heterocycloalkyl-C₁₋₆ alkyl, C₆₋₁₀ aryl-C₁₋₆ alkyl, or 5- to 10-membered heteroaryl-C₁₋₆ alkyl; the R_{A} is unsubstituted or selectively substituted at any position by one or more than one R_{c};
R_{B} is hydrogen, cyano, hydroxyl, or C₁₋₆ alkyl;
R_{A} and R_{B} are independent substituents, or R_{A} and R_{B} are attached to each other to form a 4- to 8-membered heterocycloalkyl group; the heterocycloalkyl is unsubstituted or selectively substituted at any position by one or more than one substituent selected from hydroxyl, cyano, amino, oxo, halogen, C₁₋₆ alkyl, halo-C₁₋₆ alkyl, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, 5- to 10-membered heteroaryl-C₁₋₄ alkyl, -C(O)R", -(CH₂)ₙOR", and -(CH₂)ₙN(R")₂;
R_{c} is hydroxyl, cyano, amino, oxo, halogen, C₁₋₆ alkyl, C₁₋₆ alkylene, halo-C₁₋₆ alkyl, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, 5- to 10-membered heteroaryl-C₁₋₄ alkyl, -(CH₂)ₙOR', -(CH₂)ₙN(R')₂, -(CH₂)ₙ-N(CN)R', - (CH₂)ₙ-C(O)R', -(CH₂)ₙ-C(O)N(R')₂, -(CH₂)ₙ-S(O)₂N(R')₂, -(CH₂)ₙ-NR"C(O)R', -(CH₂)ₙ-NR"S(O)₂R', -(CH₂)ₙ-N(NH)N(R')₂, -(CH₂)ₙ-NR"C(O)N(R')₂, -(CH₂)ₙ-NR"C(O)OR', -(CH₂)ₙ-OC(O)R', -(CH₂)ₙ-OC(O)N(R')₂, or -(CH₂)ₙ-B(OR")₂; in R_{c}, the C₁₋₆ alkylene, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, or 5- to 10-membered heteroaryl-C₁₋₄ alkyl is unsubstituted or selectively substituted at any position by one or more than one substituent selected from halogen, hydroxyl, amino, cyano, C₁₋₆ alkoxy, C₁₋₆ alkylamino, and C₁₋₆ alkyl;
each R' is independently hydrogen, C₁₋₄ alkyl, halo-C₁₋₄ alkyl, hydroxy-C₁₋₄ alkyl, cyano-C₁₋₄ alkyl, C₁₋₄ alkoxy-C₁₋₄ alkyl, amino-C₁₋₄ alkyl, C₁₋₆ alkylamino-C₁₋₄ alkyl, C₃₋₈ cycloalkyl, 3- to 8-membered heterocycloalkyl, C₆₋₁₀ aryl, 6- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, or 6-to 10-membered heteroaryl-C₁₋₄ alkyl;
each R" is independently hydrogen or C₁₋₆ alkyl;
n is 0, 1, 2, 3, or 4;
q is 0, 1, 2, or 3;
t is 0, 1, 2, or 3.

In some embodiments, U is N and R₆ is absent.

In some embodiments, U is CH.

In some embodiments, each m is independently 0, 1, or 2.

In some embodiments, W is a linkage bond, 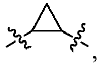 or -CH=CH-.

In some embodiments, W is a linkage bond or -CH=CH-.

In some embodiments, L is -CH₂CH₂-, 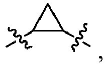 -CH₂CH₂CH₂-, -CH₂OCH₂-, - CH₂NHCH₂-, or -CH=CH-.

In some embodiments, L is -CH₂CH₂-, -CH₂CH₂CH₂-, -CHzOCHz-, -CH₂NHCH₂-, or -CH=CH-.

In some embodiments, L is -CH₂CH₂- or

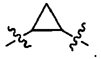

In some embodiments, L is -CH₂CH₂-.

In some embodiments, V is -O-.

In some embodiments, Z is C; Z₁ is N; Z₂ is CR₈ or N.

In some embodiments, Z is C; Z₁ is CR₈; Z₂ is N.

In some embodiments, Z is C; Z₁ is CR₈; Z₂ is CR₈.

In some embodiments, X₃ is C.

In some embodiments, X is N.

In some embodiments, X₁ is N.

In some embodiments, each R₉ is independently hydrogen, hydroxyl, halogen, cyano, amino, C₁₋₆ alkyl, C₂₋₆ alkenyl, C₂₋₆ alkynyl, C₃₋₆ cycloalkyl, 3- to 6-membered heterocycloalkyl, -N(R')₂, -OR', or -SR'; in R₉, the C₁₋₆ alkyl or C₃₋₆ cycloalkyl is unsubstituted or selectively substituted at any position by 1 to 3 substituents selected from halogen, hydroxyl, cyano, and amino.

In some embodiments, each R₉ is independently hydrogen, hydroxyl, halogen, cyano, amino, C₁₋₆ alkyl, C₂₋₆ alkenyl, C₂₋₆ alkynyl, C₃₋₆ cycloalkyl, 3- to 6-membered heterocycloalkyl, -N(R')₂, -OR', or -SR'; in R₉, the C₁₋₆ alkyl is unsubstituted or selectively substituted at any position by 1 to 3 substituents selected from halogen, hydroxyl, cyano, and amino.

In some embodiments, R₁ is C₆₋₁₂ aryl or 5- to 12-membered heteroaryl; the R₁ is unsubstituted or selectively substituted at any position by 1 to 3 R₉.

In some embodiments, R₁ is phenyl, naphthyl, quinolinyl, isoquinolinyl, benzothienyl, benzothiazolyl, 1*H*-indolyl, 1*H*-indazolyl, pyrazolo[1,5-*a*]pyridyl, or imidazo[1,2-*a*]pyridyl; the R₁ is unsubstituted or selectively substituted at any position by 1 to 3 R₉.

In some embodiments, R₁ is phenyl, naphthyl, quinolinyl, isoquinolinyl, benzothienyl, benzothiazolyl, 1*H*-indolyl, or 1*H*-indazolyl; the R₁ is unsubstituted or selectively substituted at any position by 1 to 3 R₉.

In some embodiments, the R₁ has any one of the following structures:

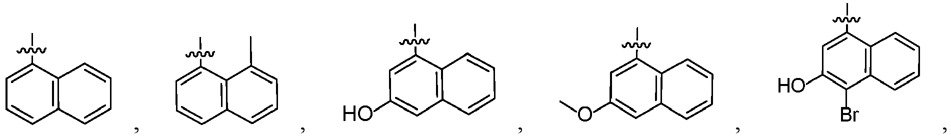

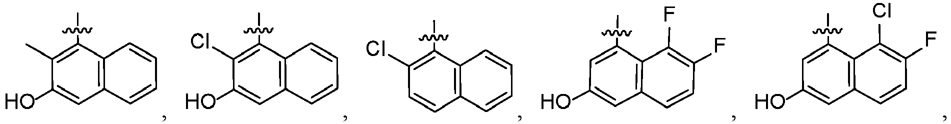

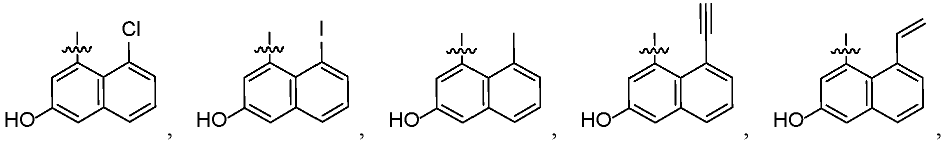

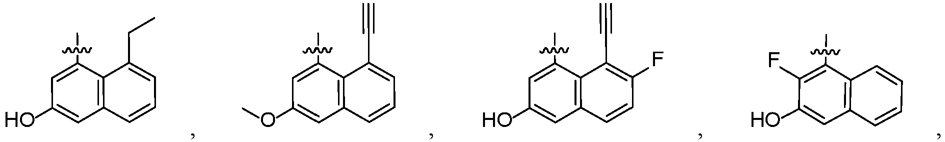

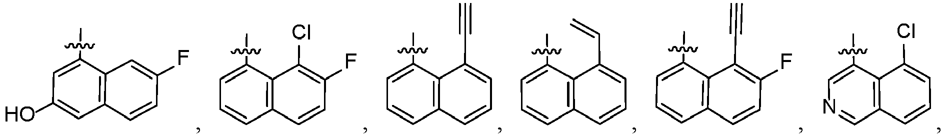

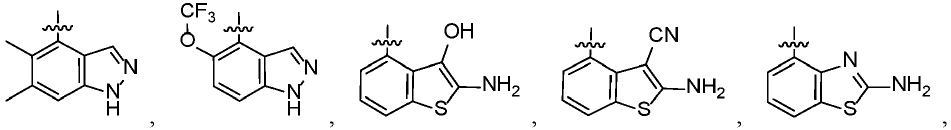

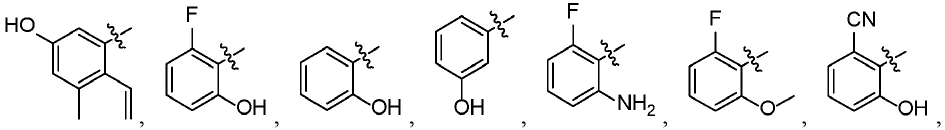

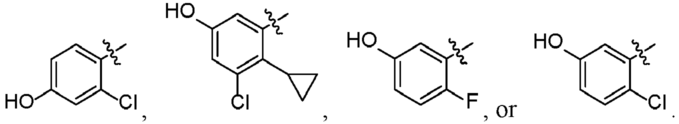

In some embodiments, the R₁ has any one of the following structures: 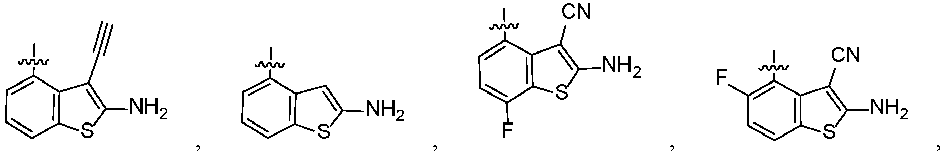 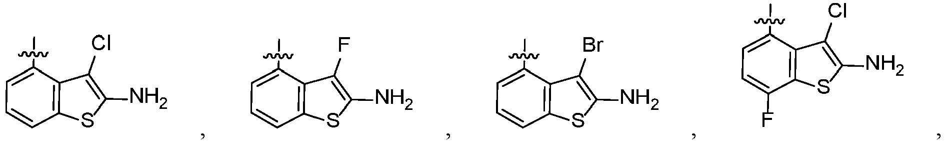 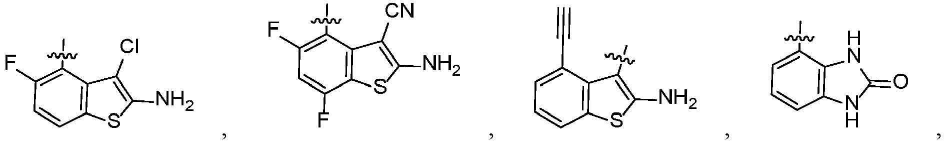 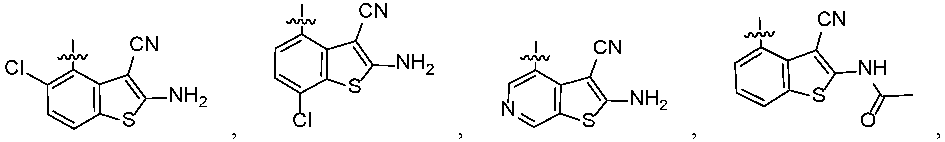 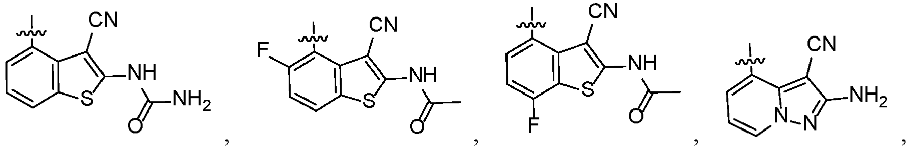 or

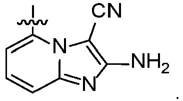

In some embodiments, the R₁ has any one of the following structures:

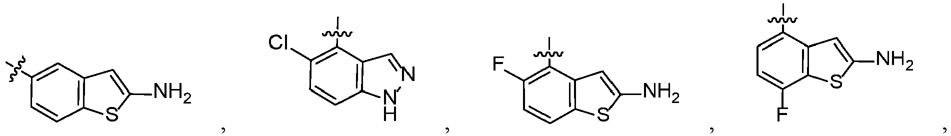

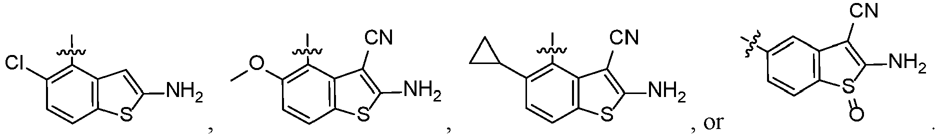

In some embodiments, the R₁ has any one of the following structures:

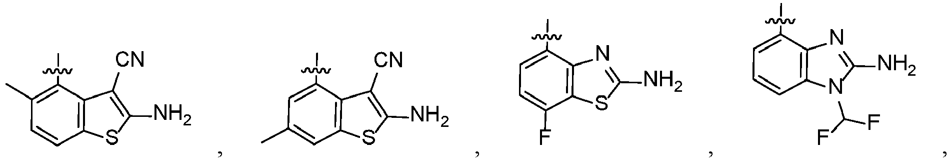

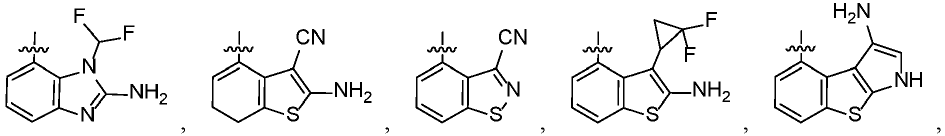

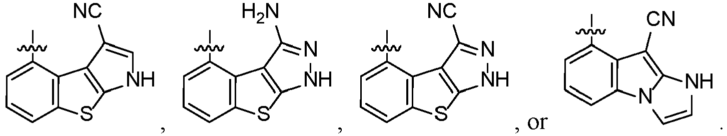

In some embodiments, the R_{c} is hydroxyl, cyano, amino, oxo, halogen, C₁₋₆ alkyl, C₁₋₆ alkylene, halo-C₁₋₆ alkyl, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, 5- to 10-membered heteroaryl-C₁₋₄ alkyl, -(CH₂)ₙOR', or - (CH₂)ₙN(R')₂; in R_{c}, the C₁₋₆ alkylene, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, or 5- to 10-membered heteroaryl-C₁₋₄ alkyl is unsubstituted or selectively substituted at any position by 1 to 3 substituents selected from halogen, hydroxyl, amino, cyano, C₁₋₆ alkoxy, C₁₋₆ alkylamino, and C₁₋₆ alkyl.

In some embodiments, R_{c} is deuterium, hydroxyl, cyano, amino, oxo, halogen, C₁₋₆ alkyl, halo-C₁₋₆ alkyl, phenyl, -(CH₂)ₙOR', or -(CH₂)ₙN(R')₂; each R' is independently hydrogen, C₁₋₄ alkyl, or halo-C₁₋₄ alkyl.

In some embodiments, R_{c} is hydroxyl, cyano, amino, oxo, halogen, C₁₋₆ alkyl, halo-C₁₋₆ alkyl, phenyl, -(CH₂)ₙOR', or -(CH₂)ₙN(R')₂; each R' is independently hydrogen, C₁₋₄ alkyl, or halo-C₁₋₄ alkyl.

In some embodiments, R_{A} is hydrogen, C₁₋₆ alkyl, C₂₋₆ alkenyl, C₂₋₆ alkynyl, C₃₋₁₀ cycloalkyl, 3- to 10-membered heterocycloalkyl, C₃₋₁₀ cycloalkyl-C₁₋₆ alkyl, 3- to 10-membered heterocycloalkyl-C₁₋₆ alkyl, C₆₋₁₀ aryl-C₁₋₆ alkyl, or 5- to 10-membered heteroaryl-C₁₋₆ alkyl; the R_{A} is unsubstituted or selectively substituted at any position by 1 to 5 R_{c}.

In some embodiments, R_{A} is 3- to 10-membered heterocycloalkyl-C₁₋₆ alkyl; the R_{A} is unsubstituted or selectively substituted at any position by 1 to 5 R_{c}.

In some embodiments, R_{A} is 3- to 10-membered heterocycloalkyl-C₁₋₆ alkyl; the R_{A} is unsubstituted or selectively substituted at any position by 1 to 3 R_{c}.

In some embodiments, R_{A} is 3- to 10-membered heterocycloalkyl-C₁₋₄ alkyl; the R_{A} is unsubstituted or selectively substituted at any position by 1 to 5 R_{c}.

In some embodiments, R_{A} is 3- to 10-membered heterocycloalkyl-C₁₋₄ alkyl; the R_{A} is unsubstituted or selectively substituted at any position by 1 to 3 R_{c}.

In some embodiments, R_{A} is 3- to 10-membered heterocycloalkyl-C₁₋₄ alkyl, and the 3- to 10-membered heterocycloalkyl is pyrrolidinyl or hexahydro-1/7-pyrrolizinyl; the R_{A} is unsubstituted or selectively substituted at any position by 1 to 5 R_{c}.

In some embodiments, R_{A} is 3- to 10-membered heterocycloalkyl-C₁₋₄ alkyl, and the 3- to 10-membered heterocycloalkyl is pyrrolidinyl or hexahydro-1/7-pyrrolizinyl; the R_{A} is unsubstituted or selectively substituted at any position by 1 to 3 R_{c}.

In some embodiments, R_{A} is 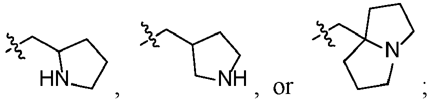 the R_{A} is unsubstituted or selectively substituted at any position by 1 to 5 R_{c}.

In some embodiments, R_{A} is 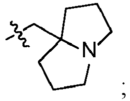 the R_{A} is unsubstituted or selectively substituted at any position by 1 to 5 fluorine, deuterium, and methyl.

In some embodiments, R_{A} is 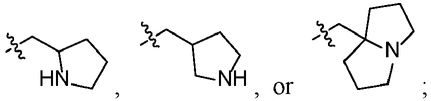 the R_{A} is unsubstituted or selectively substituted at any position by 1 to 3 R_{c}.

In some embodiments, the R₂ is -R_{A}, -NR_{A}R_{B}, or -OR_{A};

in some embodiments, the R₂ is -OR_{A}; R_{A} is defined as above.

In some embodiments, the R₂ has any one of the following structures:

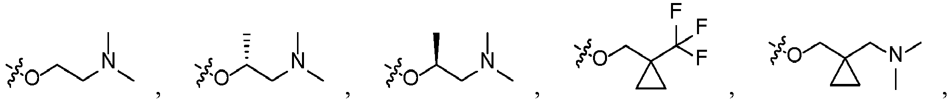

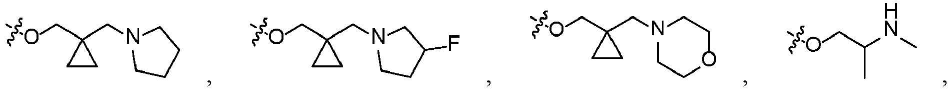

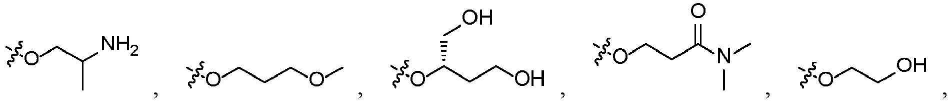

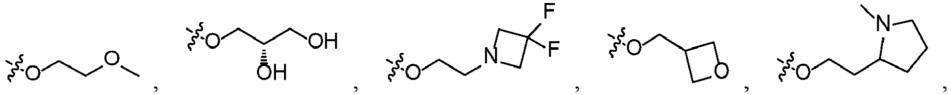

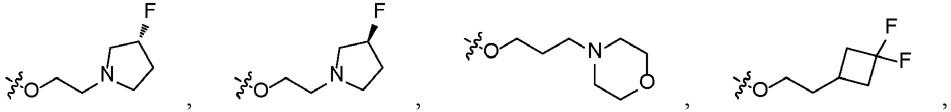

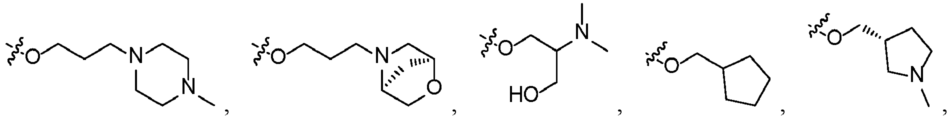

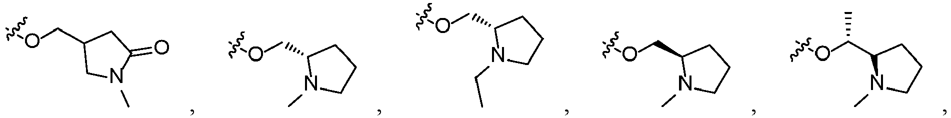

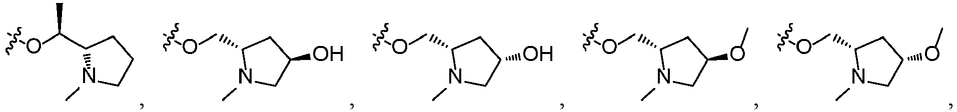

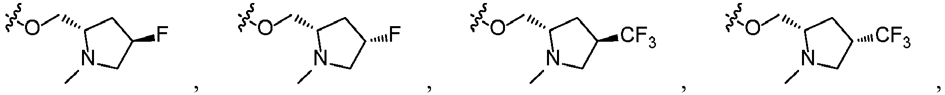

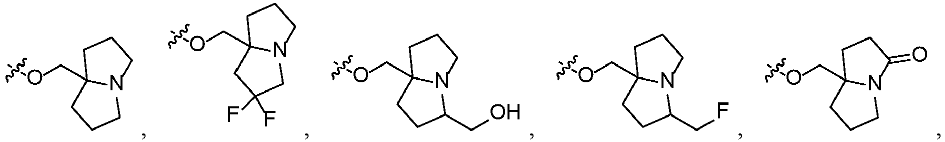

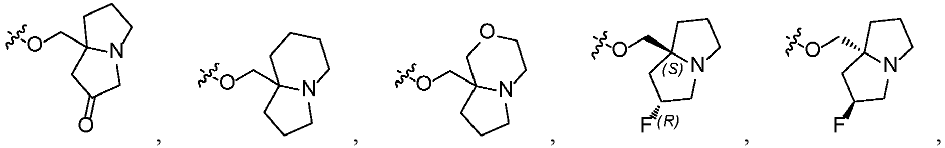

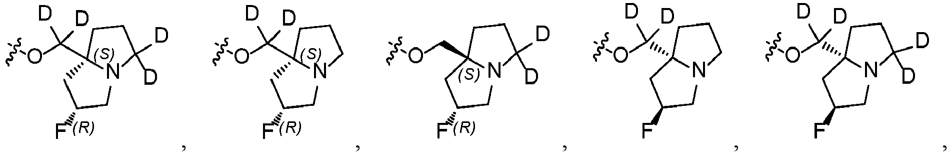

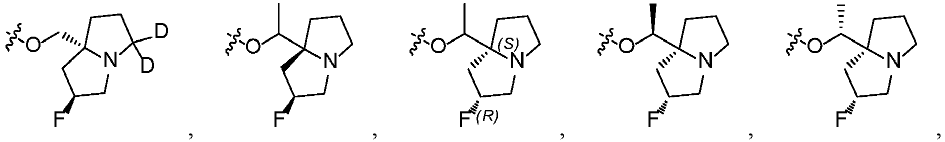

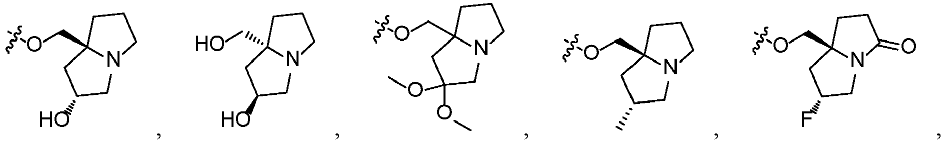

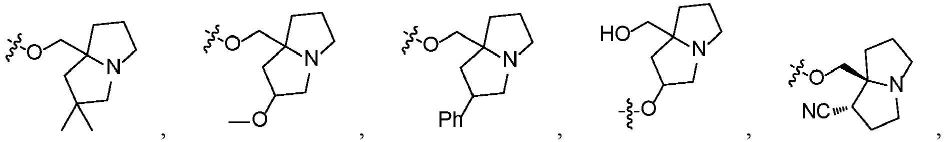

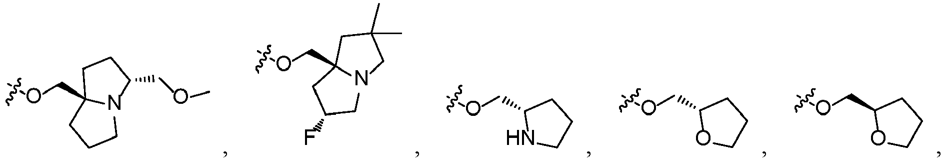

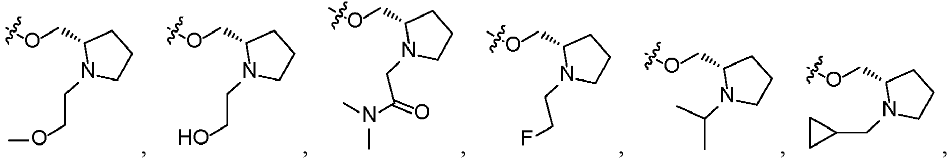

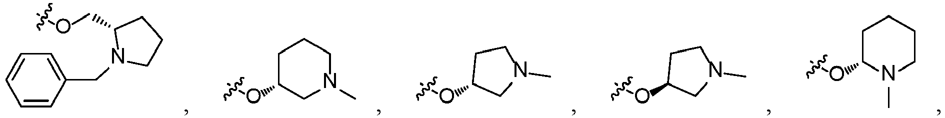

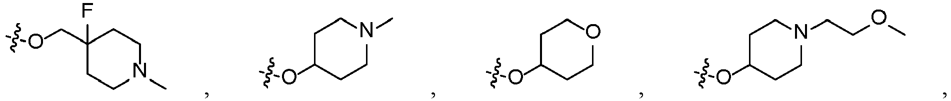

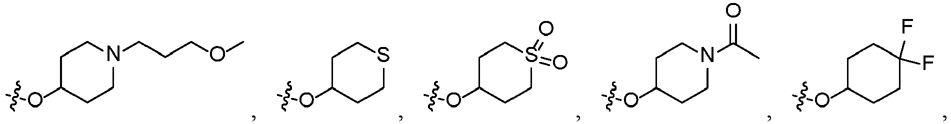

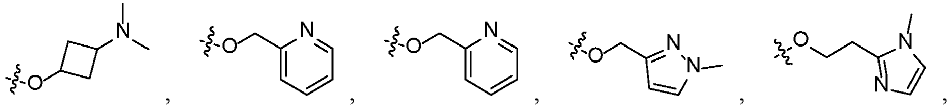

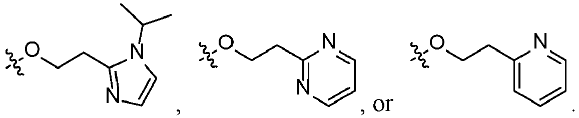

In some embodiments, the R₂ has any one of the following structures:

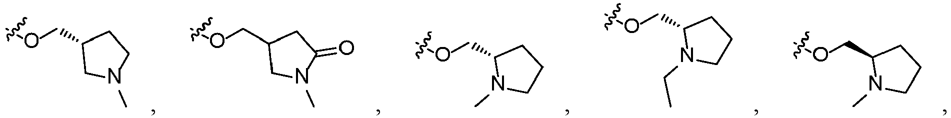

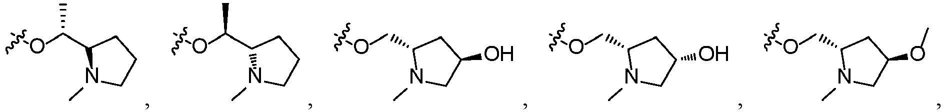

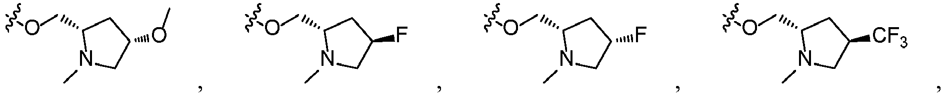

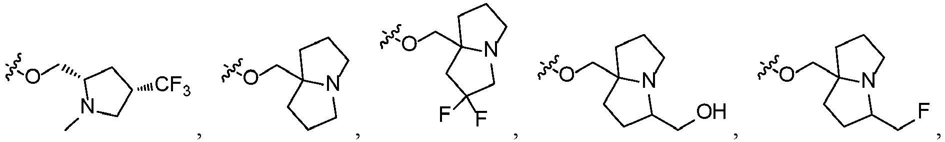

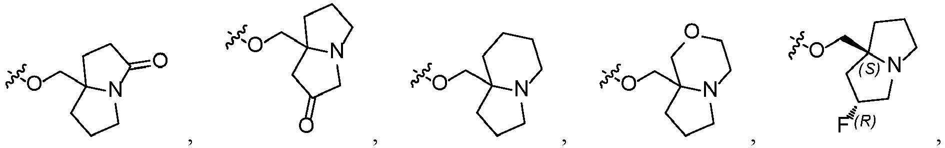

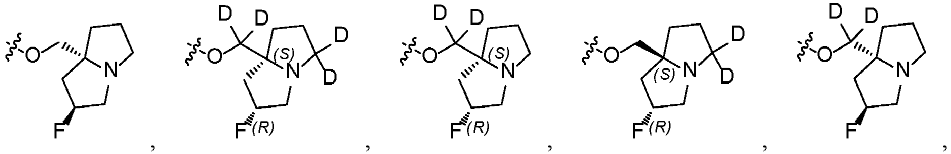

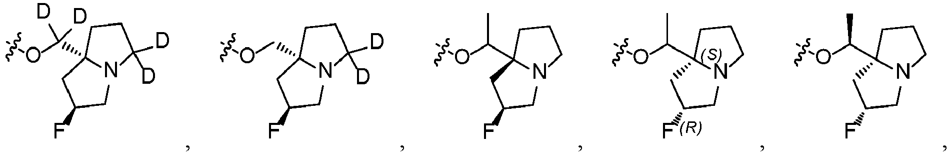

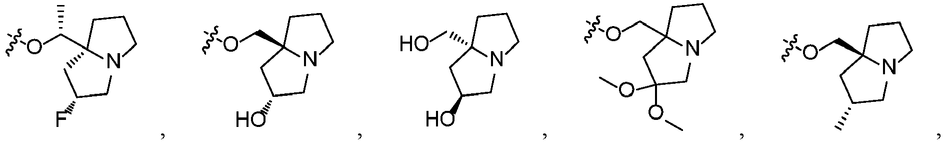

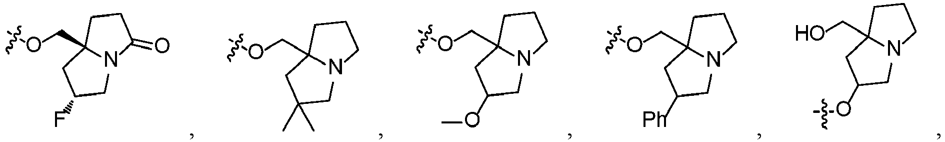

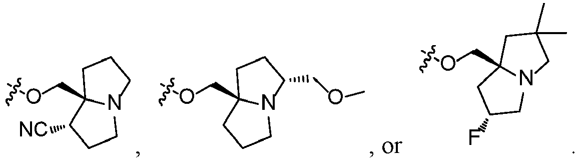

In some embodiments, the R₂ is -NR_{A}R_{B}; R_{A} and R_{B} are defined as above.

In some embodiments, the R₂ has any one of the following structures:

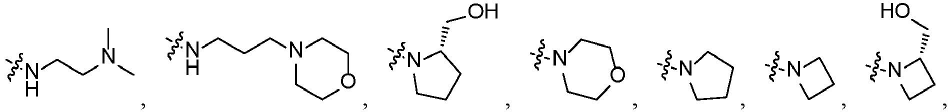

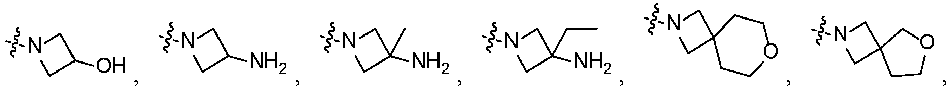

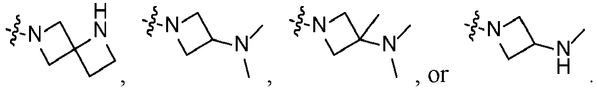

In some embodiments, the R₂ is R_{A}; R_{A} is defined as above.

In some embodiments, the R₂ has any one of the following structures:

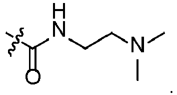

In some embodiments, the R₂ is H.

In some embodiments, R₃ and R₄ are each independently hydrogen.

In some embodiments, R₃ is hydrogen or hydroxyl; R₄ is hydrogen.

In some embodiments, R₃ is deuterium, R₄ is deuterium, and R₃ and R₄ are substituted at the same carbon atom.

In some embodiments, R₇ is hydrogen, cyano, fluorine, or chlorine.

In some embodiments, R₇ is hydrogen; other groups are defined as above.

In some embodiments, each R₈ is independently hydrogen or halogen.

In some embodiments, each R₈ is independently fluorine or chlorine.

In some embodiments, each R₁₀ is independently hydrogen, halogen, C₁₋₆ alkyl, or oxo; in R₁₀, the C₁₋₆ alkyl, C₁₋₆ alkylene, C₂₋₆ alkenyl, or C₂₋₆ alkynyl is unsubstituted or selectively substituted at any position by 1 to 3 substituents selected from deuterium, halogen, hydroxyl, amino, cyano, -N(R')₂, and -OR'.

In some embodiments, R₁₀ is hydrogen.

In some embodiments, R₁₀ is deuterium; t is 2.

In some embodiments, R₁₀ₐ is hydrogen or C₁₋₆ alkyl; the C₁₋₆ alkyl is unsubstituted or selectively substituted at any position by 1 to 3 substituents selected from halogen, hydroxyl, amino, cyano, -N(R')₂, and -OR'.

In some embodiments, R₁₀ₐ is hydrogen.

In some embodiments, R' is hydrogen or C₁₋₄ alkyl.

In some embodiments, q is 1.

In some embodiments, the compound of formula (I), the stereoisomer thereof, the stable deuterated derivative thereof, or the pharmaceutically acceptable salt thereof is a compound of formula (II), a stereoisomer thereof, a stable deuterated derivative thereof, or a pharmaceutically acceptable salt thereof; 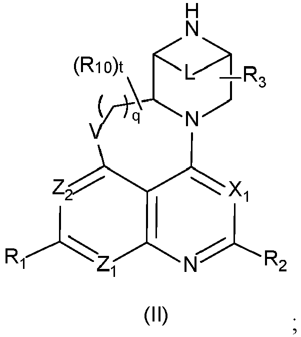 wherein V, R₁, R₂, R₃, R₁₀, L, Z₁, Z₂, X₁, q, and t are defined as above.

In some embodiments, V is -O-; q is 1.

In some embodiments, L is -CH₂CH₂-.

In some embodiments, X₁ is N.

In some embodiments, t is 0.

In some embodiments, Z₁ is N; Z₂ is CR₈.

In some embodiments, Z₁ and Z₂ are each independently CR₈.

In some embodiments, Z₁ is CR₈; Z₂ is N.

In some embodiments, each R₈ is independently hydrogen or halogen.

In some embodiments, each R₈ is independently chlorine or fluorine.

In some embodiments, R₂ is 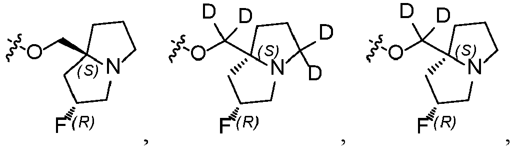 or

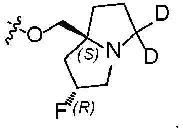

In some embodiments, a compound of formula (II), a stereoisomer thereof, a stable deuterated derivative thereof, or a pharmaceutically acceptable salt thereof; 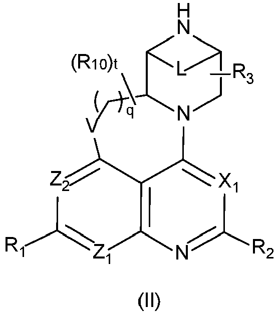
wherein V is -O-;
Z₁ and Z₂ are each independently CR₈ or N;
X₁ is N;
R₁ is ring A-fused ring B, ring A is phenyl, naphthyl, 5- to 6-membered cycloalkyl, or 9- to 10-membered heteroaryl; ring B is 5- to 6-membered heteroaryl or 5- to 6-membered heterocycloalkyl; the R₁ is unsubstituted or selectively substituted at any position by one or more than one R₉;
R₂ is hydrogen or -OR_{A};
R₃ is hydrogen;
L is -CH₂CH₂-;
R₈ is hydrogen or halogen;
each R₉ is independently hydrogen, hydroxyl, halogen, cyano, amino, C₁₋₆ alkyl, C₂₋₆ alkenyl, C₂₋₆ alkynyl, C₃₋₆ cycloalkyl, 3- to 6-membered heterocycloalkyl, -N(R')₂, -OR', or - SR'; in R₉, the C₁₋₆ alkyl or C₃₋₆ cycloalkyl is unsubstituted or selectively substituted at any position by 1 to 3 substituents selected from halogen, hydroxyl, cyano, and amino;
each R₁₀ is independently hydrogen, halogen, C₁₋₆ alkyl, or oxo;
R_{A} is hydrogen, C₁₋₆ alkyl, C₂₋₆ alkenyl, C₂₋₆ alkynyl, C₆₋₁₀ aryl, 5- to 10-membered heteroaryl, C₃₋₁₀ cycloalkyl, 3- to 10-membered heterocycloalkyl, C₃₋₁₀ cycloalkyl-C₁₋₆ alkyl, 3- to 10-membered heterocycloalkyl-C₁₋₆ alkyl, C₆₋₁₀ aryl-C₁₋₆ alkyl, or 5- to 10-membered heteroaryl-C₁₋₆ alkyl; the R_{A} is unsubstituted or selectively substituted at any position by one or more than one Rₑ;
R_{c} is hydroxyl, cyano, amino, oxo, halogen, C₁₋₆ alkyl, C₁₋₆ alkylene, halo-C₁₋₆ alkyl, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, 5- to 10-membered heteroaryl-C₁₋₄ alkyl, -(CH₂)ₙOR', or -(CH₂)ₙN(R')₂; in R_{c}, the C₁₋₆ alkylene, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, or 5- to 10-membered heteroaryl-C₁₋₄ alkyl is unsubstituted or selectively substituted at any position by 1 to 3 substituents selected from halogen, hydroxyl, amino, cyano, C₁₋₆ alkoxy, C₁₋₆ alkylamino, and C₁₋₆ alkyl;
each R' is independently hydrogen, C₁₋₄ alkyl, or halo-C₁₋₄ alkyl;
n is 0, 1, 2, 3, or 4;
q is 0, 1, 2, or 3;
t is 0, 1, 2, or 3.

In some embodiments, in R₁, the ring A is phenyl, naphthyl, benzothienyl, 1*H*-indolyl, or 1,3-cyclohexadienyl, and ring B is 5- to 6-membered heteroaryl or 5- to 6-membered heterocycloalkyl; in the 5- to 6-membered heteroaryl, the heteroatom is selected from one or more than one of N, O, and S, and the number of heteroatoms is 1, 2, or 3; in the 5- to 6-membered heterocycloalkyl, the heteroatom is selected from one or more than one of N, O, and S, and the number of heteroatoms is 1, 2, or 3.

In some embodiments, in R₁, the ring A is phenyl, naphthyl, benzothienyl, 1*H*-indolyl, or 1,3-cyclohexadienyl, and ring B is 5- to 6-membered heteroaryl; in the 5- to 6-membered heteroaryl, the heteroatom is selected from one or more than one of N, O, and S, and the number of heteroatoms is 1, 2, or 3.

In some embodiments, R₁ is attached to the parent molecule via ring A or ring B; preferably via a carbon atom on ring A.

In some embodiments, in R₁, ring A is 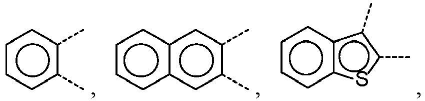 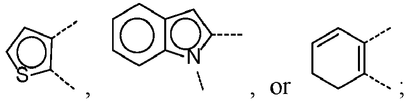 ring B is

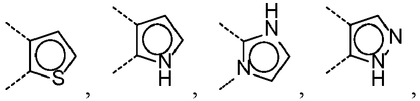

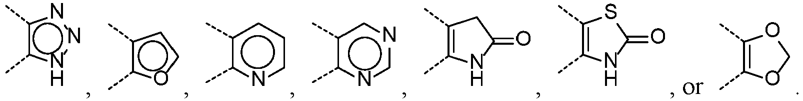

In some embodiments, in R₁, ring A is 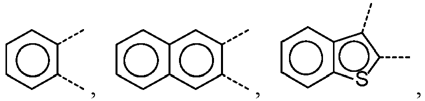 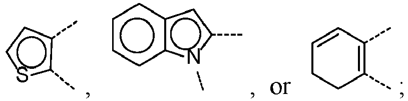 ring B is

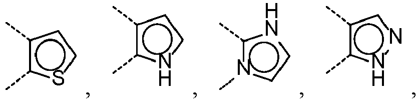

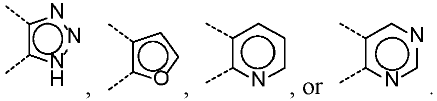

In some embodiments, R₁ is 1*H*-benzo[4,5]thieno[2,3-*c*]pyrazolyl, *1H-*benzo[4,5]thieno[2,3-*b*]pyrrolyl, 3*H*-benzo[4,5[thieno[2,3-*d*][1,2,3]triazolyl, benzo[4,5]thieno[2,3-*b*]pyridyl, benzo[4,5]thieno[2,3-*d*]pyrimidinyl, 1*H*-imidazo[1,2-*a*]indolyl, naphtho[2,3-*b*]furyl, 1*H*-benzo[f]indolyl, 1*H*-benzo[*f*]indazolyl, naphtho[2,1-*b*]furyl, 6,7-dihydrobenzo[*b*]thienyl, or 6,7-dihydrobenzo[*b*]thienyl, the R₁ is unsubstituted or selectively substituted at any position by 1 to 3 R₉.

In some embodiments, R₁ has any one of the following structures:

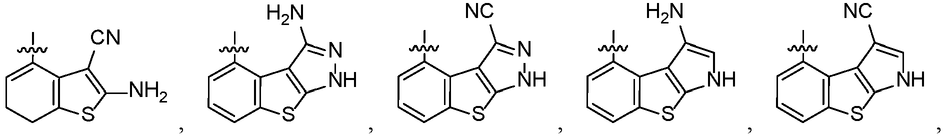

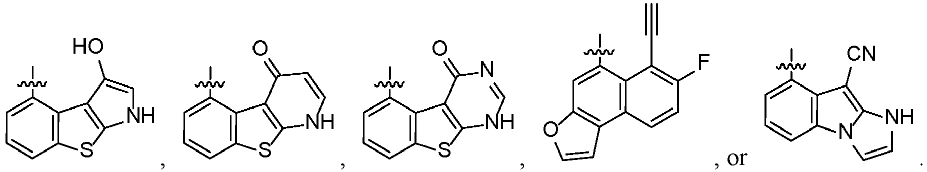

In some embodiments, R_{c} is hydroxyl, cyano, amino, oxo, halogen, C₁₋₆ alkyl, halo-C₁₋₆ alkyl, phenyl, -(CH₂)ₙOR', or -(CH₂)ₙN(R')₂.

In some embodiments, R_{c} is deuterium, hydroxyl, cyano, amino, oxo, halogen, C₁₋₆ alkyl, halo-C₁₋₆ alkyl, phenyl, -(CH₂)ₙOR', or -(CH₂)ₙN(R')₂.

In some embodiments, R_{A} is 3- to 10-membered heterocycloalkyl-C₁₋₆ alkyl; the R_{A} is unsubstituted or selectively substituted at any position by 1 to 5 R_{c}.

In some embodiments, R_{A} is 3- to 10-membered heterocycloalkyl-C₁₋₆ alkyl; the R_{A} is unsubstituted or selectively substituted at any position by 1 to 3 R_{c}.

In some embodiments, R_{A} is 3- to 10-membered heterocycloalkyl-C₁₋₄ alkyl; the 3- to 10-membered heterocycloalkyl is pyrrolidinyl or hexahydro-1*H*-pyrrolizinyl; the R_{A} is unsubstituted or selectively substituted at any position by 1 to 5 R_{c}.

In some embodiments, R_{A} is 3- to 10-membered heterocycloalkyl-C₁₋₄ alkyl; the 3- to 10-membered heterocycloalkyl is pyrrolidinyl or hexahydro-1*H*-pyrrolizinyl; the R_{A} is unsubstituted or selectively substituted at any position by 1 to 3 R_{c}.

In some embodiments, R_{A} is 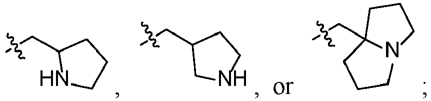 the R_{A} is unsubstituted or selectively substituted at any position by 1 to 5 R_{c}.

In some embodiments, R_{A} is 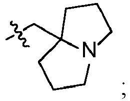 the R_{A} is unsubstituted or selectively substituted at any position by 1 to 5 fluorine, deuterium, and methyl.

In some embodiments, R_{A} is 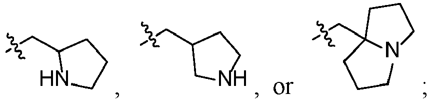 the R_{A} is unsubstituted or selectively substituted at any position by 1 to 3 R_{c}.

In some embodiments, the R₂ has any one of the following structures:

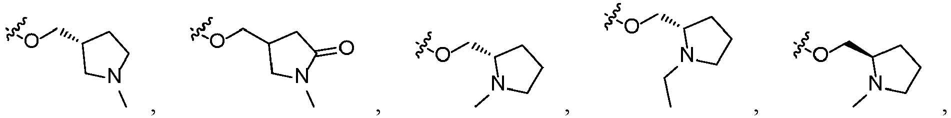

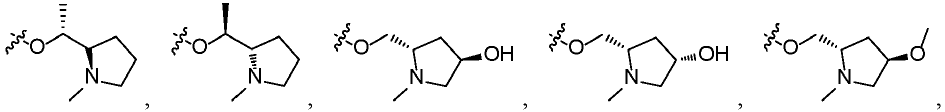

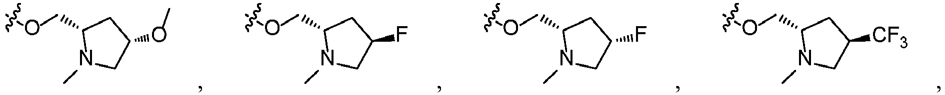

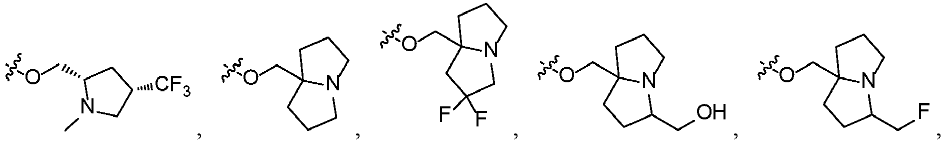

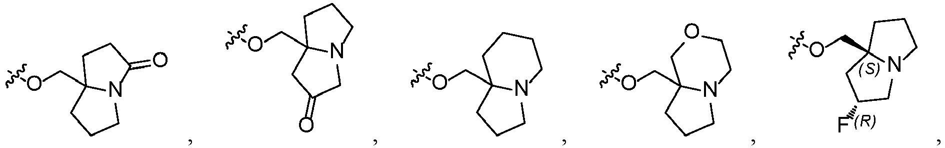

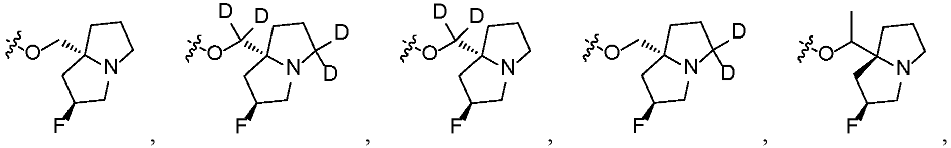

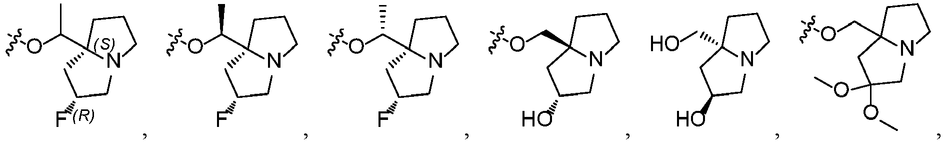

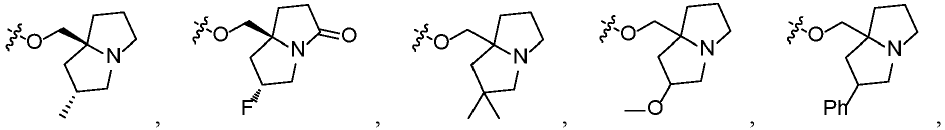

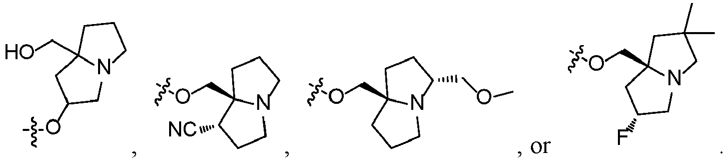

In some embodiments, R₈ is fluorine or chlorine; R₁₀ is hydrogen.

In some embodiments, the compound of formula (I), the stereoisomer thereof, the stable deuterated derivative thereof, or the pharmaceutically acceptable salt thereof is a compound of formula (III), a stereoisomer thereof, a stable deuterated derivative thereof, or a pharmaceutically acceptable salt thereof; 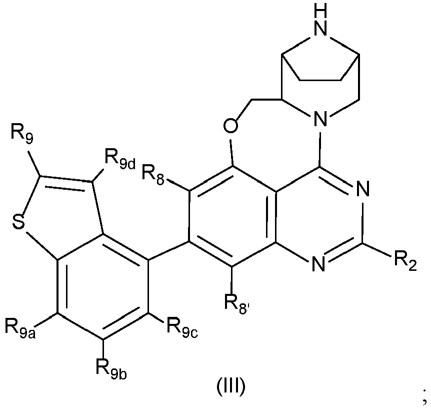
wherein R₂ is H or -OR_{A}; R_{A} is 3- to 10-membered heterocycloalkyl-C₁₋₆ alkyl; the R_{A} is unsubstituted or selectively substituted at any position by 1 to 3 R_{c};
R₈ and R_{8'} are each independently hydrogen or halogen;
R₉ is -N(R')₂, -NHC(O)R", or -NHC(O)N(R")₂;
R₉ₐ, R_{9b}, R_{9c}, and R_{9d} are each independently hydrogen, hydroxyl, halogen, cyano, amino, C₁₋₆ alkyl, C₂₋₆ alkenyl, C₂₋₆ alkynyl, C₃₋₆ cycloalkyl, 3- to 6-membered heterocycloalkyl, -N(R')₂, -OR', or -SR'; in R₉ₐ, R_{9b}, R_{9c}, and R_{9d}, the C₁₋₆ alkyl is unsubstituted or selectively substituted at any position by 1 to 3 substituents selected from halogen, hydroxyl, cyano, and amino;
R', R", and R_{c} are defined as above.

In some embodiments, R₉ₐ, R_{9b}, R_{9c}, and R_{9d} are each independently H, F, Cl, Br, cyano, hydroxyl, methyl, ethyl, isopropyl, methoxy, ethoxy, trifluoromethoxy, difluoromethoxy, trifluoromethyl, difluoromethyl, cyclopropyl, or ethynyl.

In some embodiments, R₉ is -NH₂.

In some embodiments, R₈ and R_{8'} are each independently fluorine or chlorine.

In some embodiments, R' is hydrogen, C₁₋₄ alkyl, halo-C₁₋₄ alkyl, hydroxy-C₁₋₄ alkyl, C₁₋₄ alkoxy-C₁₋₄ alkyl, amino-C₁₋₄ alkyl, C₁₋₆ alkylamino-C₁₋₄ alkyl, C₃₋₈ cycloalkyl, 3- to 8-membered heterocycloalkyl, C₃₋₈ cycloalkyl-C₁₋₄ alkyl, or 3- to 8-membered heterocycloalkyl-C₁₋₄ alkyl; R" is hydrogen or C₁₋₆ alkyl.

In some embodiments, R_{A} is 3- to 10-membered heterocycloalkyl-C₁₋₄ alkyl; the R_{A} is unsubstituted or selectively substituted at any position by 1 to 5 R_{c};
R_{c} is deuterium, hydroxyl, cyano, amino, oxo, halogen, C₁₋₆ alkyl, halo-C₁₋₆ alkyl, phenyl, -(CH₂)ₙOR', or -(CH₂)ₙN(R')₂.

In some embodiments, R₂ is 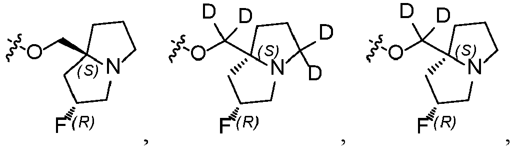 or

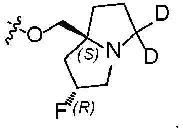

In some embodiments, the compound of formula (I), the stereoisomer thereof, the stable deuterated derivative thereof, or the pharmaceutically acceptable salt thereof is a compound of formula (IIIA) or (IIIB), a stereoisomer thereof, a stable deuterated derivative thereof, or a pharmaceutically acceptable salt thereof; 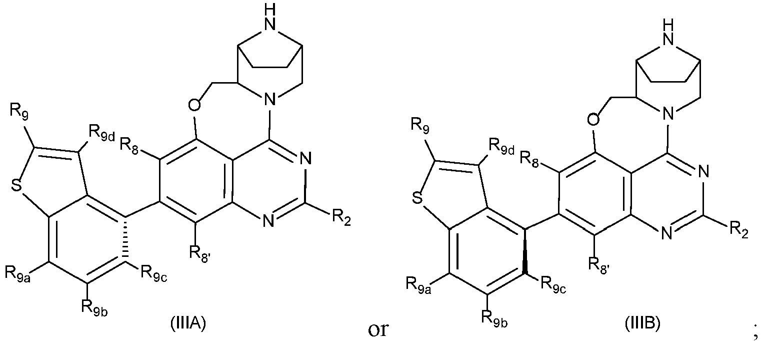 wherein R₂, R₉, R₉ₐ, R_{9b}, R_{9c}, R_{9d}, R₈, and R_{8'} are defined as above.

In some embodiments, the group 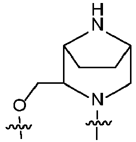 is:

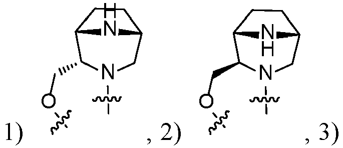

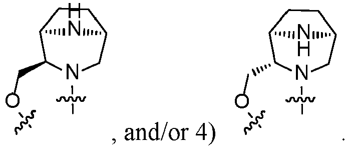

The compound of formula (I), the stereoisomer thereof, the stable deuterated derivative thereof, or the pharmaceutically acceptable salt thereof has any one of the following structures: 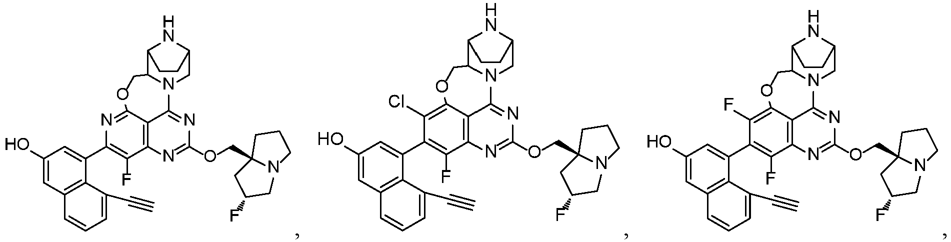 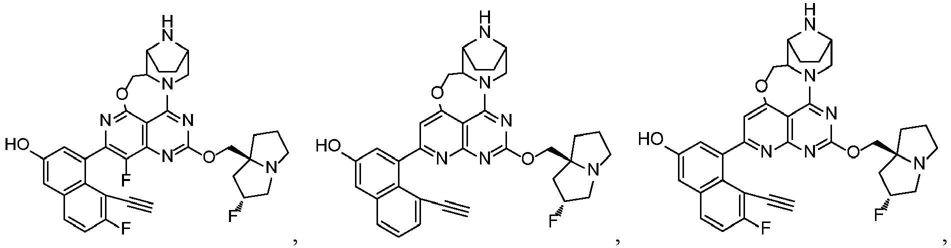 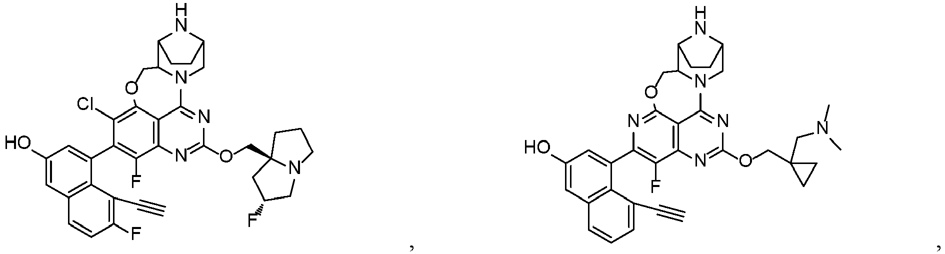 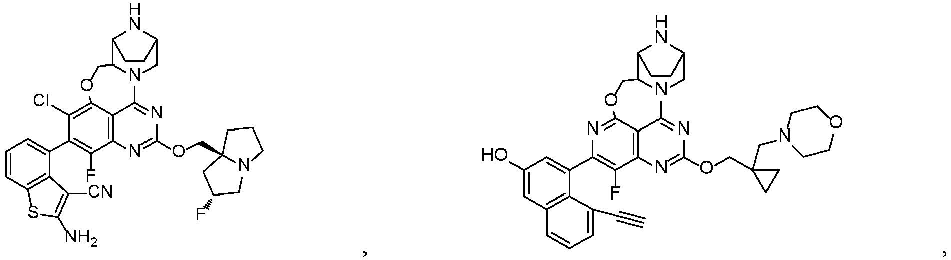 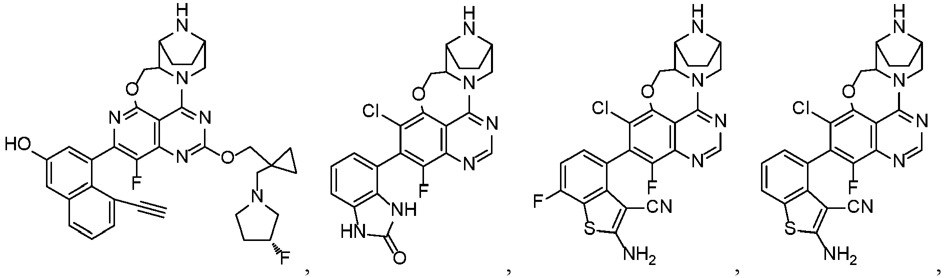 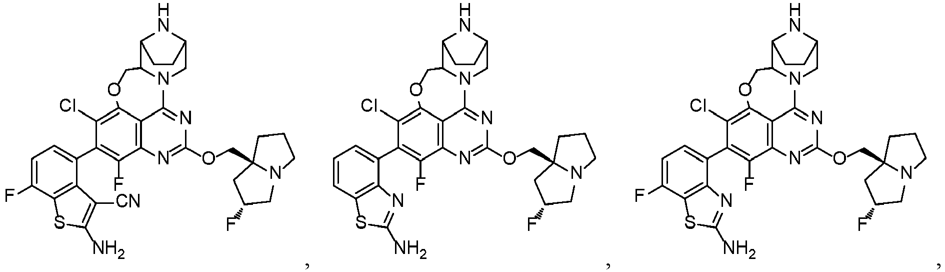 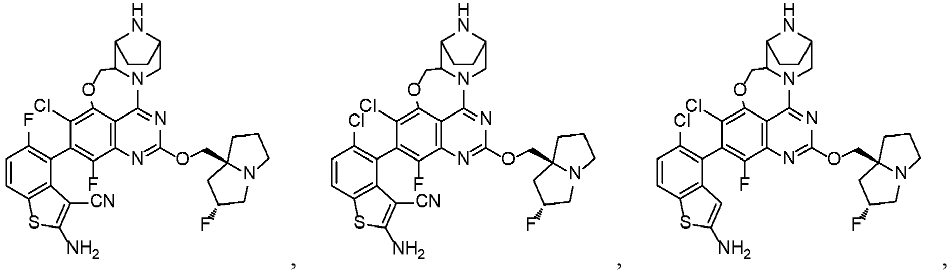 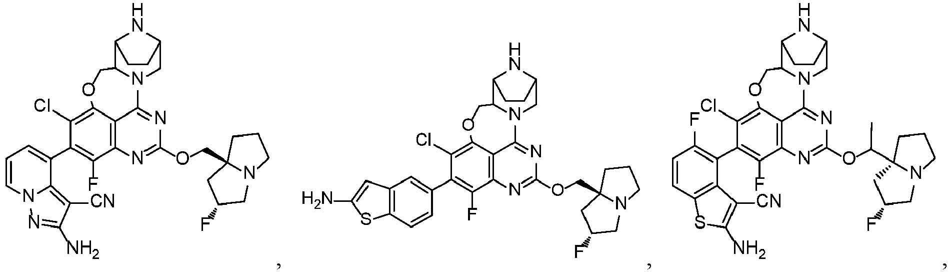 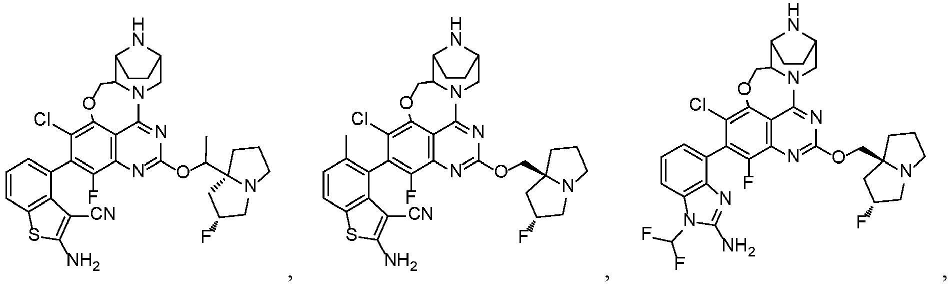 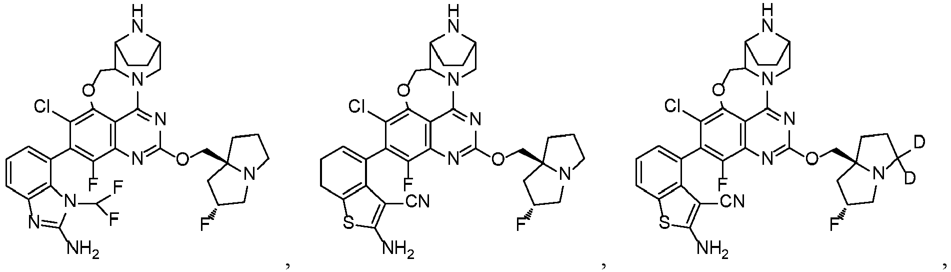 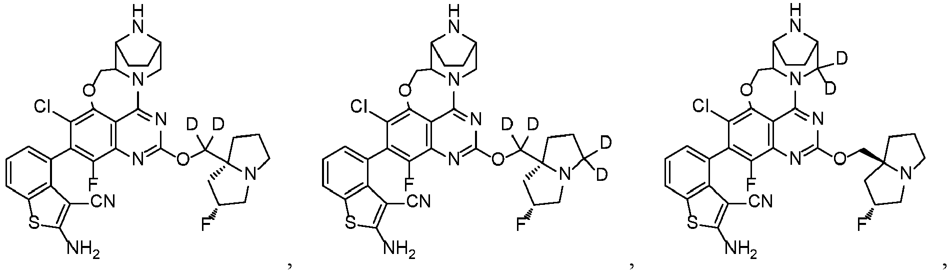 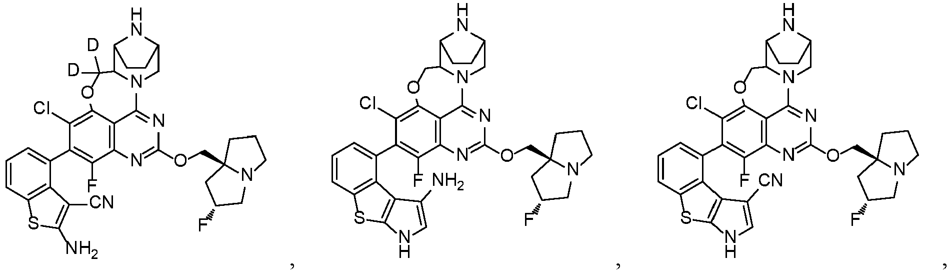 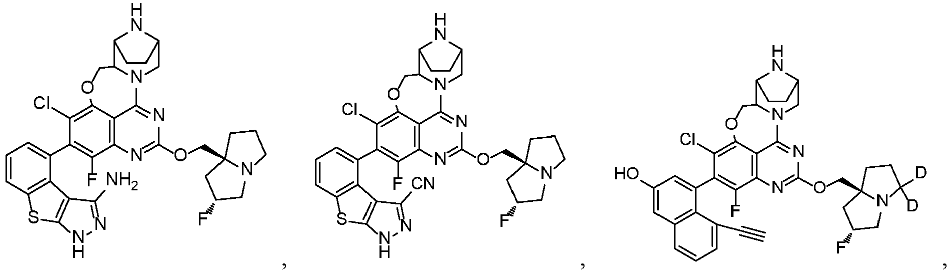 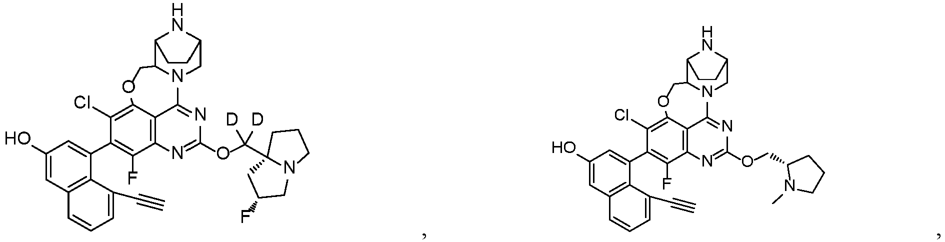 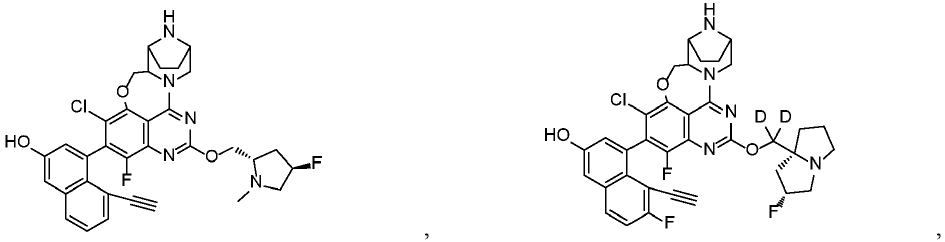 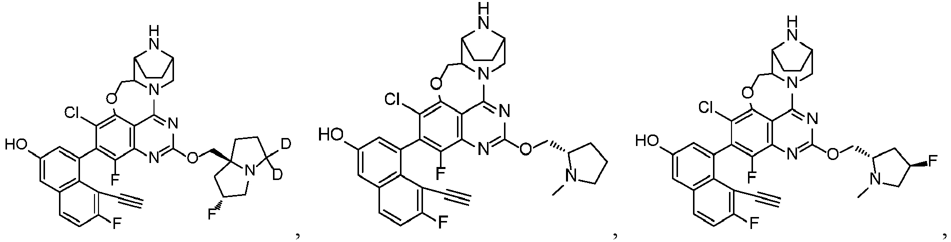 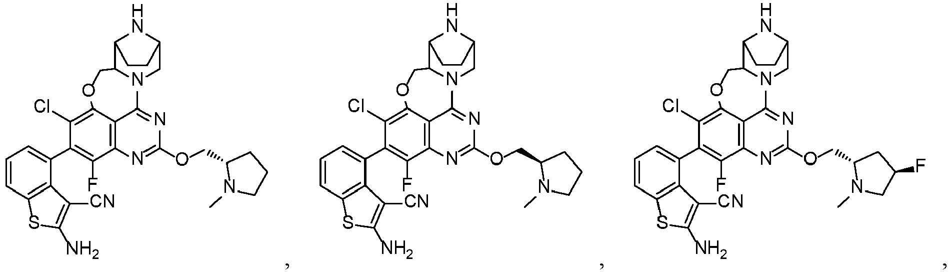 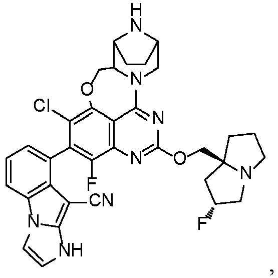 or a pharmaceutically acceptable salt.

The compound of formula (I), the stereoisomer thereof, the stable deuterated derivative thereof, or the pharmaceutically acceptable salt thereof has any one of the following structures: 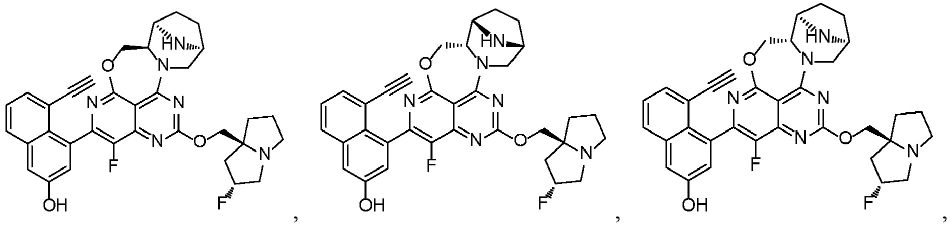 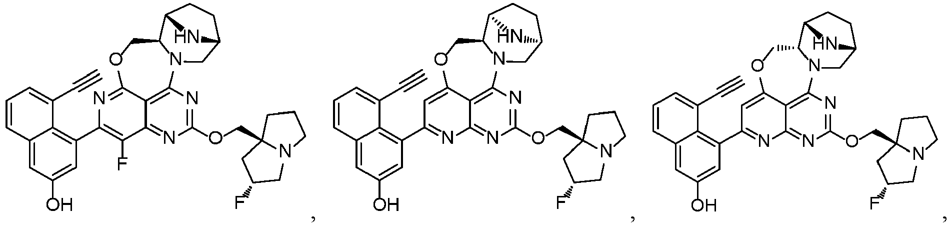 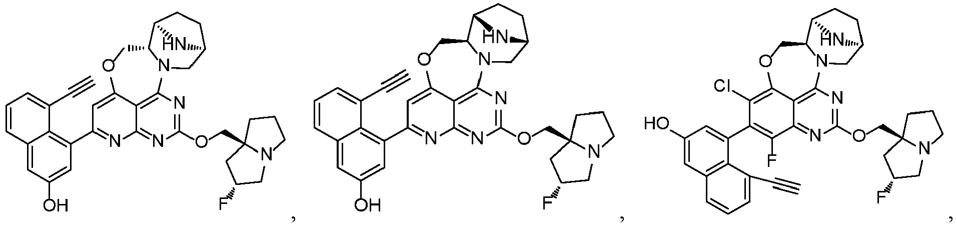 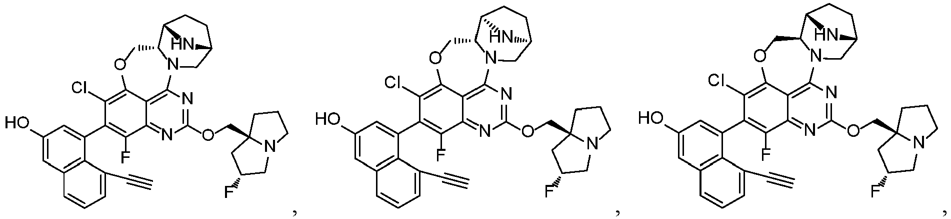 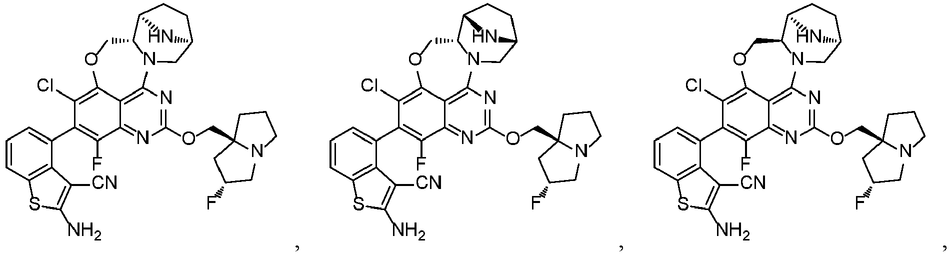 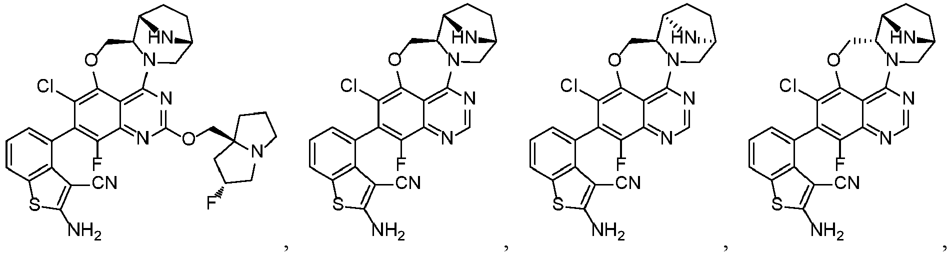 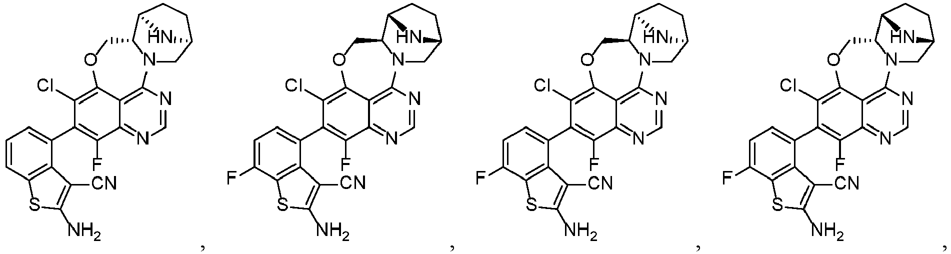 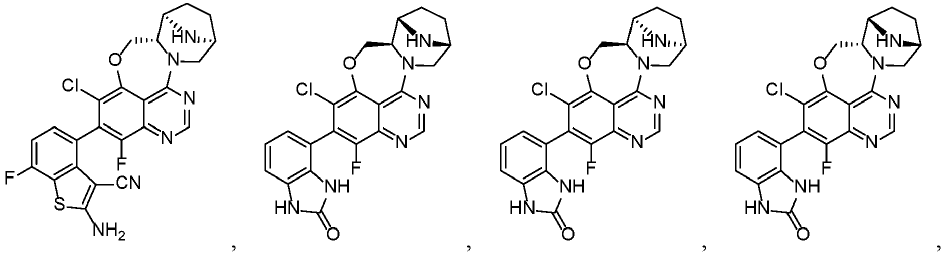 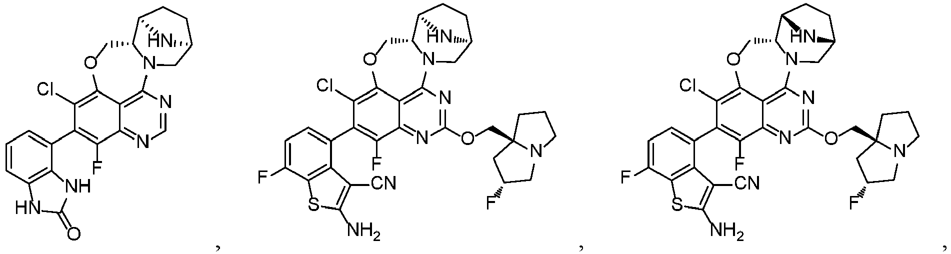 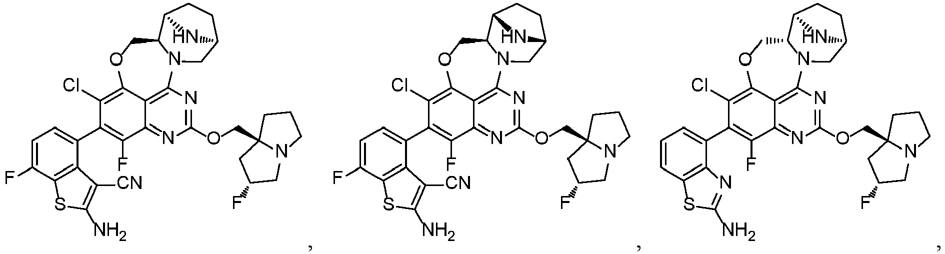 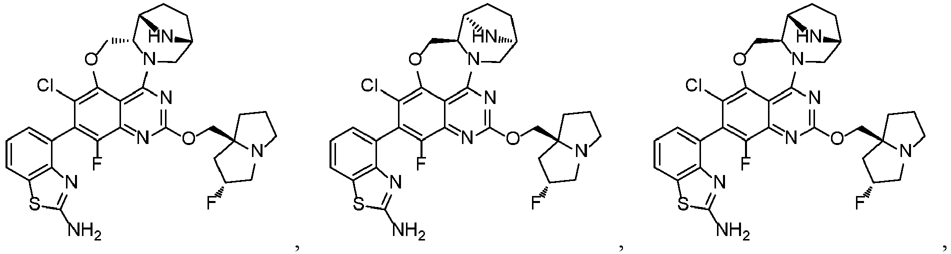 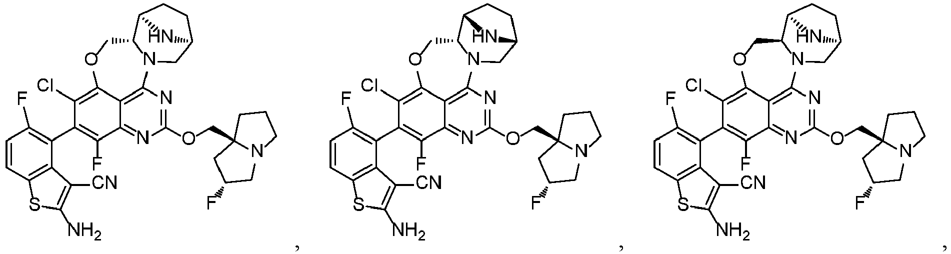 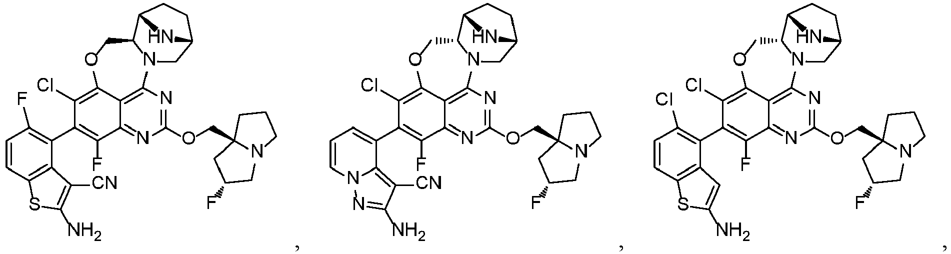 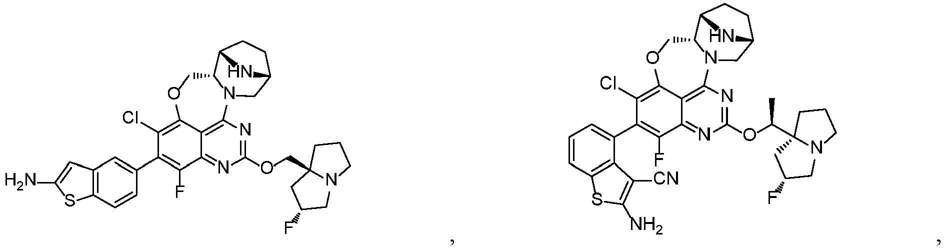 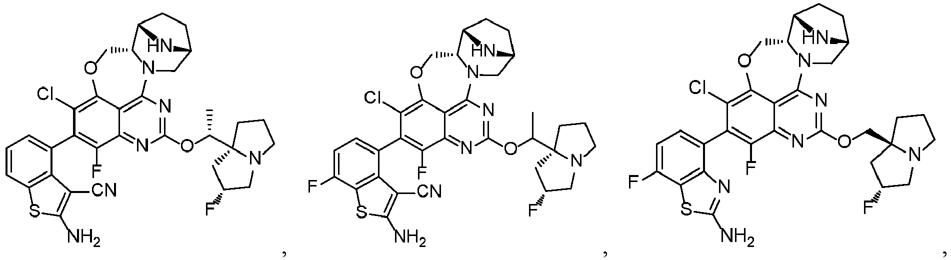 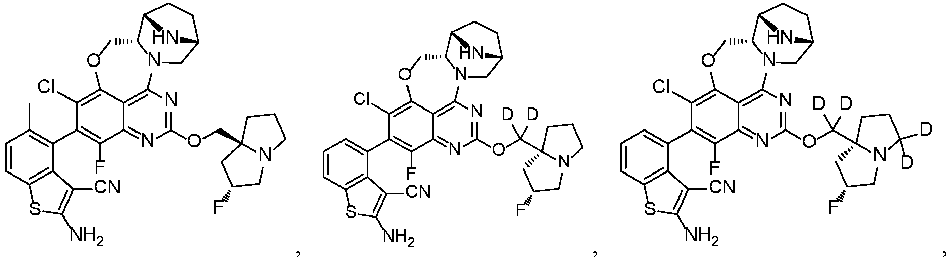 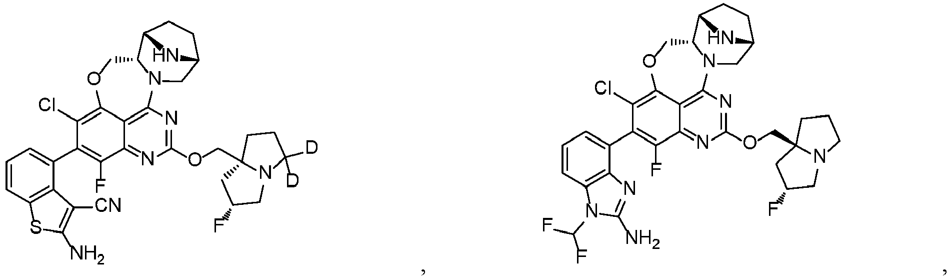 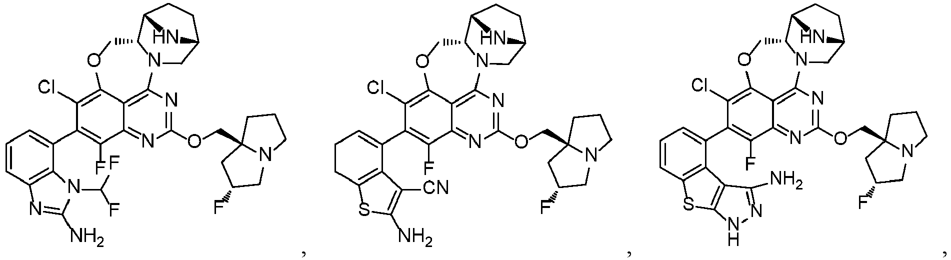 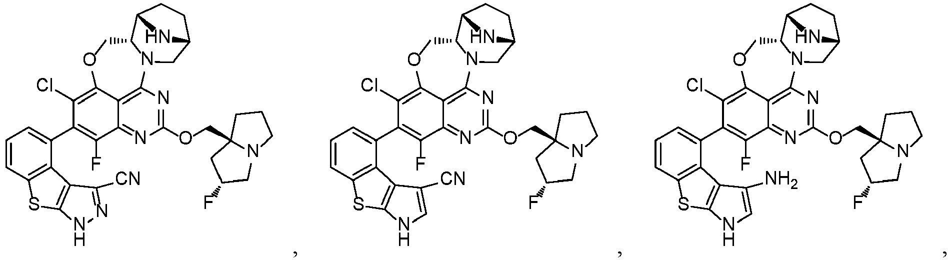 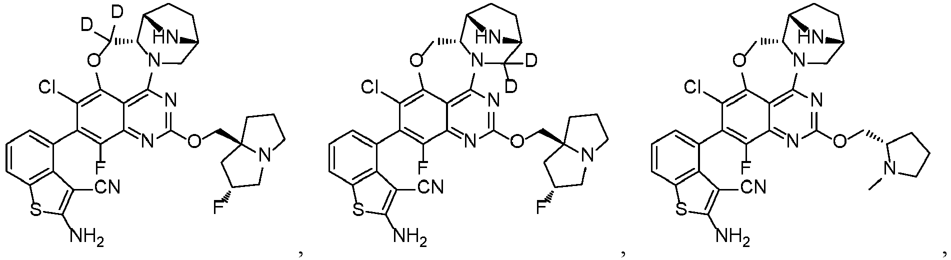 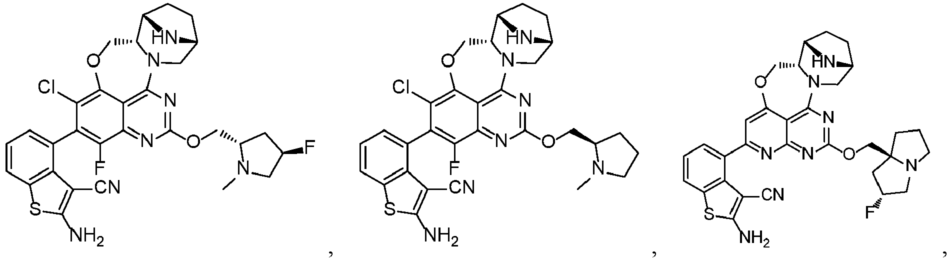 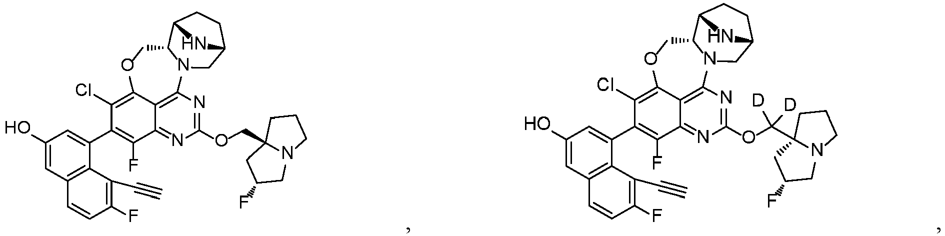 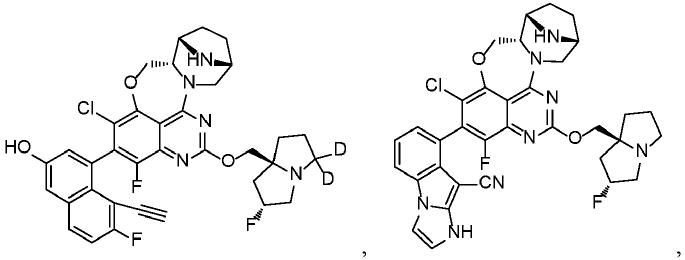 or a pharmaceutically acceptable salt.

The compound of formula (I), the stereoisomer thereof, the stable deuterated derivative thereof, or the pharmaceutically acceptable salt thereof has any one of the following structures: 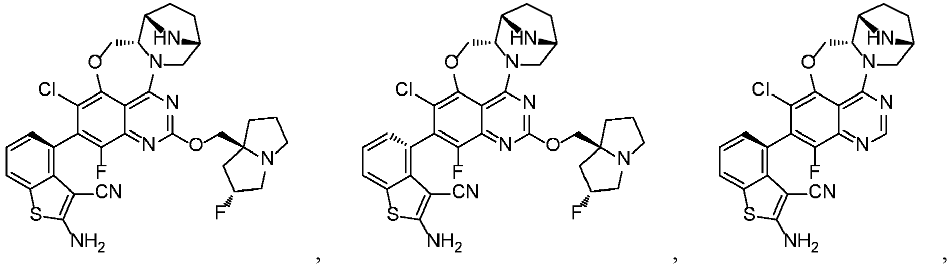 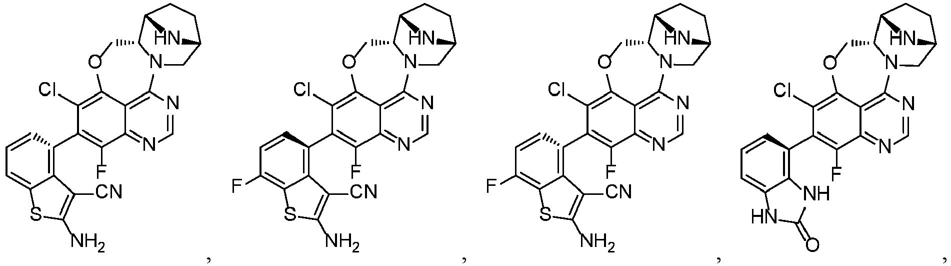 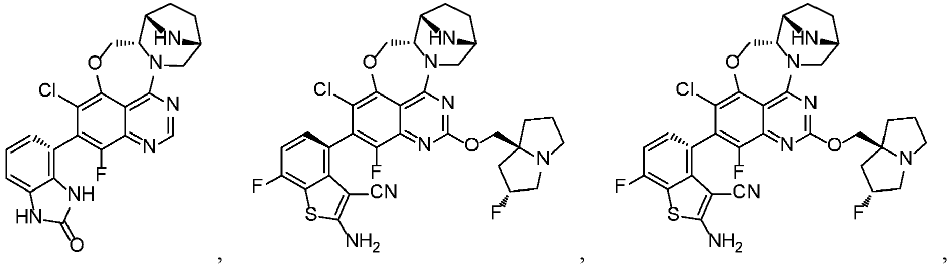 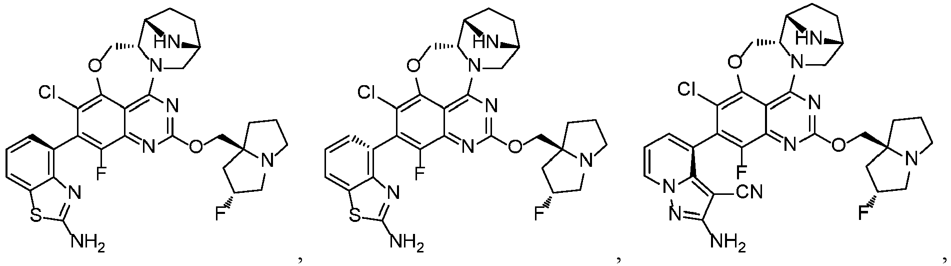 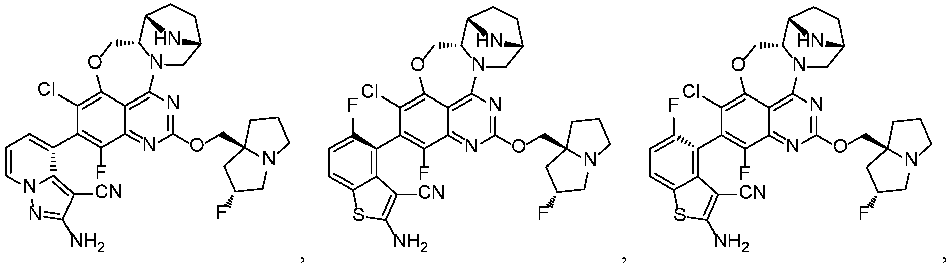 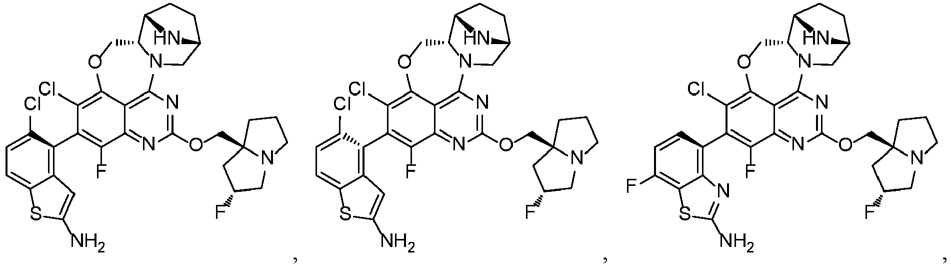 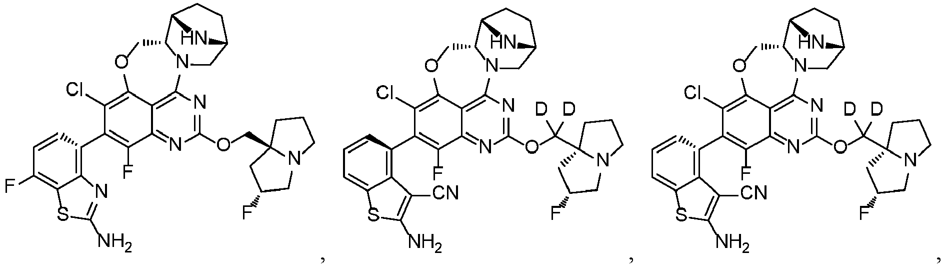 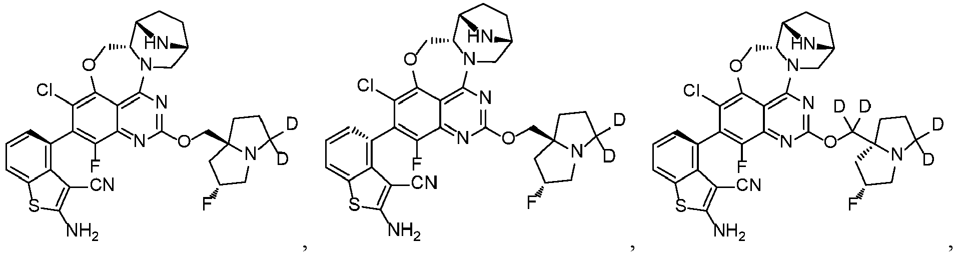 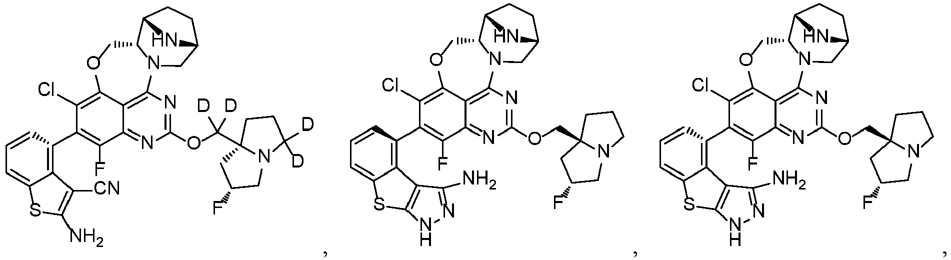 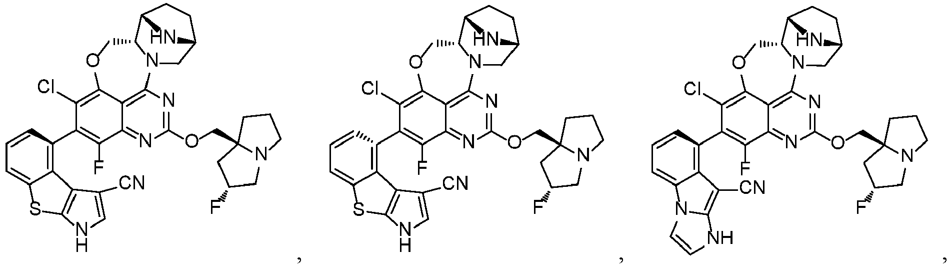 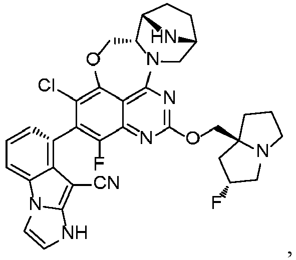 or a pharmaceutically acceptable salt.

The compound of formula (I), the stereoisomer thereof, the stable deuterated derivative thereof, or the pharmaceutically acceptable salt thereof has any one of the following structures: 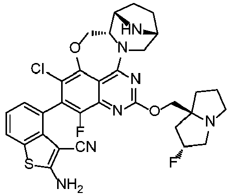 atropisomer 1, HPLC retention time: 16.724 minutes; 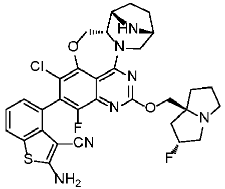 atropisomer 2, HPLC retention time: 17.162 minutes; 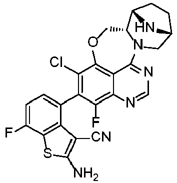 atropisomer 1, HPLC retention time: 16.118 minutes; 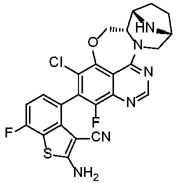 atropisomer 2, HPLC retention time: 16.489 minutes; 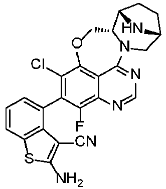 atropisomer 1, HPLC retention time: 15.584 minutes; 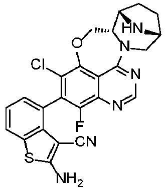 atropisomer 2, HPLC retention time: 15.966 minutes; 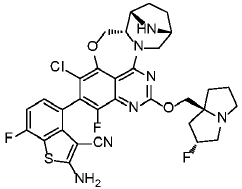 atropisomer 1, HPLC retention time: 17.403 minutes; 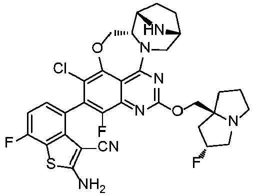 atropisomer 2, HPLC retention time: 18.023 minutes; 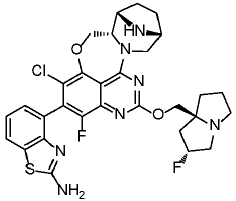 atropisomer 1, HPLC retention time: 16.558 minutes; 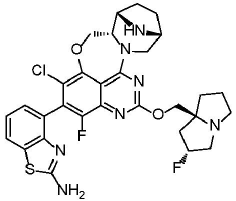 atropisomer 2, HPLC retention time: 17.076 minutes; 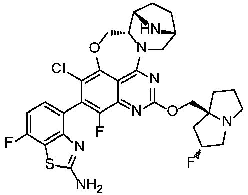 atropisomer 1, HPLC retention time: 17.152 minutes; 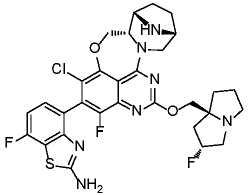 atropisomer 2, HPLC retention time: 17.710 minutes; 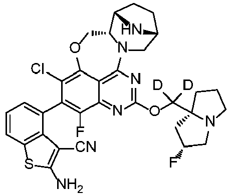 atropisomer 1, HPLC retention time: 16.821 minutes; 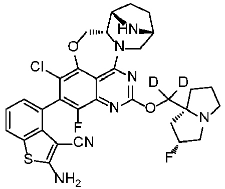 atropisomer 2, HPLC retention time: 17.354 minutes; 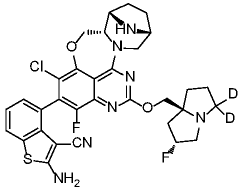 atropisomer 1, HPLC retention time: 16.793 minutes; 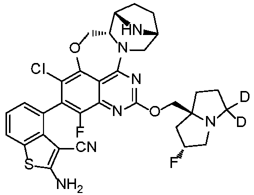 atropisomer 2, HPLC retention time: 17.322 minutes; 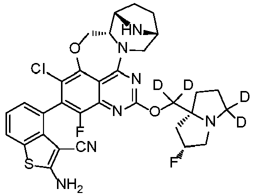 atropisomer 1, HPLC retention time: 16.758 minutes; 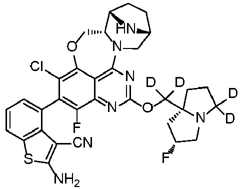 atropisomer 2, HPLC retention time: 17.293 minutes; 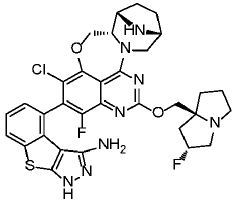 atropisomer 1, HPLC retention time: 15.452 minutes; 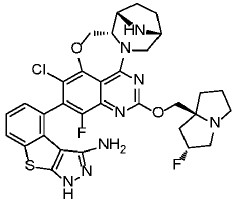 atropisomer 2, HPLC retention time: 15.838 minutes; 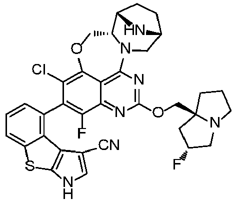 atropisomer 1, HPLC retention time: 17.687 minutes; 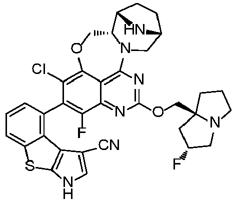 atropisomer 2, HPLC retention time: 18.251 minutes; 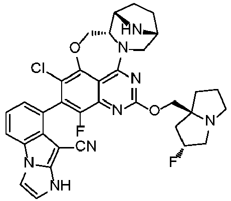 atropisomer 1, HPLC retention time: 16.574 minutes; 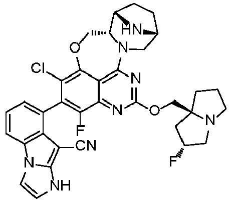 atropisomer 2, HPLC retention time: 17.147 minutes;
wherein the HPLC analysis method is as follows: chromatographic column: Welch Xtimate C18 4.6 mm * 150 mm, 5 µm, mobile phase A: acetonitrile, mobile phase B: 10 mM potassium dihydrogen phosphate buffer, with the pH adjusted to 8.0 with ammonia water. Gradient elution: mobile phase B holds at 90% for 5 minutes, B from 90% to 70%, elution time: 5 minutes, B from 70% to 40%, elution time: 4 minutes, B from 40% to 15%, elution time: 12 minutes, B from 15% to 90%, elution time: 2 minutes, B holds at 90% for 2 minutes. Detection wavelength: 214 and/or 262 nm; column temperature: 35°C. Flow rate: 1 mL/min.

The present disclosure also provides any one of the following compounds: 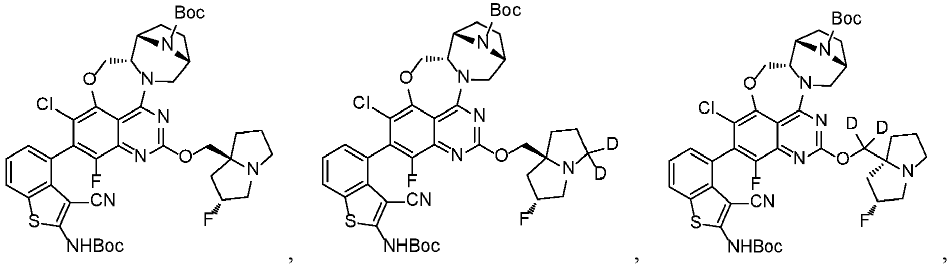 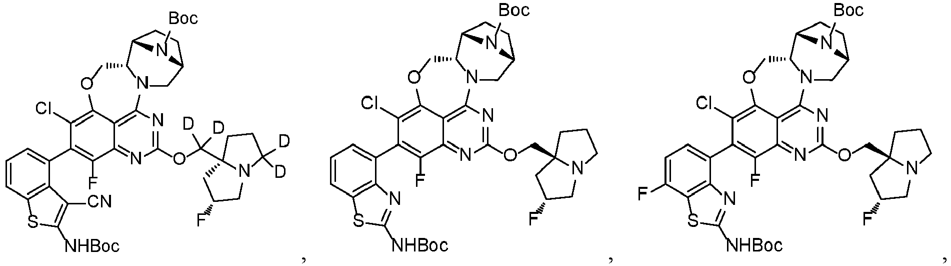 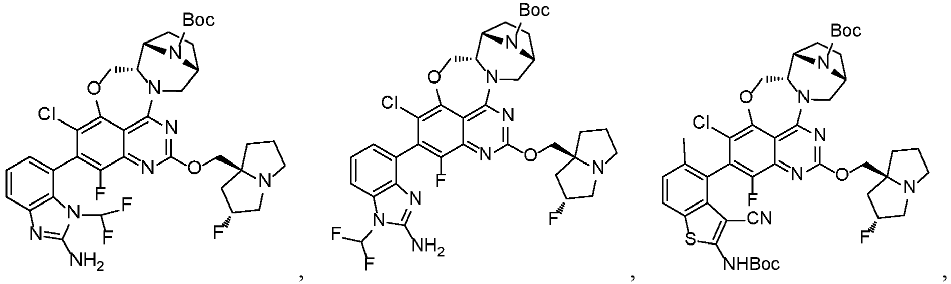 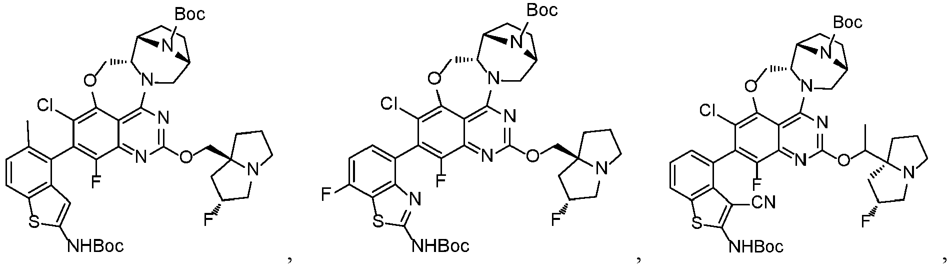 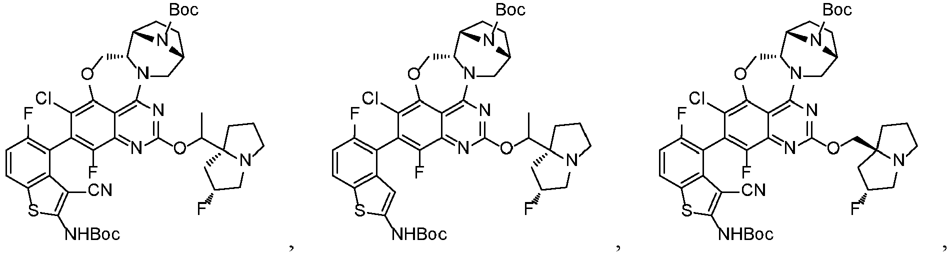 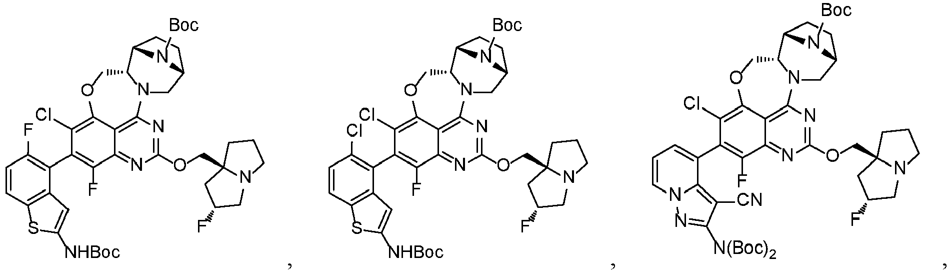 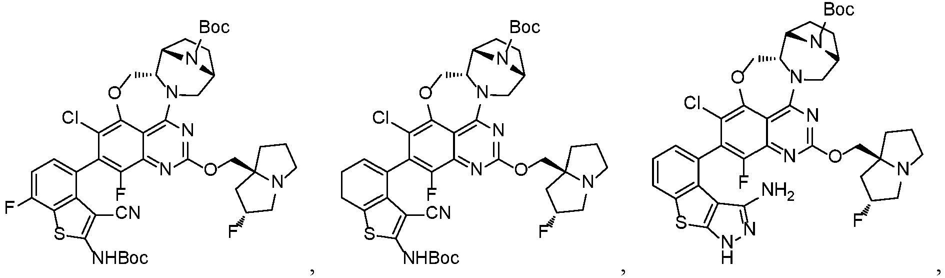 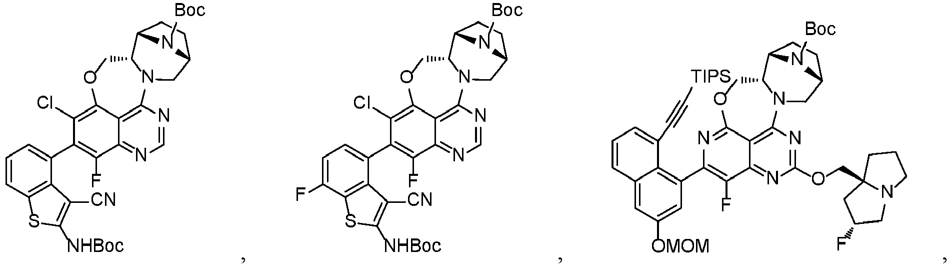 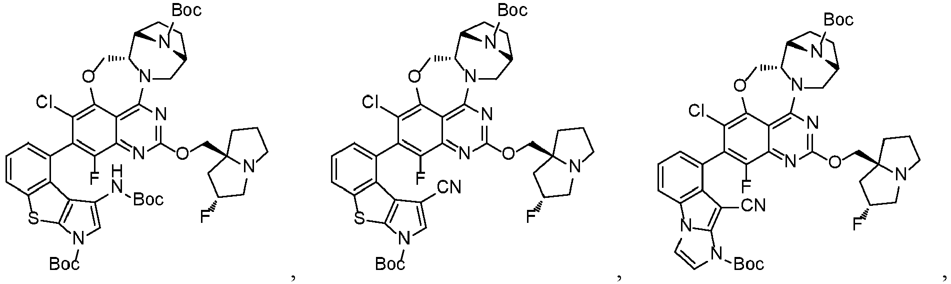 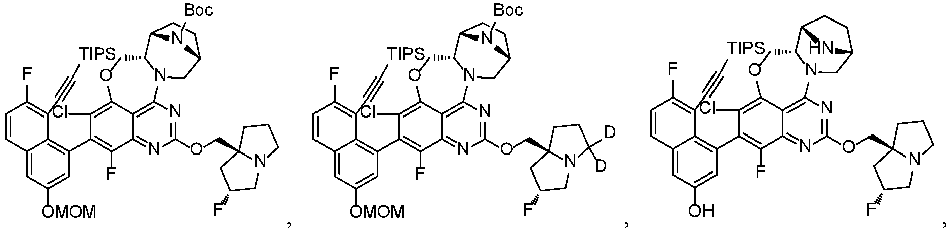 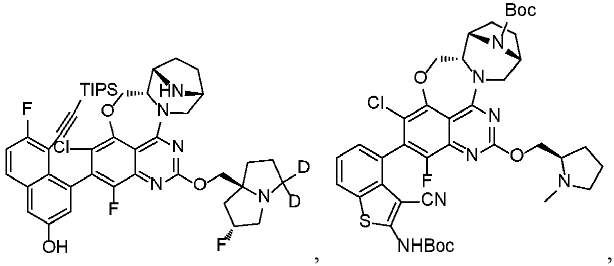 or a pharmaceutically acceptable salt.

The compound of formula (I) or the pharmaceutically acceptable salt thereof according to the present disclosure comprises all isotopically substituted derivatives of the compound of formula (I) or the pharmaceutically acceptable salt thereof.

The compound of formula (I) in any embodiment of the present disclosure comprises an isotopically substituted derivative thereof. The isotopically substituted derivative comprises: an isotopically substituted derivative obtained by substitution of any hydrogen atom (1 to 5) in formula (I) by 1 to 5 deuterium atoms (D or ²H), an isotopically substituted derivative obtained by substitution of any carbon atom (1 to 3) in formula (I) by 1 to 3 ¹⁴C atoms, or an isotopically substituted derivative obtained by substitution of any oxygen atom in formula (I) by 1 to 3 ¹⁸O atoms. In the present disclosure, the stable isotopically substituted derivative is preferably a stable deuterated derivative obtained by substitution of any hydrogen atom (1 to 5) in formula (I) by 1 to 5 deuterium atoms, and the stable deuterated derivative of the compound of formula (I) is more preferably:
a) A compound of formula (I) in which the R₂ group is substituted by 1 to 5 deuterium atoms to form a stable deuterated (²H₂ or ²H₄) R₂ group; e.g., 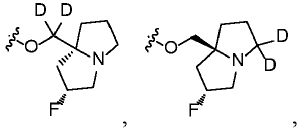 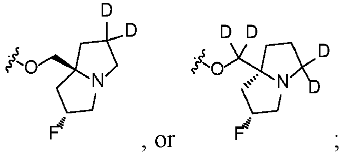 other groups are defined as above.
b) A compound of formula (IC), 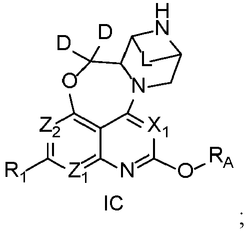 wherein Z₁, Z₂, X₁, L, R₁, and R_{A} are defined as above.
   or, c) a compound of formula (ID), 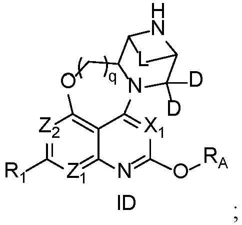 wherein q, Z₁, Z₂, X₁, L, R₁, and R_{A} are defined as above.

The compound of formula (I) or the pharmaceutically acceptable salt thereof according to the present disclosure comprises a prodrug of the compound of formula (I) or the pharmaceutically acceptable salt thereof.

The compound of formula (I) or the pharmaceutically acceptable salt thereof according to the present disclosure comprises all possible isomers of the compound of formula (I) or the pharmaceutically acceptable salt thereof. The isomers include, but are not limited to, forms of a stereoisomer, a tautomer, or a mixture thereof.

Stereochemical definitions and rules used in the present disclosure generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. The "stereoisomer" of the present disclosure refers to an optical isomer, a geometric isomer, or an atropisomer. These stereoisomers can be separated, purified, and enriched by asymmetric synthesis methods, chromatography, or chiral separation methods (including but not limited to thin-layer chromatography, rotary chromatography, column chromatography, gas chromatography, high-pressure liquid chromatography, etc.), and can also be obtained by chiral resolution through bonding with other chiral compounds (chemical bonding, etc.) or salt formation (physical bonding, etc.). The term "individual stereoisomer" means that one stereoisomer of a compound of the present disclosure is present in an amount of not less than 90% by mass, preferably not less than 95% by mass, relative to all stereoisomers of the compound. Individual stereoisomers and mixtures thereof are included within the scope of the present disclosure. The present disclosure includes all combinations and subsets of stereoisomers of all specific groups defined as above. The "stereoisomer" of the present disclosure is preferably an optical isomer and/or an atropisomer.

The term "optical isomer" includes an enantiomer, a diastereomer, and a mixture thereof. For example, a racemic mixture. The term "enantiomer" refers to two isomers of a compound that are non-superimposable mirror images of each other. The term "diastereomer" refers to a stereoisomer that has two or more chiral centers and whose molecules are not mirror images of each other. Diastereomers usually have different physical properties such as melting point, boiling point, spectral properties, and reactivity.

The term "geometric isomer" includes *cis-trans* isomers. It is a kind of stereoisomerism that is present in some compounds containing a double bond or cyclic compounds. The two different stereoisomers resulting from the presence of a double bond or ring, in which the free rotation of these molecules is hindered, are called *cis* and *trans* isomers.

The term "atropisomer" refers to a stereoisomer resulting from hindered rotation around a single bond, which is a conformational isomer that can be separated from each other resulting from hindered rotation of a single bond due to energy differences caused by steric strain or other contributing factors. For example, a structure of formula IIIA or IIIB: 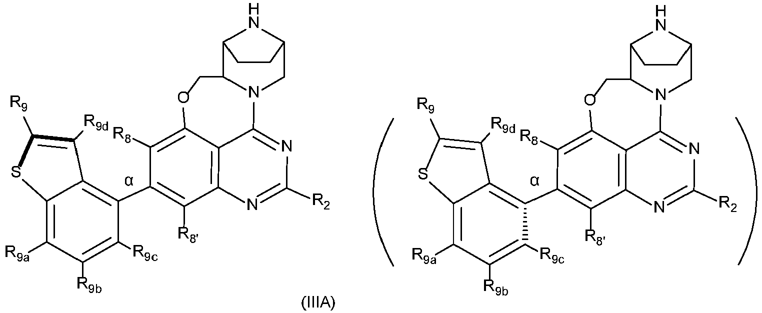 or 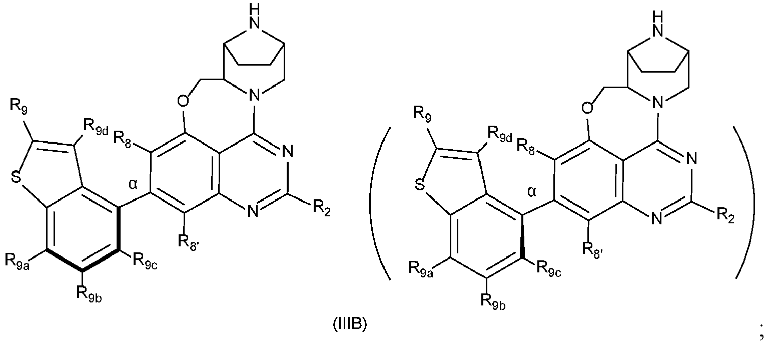 wherein R₂, R₉ₐ, R_{9b}, R_{9c}, and R_{9d} are defined as above; when R₈ and R_{8'} are not both H, rotation of α bond is hindered, and then an atropisomer of formula IIIA or IIIB is obtained.

The term "tautomer" refers to structural isomers with different energies can be interconvertible via a low energy barrier. For example, proton tautomers (also known as proton transfer tautomers) include interconversions via proton transfer, such as keto-enol tautomerism.

The present disclosure also provides a method for preparing the compound of formula (I), the stereoisomer thereof, the stable deuterated derivative thereof, or the pharmaceutically acceptable salt thereof, which is any one of the following methods:

### Method 1: As shown in reaction general formulas 1 and 2:

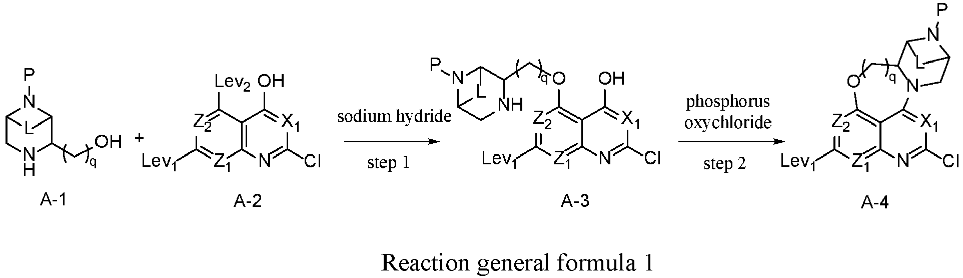

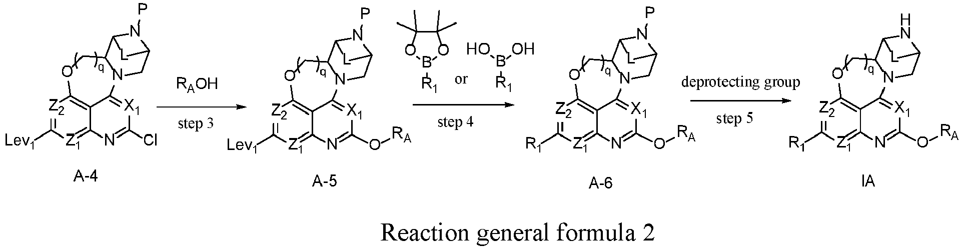

In method 1, Lev₁ and Lev₂ are each halogen, wherein Lev₁ is preferably chlorine or bromine; Lev₂ is preferably chlorine or fluorine; P is an amino protecting group, preferably Boc; q, Z₁, Z₂, X₁, L, R₁, and R_{A} are defined as above. Step 1: In a solvent (e.g., tetrahydrofuran), under alkaline conditions (e.g., sodium hydride), performing a nucleophilic substitution between A-1 and A-2 to obtain A-3; step 2: in a solvent (e.g., acetonitrile or tetrahydrofuran), under alkaline conditions (e.g., *N*,*N-*diisopropylethylamine), cyclizing A-3 under the action of phosphorus oxychloride to obtain A-4; step 3: in a solvent (e.g., tetrahydrofuran), under alkaline conditions (e.g., sodium hydride), performing a nucleophilic substitution between A-4 and R_{A}OH to obtain A-5; step 4: in a solvent (e.g., 1,4-dioxane or toluene), under alkaline conditions (e.g., cesium carbonate, sodium carbonate, or potassium phosphate), in the presence of a catalyst (e.g., tetrakis(triphenylphosphine)palladium, [1,1'-bis(diphenylphosphino)ferrocene]dichloropalladium(II) dichloromethane complex, [1,1'-bis(di-*tert*-butylphosphino)ferrocene]dichloropalladium(II), or dichloro[bis(diphenylphosphinophenyl)ether]palladium(II)), performing a Suzuki coupling between A-5 and boronic acid or boronic acid pinacol ester of R₁ to obtain A-6; step 5: reacting A-6 through a one-step or multi-step deprotection to obtain a compound of formula IA.

### Method 2: As shown in reaction general formulas 3 and 4:

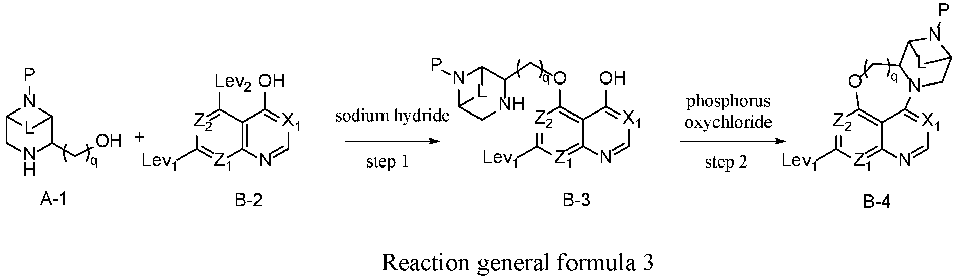

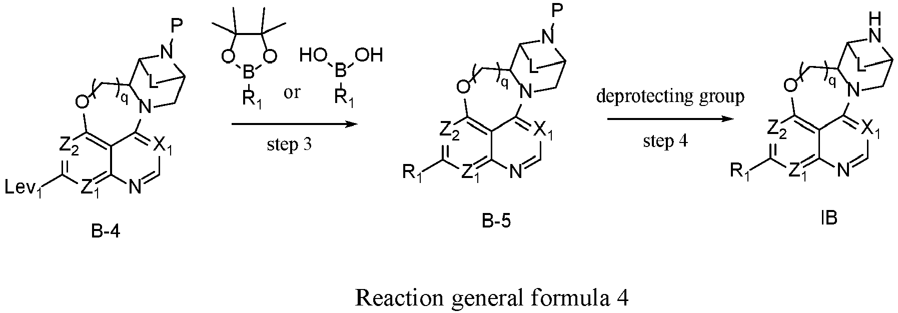

In method 2, Lev₁ is halogen, preferably chlorine or bromine; P is an amino protecting group, preferably Boc; q, Z₁, Z₂, X₁, L, and R₁ are defined as above. Step 1: In a solvent (e.g., tetrahydrofuran), under alkaline conditions (e.g., sodium hydride), performing a nucleophilic substitution between A-1 and B-2 to obtain B-3; step 2: in a solvent (e.g., acetonitrile or tetrahydrofuran), under alkaline conditions (e.g., *N*,*N-*diisopropylethylamine), cyclizing B-3 under the action of phosphorus oxychloride to obtain B-4; step 3: in a solvent (e.g., 1,4-dioxane or toluene), under alkaline conditions (e.g., cesium carbonate, sodium carbonate, or potassium phosphate), in the presence of a catalyst (e.g., tetrakis(triphenylphosphine)palladium, [1,1'-bis(diphenylphosphino)ferrocene]dichloropalladium(II) dichloromethane complex, [1,1'-bis(di-*tert*-butylphosphino)ferrocene]dichloropalladium(II), or dichloro[bis(diphenylphosphinophenyl)ether]palladium(II)), performing a Suzuki coupling between B-4 and boronic acid or boronic acid pinacol ester of R₁ to obtain B-5; step 4: reacting B-5 through a one-step or multi-step deprotection to obtain a compound of formula IB.

### Method 3: As shown in reaction general formula 5:

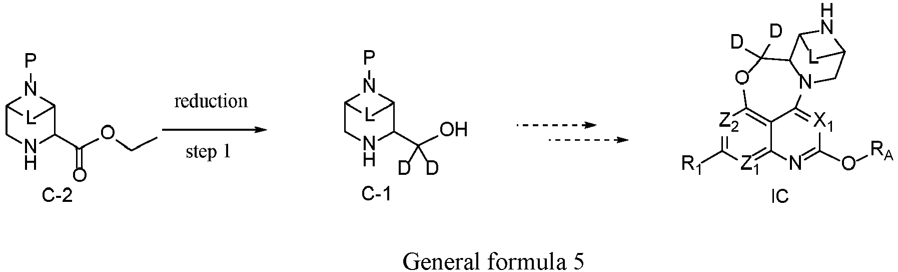

In method 3, P is an amino protecting group, preferably Boc; Z₁, Z₂, X₁, L, R₁, and R_{A} are defined as above. Step 1: In a solvent (e.g., tetrahydrofuran), reducing C-2 with a deuterated reducing agent (e.g., lithium aluminum deuteride (LiAlD₄)) to obtain C-1. C-1 is used as the starting material to synthesize a compound of formula IC according to the synthesis method of steps 1 to 5 in method 1.

### Method 4: As shown in reaction general formula 6:

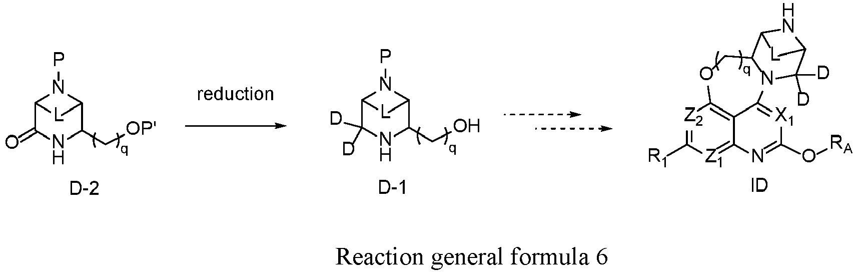

In method 4, P' is a hydroxyl protecting group, preferably an organosilicon group (e.g., tert-butyldimethylsilyl, *tert*-butyldiphenylsilyl); P is an amino protecting group, preferably Boc; q, Z₁, Z₂, X₁, L, R₁, and R_{A} are defined as above. Step 1: In a solvent (e.g., tetrahydrofuran), reducing D-2 with a deuterated reducing agent (e.g., lithium aluminum deuteride (LiAlD₄)) to obtain D-1. C-1 is used as the starting material to synthesize a compound of formula ID according to the synthesis method of steps 1 to 5 in method 1.

In the above methods, when there is an amino group, a hydroxyl group, or a carboxyl group in A-1 to A-6, B-1 to B-5, C-1 to C-2, D-1 to D-2, -R₁, or -R_{A}, the amino group, hydroxyl group, or carboxyl group can be protected by a protecting group to avoid any side reactions. If the above amino protecting group, hydroxyl protecting group, or carboxyl protecting group is present, a subsequent deprotection step is required to obtain the compounds of formulas IA to ID. Any suitable amino protecting group, such as *tert*-butoxycarbonyl (Boc), can be used to protect the amino group. If Boc is used as a protecting group, the subsequent deprotection reaction can be carried out under standard conditions, for example, p-toluenesulfonic acid/methanol system, dichloromethane/trifluoroacetic acid system, organic solution system of hydrogen chloride (organic solution includes, but is not limited to, ether solution, 1,4-dioxane solution, methanol solution, ethanol solution, isopropanol solution) or trimethylsilyl trifluoromethanesulfonate/2,6-lutidine/dichloromethane system; any suitable hydroxyl protecting group, for example, benzyl, methoxymethyl (MOM-), organosilicon groups (including, but not limited to, tert-butyldimethylsilyl (TBS-), tert-butyldiphenylsilyl (TBDPS-), trimethylsilyl (TMS-), triisopropylsilyl (TIPS-)) can be used as hydroxyl protecting groups, and the subsequent deprotection reaction can be carried out under standard conditions. For example, benzyl can be deprotected with palladium on carbon/hydrogen system; methoxymethyl can be deprotected with organic solution system of hydrogen chloride (organic solution includes, but is not limited to, ether solution, 1,4-dioxane solution, methanol solution, ethanol solution, isopropanol solution); organosilicon group can be deprotected with tetrabutylammonium fluoride/tetrahydrofuran system. Any suitable carboxyl protecting group, for example, forming a carboxylate group (e.g., methyl carboxylate, ethyl carboxylate), can be used to protect the carboxyl group, and the subsequent deprotection reaction can be carried out under standard conditions, for example, sodium hydroxide, potassium hydroxide, and lithium hydroxide in tetrahydrofuran, water, and/or methanol solvents for deprotection. The above deprotection reaction is preferably carried out in the last step.

The compound of formula (I), the stereoisomer thereof, the stable deuterated derivative thereof, or the pharmaceutically acceptable salt thereof can be synthesized by general chemical methods.

In general, salts can be prepared by reacting free bases or acids with an equivalent or excess amount of acids (inorganic or organic) or bases (inorganic or organic) in a suitable solvent or solvent composition.

The present disclosure also provides a pharmaceutical composition, comprising a therapeutically effective amount of an active component and a pharmaceutically acceptable excipient; the active component comprises one or more than one of the compound of formula (I), the stereoisomer thereof, the stable deuterated derivative thereof, or the pharmaceutically acceptable salt thereof.

In the pharmaceutical composition, the active component may also comprise other therapeutic agents for cancer.

In the pharmaceutical composition, the pharmaceutically acceptable excipient may comprise a pharmaceutically acceptable vehicle, diluent, and/or formulating agent.

According to the therapeutic purpose, the pharmaceutical composition can be made into various types of administration unit dosage forms, such as tablets, pills, powders, liquids, suspensions, emulsions, granules, capsules, suppositories, and injections (solutions and suspensions), preferably liquids, suspensions, emulsions, suppositories, and injections (solutions and suspensions).

To shape the pharmaceutical composition in the form of a tablet, any formulating agent known and widely used in the art can be used. For example, vehicles, such as lactose, white sugar, sodium chloride, glucose, urea, starch, calcium carbonate, kaolin, crystalline cellulose, and silicic acid; binders, such as water, ethanol, propanol, common syrup, glucose solution, starch solution, gelatin solution, carboxymethylcellulose, shellac, methylcellulose, potassium phosphate, and polyvinylpyrrolidone; disintegrants, such as dry starch, sodium alginate, agar powder, kelp powder, sodium bicarbonate, calcium carbonate, fatty acid ester of polyethylene dehydrated sorbitol, sodium dodecyl sulfate, glycerol monostearate, starch, and lactose; disintegration inhibitors, such as white sugar, glycerol tristearate, coconut oil, and hydrogenated oil; adsorption promoters, such as quaternary amine base and sodium dodecyl sulfate; wetting agents, such as glycerin and starch; adsorbents, such as starch, lactose, kaolin, bentonite, and colloidal silicic acid; and lubricants, such as pure talc, stearate, boric acid powder, and polyethylene glycol. Common coating materials can also be selected to make sugar-coated tablets, gelatin-coated tablets, enteric-coated tablets, film-coated tablets, double-layer tablets, and multi-layer tablets as required.

To shape the pharmaceutical composition in the form of a pill, any formulating agent known and widely used in the art may be used, for example, vehicles, such as lactose, starch, coconut oil, hardened vegetable oil, kaolin, and talc; binders, such as gum arabic powder, tragacanth powder, gelatin, and ethanol; disintegrants, such as agar and kelp powder.

To shape the pharmaceutical composition in the form of a suppository, any formulating agent known and widely used in the art may be used, for example, polyethylene glycol, coconut oil, higher alcohol, esters of higher alcohol, gelatin, and semi-synthetic glyceride.

To prepare the pharmaceutical composition in the form of an injection, the solution or suspension can be sterilized (preferably by adding an appropriate amount of sodium chloride, glucose, or glycerol) to make an injection isotonic with blood. Any vehicle commonly used in the art can also be used in the preparation of an injection. For example, water, ethanol, propylene glycol, ethoxylated isostearyl alcohol, polyoxylated isostearyl alcohol, and fatty acid ester of polyethylene dehydrated sorbitol. In addition, common dissolving agents, buffers, analgesics, etc. can also be added.

In the present disclosure, the content of the composition in the pharmaceutical composition is not particularly limited, and can be selected within a wide range, usually 5wt.% to 95wt.%, preferably 30wt.% to 80wt.%.

In the present disclosure, the administration method of the pharmaceutical composition is not particularly limited. Various dosage forms of preparations can be selected for administration according to the patient's age, gender, and other conditions and symptoms. For example, tablets, pills, solutions, suspensions, emulsions, granules, or capsules are administered orally; injections can be administered alone, or mixed with injectable delivery solutions (such as glucose solution and amino acid solution) for intravenous injection; suppositories are administered to the rectum.

The present disclosure also provides a use of the compound of formula (I), the stereoisomer thereof, the stable deuterated derivative thereof, or the pharmaceutically acceptable salt thereof, or the pharmaceutical composition in the manufacture of a KRAS G12D inhibitor.

The present disclosure also provides a use of the compound of formula (I), the stereoisomer thereof, the stable deuterated derivative thereof, or the pharmaceutically acceptable salt thereof, or the pharmaceutical composition in the manufacture of a medicament for treating a related disease mediated by KRAS G12D. The related disease is cancer. Further, the cancer is a cancer containing a G12D mutated KRAS gene in tumor cell DNA.

The present disclosure also provides a use of the compound of formula (I), the stereoisomer thereof, the stable deuterated derivative thereof, or the pharmaceutically acceptable salt thereof, or the pharmaceutical composition in the manufacture of a medicament for treating and/or alleviating cancer.

The present disclosure also provides a use of the compound of formula (I), the stereoisomer thereof, the stable deuterated derivative thereof, or the pharmaceutically acceptable salt thereof, or the pharmaceutical composition in the manufacture of a medicament for treating and/or alleviating pancreatic cancer, endometrial cancer, lung cancer (e.g., small cell lung cancer, non-small cell lung cancer), rectal cancer, and/or colorectal cancer.

The present disclosure also provides the compound of formula (I), the stereoisomer thereof, the stable deuterated derivative thereof, or the pharmaceutically acceptable salt thereof, or the pharmaceutical composition for use in the treatment of cancer.

The present disclosure also provides the compound of formula (I), the stereoisomer thereof, the stable deuterated derivative thereof, or the pharmaceutically acceptable salt thereof, or the pharmaceutical composition for use in the treatment and/or alleviation of pancreatic cancer, endometrial cancer, lung cancer (e.g., small cell lung cancer, non-small cell lung cancer), rectal cancer, and/or colorectal cancer.

The present disclosure further provides a method for treating cancer with the compound of formula (I), the stereoisomer thereof, the stable deuterated derivative thereof, or the pharmaceutically acceptable salt thereof, or the pharmaceutical composition, comprising administering to a mammal a therapeutically effective amount of the compound of formula (I) or the pharmaceutically acceptable salt thereof, or the pharmaceutical composition.

The mammal is preferably a human.

The present disclosure further provides the compound of formula (I), the stereoisomer thereof, the stable deuterated derivative thereof, or the pharmaceutically acceptable salt thereof, or the pharmaceutical composition in combination with one or more than one other therapeutic agent and/or therapeutic method for use in the treatment and/or alleviation of a related disease mediated by KRAS G12D. The related disease mediated by KRAS G12D is cancer. Further, the cancer is a cancer containing a G12D mutated KRAS gene in tumor cell DNA.

In the present disclosure, the cancer comprises metastatic and non-metastatic cancers, as well as familial and sporadic cancers, and may also comprise solid tumors and non-solid tumors.

Specific examples of the cancer may include, but are not limited to, eye cancer, bone cancer, lung cancer (including small cell lung cancer and non-small cell lung cancer), gastric cancer, pancreatic cancer, breast cancer, prostate cancer, brain cancer (including glioblastoma and medulloblastoma), ovarian cancer, bladder cancer, cervical cancer, endometrial cancer, fallopian tube cancer, peritoneal cancer, testicular cancer, renal cancer (including adenocarcinoma and nephroblastoma), oral cancer (including squamous cell carcinoma), tongue cancer, laryngeal cancer, nasopharyngeal cancer, head and neck cancer, colon cancer, small bowel cancer, rectal cancer, parathyroid cancer, thyroid cancer, esophageal cancer, gallbladder cancer, cholangiocarcinoma, liver cancer, sarcoma, skin cancer, lymphoid leukemia (including acute lymphoblastic leukemia, lymphoma, myeloma, chronic lymphocytic leukemia, Hodgkin lymphoma, non-Hodgkin lymphoma, T-cell chronic lymphocytic leukemia, and B-cell chronic lymphocytic leukemia), myeloid-related leukemia (including acute myeloid leukemia and chronic myeloid leukemia), and one or more than one of AIDs-related leukemias.

Unless otherwise specified, the terms used in the description and claims of the present disclosure have the following meanings:

The term "C_{t-q}" refers to the range from the starting point to the end point, wherein t, q, and each point in the range are integers, indicating the number of the carbon atom, for example, C₁₋₄ means that the number of the carbon atom is 1, 2, 3, or 4; C₁₋₆ means that the number of the carbon atom is 1, 2, 3, 4, 5, or 6; C₃₋₈ means that the number of the carbon atom is 3, 4, 5, 6, 7, or 8; C_{t-q} can be used in combination with any group containing the carbon atom for the purpose of defining the number of the carbon atom, such as C₁₋₆ alkyl, C₁₋₄ alkylene, C₃₋₈ cycloalkyl, C₆₋₁₀ aryl, C₁₋₄ alkoxy, and C₃₋₈ cycloalkyl-C₁₋₄ alkyl.

The term "alkyl" means a saturated linear or branched hydrocarbon group containing 1 to 20 carbon atoms, preferably 1 to 10 carbon atoms, more preferably 1 to 8, 1 to 6, 1 to 4, or 1 to 3 carbon atoms. Representative examples of alkyl include, but are not limited to, methyl, ethyl, n-propyl, isopropyl, n-butyl, sec-butyl, tert-butyl, isobutyl, n-pentyl, n-hexyl, n-heptyl, octyl, nonyl, decyl, 1,1-dimethylpropyl, 1,2-dimethylpropyl, 2,2-dimethylpropyl, 1-ethylpropyl, 2-methylbutyl, 3-methylbutyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2,3-dimethylbutyl, 2-ethylbutyl, 2-methylpentyl, 3-methylpentyl, 4-methylpentyl, 4,4-dimethylpentyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylhexyl, 3-ethylhexyl, 2,2,4-trimethylpentyl, undecyl, dodecyl, and various isomers thereof.

The term "alkylene" means a saturated linear or branched unbridged divalent alkyl group containing 1 to 20 carbon atoms, preferably 1 to 6 carbon atoms, more preferably 1 to 4 or 1 to 3 carbon atoms, such as methylene (=CH₂), ethylene (=CHCH₃), and 2-isopropylidene (=CH(CH₃)₂).

The term "cycloalkyl" means a saturated or partially unsaturated (containing 1 or 2 double bonds) monocyclic or polycyclic group containing 3 to 20 carbon atoms. The "cycloalkyl" is preferably a 3- to 10-membered monocycloalkyl group, more preferably a 3- to 8-membered or 3- to 6-membered monocycloalkyl group, such as cyclopropyl, cyclobutyl, cyclopentyl, cyclohexyl, cycloheptyl, cyclooctyl, cyclodecyl, cyclododecyl, cyclobutenyl, cyclopentenyl, cyclopentadienyl, cyclohexenyl, and 1,3-cyclohexadienyl. "Polycyclic cycloalkyl" includes "bridged cycloalkyl", "fused cycloalkyl", and "spiro cycloalkyl", such as tetrahydronaphthyl and 2,3-dihydroindenyl. Monocyclic cycloalkyl or polycyclic cycloalkyl can be attached to the parent molecule via any carbon atom on the ring.

The term "heterocycloalkyl" means a saturated or partially unsaturated (containing 1 or 2 double bonds) non-aromatic cyclic group consisting of carbon atoms and heteroatoms selected from nitrogen, oxygen, sulfur, boron, etc., which may be a monocyclic or polycyclic group, preferably a monocyclic, bicyclic, or tricyclic group. In the present disclosure, the number of heteroatoms in the "heterocycle" or "heterocycloalkyl" is preferably 1, 2, 3, or 4, and the nitrogen, sulfur, or boron atom in the heterocycloalkyl group can be selectively substituted by oxo (=O). The carbon atom can be further substituted by oxo (=O) or thio (=S). The nitrogen atom can selectively be further substituted by other groups to form tertiary amines or quaternary ammonium salts. The "heterocycle" or "heterocycloalkyl" is preferably a 3- to 10-membered monocyclic group, more preferably a 3- to 8-membered, 3- to 6-membered, or 4- to 8-membered monocyclic group. Representative examples include, but are not limited to, aziridinyl, azetidinyl, tetrahydrofuran-2-yl, morpholinyl, thiomorpholinyl, thiomorpholin-S-oxide-4-yl, piperidinyl, pyrrolidinyl, piperazinyl, homopiperazinyl, 1,4-dioxanyl, pyranyl, tetrahydropyranyl, tetrahydrothienyl, dihydroimidazolyl, 2,3-dihydrofuryl, 2,5-dihydrofuryl, dihydropyrazolyl, 2,3-dihydro-1*H*-pyrrolidinyl, 2,5-dihydro-1*H*-pyrrolidinyl, 1,1-dioxotetrahydro-2*H*-thiopyranyl, 1-imino-1-oxotetrahydro-2*H*-thiopyranyl, 1,1-dioxotetrahydrothienyl, 1-imino-1-oxotetrahydrothienyl, 1,1-dioxo-3,4-dihydro-2*H-*thiopyranyl, 1-imino-1-oxo-3,4-dihydro-2*H*-thiopyranyl, 1,1-dioxo-2,3-dihydrothienyl, 1-imino-1-oxo-2,3-dihydrothienyl, 1,3-dioxolanyl, 1,3-dioxolyl, 1,3-oxathiolanyl, 1,3-oxathiolyl, 1,4-dioxa-2-hexenyl, 3,4-dihydro-2*H*-pyranyl, 3,6-dihydro-2*H*-pyranyl, 1,2,3,4-tetrahydropyrazinyl, 1,2,3,4-tetrahydropyridyl, 1,2,3,6-tetrahydropyridyl, etc. "Polycyclic heterocycle" or "polycyclic heterocycloalkyl" includes "fused heterocycloalkyl", "spiro heterocycloalkyl", and "bridged heterocycloalkyl". "Fused heterocycloalkyl" includes a monocyclic heterocycloalkyl ring fused to aryl, cycloalkyl, heterocycloalkyl, or heteroaryl. "Fused heterocycloalkyl" includes, but is not limited to, indolinyl, 2,3-dihydrobenzofuryl, 1,3-dihydroisobenzofuryl, 2,3-dihydrobenzo[*b*]thienyl, dihydrobenzopyranyl, 1,2,3,4-tetrahydroquinolinyl, benzo[*d*][1,3]dioxolanyl, 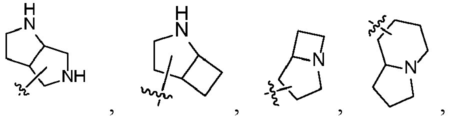 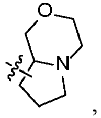 hexahydro-1*H*-pyrrolizinyl 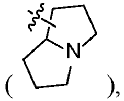 etc. "Spiro heterocycloalkyl" means a bicyclic group formed by two heterocycloalkyl groups or one cycloalkyl group and one heterocycloalkyl group sharing one carbon atom. "Bridged heterocycloalkyl" means a monocyclic heterocycloalkyl group in which any two unlinked ring atoms are bridged by a linear group formed by 1 to 3 additional carbon atoms or heteroatoms (the linear group is selected from, but not limited to, -CH₂-, -O-, -NH-, -S-, -CH₂CH₂-, -CH₂O-, -CH₂S-, -CH₂NH-, -CH₂CH₂CH₂-, -CH₂OCH₂-, -CH₂CH₂O-, -CH₂CH₂NH-, and -CH₂NHCH₂-). Bridged heterocycloalkyl includes, but is not limited to, 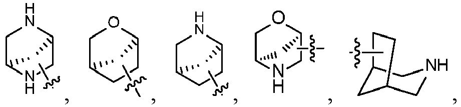 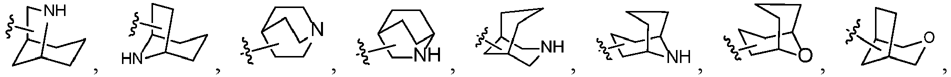 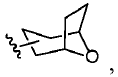 etc. The monocyclic heterocycloalkyl and bicyclic heterocycloalkyl can be attached to the parent molecule via any ring atom on the ring. The ring atom specifically means the carbon atom and/or nitrogen atom constituting the ring skeleton.

The term "cycloalkylalkyl" means the cycloalkyl is attached to the parent structure via an alkyl group. Thus, "cycloalkylalkyl" includes the above definitions of alkyl and cycloalkyl.

The term "heterocycloalkylalkyl" means the heterocycloalkyl is attached to the parent structure via an alkyl group. Thus, "heterocycloalkylalkyl" includes the above definitions of alkyl and heterocycloalkyl.

The term "alkenyl" means a linear, branched, or cyclic non-aromatic hydrocarbon group containing at least one carbon-carbon double bond. There can be 1 to 3 carbon-carbon double bonds, preferably one carbon-carbon double bond. The term "C₂₋₄ alkenyl" means an alkenyl group with 2 to 4 carbon atoms, and the term "C₂₋₆ alkenyl" means an alkenyl group with 2 to 6 carbon atoms, including vinyl, propenyl, butenyl, 2-methylbutenyl, and cyclohexenyl. The alkenyl can be substituted.

The term "alkenylene" means a linear or branched unbridged divalent alkenyl group containing 2 to 6 carbon atoms, preferably 2 to 4 carbon atoms, such as vinylene (-CH=CH-) and propenylene (-CH=CHCH₂-).

The term "alkynyl" means a linear, branched, or cyclic hydrocarbon group containing at least one carbon-carbon triple bond. There can be 1 to 3 carbon-carbon triple bonds, preferably one carbon-carbon triple bond. The term "C₂₋₆ alkynyl" means an alkynyl group with 2 to 6 carbon atoms, including ethynyl, propynyl, butynyl, and 3-methylbutynyl.

The term "alkoxy" means a cyclic or non-cyclic alkyl group having the stated number of the carbon atoms attached via an oxygen bridge, including alkyloxy, cycloalkyloxy, and heterocycloalkyloxy. Thus, "alkoxy" includes the above definitions of alkyl, heterocycloalkyl, and cycloalkyl.

The term "aryl" means any stable 6- to 14-membered all-carbon monocyclic or fused bicyclic aromatic group with a conjugated π-electron system. At least one ring of the fused bicyclic aromatic group is an aromatic ring, and the aryl is preferably a 6- to 10-membered aryl group, such as phenyl, naphthyl, tetrahydronaphthyl, 2,3-dihydroindenyl, or biphenyl.

The term "heteroaromatic ring" or "heteroaryl" means an aromatic ring group formed by replacing at least one carbon atom on the ring by a heteroatom selected from nitrogen, oxygen, or sulfur. The heteroaryl group may be a 5- to 6-membered monocyclic structure or a 7- to 12-membered bicyclic structure, preferably a 5- to 10-membered heteroaryl group; the heteroaryl group may also be an 11- to 13-membered tricyclic structure, preferably an 11- to 12-membered tricyclic structure. In the present disclosure, the number of heteroatoms is preferably 1, 2, or 3. The heteroaryl group includes, but is not limited to, pyridyl, pyrimidinyl, pyrazinyl, pyridazinyl, pyridazin-3(2*H*)-one, furyl, thienyl, thiazolyl, isothiazolyl, pyrrolyl, imidazolyl, pyrazolyl, oxazolyl, isoxazolyl, 1,2,5-oxadiazolyl, 1,2,4-oxadiazolyl, 1,2,4-triazolyl, 1,2,3-triazolyl, tetrazolyl, 1*H*-benzo[*d*]imidazol-2(3*H*)-one 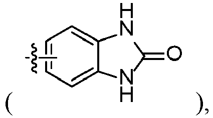 benzo[*d*]thiazol-2(3*H*)-one 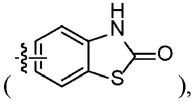 1*H*-indazolyl 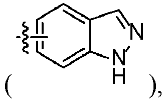 isoindazolyl, 1*H-*indolyl 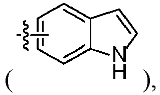 isoindolyl, benzofuranyl 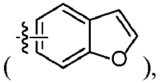 benzothienyl 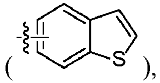 benzothiazolyl 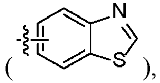 benzoisothiazolyl 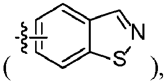 benzoxazolyl 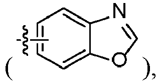 quinolyl 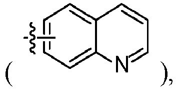 isoquinolinyl 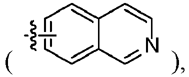 quinazolinyl 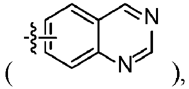 pyrazolo[1,5-*a*]pyridyl 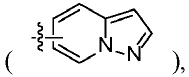 imidazo[1,2-*a*]pyridyl 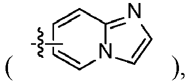 *1H-*benzo[4,5]thieno[2,3-*c*]pyrazolyl 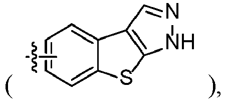 1*H*-benzo[4,5]thieno[2,3-*b*]pyrrolyl 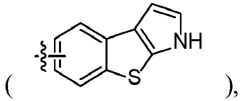 3H-benzo[4,5]thieno[2,3-d][1,2,3]triazolyl, benzo[4,5]thieno[2,3-*b*]pyridyl, benzo[4,5]thieno[2,3-*d*]pyrimidinyl, 1*H*-imidazo[1,2-*a*]indolyl 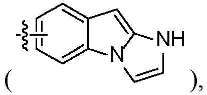 naphtho[2,3-*b*]furyl, 1*H*-benzo[*f*]indolyl, 1*H*-benzo[*f*]indazolyl, naphtho[2,1-*b*]furyl, etc.

The term "arylalkyl" means the aryl group is attached to the parent structure via an alkyl group. Thus, "arylalkyl" includes the above definitions of alkyl and aryl.

The term "heteroarylalkyl" means the heterocycloalkyl group is attached to the parent structure via an alkyl group. Thus, "heteroarylalkyl" includes the above definitions of alkyl and heteroaryl.

The term "halogen" means fluorine, chlorine, bromine, or iodine.

The term "haloalkyl" means an alkyl group selectively substituted by halogen. Thus, "haloalkyl" includes the above definitions of halogen and alkyl.

The term "haloalkoxy" means an alkoxy group selectively substituted by halogen. Thus, "haloalkoxy" includes the above definitions of halogen and alkoxy.

The term "amino" means -NH₂. The term "alkylamino" means an amino group in which at least one hydrogen atom is substituted by an alkyl group, including "monoalkylamino" and "dialkylamino". Examples of alkylamino include, but are not limited to, -NHCH₃, - N(CH₃)₂, -NHCH₂CH₃, -N(CH₂CH₃)₂, -N(CH₂CH₂CH₃)₂, -N(CH₃)(CH₂CH₃), and - N(CH₃)(CH₂CH₂CH₃). The term "aminoalkyl" means an alkyl group in which any hydrogen atom is substituted by an amino group. Examples of aminoalkyl include, but are not limited to, -CH₂NH₂ and -CH₂CH₂NH₂. Thus, "aminoalkyl" and "alkylamino" include the above definitions of alkyl and amino.

The term "alkylaminoalkyl" means an alkyl group in which any hydrogen atom is substituted by an alkylamino group. Examples of alkylaminoalkyl include, but are not limited to, -CH₂N(CH₃)₂, -CH₂CH₂N(CH₃)₂, -CH₂CH₂CH₂N(CH₃)₂, and -CH₂NHCH₃. Thus, "alkylaminoalkyl" includes the above definitions of alkylamino and alkyl.

The term "hydroxyl" means -OH. The term "hydroxyalkyl" means an alkyl group in which any hydrogen atom is substituted by a hydroxyl group. Examples of hydroxyalkyl include, but are not limited to, -CH₂OH, -CH₂CH₂OH, -CH₂CH₂C(CH₃)₂OH, and - CH(CH₃)₂OH. Thus, "hydroxyalkyl" includes the above definitions of hydroxyl and alkyl.

The term "alkoxyalkyl" means an alkyl group in which any hydrogen atom is substituted by an alkoxy group. Examples of alkoxyalkyl include, but are not limited to, - CH₂OCH₃ and -CH₂CH₂OCH₃. Thus, "alkoxyalkyl" includes the above definitions of alkoxy and alkyl.

The term "oxo" means =O, such as the =O portion of carbonyl (-CO-), nitroso (-N=O), sulfinyl (-SO-), or sulfonyl (-SO₂-).

The term "cyano" means -CN.

The term "carboxyl" means -C(O)OH.

The "room temperature" in the present disclosure means 15 to 30°C.

In the present disclosure, unless otherwise specified, the term "selectively substituted at any position by one or more than one substituent" means that any 1, 2, 3, or more hydrogen atoms of 1, 2, 3, or more atoms designated on the group are substituted with the designated substituent provided that the normal valence of the designated atom is not exceeded, and that the substitution is a common and reasonable substitution in the art, for example, =O cannot be substituted on a tertiary carbon. In the present disclosure, "selectively substituted at any position by one or more than one substituent" is preferably "selectively substituted at any position by 1 to 3 substituents".

In the present disclosure, when the bonding to a substituent is shown to intersect the bonding of two atoms in a linking ring, then such a substituent can be bonded to any bondable ring atom on the ring.

In the present disclosure, any combination of variables is allowed only if such combination results in a stable compound. For example, various combinations of X, X₁, and X₃ or various combinations of Z, Z₁, and Z₂ constituting a ring are allowed only if they result in a stable ring.

In the present disclosure, 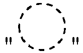 in the ring means that the bond forming the ring may be a single bond or a double bond. The formed ring may be an aromatic or non-aromatic ring, and the non-aromatic ring is a saturated or partially unsaturated non-aromatic ring. In the present disclosure, when any variable occurs in the constitution or structure of the compound more than once, the definition of the variable at each occurrence is independent. For example, R₄ and R₆ are attached to each other to form a C₃₋₈ cycloalkyl or 3- to 8-membered heterocycloalkyl group; when the C₃₋₈ cycloalkyl or 3- to 8-membered heterocycloalkyl is substituted by one or more than one R₁₀, each R₁₀ substituent is an independent substituent and may be the same or different.

In the present disclosure, " 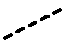 " in ring A and ring B means the site where ring A and ring B are attached.

The "pharmaceutically acceptable salt" of the present disclosure is discussed in Berge, et al., "Pharmaceutically acceptable salts", J. Pharm. Sci., 66, 1-19 (1977), and it is obvious to the medicinal chemist that the salts are essentially non-toxic and can provide the desired pharmacokinetic properties, palatability, absorption, distribution, metabolism, excretion, etc. The compound of the present disclosure can have an acidic group, a basic group, or an amphoteric group. Typical pharmaceutically acceptable salts include salts prepared by reacting the compound of the present disclosure with an acid, such as hydrochloride, hydrobromide, sulfate, pyrosulfate, bisulfate, sulfite, bisulfite, phosphate, monohydrogen phosphate, dihydrogen phosphate, metaphosphate, pyrophosphate, nitrate, acetate, propionate, caprate, caprylate, formate, acrylate, isobutyrate, caproate, enanthate, oxalate, malonate, succinate, suberate, benzoate, methylbenzoate, phthalate, maleate, mesylate, *p-*toluenesulfonate, (*D*, *L*)-tartaric acid, citric acid, maleic acid, (*D*, *Z*)-malic acid, fumaric acid, succinic acid, succinate, lactate, triflate, naphthalene-1-sulfonate, mandelate, pyruvate, stearate, ascorbate, and salicylate. When the compound of the present disclosure contains an acidic group, its pharmaceutically acceptable salts may also include: alkali metal salts, such as sodium or potassium salts; alkaline earth metal salts, such as calcium or magnesium salts; organic base salts, such as salts formed with ammonia, alkylammonia, hydroxyalkylammonia, amino acids (lysine, arginine), *N*-methylglucamine, etc.

On the basis of not violating common knowledge in the art, the above-mentioned preferred conditions can be combined arbitrarily to obtain preferred examples of the present disclosure.

The reagents and raw materials used in the present disclosure are all commercially available.

### DETAILED DESCRIPTION OF THE PREFERRED EMBODIMENT

The structures of all compounds of the present disclosure can be identified by nuclear magnetic resonance (¹H NMR) and/or mass spectrometry (MS).

¹H NMR chemical shifts (δ) were recorded in ppm (10⁻⁶). NMR was performed with a Bruker AVANCE-400 spectrometer. Suitable solvents were deuterated chloroform (CDCl₃), deuterated methanol (CD₃OD), deuterated dimethyl sulfoxide (DMSO-*d*₆), with tetramethylsilane (TMS) as an internal standard.

Liquid chromatography-mass spectrometry (LCMS) was measured by an Agilent 1200HPLC/6120 mass spectrometer, using a chromatographic column: Xtimate C18, 3.0 × 50 mm, 3 µm, with a column temperature of 40°C; or by a Thermo UltiMate 3000HPLC/MSQ PLUS mass spectrometer using a chromatographic column of XBridge C18, 3.0 × 50 mm, 3.5 µm, with a column temperature of 30°C. Agilent gradient elution condition I: 95% to 5% solvent A₁ and 5% to 95% solvent B₁ (0 to 2.0 minutes), then 95% solvent B₁ and 5% solvent A₁ (held for 1.1 minutes), the percentage was the volume percentage of a specific solvent in the total solvent volume. Solvent A₁: 0.01% trifluoroacetic acid (TFA) aqueous solution; solvent B₁: 0.01% acetonitrile solution of trifluoroacetic acid; the percentage was the volume percentage of the solute in the solution. Thermo gradient elution condition II: 95% to 5% solvent A₂ and 5% to 95% solvent B₂ (0 to 2 minutes), then 95% solvent B₂ and 5% solvent A₂ (held for 1.8 minutes), the percentage was the volume percentage of a specific solvent in the total solvent volume. Solvent A₂: 10 mM ammonium bicarbonate aqueous solution; solvent B₂: acetonitrile.

All compounds of the present disclosure can be separated by preparative high-performance liquid chromatography or flash column chromatography.

Preparative high-performance liquid chromatography (*prep-HPLC*) was performed on Shimadzu LC-20 preparative liquid chromatography. 1) Chromatographic column: Welch Xtimate C18, 21.2 × 250 mm, 10 µm. Alkaline conditions: mobile phase A: 10 mmol/L ammonium bicarbonate aqueous solution, mobile phase B: acetonitrile, gradient elution condition 1: mobile phase B from 15% to 30%, elution time: 5 minutes, mobile phase B from 30% to 80%, elution time: 15 minutes. Gradient elution condition 3: mobile phase B from 25% to 90%, elution time: 70 minutes. Gradient elution condition 6: mobile phase B from 15% to 65%, elution time: 20 minutes, mobile phase B from 65% to 90%, elution time: 5 minutes. Gradient elution condition 7: mobile phase B from 5% to 15%, elution time: 5 minutes, mobile phase B from 15% to 40%, elution time: 15 minutes. Acidic conditions: mobile phase A: 0.1% trifluoroacetic acid aqueous solution, mobile phase B: acetonitrile, gradient elution condition 2: mobile phase B from 15% to 30%, elution time: 5 minutes, from 30% to 65%, elution time: 15 minutes. Gradient elution condition 4: mobile phase B from 10% to 20%, elution time: 5 minutes, from 20% to 55%, elution time: 15 minutes. Gradient elution condition 8: mobile phase B from 10% to 20%, elution time: 5 minutes, from 25% to 45%, elution time: 25 minutes. Detection wavelength: 214 nm & 254 nm; flow rate: 15.0 mL/min. 2) Chromatographic column: Welch Xtimate XB-C18, 30 × 250 mm, 10 µm. Alkaline conditions: mobile phase A: 0.1% ammonia aqueous solution, mobile phase B: acetonitrile, gradient elution condition 5: mobile phase B from 15% to 35%, elution time: 5 minutes, mobile phase B from 35% to 65%, elution time: 15 minutes, detection wavelength: 214 nm & 254 nm; flow rate: 40 mL/min.

Flash column chromatography (flash system/CheetahTM) was performed on Agela Technologies MP200. The accompanying normal-phase separation column was Flash column Silica-CS (25 g, 40 g, 80 g, 120 g, or 330 g) from Tianjin Bonna-Agela, and the elution system was ethyl acetate/petroleum ether or dichloromethane/methanol; the reverse-phase separation column was C18 reverse-phase column (12 g, 20 g, or 40 g) from Changzhou Santai Technologies, and the elution system was acetonitrile/0.1% trifluoroacetic acid aqueous solution or acetonitrile/10 mmol/L ammonium bicarbonate aqueous solution.

The preparative thin-layer chromatography silica gel plate (*prep*-TLC) was Yantai Huanghai HSGF254 or Qingdao GF254 silica gel plate.

All compounds of the present disclosure can be analyzed by ultra-performance liquid chromatography or high-performance liquid chromatography.

Ultra-performance liquid chromatography (UPLC) was performed on Waters ACQUITY Hclass; chromatographic column: Waters ACQUITY UPLC BEH Shield RP18 2.1 mm * 100 mm, 1.7 µm, 5 µm, mobile phase A: acetonitrile, mobile phase B: 5 mM potassium dihydrogen phosphate buffer, with the pH adjusted to 2.5 with phosphoric acid. Gradient elution: mobile phase B from 10% to 90%, elution time: 7 minutes, B held at 90% for 6 minutes, B from 90% to 10%, elution time: 2 minutes. Detection wavelength: 214 nm; column temperature: 40°C. Flow rate: 0.4 mL/min.

High-performance liquid chromatography (HPLC) was performed on Waters e2695, 2498 UV/VIS Detector; chromatographic column: Welch Xtimate C18 4.6 mm * 150 mm, 5 µm, mobile phase A: acetonitrile, mobile phase B: 10 mM potassium dihydrogen phosphate buffer, with the pH adjusted to 8.0 with ammonia water. Gradient elution: mobile phase B held at 90% for 5 minutes, B from 90% to 70%, elution time: 5 minutes, B from 70% to 40%, elution time: 4 minutes, B from 40% to 15%, elution time: 12 minutes, B from 15% to 90%, elution time: 2 minutes, B held at 90% for 2 minutes. Detection wavelength: 214 & 262 nm; column temperature: 35°C. Flow rate: 1 mL/min.

The chiral compounds or intermediates covered by the present disclosure can be separated and analyzed by chiral high-performance liquid chromatography (Chiral HPLC).

The chiral analysis of the compounds of the present disclosure was performed on high-performance liquid chromatography Agilent 1200 HPLC with a chiral analysis column: R,R-WHELK-O1 (4.6 * 250 mm, 5 µm), flow rate: 1.0 mL/min; column temperature: 40°C, injection volume: 5 µL; detection wavelength: 214 and/or 254 nm; mobile phase: n-hexane (0.1% diethylamine)/ethanol (0.1% diethylamine) = 50:50.

The microwave reaction in the examples of the present disclosure was carried out using a Biotage^{®} Initiator + Microwave System EU (356006) microwave reactor.

Unless otherwise specified in the present disclosure, the reactions in all examples were carried out under nitrogen atmosphere or argon atmosphere.

The hydrogen atmosphere can be achieved by 1) connecting a hydrogen balloon with a volume of about 1 L to the reaction system; 2) directly and continuously introducing hydrogen into the reaction system under normal pressure; and 3) sealing after hydrogen replacement in a sealed tube.

### Synthesis of intermediates:

### Intermediate 1: Synthesis of tert-butyl 2-(hydroxymethyl)-3,8-diazabicyclo[3.2.1]octane-8-carboxylate

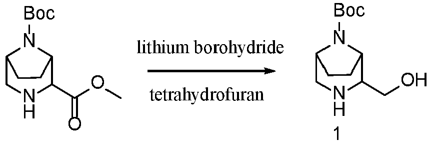

To a solution of 8-(*tert*-butyl) 2-methyl 3,8-diazabicyclo[3.2.1]octane-2,8-dicarboxylate (3.50 g, 12.3 mmol) in anhydrous tetrahydrofuran (20 mL) was added dropwise a solution of lithium borohydride (407 mg, 18.5 mmol) in tetrahydrofuran (40 mL) in an ice bath. After the addition was completed, the reaction system was stirred at 80°C for 3 hours. The reaction mixture was cooled to room temperature, then diluted with tetrahydrofuran (170 mL), slowly added with saturated ammonium chloride aqueous solution (10 mL) to quench, concentrated under reduced pressure, and the residue was purified by flash column chromatography (methanol/dichloromethane = 1/10) to obtain intermediate 1 (0.8 g) as a white solid. m/z: [M+H]⁺ 243.2.

### Intermediate 1 (trans): Synthesis of tert-butyl 2-(hydroxymethyl)-3,8-diazabicyclo[3.2.1]octane-8-carboxylate (trans)

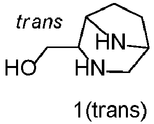

Intermediate 1 (*trans*) was obtained by using 8-(*tert*-butyl) 2-methyl 3,8-diazabicyclo[3.2.1]octane-2,8-dicarboxylate (*trans*) as the starting material using the synthesis method of intermediate 1.

### Intermediate 2: Synthesis of tert-butyl (1S,2S,5S)-2-(hydroxymethyl)-3,8-diazabicyclo[3.2.1]octane-8-carboxylate

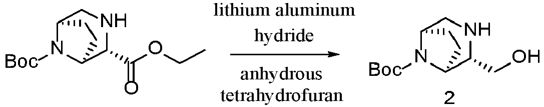

To a solution of 8-(*tert*-butyl) 2-ethyl (1*S*,2*S*,5*R*)-3,8-diazabicyclo[3.2.1]octane-2,8-dicarboxylate (with the synthesis method referring to that of compound 6# in WO2019/179515A1) (4.50 g, 15.8 mmol) in anhydrous tetrahydrofuran (80 mL) was added in batches a solution of lithium aluminum hydride in tetrahydrofuran (2.5 M, 8.3 mL, 20.6 mmol) in an ice bath, and the reaction system was stirred at 0°C for 3 hours. The reaction system was then sequentially added with water (1 mL) and sodium hydroxide aqueous solution (15%, 2 mL). The resulting mixture was concentrated under reduced pressure, and the residue was directly purified by flash column chromatography (methanol/dichloromethane = 1/10) to obtain intermediate 2 (2.5 g, yield: 65%) as a white solid. m/z: [M+H]⁺ 243.2.

### Intermediate 3: Synthesis of tert-butyl (1R,2R,5S)-2-(hydroxymethyl)-3,8-diazabicyclo[3.2.1]octane-8-carboxylate

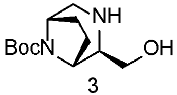

Intermediate 3 was synthesized using the synthesis method of intermediate 2. m/z: [M+H]⁺ 243.2.

### Intermediate 6: Synthesis of 2,5,7-trichloropyrido[2,3-d]pyrimidin-4-ol

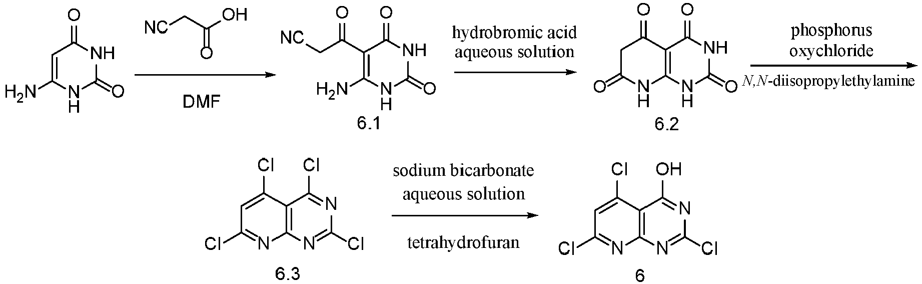

Step 1: 4-Aminouracil (15.3 g, 0.12 mol) and cyanoacetic acid (12.8 g, 0.15 mol) were suspended in anhydrous *N*,*N-*dimethylformamide (80 mL), and phosphorus oxychloride (6.4 g, 42 mmol) was slowly added thereto. The reaction mixture was stirred at 60°C for 2 hours, cooled to room temperature, slowly added with ice water to quench, and filtered. The filter cake was washed with ice water and dried under vacuum to obtain intermediate 6.1 (15 g) as a light yellow solid. m/z: [M+H]⁺ 195.1.

Step 2: Intermediate 6.1 (15 g, 77 mmol) was suspended in hydrobromic acid aqueous solution (40%, 100 mL). The reaction mixture was stirred at 90°C for 2 hours, cooled to room temperature, slowly diluted with ice water, and filtered. The filter cake was washed with ice water and dried under vacuum to obtain intermediate 6.2 (10.3 g) as a yellow solid. m/z: [M+H]⁺ 196.2.

Step 3: Intermediate 6.2 (6.6 g, 33 mmol) was suspended in phosphorus oxychloride (50 mL), and *N*,*N-*diisopropylethylamine (8.6 g, 66 mmol) was slowly added dropwise thereto. The reaction mixture was stirred at 110°C for overnight, concentrated under reduced pressure, diluted with acetonitrile (20 mL), then slowly poured into ice water, and the resulting solid was filtered. The filter cake was washed with ice water and dried under vacuum to obtain intermediate 6.3 (6.14 g) as a brown solid.

Step 4: To a suspension of intermediate 6.3 (6.14 g, 23 mmol) in tetrahydrofuran (120 mL) was slowly added dropwise sodium bicarbonate aqueous solution (1.0 M, 30 mL) in an ice bath. The reaction mixture was stirred at room temperature for overnight and then diluted with ethyl acetate (150 mL). The organic phase was separated, washed with saturated brine, dried over anhydrous sodium sulfate, and filtered. The filtrate was concentrated under reduced pressure, then the residue was triturated with a mixture of petroleum ether/ethyl acetate and filtered, and the filter cake was dried under vacuum to obtain intermediate 6 (4.94 g) as a brown solid. m/z: [M+H]⁺ 250.0.

### Intermediate 7: Synthesis of 2,5,7-trichloro-8-fluoropyrido[4,3-d]pyrimidin-4-ol

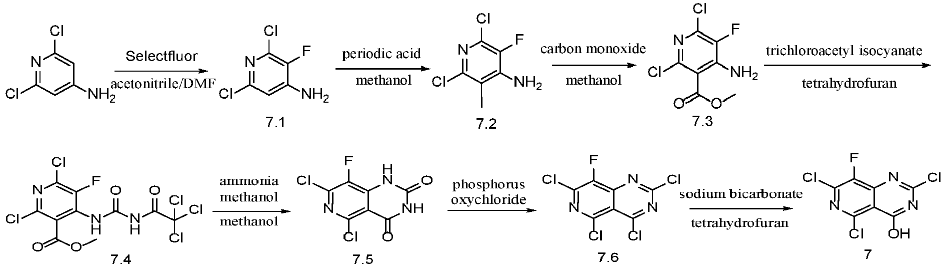

Step 1: A mixed solution of 2,6-dichloropyridin-4-amine (50 g, 0.31 mol) and 1-fluoro-4-methyl-1,4-diazabicyclo[2.2.2]octane tetrafluoroborate (131 g, 0.37 mol) in anhydrous acetonitrile (500 mL) and *N*,*N*-dimethylformamide (500 mL) was stirred at 80°C for 1 hour. The reaction system was cooled to room temperature and then diluted with ethyl acetate (1700 mL). The organic phase was washed with saturated brine, dried over anhydrous sodium sulfate, filtered, and the filtrate was concentrated under reduced pressure to obtain intermediate 7.1 (52 g) as a yellow solid. m/z: [M+H]⁺ 181.0.

Step 2: A solution of intermediate 7.1 (52 g, 0.29 mol), periodic acid (26.2 g, 0.11 mol), and iodine (73 g, 0.29 mol) in anhydrous methanol (500 mL) was stirred at 60°C for 5 hours, and then directly concentrated under reduced pressure. The residue was added with ethyl acetate (1000 mL), filtered, and the filtrate was sequentially washed with saturated sodium bicarbonate aqueous solution (4 L), saturated sodium bisulfite aqueous solution (4 L), and saturated brine (0.4 L). The organic phase was separated, dried over anhydrous sodium sulfate, filtered, and the filtrate was concentrated under reduced pressure to obtain intermediate 7.2 (45 g) as a yellow solid. m/z: [M+H]⁺ 306.8.

Step 3: A solution of intermediate 7.2 (10 g, 32.6 mmol), [1,1'-bis(diphenylphosphino)ferrocene]dichloropalladium(II) dichloromethane complex (2.7 g, 3.26 mmol), and triethylamine (23 mL, 163 mmol) in methanol (400 mL) was replaced with carbon monoxide, and then stirred at 50°C for 20 hours under carbon monoxide atmosphere (30 psi). The reaction mixture was then directly concentrated under reduced pressure to obtain intermediate 7.3 (4 g) as a yellow solid. m/z: [M+H]⁺ 239.0.

Step 4: To a solution of intermediate 7.3 (3.7 g, 15.5 mmol) in tetrahydrofuran (40 mL) was added a solution of trichloroacetyl isocyanate (3.78 g, 46.4 mmol) in tetrahydrofuran (5 mL) in an ice bath. The reaction system was stirred at room temperature for 2 hours and then directly concentrated under reduced pressure. The residue was triturated with methyl *tert-*butyl ether (100 mL), filtered, and the filter cake was dried under vacuum to obtain intermediate 7.4 (5 g) as a yellow solid.

Step 5: To a solution of intermediate 7.4 (5 g, 11.7 mmol) in methanol (150 mL) was added a solution of ammonia in methanol (20 mL, 7 M, 140 mmol). The reaction mixture was stirred at room temperature for 2 hours, filtered, and the filter cake was dried under vacuum to obtain intermediate 7.5 (2.9 g) as a light yellow solid.

Step 6: To a solution of intermediate 7.5 (2 g, 8.0 mmol) in phosphorus oxychloride (25 mL) was added *N*,*N-*diisopropylethylamine (5.7 mL, 32 mmol). The reaction system was stirred at 80°C for 1 hour and then directly concentrated under reduced pressure. The residue was poured into ice water (50 mL), and the aqueous phase was extracted with ethyl acetate. The organic phases were combined, washed with saturated brine, separated, dried over anhydrous sodium sulfate, and filtered. The filtrate was concentrated under reduced pressure to obtain intermediate 7.6 (2.3 g) as a brown solid.

Step 7: To a solution of intermediate 7.6 (2.3 g, 8.0 mmol) in tetrahydrofuran (50 mL) was added sodium bicarbonate aqueous solution (24 mL, 1 M) in an ice bath. The reaction system was stirred at 0°C for 1 hour, then added with dilute hydrochloric acid (2 M) to adjust the pH to 5, and the aqueous phase was extracted with ethyl acetate. The organic phases were combined, washed with saturated brine, dried over anhydrous sodium sulfate, and filtered. The filtrate was concentrated under reduced pressure, and the residue was purified by flash column chromatography (ethyl acetate/petroleum ether = 1/1) to obtain intermediate 7 (1.5 g) as a white solid. m/z: [M+H]⁺ 267.8.

### Intermediate 8: Synthesis of 7-bromo-2,6-dichloro-5,8-difluoroquinazolin-4-ol

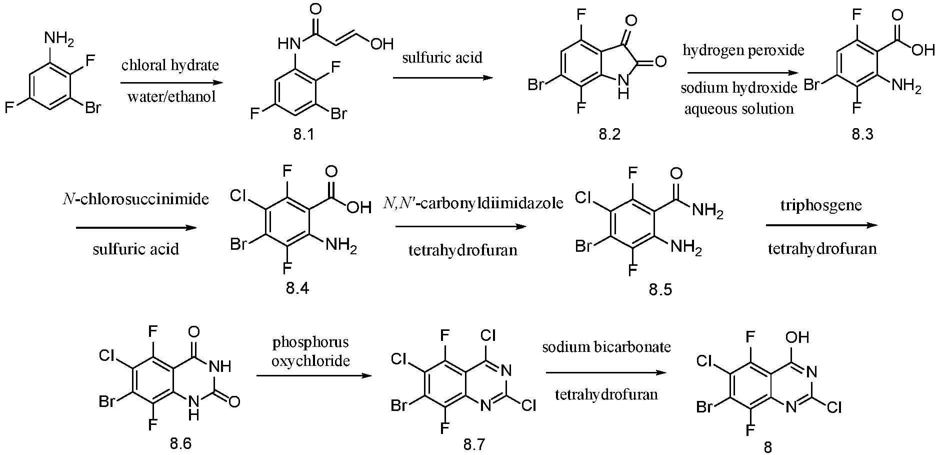

Step 1: To a solution of chloral hydrate (15 g, 90.7 mmol) in water (300 mL) were added anhydrous sodium sulfate (69 g, 486 mmol) and hydroxylamine hydrochloride (15 g, 215 mmol), respectively. 3-Bromo-2,5-difluoroaniline (15 g, 72.1 mmol) was dissolved in a mixed solution of water (30 mL), ethanol (45 mL), and hydrochloric acid (10.5 mL) and added to the above reaction system. The resulting mixture was stirred at 60°C for 18 hours, then cooled to room temperature, filtered, and the filter cake was dried under vacuum to obtain intermediate 8.1 (12.5 g) as a gray solid.

Step 2: To concentrated sulfuric acid (100 mL) was added intermediate 8.1 (12.5 g, 44.8 mmol) at 60°C, and the reaction mixture was stirred at 90°C for 1 hour. The reaction mixture was cooled to room temperature, slowly added to ice water, filtered, and the filter cake was dried under vacuum to obtain intermediate 8.2 (11.1 g) as a reddish black solid. m/z: [M+H]⁺ 262.0.

Step 3: To a sodium hydroxide aqueous solution (190 mL, 380 mmol) of intermediate 8.2 (11.1 g, 42.4 mmol) was added hydrogen peroxide (21 mL, 191 mmol) in an ice bath, and the reaction system was stirred at 0°C for 2 hours. The reaction mixture was slowly added with solid sodium sulfite to quench, then added with concentrated hydrochloric acid to adjust the pH to 2 to 3, filtered, and the filter cake was dried under vacuum to obtain intermediate 8.3 (7.6 g) as a gray solid. m/z: [M+H]⁺ 252.0.

Step 4: To a solution of intermediate 8.3 (8.7 g, 34.5 mmol) in concentrated sulfuric acid (85 mL) was added N-chlorosuccinimide (9.2 g, 69.0 mmol). The reaction mixture was stirred at 80°C for 16 hours, cooled to room temperature, slowly added to ice water, filtered, and the filter cake was dried under vacuum to obtain intermediate 8.4 (9 g) as a gray solid. m/z: [M+H]⁺ 285.9.

Step 5: To a solution of intermediate 8.4 (9 g, 31.5 mmol) in tetrahydrofuran (100 mL) was added *N*,*N'*-carbonyldiimidazole (10 g, 63.1 mmol). The reaction mixture was stirred at 50°C for 2 hours, cooled to room temperature, and slowly added with concentrated ammonia water (200 mL). The resulting mixture was stirred at room temperature for another 16 hours, added with water to quench, and the aqueous phase was extracted with ethyl acetate (450 mL). The organic phase was separated and concentrated under reduced pressure to obtain intermediate 8.5 (10 g) as a gray solid. m/z: [M+H]⁺ 285.0.

Step 6: To a solution of intermediate 8.5 (4.2 g, 14.7 mmol) in tetrahydrofuran (100 mL) was added triphosgene (3.5 g, 11.8 mmol). The reaction mixture was stirred at 70°C for 2 hours, cooled to room temperature, added with water to quench, and the aqueous phase was extracted with ethyl acetate. The organic phase was separated and concentrated under reduced pressure to obtain intermediate 8.6 (3.3 g) as a gray solid. m/z: [M+H]⁺ 310.8.

Step 7: To a solution of intermediate 8.6 (1.7 g, 5.45 mmol) in phosphorus oxychloride (17 mL) was added *N*,*N-*diisopropylethylamine (4 mL). The reaction mixture was stirred at 110°C for 2 hours and concentrated under reduced pressure. The residue was diluted with dichloromethane, and then washed with saturated sodium bicarbonate aqueous solution and saturated brine, respectively. The organic phase was separated and concentrated under reduced pressure to obtain intermediate 8.7 (1.9 g) as a black solid. m/z: [M+H]⁺ 346.8.

Step 8: To a solution of intermediate 8.7 (1.9 g, 5.45 mmol) in tetrahydrofuran (40 mL) was added sodium bicarbonate aqueous solution (11 mL, 10.91 mmol) in an ice bath, and the reaction mixture was stirred at room temperature for 3 hours. The reaction mixture was added with hydrochloric acid (1.0 M) to adjust the pH to 6 to 7, and the aqueous phase was extracted with ethyl acetate (300 mL). The organic phase was separated and concentrated under reduced pressure, and the residue was purified by flash column chromatography (dichloromethane/methanol = 10/1) to obtain intermediate 8 (1.33 g) as a gray solid. m/z: [M+H]⁺ 330.8.

### Intermediate 9: Synthesis of 7-bromo-6-chloro-5,8-difluoroquinazolin-4-ol

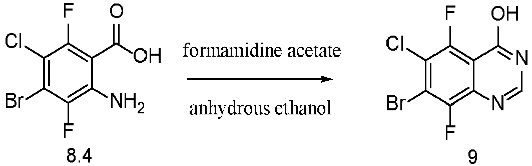

A solution of intermediate 8.4 (10.0 g, 34.9 mmol) and formamidine acetate (36.3 g, 349 mmol) in anhydrous ethanol (150 mL) was stirred at 80°C for overnight, cooled to room temperature, then poured into water, filtered, and the filter cake was dried under vacuum to obtain intermediate 9 (9.0 g) as a gray solid. m/z: [M+H]⁺ 295.0.

### Intermediate 10: Synthesis of 2-bis(tert-butoxycarbonyl)amino-4-(4,4,5,5-tetramethyl-1,3,2-dioxaborolan-2-yl)pyrazolo[1,5-a]pyridine-3-carbonitrile

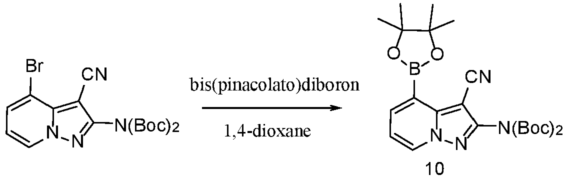

A solution of 2-bis(*tert*-butoxycarbonyl)amino-4-bromopyrazolo[1,5-*a*]pyridine-3-carbonitrile (Cat. No.: PCS0834, Nanjing PharmaBlock Sciences) (300 mg, 0.69 mmol), [1,1'-bis(diphenylphosphino)ferrocene]dichloropalladium(II) dichloromethane complex (56.0 mg, 0.07 mmol), potassium acetate (203 mg, 2.07 mmol), and bis(pinacolato)diboron (350 mg, 1.38 mmol) in anhydrous 1,4-dioxane (10 mL) was replaced with nitrogen and stirred at 100°C for 5 hours, cooled to room temperature and concentrated under reduced pressure, and the residue was purified by flash column chromatography (ethyl acetate/petroleum ether = 10/1) to obtain intermediate 10 (0.15 g) as a yellow solid.

### Intermediate 11: Synthesis of tert-butyl (3-cyano-4-(4,4,5,5-tetramethyl-1,3,2-dioxaborolan-2-yl)benzo[b]thiophen-2-yl)carbamate

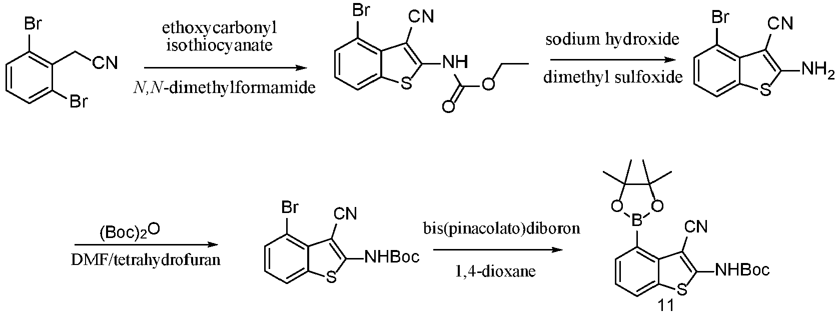

Step 1: To a solution of 2-(2,6-dibromophenyl)acetonitrile (3.8 g, 13.8 mmol) in *N,N-*dimethylformamide (10 mL) was added sodium hydride (0.55 g, 60%) in an ice bath under nitrogen atmosphere. The reaction system was stirred at 0°C for 10 minutes, and then ethoxycarbonyl isothiocyanate (2.1 g, 15.2 mmol) was added thereto. The reaction system was stirred at 95°C for 4 hours, concentrated under reduced pressure, and then triturated with water and ethyl acetate. The mixture was filtered, and the filter cake was dried under vacuum to obtain ethyl (4-bromo-3-cyanobenzo[b]thiophen-2-yl)carbamate (1.7 g) as an orange solid.

Step 2: To a solution of ethyl (4-bromo-3-cyanobenzo[b]thiophen-2-yl)carbamate (1.7 g, 5.2 mmol) in dimethyl sulfoxide (170 mL) was added sodium hydroxide (1.66 g, 41.6 mmol), and the reaction system was stirred at 120°C for 16 hours. The reaction mixture was cooled to room temperature, directly poured into ice water, filtered, and the filter cake was dried under vacuum to obtain 2-amino-4-bromobenzo[b]thiophene-3-carbonitrile (0.79 g) as a yellow solid. m/z: [M+H]⁺ 254.9.

Step 3: To a mixed solution of 2-amino-4-bromobenzo[b]thiophene-3-carbonitrile (0.77 g, 3.06 mmol) in *N*,*N*-dimethylformamide (10 mL) and tetrahydrofuran (2 mL) were sequentially added di-*tert*-butyl dicarbonate (1 g, 4.59 mmol), 4-dimethylaminopyridine (75 mg, 0.61 mmol), and diisopropylethylamine (0.79 g, 6.12 mmol), and the reaction system was stirred at room temperature for 8 hours under nitrogen atmosphere. The reaction mixture was diluted with ethyl acetate and washed with water, and the organic phase was separated and concentrated under reduced pressure to obtain *tert*-butyl (4-bromo-3-cyanobenzo[b]thiophen-2-yl)carbamate (700 mg) as a light yellow solid.

Step 4: A solution of *tert*-butyl (4-bromo-3-cyanobenzo[*b*]thiophen-2-yl)carbamate (700 mg, 1.97 mmol), [1,1'-bis(diphenylphosphino)ferrocene]dichloropalladium(II) dichloromethane complex (160 mg, 0.2 mmol), cesium carbonate (1.28 g, 3.94 mmol), and bis(pinacolato)diboron (750 mg, 2.96 mmol) in anhydrous 1,4-dioxane (15 mL) was replaced with nitrogen and stirred at 105°C for 6 hours, cooled to room temperature and concentrated under reduced pressure, and the residue was purified by silica gel column chromatography (dichloromethane/petroleum ether = 0 to 1/1) to obtain intermediate 11 (600 mg) as a light yellow solid. m/z: [M+Na]⁺423.0.

### Intermediate 12: Synthesis of tert-butyl (5-chloro-4-(4,4,5,5-tetramethyl-1,3,2-dioxaborolan-2-yl)benzo[b]thiophen-2-yl)carbamate

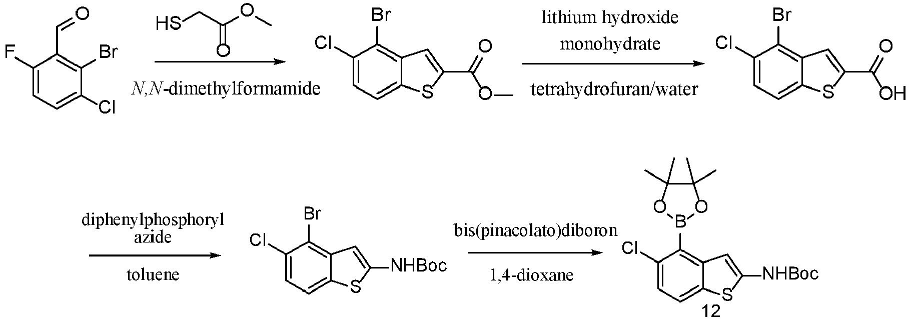

Step 1: To a solution of 2-bromo-3-chloro-6-fluorobenzaldehyde (1 g, 4.21 mmol) in *N*,*N*-dimethylformamide (120 mL) were sequentially added potassium carbonate (1.16 g, 8.42 mmol) and methyl 2-mercaptoacetate (460 mg, 4.34 mmol), and the reaction system was stirred at 60°C for overnight. The reaction system was diluted with ethyl acetate and washed with water, and the organic phase was separated and concentrated under reduced pressure. The residue was purified by flash column chromatography (ethyl acetate/petroleum ether = 0 to 30%) to obtain methyl 4-bromo-5-chlorobenzo[b]thiophene-2-carboxylate (760 mg) as a white solid. m/z: [M+H]⁺ 307.0.

Step 2: To a solution of methyl 4-bromo-5-chlorobenzo[b]thiophene-2-carboxylate (750 mg, 2.44 mmol) in tetrahydrofuran (10 mL) and water (2 mL) was added lithium hydroxide monohydrate (512 mg, 12.2 mmol), and the reaction system was stirred at room temperature for overnight. The reaction mixture was added with hydrochloric acid (1 M) to adjust the pH to 3, and the aqueous phase was extracted with ethyl acetate. The organic phases were combined and concentrated under reduced pressure to obtain 4-bromo-5-chlorobenzo[*b*]thiophene-2-carboxylic acid (675 mg) as a light yellow solid.

Step 3: To a solution of 4-bromo-5-chlorobenzo[*b*]thiophene-2-carboxylic acid (670 mg, 2.3 mmol), triethylamine (354 mg, 3.5 mmol), diphenylphosphoryl azide (943 mg, 3.4 mmol) in toluene (8 mL) was added *tert*-butanol (256 mg, 3.45 mmol). The reaction system was stirred at 100°C for 12 hours and directly concentrated under reduced pressure, and the residue was purified by flash column chromatography (ethyl acetate/petroleum ether = 0 to 40%) to obtain *tert*-butyl (4-bromo-5-chlorobenzo[*b*]thiophen-2-yl)carbamate (1 g) as a white solid.

Step 4: A solution of *tert*-butyl (4-bromo-5-chlorobenzo[*b*]thiophen-2-yl)carbamate (480 mg, 1.32 mmol), [1,1'-bis(diphenylphosphino)ferrocene]dichloropalladium(II) dichloromethane complex (108 mg, 0.13 mmol), potassium acetate (324 mg, 3.3 mmol), and bis(pinacolato)diboron (402 mg, 1.58 mmol) in anhydrous 1,4-dioxane (8 mL) was replaced with nitrogen and stirred at 100°C for 3 hours, cooled to room temperature and concentrated under reduced pressure, and the residue was purified by silica gel column chromatography (dichloromethane/petroleum ether = 0 to 4/1) to obtain intermediate 12 (223 mg) as a yellow solid. m/z: [M+Na]⁺ 432.2.

### Intermediate 13: Synthesis of tert-butyl (5-fluoro-4-(4,4,5,5-tetramethyl-1,3,2-dioxaborolan-2-yl)benzo[b]thiophen-2-yl)carbamate

Intermediate 13 was obtained by reacting 2-bromo-3,6-difluorobenzaldehyde using the synthesis method of intermediate 12. m/z: [M-56+H]⁺ 338.2.

### Intermediate 14: Synthesis of tert-butyl (5-methyl-4-(4,4,5,5-tetramethyl-1,3,2-dioxaborolan-2-yl)benzo[b]thiophen-2-yl)carbamate

Intermediate 14 was obtained by reacting 2-bromo-6-fluoro-3-methylbenzaldehyde using the synthesis method of intermediate 12. m/z: [M-56+H]⁺334.2.

### Intermediate 15: Synthesis of tert-butyl (3-cyano-7-fluoro-4-(4,4,5,5-tetramethyl-1,3,2-dioxaborolan-2-yl)benzo[b]thiophen-2-yl)carbamate

Intermediate 15 was obtained by reacting 2-(6-bromo-2,3-difluorophenyl)acetonitrile using the synthesis method of intermediate 11. m/z: [M+Na]⁺ 441.2.

### Intermediate 16: Synthesis of (2-((tert-butoxycarbonyl)amino)benzo[d]thiazol-4-yl)boronic acid

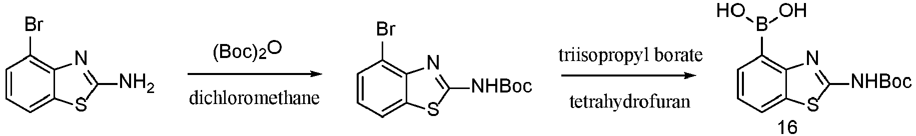

Step 1: To a solution of 4-bromobenzo[*d*]thiazol-2-amine (5 g, 21.8 mmol) in dichloromethane (50 mL) were sequentially added di-*tert*-butyl dicarbonate (7.14 g, 32.7 mmol), 4-dimethylaminopyridine (0.53 g, 4.36 mmol), and diisopropylethylamine (5.64 g, 43.6 mmol). The reaction system was stirred at room temperature for 2 hours under nitrogen atmosphere, and then diluted with dichloromethane. The organic phase was washed with water, separated, and concentrated under reduced pressure. The residue was purified by flash column chromatography (dichloromethane/petroleum ether = 0 to 80%) to obtain *tert*-butyl (4-bromobenzo[*d*]thiazol-2-yl)carbamate (6.6 g) as a light yellow solid.

Step 2: To a solution of *tert*-butyl (4-bromobenzo[*d*]thiazol-2-yl)carbamate (1.3 g, 3.95 mmol) in tetrahydrofuran (10 mL) was added sodium hydride (0.14 g, 5.93 mmol), and the reaction system was stirred at room temperature for 10 minutes. The above reaction system was added dropwise with a solution of n-butyllithium in *n*-hexane (2.5 M, 2.37 mL) at -70°C. After the addition was completed, the reaction mixture was stirred for 25 minutes, and then added with triisopropyl borate (2.23 g, 11.9 mmol). The reaction mixture was stirred at this temperature for another 25 minutes, then warmed to room temperature, and added with saturated ammonium chloride aqueous solution to quench. The aqueous phase was extracted with ethyl acetate, and the organic phases were combined and concentrated under reduced pressure. The residue was triturated with petroleum ether, filtered, and the filter cake was dried under vacuum to obtain intermediate 16 (1.5 g) as a white solid. m/z: [M+H]⁺ 295.2.

### Intermediate 17: Synthesis of (2-((tert-butoxycarbonyl)amino)-7-fluorobenzo[d]thiazol-4-yl)boronic acid

Intermediate 17 was obtained by reacting 4-bromo-7-fluorobenzo[*d*]thiazol-2-amine using the synthesis method of intermediate 16. m/z: [M+H]⁺ 313.0.

### Intermediate 18: Synthesis of tert-butyl (3-cyano-4-(4,4,5,5-tetramethyl-1,3,2-dioxaborolan-2-yl)-6,7-dihydrobenzo[b]thiophen-2-yl)carbamate

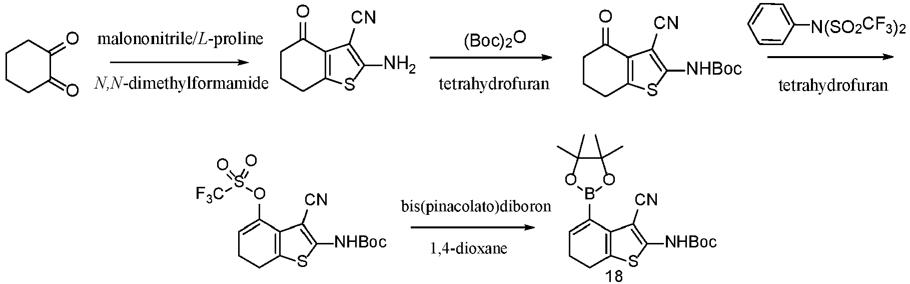

Step 1: To a solution of 1,2-cyclohexanedione (5.2 g, 46.4 mmol) in *N,N-*dimethylformamide (50 mL) were sequentially added malononitrile (3.37 g, 51.0 mmol), sulfur powder (2.23 g, 70.0 mmol), and *L*-proline (0.53 g, 4.64 mmol). The reaction system was stirred at 60°C for overnight. The reaction mixture was poured into ice water, filtered, then the filter cake was triturated with ethyl acetate, filtered, and the filter cake was dried under vacuum to obtain 2-amino-4-oxo-4,5,6,7-tetrahydrobenzo[*b*]thiophene-3-carbonitrile (2 g) as a brown solid.

Step 2: To a solution of 2-amino-4-oxo-4,5,6,7-tetrahydrobenzo[*b*]thiophene-3-carbonitrile (1.5 g, 7.8 mmol) in tetrahydrofuran (50 mL) were sequentially added di-*tert*-butyl dicarbonate (5.11 g, 23.4 mmol) and 4-dimethylaminopyridine (1.91 g, 15.6 mmol). The reaction system was stirred at 60°C for overnight and then directly concentrated under reduced pressure. The residue was purified by silica gel column chromatography (petroleum ether/ethyl acetate = 3/1) to obtain *tert*-butyl (3-cyano-4-oxo-4,5,6,7-tetrahydrobenzo[*b*]thiophen-2-yl)carbamate (350 mg) as an off-white solid. m/z: [M+Na]⁺ 315.2.

Step 3: To a solution of *tert*-butyl (3-cyano-4-oxo-4,5,6,7-tetrahydrobenzo[*b*]thiophen-2-yl)carbamate (60 mg, 0.21 mmol) and *N-*phenylbis(trifluoromethanesulfonimide) (0.11 g, 0.32 mmol) in anhydrous tetrahydrofuran (2 mL) was added dropwise a solution of lithium bis(trimethylsilyl)amide in tetrahydrofuran (1 M, 0.32 mL) at -78°C. The reaction system was stirred at this temperature for another 3 hours, added with saturated ammonium chloride aqueous solution to quench, concentrated under reduced pressure, and the residue was purified by *prep-TLC* (petroleum ether/ethyl acetate = 3/1) to obtain 2-((*tert*-butoxycarbonyl)amino)-3-cyano-6,7-dihydrobenzo[*b*]thiophen-4-yl trifluoromethanesulfonate (27 mg) as an off-white solid. m/z: [M+H]⁺ 425.0.

Step 4: A solution of 2-((*tert*-butoxycarbonyl)amino)-3-cyano-6,7-dihydrobenzo[b]thiophen-4-yl trifluoromethanesulfonate (27 mg, 0.06 mmol), [1,1'-bis(diphenylphosphino)ferrocene]dichloropalladium(II) dichloromethane complex (2.6 mg), potassium acetate (19 mg, 0.19 mmol), and bis(pinacolato)diboron (24 mg, 0.10 mmol) in anhydrous 1,4-dioxane (2 mL) was replaced with nitrogen and stirred at 85°C for 7 hours, cooled to room temperature, filtered through diatomite, and the filtrate was concentrated under reduced pressure to obtain intermediate 18 (25 mg) as a yellow oil. m/z: [M+Na]⁺ 425.2.

### Intermediate 19: Synthesis of a mixture of tert-butyl (1-(difluoromethyl)-7-(4,4,5,5-tetramethyl-1,3,2-dioxaborolan-2-yl)-1H-benzo[d]imidazol-2-yl)carbamate and tert-butyl (1-(difluoromethyl)-4-(4,4,5,5-tetramethyl-1,3,2-dioxaborolan-2-yl)-1H-benzo[d]imidazol-2-yl)carbamate

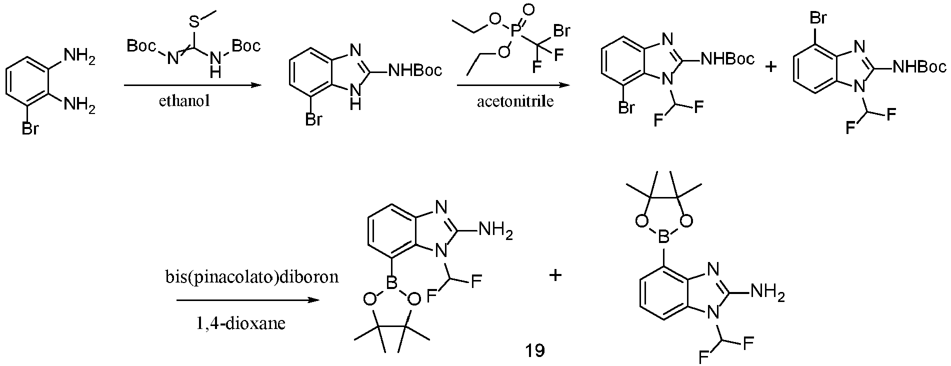

Step 1: A solution of 3-bromo-1,2-diaminobenzene (3 g, 15.6 mmol), *N,N*'-di-Boc-*S-*methylisothiourea (9.23 g, 31.1 mmol), and *D*-(+)-camphorsulfonic acid (0.37 g, 1.56 mmol) in ethanol (120 mL) was stirred at room temperature for 12 hours, then the reaction mixture was directly concentrated under reduced pressure, and the residue was purified by flash column chromatography (petroleum ether/ethyl acetate = 0 to 60%) to obtain *tert*-butyl (7-bromo-1*H-*benzo[*d*]imidazol-2-yl)carbamate (4.9 g) as a yellow solid. m/z: [M+H]⁺ 312.0.

Step 2: To a solution of *tert*-butyl (7-bromo-1*H*-benzo[*d*]imidazol-2-yl)carbamate (2.5 g, 7.94 mmol) and potassium fluoride (1.25 g, 21.3 mmol) in acetonitrile (60 mL) was added diethyl bromodifluoromethanephosphonate (2.49 g, 9.05 mmol). The reaction mixture was stirred at room temperature for 18 hours and added with ice water to quench. The aqueous phase was extracted with ethyl acetate, then the organic phase was concentrated under reduced pressure, and the residue was purified by flash column chromatography (petroleum ether/ethyl acetate = 0 to 50%) to obtain a mixture of *tert*-butyl (7-bromo-1-(difluoromethyl)-1*H-*benzo[*d*]imidazol-2-yl)carbamate and *tert*-butyl (4-bromo-1-(difluoromethyl)-1*H-*benzo[*d*]imidazol-2-yl)carbamate (1.4 g) as a yellow oil.

Step 3: A solution of the product obtained from step 2 (1.4 g, 3.87 mmol), [1,1'-bis(diphenylphosphino)ferrocene]dichloropalladium(II) dichloromethane complex (0.67 g, 0.77 mmol), potassium acetate (1.15 g, 11.6 mmol), and bis(pinacolato)diboron (1.5 g, 5.8 mmol) in anhydrous 1,4-dioxane (20 mL) was replaced with nitrogen and stirred at 100°C for 16 hours, cooled to room temperature and concentrated under reduced pressure, and the residue was purified by flash column chromatography (ethyl acetate/petroleum ether = 0 to 100%) to obtain intermediate 19 (360 mg) as a yellow oil. m/z: [M+H]⁺ 310.2.

### Intermediate 20: Synthesis of tert-butyl 3-cyano-4-(4,4,5,5-tetramethyl-1,3,2-dioxaborolan-2-yl)-1H-benzo[4,5]thieno[2,3-b]pyrrole-1-carboxylate

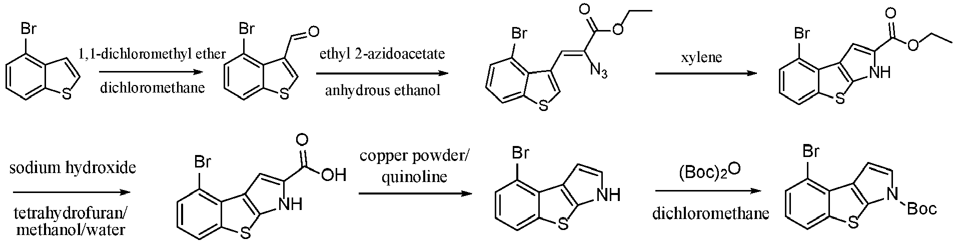

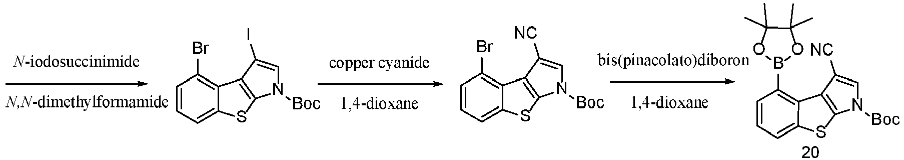

Step 1: To a solution of 4-bromobenzo[b]thiophene (3.8 g, 17.8 mmol) and 1,1-dichloromethyl ether (2.05 g, 17.8 mmol) in dichloromethane (150 mL) was slowly added dropwise titanium tetrachloride (24.6 mL, 225 mmol) in an ice bath. After the addition was completed, the reaction mixture was stirred at room temperature for overnight. The reaction mixture was added with saturated ammonium chloride aqueous solution to quench at 0°C. The aqueous phase was extracted with dichloromethane, then the organic phases were combined, washed with saturated brine, dried over anhydrous sodium sulfate, filtered, and the filtrate was concentrated under reduced pressure. The residue was dispersed in a small amount of dichloromethane, filtered, and the filter cake was dried under vacuum to obtain 4-bromobenzo[*b*]thiophene-3-carbaldehyde (1.8 g) as an off-white solid.

Step 2: A sodium ethoxide solution was prepared by dissolving sodium (610 mg, 26.6 mmol) in anhydrous ethanol (15 mL) in an ice bath. To a solution of 4-bromobenzo[b]thiophene-3-carbaldehyde (1.6 g, 6.6 mmol) and ethyl 2-azidoacetate (3.4 g, 26.6 mmol) in tetrahydrofuran (40 mL) was slowly added dropwise the above sodium ethoxide solution in an ice bath. After the addition was completed, the reaction mixture was stirred at 0°C for 2 hours. The reaction mixture was added with saturated ammonium chloride aqueous solution to quench, and the aqueous phase was extracted with ethyl acetate. The organic phase was dried over anhydrous sodium sulfate, filtered, and the filtrate was concentrated under reduced pressure. The residue was purified by flash column chromatography (petroleum ether/dichloromethane = 4/1) to obtain ethyl 2-azido-3-(4-bromobenzo[*b*]thiophen-3-yl)acrylate (660 mg) as a light brown solid. ¹H NMR (400 MHz, CDCl₃): δ 8.42 (s, 1H), 8.27 (d, *J* = 0.8Hz, 1H), 7.83 (dd, *J* = 8.0, 1.2Hz, 1H), 7.64 (dd, *J* = 7.6, 0.8 Hz, 1H), 7.20 (t, *J* = 8.0Hz, 1H), 4.40 (q, *J* = 7.2Hz, 2H), 1.42 (t, *J* = 7.2Hz, 3H).

Step 3: Ethyl 2-azido-3-(4-bromobenzo[*b*]thiophen-3-yl)acrylate (829 mg, 2.35 mmol) was dissolved in xylene (40 mL), and the reaction system was replaced with nitrogen and stirred at 145°C for 0.5 hours. The reaction mixture was concentrated under reduced pressure to obtain ethyl 4-bromo-1*H*-benzo[4,5]thieno[2,3-*b*]pyrrole-2-carboxylate (730 mg) as a light brown solid. m/z: [M+H]⁺ 324.0.

Step 4: To a mixed solution of ethyl 4-bromo-1*H*-benzo[4,5]thieno[2,3-*b*]pyrrole-2-carboxylate (350 mg, 1.08 mmol) in tetrahydrofuran (5 mL), methanol (5 mL), and water (5 mL) was added sodium hydroxide (216 mg, 5.4 mmol). The reaction system was stirred at 90°C for 13 hours. The reaction mixture was cooled to room temperature and concentrated under reduced pressure to remove the organic solvent. The aqueous phase was added with saturated citric acid aqueous solution to adjust the pH to 4, and a large amount of solid was precipitated. The solid was filtered, and the filter cake was washed with water and dried under vacuum to obtain 4-bromo-1*H*-benzo[4,5]thieno[2,3-*b*]pyrrole-2-carboxylic acid (310 mg) as a light purple solid. m/z: [M+H]⁺ 296.0.

Step 5: 4-Bromo-1*H*-benzo[4,5]thieno[2,3-*b*]pyrrole-2-carboxylic acid (260 mg, 0.79 mmol), copper powder (75 mg, 1.19 mmol), and quinoline (13 mL) were placed in a microwave tube. The reaction system was stirred at 160°C for 5 minutes. The reaction mixture was cooled to room temperature, added with hydrochloric acid (3 M) to adjust the pH to 5, and diluted with ethyl acetate. The reaction mixture was filtered, and the filtrate was extracted with ethyl acetate. The organic phases were combined and concentrated under reduced pressure. The residue was purified by *prep-TLC* (petroleum ether/ethyl acetate = 4/1) to obtain 4-bromo-1*H*-benzo[4,5]thieno[2,3-*b*]pyrrole (70 mg) as a light brown solid. m/z: [M+H]⁺ 252.0.

Step 6: To a solution of 4-bromo-1*H*-benzo[4,5]thieno[2,3-*b*]pyrrole (70 mg, 0.28 mmol) in dichloromethane (5 mL) were sequentially added triethylamine (56 mg, 0.56 mmol), 4-dimethylaminopyridine (3 mg, 0.03 mmol), and (Boc)₂O (91 mg, 0.42 mmol). The reaction mixture was stirred at room temperature for 1 hour and concentrated under reduced pressure. The residue was purified by flash column chromatography (petroleum ether/ethyl acetate = 10/1) to obtain *tert*-butyl 4-bromo-1*H*-benzo[4,5]thieno[2,3-*b*]pyrrole-1-carboxylate (86 mg) as a light brown solid.

Step 7: A solution of *tert*-butyl 4-bromo-1*H*-benzo[4,5]thieno[2,3-*b*]pyrrole-1-carboxylate (76 mg, 0.22 mmol) and *N*-iodosuccinimide (74 mg, 0.33 mmol) in *N,N-*dimethylformamide (5 mL) was stirred at room temperature for 15 hours. The reaction mixture was added with water to quench, and the aqueous phase was extracted with ethyl acetate. The organic phases were combined and concentrated under reduced pressure. The residue was purified by *prep-TLC* (petroleum ether/dichloromethane = 4/1) to obtain *tert*-butyl 4-bromo-3-iodo-1*H*-benzo[4,5]thieno[2,3-*b*]pyrrole-1-carboxylate (90 mg) as a white solid. m/z: [M-56+H]⁺ 422.0.

Step 8: A reaction system of *tert*-butyl 4-bromo-3-iodo-1*H*-benzo[4,5]thieno[2,3-b]pyrrole-1-carboxylate (90 mg, 0.19 mmol), cuprous cyanide (51 mg, 0.57 mmol), tris(dibenzylideneacetone)dipalladium (8.7 mg, 9.5 µmol), and 1,1'-bis(diphenylphosphino)ferrocene (18.4 mg. 0.033 mmol) in 1,4-dioxane (6 mL) was replaced with nitrogen for three times, and the reaction mixture was stirred at 100°C for 1 hour. The reaction mixture was cooled to room temperature and diluted with ethyl acetate. The reaction mixture was filtered, and the filtrate was concentrated under reduced pressure. The residue was purified by *prep-TLC* (petroleum ether/ethyl acetate = 4/1) to obtain *tert*-butyl 4-bromo-3-cyano-1*H*-benzo[4,5]thieno[2,3-*b*]pyrrole-1-carboxylate (50 mg) as a white solid. m/z: [M+H]⁺ 377.0. ¹H NMR (400 MHz, DMSO-*d₆*): δ 8.14 (d, *J=* 8.0Hz, 1H), 8.05 (s, 1H), 7.73 (d, *J=* 7.6Hz, 1H), 7.37 (t, *J* = 8.0Hz, 1H), 1.69 (s, 9H).

Step 9: A solution of the product obtained from step 8 (40 mg, 0.11 mmol), potassium acetate (32 mg, 0.33 mmol), bis(pinacolato)diboron (84 mg, 0.33 mmol), and [1,1'-bis(diphenylphosphino)ferrocene]dichloropalladium(II) dichloromethane complex (13.5 mg, 0.017 mmol) in anhydrous 1,4-dioxane (8 mL) was replaced with nitrogen for three times, and the reaction system was stirred at 100°C for 3 hours. The reaction mixture was cooled to room temperature and diluted with ethyl acetate. The reaction mixture was filtered, and the filtrate was concentrated under reduced pressure. The residue was purified by flash column chromatography (ethyl acetate/petroleum ether = 4/1) to obtain intermediate 20 (43 mg) as a light brown solid. m/z: [M+H]⁺ 425.2.

### Intermediate 25: Synthesis of tert-butyl 3-((tert-butoxycarbonyl)amino)-4-(4,4,5,5-tetramethyl-1,3,2-dioxaborolan-2-yl)-1H-benzo[4,5]thieno[2,3-b]pyrrole-1-carboxylate

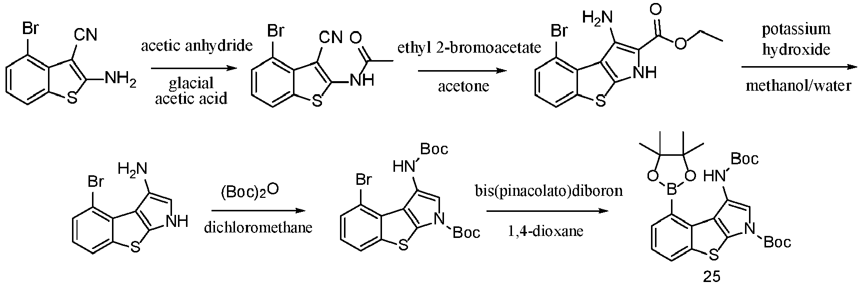

Step 1: 2-Amino-4-bromobenzo[*b*]thiophene-3-carbonitrile (500 mg, 1.96 mmol), acetic anhydride (3 mL), and acetic acid (3 mL) were placed in a sealed tube. The reaction mixture was stirred at 140°C for 2 hours, cooled to room temperature, and then concentrated under reduced pressure. The residue was added with a mixed solution of petroleum ether/ethyl acetate (1/1), stirred for 0.5 hours, filtered, and the filter cake was dried under vacuum to obtain *N-*(4-bromo-3-cyanobenzo[*b*]thiophen-2-yl)acetamide (582 mg) as a white solid.

Step 2: To a solution of *N-*(4-bromo-3-cyanobenzo[*b*]thiophen-2-yl)acetamide (530 mg, 1.78 mmol) and potassium carbonate (492 mg, 3.56 mmol) in acetone (10 mL) was added ethyl 2-bromoacetate (357 mg, 2.14 mmol). The reaction system was stirred at 65°C for 16 hours, then transferred to a sealed tube, and stirred at 125°C for another 16 hours. The reaction mixture was cooled to room temperature and concentrated under reduced pressure to obtain ethyl 3-amino-4-bromo-1*H*-benzo[4,5]thieno[2,3-*b*]pyrrole-2-carboxylate (550 mg). m/z: [M+H]⁺ 339.2.

Steps 3 & 4: To a mixed solution of ethyl 3-amino-4-bromo-1*H*-benzo[4,5]thieno[2,3-b]pyrrole-2-carboxylate (550 mg, 1.6 mmol) in methanol (8 mL) and water (2 mL) was added potassium hydroxide (411 mg, 7.3 mmol), and the reaction system was heated under reflux and stirred for overnight. The reaction system was cooled to room temperature, added with (Boc)₂O (1.76 g, 8.06 mmol), and stirred at room temperature for overnight. The reaction mixture was extracted with ethyl acetate, then the organic phases were combined and concentrated under reduced pressure, and the residue was purified by flash column chromatography (petroleum ether/ethyl acetate = 10/1 to 4/1) to obtain *tert*-butyl 4-bromo-3-((*tert*-butoxycarbonyl)amino)-1*H*-benzo[4,5]thieno[2,3-*b*]pyrrole-1-carboxylate (35 mg) as a yellow oil. m/z: [M+H]⁺ 469.0.

Step 5: A solution of the product obtained from step 4 (15 mg, 32 µmol), [1,1'-bis(diphenylphosphino)ferrocene]dichloropalladium(II) dichloromethane complex (4 mg), potassium acetate (9.5 mg, 96 µmol), and bis(pinacolato)diboron (24 mg, 96 µmol) in anhydrous 1,4-dioxane (3 mL) was replaced with nitrogen and stirred at 100°C for 2 hours, cooled to room temperature and concentrated under reduced pressure, and the residue was purified by *prep-TLC* (ethyl acetate/petroleum ether = 4/1) to obtain intermediate 25 (15 mg) as a brown solid.

### Intermediate 26: Synthesis of tert-butyl 9-cyano-8-(4,4,5,5-tetramethyl-1,3,2-dioxaborolan-2-yl)-1H-imidazo[1,2-a]indole-1-carboxylate

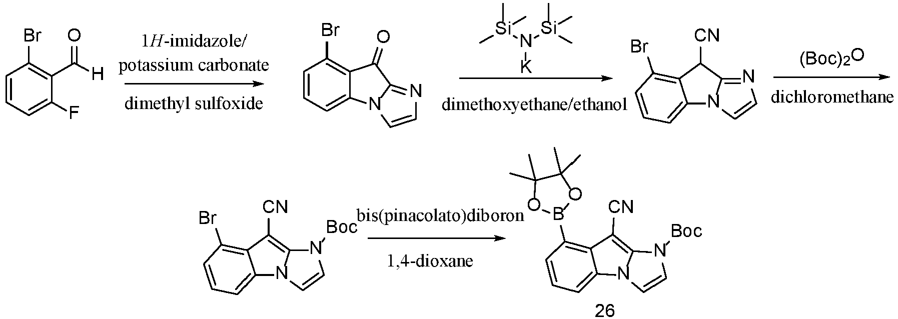

Step 1: A solution of 2-bromo-6-fluorobenzaldehyde (200 mg, 0.99 mmol), 1*H-*imidazole (67 mg, 0.99 mmol), and potassium carbonate (410 mg, 2.97 mmol) in dimethyl sulfoxide (20 mL) was stirred at 80°C for 4 hours. The reaction mixture was diluted with ethyl acetate, and the organic phase was washed with water and saturated brine, respectively, separated, and concentrated under reduced pressure. The residue was purified by silica gel column chromatography (petroleum ether/ethyl acetate = 1/1) to obtain 8-bromo-9*H-*imidazo[1,2-*a*]indol-9-one (20 mg) as a yellow solid. m/z: [M+H]⁺ 249.0.

Step 2: To a mixed solution of 8-bromo-9*H*-imidazo[1,2-*a*]indol-9-one (500 mg, 2.01 mmol) in dimethoxyethane (7 mL) and ethanol (0.2 mL) were added *p*-toluenesulfonyl isocyanate (520 mg, 2.61 mmol) and potassium bis(trimethylsilyl)amide (1 g, 5.02 mmol), and the reaction mixture was stirred at 35°C for 4 hours. The reaction mixture was added with water (1 mL) to quench, and the aqueous phase was extracted with ethyl acetate. The organic phases were combined and concentrated under reduced pressure. The residue was purified by silica gel column chromatography (dichloromethane/methanol = 20/1) to obtain 8-bromo-9*H-*imidazo[1,2-*a*]indole-9-carbonitrile (300 mg) as a brown solid. m/z: [M+H]⁺ 260.0.

Step 3: To a solution of 8-bromo-9*H*-imidazo[1,2-*a*]indole-9-carbonitrile (68 mg, 0.31 mmol) in dichloromethane (10 mL) were sequentially added triethylamine (53 mg, 0.52 mmol), 4-dimethylaminopyridine (3 mg, 0.03 mmol), and (Boc)₂O (68 mg, 0.31 mmol). The reaction mixture was stirred at room temperature for 1 hour and concentrated under reduced pressure to obtain *tert*-butyl 8-bromo-9-cyano-1*H*-imidazo[1,2-*a*]indole-1-carboxylate (50 mg) as a white solid. m/z: [M+H]⁺ 360.2.

Step 4: A solution of the product obtained from step 3 (50 mg, 0.14 mmol), [1,1'-bis(diphenylphosphino)ferrocene]dichloropalladium(II) dichloromethane complex (17 mg, 21 µmol), potassium acetate (41 mg, 0.42 mmol), and bis(pinacolato)diboron (107 mg, 0.42 mmol) in anhydrous 1,4-dioxane (8 mL) was replaced with nitrogen and stirred at 100°C for 3 hours, cooled to room temperature and concentrated under reduced pressure, and the residue was purified by flash column chromatography (ethyl acetate/petroleum ether = 4/1) to obtain intermediate 26 (50 mg) as a brown solid.

### Intermediate 21: Synthesis of 1-((2R,7aS)-2-fluorohexahydro-1H-pyrrolizin-7a-yl)ethanol

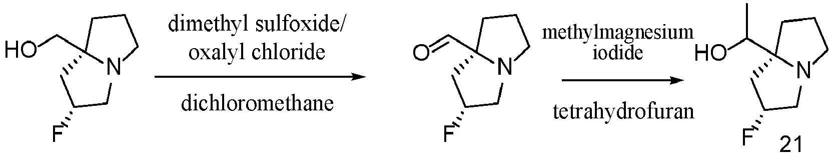

Step 1: To a solution of oxalyl chloride (7.41 g, 58.4 mmol) in dichloromethane (10 mL) was added dropwise a solution of dimethyl sulfoxide (7.61 g, 97.4 mmol) in dichloromethane (10 mL) at -78°C, and the reaction mixture was stirred for 1 hour. A solution of ((2*R*,7a*S*)-2-fluorohexahydro-1*H*-pyrrolizin-7a-yl)methanol (3.1 g, 19.5 mmol) in dichloromethane (10 mL) was then added dropwise thereto, and the reaction mixture was stirred for another 1 hour. Triethylamine (19.7 g, 195 mmol) was added thereto, and the reaction mixture was slowly warmed to room temperature and stirred for overnight. The reaction system was diluted with dichloromethane (100 mL), and the organic phase was washed with saturated sodium bicarbonate aqueous solution (50 mL) and concentrated under reduced pressure to obtain (2*R*,7a*S*)-2-fluorohexahydro-1*H*-pyrrolizine-7a-carbaldehyde (3 g) as a yellow oil.

Step 2: To a solution of (2*R*,7a*S*)-2-fluorohexahydro-1*H*-pyrrolizine-7a-carbaldehyde (3.0 g, 19.1 mmol) in tetrahydrofuran (80 mL) was slowly added a solution of methylmagnesium iodide in tetrahydrofuran (12.7 mL, 3 M) at -40°C, and the reaction system was slowly warmed to room temperature and stirred for overnight. The reaction system was added with tetrahydrofuran (200 mL) and saturated ammonium chloride aqueous solution (10 mL), respectively, then directly concentrated under reduced pressure, and the residue was purified by flash column chromatography (methanol/dichloromethane = 0 to 10%) to obtain intermediate 21 (2.5 g) as a brown oil. m/z: [M+H]⁺ 174.1.

### Intermediate 22: Synthesis of ((2R,7aS)-2-fluorohexahydro-1H-pyrrolizin-7a-yl)(²H₂)methanol

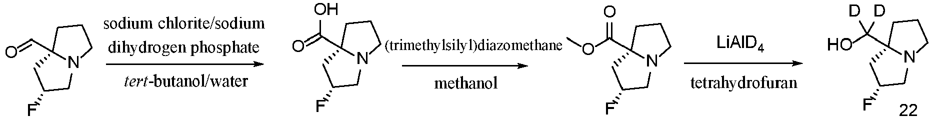

Step 1: To a mixed solution of (2*R*,7a*S*-2-fluorohexahydro-1*H*-pyrrolizine-7a-carbaldehyde (5.5 g, 35.0 mmol) in *tert*-butanol (60 mL) and water (30 mL) were sequentially added 2-methylbut-2-ene (12.3 g, 175 mmol), sodium dihydrogen phosphate (6.3 g, 52.5 mmol), and sodium chlorite (4.75 g, 52.5 mmol). The reaction mixture was stirred at room temperature for 2 hours, added with water to quench, and concentrated under reduced pressure to remove the organic solvent. The aqueous phase was extracted with ethyl acetate, and the organic phase was separated and concentrated under reduced pressure to obtain (2*R*,7a*S*)-2-fluorohexahydro-1*H*-pyrrolizine-7a-carboxylic acid (3.65 g) as a yellow oil. m/z: [M+H]⁺ 174.2.

Step 2: To a solution of (2*R*,7a*S*)-2-fluorohexahydro-1*H*-pyrrolizine-7a-carboxylic acid (3.6 g, 20.8 mmol) in methanol (30 mL) was slowly added (trimethylsilyl)diazomethane (4.75 g, 41.6 mmol) in an ice bath. The reaction mixture was stirred at room temperature for 1 hour, then directly concentrated under reduced pressure, and the residue was purified by flash column chromatography (methanol/dichloromethane = 0 to 1/20) to obtain methyl (2*R*,7a*S*)-2-fluorohexahydro-1*H*-pyrrolizine-7a-carboxylate (1.15 g) as a yellow oil. m/z: [M+H]⁺ 188.2.

Step 3: To a solution of methyl (2*R*,7a*S*)-2-fluorohexahydro-1*H*-pyrrolizine-7a-carboxylate (1.15 g, 5.61 mmol) in tetrahydrofuran (40 mL) was slowly added lithium aluminum deuteride (471 mg, 11.2 mmol) in an ice bath, and the reaction system was stirred at 0°C for 1 hour. The reaction system was slowly added with sodium sulfate decahydrate (5 g) and ethyl acetate (60 mL), stirred at room temperature for another 1 hour, filtered, and the filtrate was concentrated under reduced pressure to obtain intermediate 22 (900 mg) as a yellow oil. m/z: [M+H]⁺ 162.2.

### Intermediate 23: Synthesis of ((2R,7aS)-(5,5-²H₂)-2-fluorohexahydro-1H-pyrrolizin-7a-yl)methanol

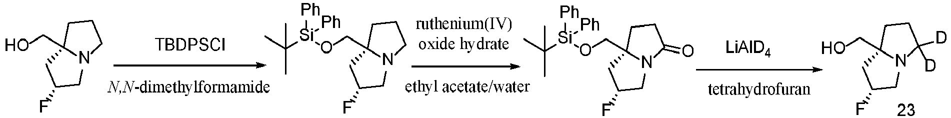

Step 1: To a solution of ((2*R*,7a*S*)-2-fluorohexahydro-1*H*-pyrrolizin-7a-yl)methanol (5 g, 31.4 mmol) in *N,N*-dimethylformamide (40 mL) were slowly added *tert-*butylchlorodiphenylsilane (11.2 g, 40.8 mmol) and 1*H*-imidazole (3.2 g, 47.1 mmol). The reaction mixture was stirred at room temperature for overnight and added with water (20 mL) to quench. The aqueous phase was extracted with ethyl acetate, and the organic phases were combined and concentrated under reduced pressure. The residue was purified by flash column chromatography (ethyl acetate/petroleum ether = 0 to 1/4) to obtain (2*R*,7a*S*)-7a-(((*tert-*butyldiphenylsilyl)oxy)methyl)-2-fluorohexahydro-1*H*-pyrrolizine (14 g) as a colorless oil.

Step 2: To a mixed solution of (2*R*,7a*S*)-7a-(((*tert*-butyldiphenylsilyl)oxy)methyl)-2-fluorohexahydro-1*H*-pyrrolizine (16.5 g, 41.5 mmol) in ethyl acetate (30 mL) and water (30 mL) were sequentially added sodium periodate (23.1 g, 108 mmol) and ruthenium(IV) oxide hydrate (313 mg, 2.08 mmol). The reaction system was stirred at room temperature for 2 hours, filtered through diatomite, and the filtrate was extracted with ethyl acetate. The organic phases were combined and concentrated under reduced pressure, and the residue was purified by flash column chromatography (petroleum ether/ethyl acetate = 0 to 1/3) to obtain (6*R*,7a*S*)-7a-(((*tert*-butyldiphenylsilyl)oxy)methyl)-6-fluorotetrahydro-1*H*-pyrrolizin-3(2H)-one (12 g) as a yellow solid. m/z: [M+H]⁺ 412.2.

Step 3: To a solution of (6*R*,7a*S*)-7a-(((*tert*-butyldiphenylsilyl)oxy)methyl)-6-fluorotetrahydro-1*H*-pyrrolizin-3(2*H*)-one (3.8 g, 9.23 mmol) in tetrahydrofuran (40 mL) was slowly added lithium aluminum deuteride (775 mg, 18.5 mmol) in an ice bath, and the reaction system was stirred at 80°C for 2 hours. The reaction system was slowly added with sodium sulfate decahydrate to quench, stirred at room temperature for another 0.5 hours, and filtered. The filtrate was concentrated under reduced pressure, and the residue was purified by flash column chromatography (methanol/dichloromethane = 1/10) to obtain intermediate 23 (600 mg) as a yellow oil. m/z: [M+H]⁺ 162.2.

**Intermediate 24:** Synthesis of ((2*R*,7a*S*)-(5,5-²H₂)-2-fluorohexahydro-1*H*-pyrrolizin-7a-yl)(²H₂)methanol

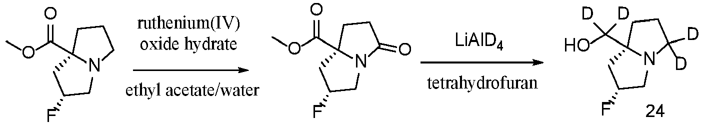

Step 1: To a mixed solution of methyl (2*R*,7a*S*)-2-fluorohexahydro-1*H*-pyrrolizine-7a-carboxylate (700 mg, 3.74 mmol) in ethyl acetate (10 mL) and water (10 mL) were sequentially added sodium periodate (2.4 g, 11.2 mmol) and ruthenium(IV) oxide hydrate (28.3 mg, 0.19 mmol). The reaction system was stirred at room temperature for 2 hours, filtered through diatomite, and the filtrate was extracted with ethyl acetate. The organic phases were combined, washed with saturated brine, separated, and concentrated under reduced pressure to obtain methyl (2*R*,7a*S*)-2-fluoro-5-oxohexahydro-1*H*-pyrrolizine-7a-carboxylate (600 mg) as a black oil. m/z: [M+H]⁺ 202.2.

Step 2: To a solution of methyl (2*R*,7a*S*)-2-fluoro-5-oxohexahydro-1*H*-pyrrolizine-7a-carboxylate (600 mg, 3.0 mmol) in tetrahydrofuran (12 mL) was slowly added lithium aluminum deuteride (375 mg, 8.9 mmol) in an ice bath, and the reaction system was stirred at 80°C for 0.5 hours. The reaction system was slowly added with sodium sulfate decahydrate to quench, stirred at room temperature for another 0.5 hours, and filtered. The filtrate was concentrated under reduced pressure, and the residue was purified by flash column chromatography (methanol/dichloromethane = 1/10) to obtain intermediate 24 (450 mg) as a yellow oil. m/z: [M+H]⁺ 164.2; ¹H NMR (400 MHz, CDCl₃): δ 5.27-5.13 (m, 1H), 3.15-3.02 (m, 2H), 2.12-2.02 (m, 2H), 1.92-1.74 (m, 4H).

### Synthesis of compounds:

### Example 1: Synthesis of 5-ethynyl-4-(1-fluoro-12-(((2R,7aS)-2-fluorohexahydro-1H-pyrrolizin-7a-yl)methoxy)-5a,6,7,8,9,10-hexahydro-5H-4-oxa-3,10a,11,13,14-pentaaza-6,9-methanonaphtho[1,8-ab]heptan-2-yl)naphthalen-2-ol (compound 1-1-1 or 1-1-2)

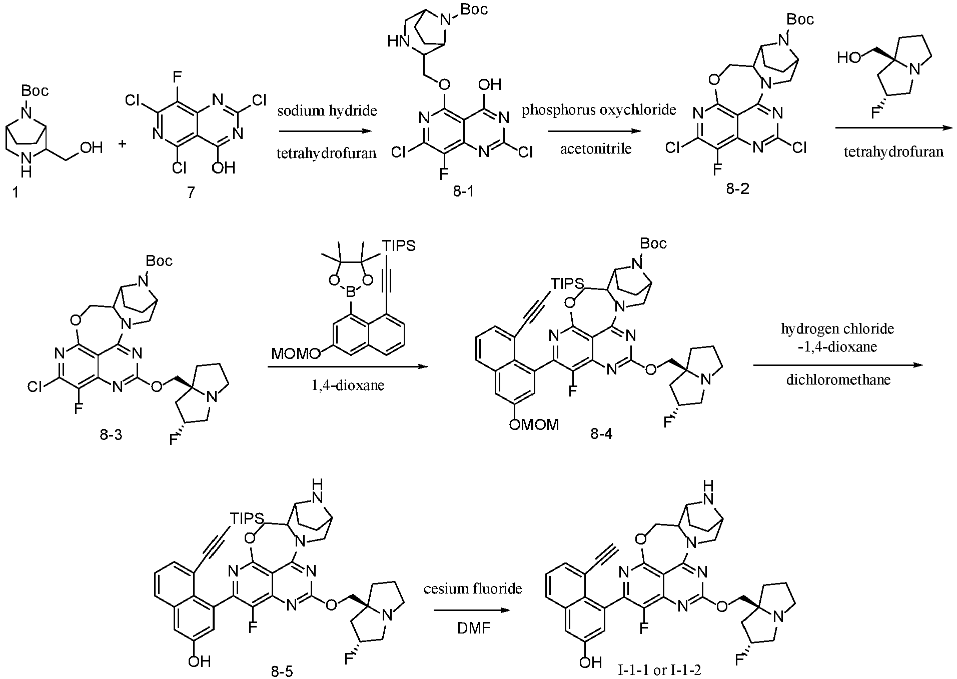

Step 1: To a solution of intermediate 1 (0.63 g, 2.61 mmol) in tetrahydrofuran (10 mL) was added a solution of sodium hydride (0.32 g, 7.83 mmol) in tetrahydrofuran (15 mL) in an ice bath. The resulting mixture was stirred at 0°C for 1 hour, and then the system was added with a solution of intermediate 7 (0.7 g, 2.61 mmol) in tetrahydrofuran (15 mL). The reaction mixture was stirred at room temperature for 1 hour, then added with water (1 mL) to quench, and the aqueous phase was extracted with ethyl acetate. The organic phases were combined, washed with saturated brine, dried over anhydrous sodium sulfate, and filtered. The filtrate was concentrated under reduced pressure, and the residue was purified by flash column chromatography (ethyl acetate/petroleum ether = 5/1) to obtain compound 8-1 (1.1 g, yield: 89%) as a yellow solid. m/z: [M+H]⁺ 474.2.

Step 2: To a solution of compound 8-1 (1.1 g, 2.32 mmol) and *N,N-*diisopropylethylamine (8.5 mL, 51.0 mmol) in acetonitrile (40 mL) was added a solution of phosphorus oxychloride (2.5 g, 16.2 mmol) in acetonitrile (5 mL). The reaction system was stirred at 100°C for 2 hours and directly concentrated under reduced pressure, and the residue was added with ethyl acetate (200 mL). The organic phase was sequentially washed with saturated sodium bicarbonate aqueous solution and saturated brine, separated, dried over anhydrous sodium sulfate, and filtered. The filtrate was concentrated under reduced pressure, and the residue was purified by flash column chromatography (ethyl acetate/petroleum ether = 1/1) to obtain compound 8-2 (0.5 g, yield: 47%) as a yellow solid. m/z: [M+H]⁺ 456.2.

Step 3: To a solution of ((2*R*,7a*S*)-2-fluorohexahydro-1*H*-pyrrolizin-7a-yl)methanol (0.14 g, 0.85 mmol) in tetrahydrofuran (5 mL) was added a solution of sodium hydride (0.11 g, 2.6 mmol) in tetrahydrofuran (5 mL) in an ice bath. The resulting mixture was stirred at 0°C for 1 hour, then a solution of compound 8-2 (0.3 g, 0.65 mmol) in tetrahydrofuran (10 mL) was added thereto, and the reaction system was stirred at room temperature for 1 hour. The reaction mixture was added with water (1 mL) to quench, and the aqueous phase was extracted with ethyl acetate. The organic phases were combined, washed with saturated brine, dried over anhydrous sodium sulfate, and filtered. The filtrate was concentrated under reduced pressure, and the residue was purified by flash column chromatography (methanol/dichloromethane = 10/1) to obtain compound 8-3 (0.24 g, yield: 65%) as a yellow solid. m/z: [M+H]⁺ 579.2.

Step 4: A mixed solution of compound 8-3 (160 mg, 0.27 mmol), triisopropyl((6-(methoxymethoxy)-8-(4,4,5,5-tetramethyl-1,3,2-dioxaborolan-2-yl)naphthalen-1-yl)ethynyl)silane (204 mg, 0.41 mmol), [1,1'-bis(diphenylphosphino)ferrocene]dichloropalladium(II) dichloromethane complex (224 mg, 0.27 mmol), and cesium carbonate (268 mg, 0.54 mmol) in 1,4-dioxane (12 mL) and water (3 mL) was reacted under microwave irradiation at 100°C for 5 hours. The reaction system was cooled to room temperature and concentrated under reduced pressure, and the residue was purified by flash column chromatography (methanol/dichloromethane = 10/1) to obtain compound 8-4 (150 mg, yield: 60%) as a black solid. m/z: [M+H]⁺ 911.4.

Step 5: To a solution of compound 8-4 (130 mg, 0.14 mmol) in dichloromethane (6 mL) was added a solution of hydrogen chloride in 1,4-dioxane (3 mL) in an ice bath, and the reaction mixture was stirred at room temperature for 2 hours and then directly concentrated under reduced pressure. The residue was added with acetonitrile (5 mL) and ammonia water (0.1 mL) and concentrated under reduced pressure again to obtain compound 8-5 (100 mg) as a black solid. m/z: [M+H]⁺ 767.4.

Step 6: Compound 8-5 (60 mg, 0.14 mmol) and cesium fluoride (108 mg, 0.71 mmol) were dissolved in *N,N-*dimethylformamide (5 mL), and the reaction system was stirred at 50°C for 2 hours and directly concentrated under reduced pressure. The residue was purified by *prep*-HPLC (alkaline conditions, gradient elution condition 1) to obtain compounds 1-1-1 (13.1 mg, a mixture of two *cis-trans* isomers) and I-1-2 (6.0 mg), both as white solids. I-1-1: UPLC RT = 3.832 minutes; m/z: [M+H]⁺ 611.2; ¹H NMR (400 MHz, DMSO-*d₆*): δ 10.14 (s, 1H), 7.88 (dd, *J* = 7.6, 2.8Hz, 1H), 7.48-7.40 (m, 2H), 7.31 (d, *J* = 2.8Hz, 1H), 7.13-7.03 (m, 1H), 5.34-5.21 (m, 2H), 4.88-4.78 (m, 1H), 4.57-4.49 (m, 1H), 4.40-4.29 (m, 1H), 4.17-3.89 (m, 3H), 3.80-3.54 (m, 4H), 3.18-2.98 (m, 4H), 2.85-2.67 (m, 2H), 2.12-1.97 (m, 3H), 1.85-1.57 (m, 5H); I-1-2: UPLC RT = 4.649 minutes; m/z: [M+H]⁺ 611.2; ¹H NMR (400 MHz, DMSO-*d₆*): δ 10.02 (s, 1H), 7.88 (d, *J* = 2.0Hz, 1H), 7.91 (d, *J* = 2.0Hz, 1H), 7.85 (d, *J* = 7.6Hz, 1H), 7.79 (d, *J* = 7.6Hz, 1H), 7.23 (d, *J* = 2.0Hz, 1H), 5.39-5.24 (m, 2H), 4.82-4.78 (m, 1H), 4.61-4.58 (m, 1H), 4.51-4.43 (m, 1H), 4.24-4.03 (m, 4H), 3.64-3.54 (m, 3H), 3.18-2.98 (m, 4H), 2.89-2.83 (m, 2H), 2.10-1.97 (m, 3H), 1.89-1.61 (m, 5H).

### Example 2: Synthesis of compound I-1-1

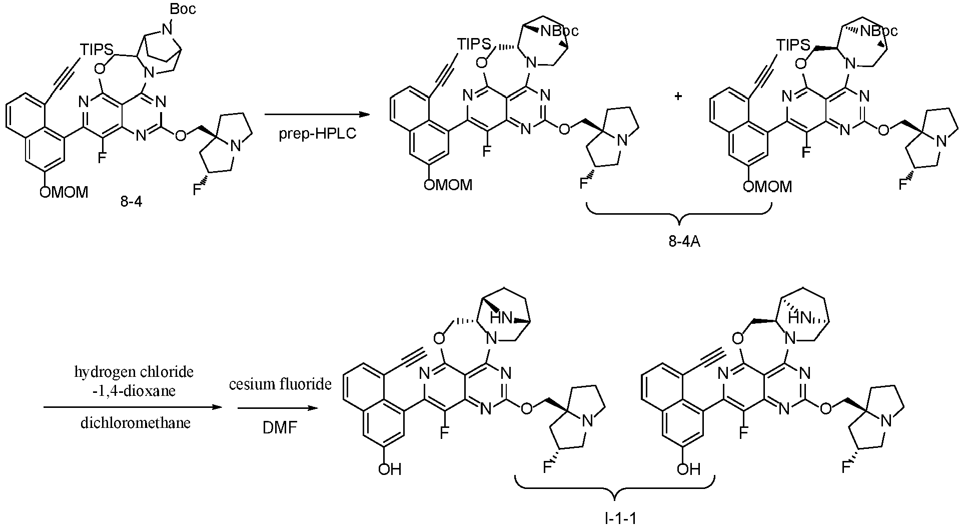

Step 1: Compound 8-4 was separated by *prep*-HPLC (alkaline conditions, gradient elution condition 1) to obtain compound 8-4A(Chiral HPLC RT = 10.462 and 17.460 minutes).

Steps 2 & 3: Compound I-1-1 was obtained by reacting compound 8-4A using the synthesis method of steps 5 to 6 in example 1, which was a mixture of two *cis-trans* isomers. m/z: [M+H]⁺ 611.2; UPLC RT = 3.784 minutes.

### Example 3: Synthesis of 5-ethynyl-4-(12-(((2R,7aS)-2-fluorohexahydro-1H-pyrrolizin-7a-yl)methoxy)-5a,6,7,8,9,10-hexahydro-5H-4-oxa-1,10a,11,13,14-pentaaza-6,9-methanonaphtho[1,8-ab]heptan-2-yl)naphthalen-2-ol (compound I-2)

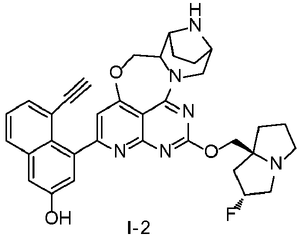

Compound I-2 was synthesized (*prep*-HPLC, alkaline conditions, gradient elution condition 1) as an earthy yellow solid using intermediates 1 and 6 as the starting materials using the synthesis method of example 1. UPLC RT = 2.446, 2.512, 2.567, 2.620 minutes; m/z: [M+H]⁺ 593.2; ¹H NMR (400 MHz, DMSO-*d₆*): δ 7.71-7.66 (m, 2H), 7.32-7.03 (m, 3H), 6.36 (s, 1 H), 5.34-5.21 (m, 2H), 4.80-4.66 (m, 2H), 4.53-4.30 (m, 2H), 4.11-3.85 (m, 4H), 3.20-2.81 (m, 5H), 2.10-1.94 (m, 4H), 1.84-1.68 (m, 3H), 1.56-1.42 (m, 3H).

### Example 4: Synthesis of 4-(3-chloro-1-fluoro-13-(((2R,7aS)-2-fluorohexahydro-1H-pyrrolizin-7a-yl)methoxy)-5a,6,7,8,9, 10-hexahydro-5H-6,9-epiminoazepino[2',1':3,4][1,4]oxazepino[5,6,7-de]quinazolin-2-yl)-5-ethynylnaphthalen-2-ol (compound I-3)

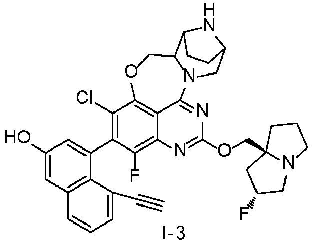

Compound I-3 was synthesized (*prep*-HPLC, alkaline conditions, gradient elution condition 1) as a yellow solid using intermediates 1 and 8 as the starting materials using the synthesis method of example 1. m/z: [M+H]⁺ 644.2.

### Example 5: Synthesis of 5-ethynyl-4-((5aS,6S,9R)-1-fluoro-12-(((2R,7aS)-2-fluorohexahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptan-2-yl)naphthalen-2-ol ditrifluoroacetate (compound I-4)

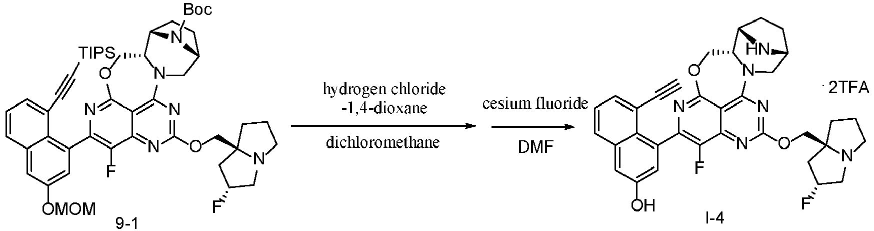

Synthesis of compound 9-1: Compound 9-1 was synthesized *(prep-HPLC,* acidic conditions, gradient elution condition 2) as a white solid using intermediates 2 and 7 as the starting materials using the synthesis method of steps 1 to 4 in example 1. Chiral HPLC RT = 10.556 minutes.

Compound I-4 was obtained as a white solid by reacting compound 9-1 using the synthesis method of steps 5 to 6 in example 1. m/z: [M+H]⁺ 611.2; ¹H NMR (400 MHz, DMSO-*d₆*): δ 10.12 (s, 1H), 7.88 (dd, *J*= 8.0, 2.0Hz, 1H), 7.48-7.38 (m, 2H), 7.31 (d, *J*= 1.2Hz, 1H), 7.12-7.03 (m, 1H), 5.34-5.21 (m, 2H), 4.87-4.77 (m, 1H), 4.56-4.48 (m, 1H), 4.39-4.28 (m, 1H), 4.08-3.96 (m, 3H), 3.62-3.48 (m, 2H), 3.13-3.01 (m, 4H), 2.85-2.80 (m, 1H), 2.15-1.91 (m, 4H), 1.89-1.51 (m, 8H).

### Example 6: Synthesis of 5-ethynyl-4-((5aR,6R,9S)-1-fluoro-12-(((2R,7aS)-2-fluorohexahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptan-2-yl)naphthalen-2-ol ditrifluoroacetate (compound I-5)

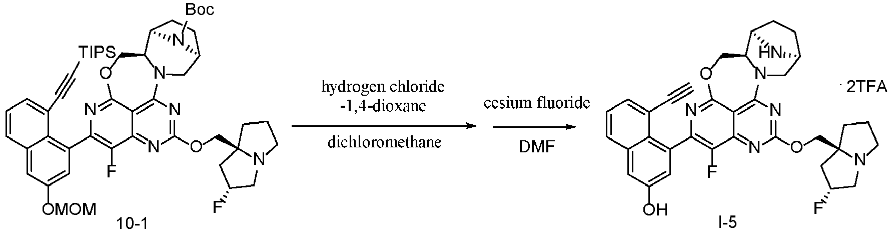

Synthesis of compound 10-1: Compound 10-1 was synthesized (*prep*-HPLC, acidic conditions, gradient elution condition 2) as a white solid using intermediates 3 and 7 as the starting materials using the synthesis method of steps 1 to 4 in example 1. Chiral HPLC RT = 17.256 minutes.

Compound I-5 was obtained (*prep*-HPLC, acidic conditions, gradient elution condition 2) as a white solid by reacting compound 10-1 using the synthesis method of steps 5 to 6 in example 1. m/z: [M+H]⁺ 611.2; ¹H NMR (400 MHz, DMSO-*d₆*): δ 10.14 (s, 1H), 7.88 (dd, *J* = 7.6, 2.8Hz, 1H), 7.48-7.40 (m, 2H), 7.31 (d, *J* = 2.8Hz, 1H), 7.13-7.03 (m, 1H), 5.34-5.21 (m, 2H), 4.88-4.78 (m, 1H), 4.57-4.49 (m, 1H), 4.40-4.29 (m, 1H), 4.17-3.89 (m, 3H), 3.80-3.54 (m, 4H), 3.18-2.98 (m, 4H), 2.85-2.67 (m, 2H), 2.12-1.97 (m, 3H), 1.85-1.57 (m, 5H).

### Example 7: Synthesis of 2-amino-4-(3-chloro-1-fluoro-5a,6,7,8,9,10-hexahydro-5H-6,9-epiminoazepino[2',1':3,4][1,4]oxazepino[5,6,7-de]quinazolin-2-yl)benzo[b]thiophene-3-carbonitrile (trans) trifluoroacetate (compound I-6-1 or I-6-2)

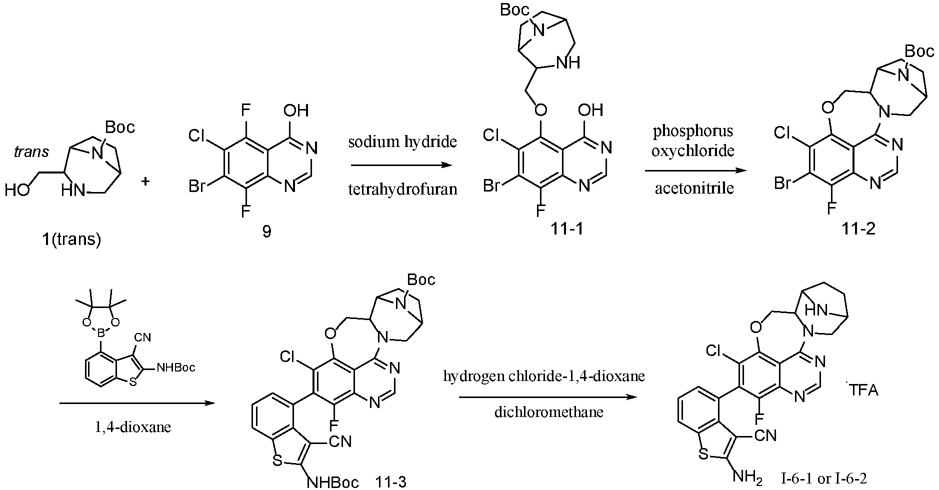

Step 1: To a solution of intermediate 1 (*trans*) (1.0 g, 4.13 mmol) in tetrahydrofuran (20 mL) was added a solution of sodium hydride (0.5 g, 12.4 mmol) in tetrahydrofuran (20 mL) in an ice bath. The reaction system was stirred at 0°C for 1 hour, and then added with a solution of intermediate 9 (1.46 g, 4.96 mmol) in tetrahydrofuran (20 mL). The reaction mixture was stirred at room temperature for 1 hour, added with water (50 mL) to quench, then added with hydrochloric acid (1 M) to adjust the pH to 6, and the aqueous phase was extracted with ethyl acetate (200 mL). The organic phases were combined, dried over anhydrous sodium sulfate, and filtered. The filtrate was concentrated under reduced pressure, and the residue was purified by flash column chromatography (methanol/dichloromethane = 10/1) to obtain compound 11-1 (1.3 g, yield: 60%) as a white solid. m/z: [M+H]⁺ 517.0.

Step 2: To a solution of compound 11-1 (1.30 g, 2.51 mmol) and *N,N-*diisopropylethylamine (8.5 mL, 55.2 mmol) in acetonitrile (150 mL) was added a solution of phosphorus oxychloride (2.69 g, 17.6 mmol) in acetonitrile (20 mL). The reaction system was stirred at 100°C for 2 hours and directly concentrated under reduced pressure, and the residue was added with ethyl acetate (200 mL). The organic phase was sequentially washed with saturated sodium bicarbonate aqueous solution and saturated brine, separated, dried over anhydrous sodium sulfate, and filtered. The filtrate was concentrated under reduced pressure, and the residue was purified by flash column chromatography (ethyl acetate/petroleum ether = 1/1) to obtain compound 11-2 (0.95 g, yield: 75%) as a white solid. m/z: [M+H]⁺ 499.0.

Step 3: A mixture of compound 11-2 (200 mg, 0.4 mmol), *tert*-butyl (3-cyano-4-(4,4,5,5-tetramethyl-1,3,2-dioxaborolan-2-yl)benzo[b]thiophen-2-yl)carbamate (200 mg, 0.40 mmol), dichloro[bis(diphenylphosphinophenyl)ether]palladium(II) (95.9 mg, 0.12 mmol), and cesium carbonate (326 mg, 1.0 mmol) in toluene (5 mL) was replaced with nitrogen and stirred at 105°C for 6 hours. The reaction system was directly concentrated under reduced pressure, and the residue was purified by flash column chromatography (methanol/dichloromethane = 10/1) to obtain compound 11-3 (150 mg, yield: 54%) as a white solid. m/z: [M+H]⁺ 693.2.

Step 4: To a solution of compound 11-3 (150 mg, 0.14 mmol) in dichloromethane (10 mL) was added trifluoroacetic acid (2 mL) in an ice bath. The reaction mixture was stirred at room temperature for 2 hours and directly concentrated under reduced pressure, and the residue was purified by *prep-HPLC* (acidic conditions, gradient elution condition 2) to obtain compounds I-6-1 (79.2 mg) and I-6-2 (56.1 mg), both as yellow solids. I-6-1: UPLC RT = 4.944 minutes; m/z: [M+H]⁺ 493.0; ¹H NMR (400 MHz, DMSO-*d₆*): δ 8.69 (s, 1H), 7.85 (s, 2H), 7.80 (dd, *J* = 8.0, 1.6Hz, 1H), 7.27-7.18 (m, 2H), 4.98 (dd, *J* = 14.4, 2.8Hz, 1H), 4.77 (dd, *J* = 13.2, 3.2Hz, 1H), 4.56-4.52 (m, 1H), 4.36-4.28 (m, 4H), 3.48 (d, *J* = 14.0Hz, 1H), 2.17-2.12 (m, 1H), 2.00-1.83 (m, 3H). I-6-2: UPLC RT = 5.150 minutes; m/z: [M+H]⁺ 493.0; ¹H NMR (400 MHz, DMSO-*d₆*): δ 8.69 (s, 1H), 7.85 (s, 2H), 7.80 (dd, *J* = 8.0, 1.2Hz, 1H), 7.27-7.14 (m, 2H), 4.98 (dd, *J* = 14.4, 2.8Hz, 1H), 4.66 (d, *J=* 5.2Hz, 2H), 4.44-4.41 (m, 1H), 4.30-4.24 (m, 3H), 3.47 (d, *J=* 14.0Hz, 1H), 2.20-2.17 (m, 1H), 2.00-1.88 (m, 3H).

### Example 8: Synthesis of 2-amino-4-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)benzo[b]thiophene-3-carbonitrile ditrifluoroacetate (compound I-7)

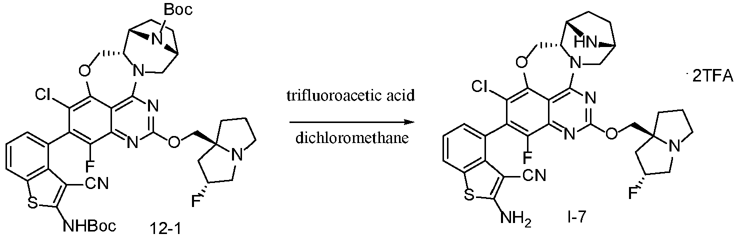

Synthesis of compound 12-1: Compound 12-1 was synthesized as a light yellow solid with corresponding boronic acid pinacol ester (intermediate 11) using intermediates 2 and 8 as the starting materials using the synthesis method of steps 1 to 4 in example 1. m/z: [M+H]⁺ 850.2.

To a solution of compound 12-1 (70 mg, 0.08 mmol) in dichloromethane (2 mL) was added trifluoroacetic acid (1 mL). The reaction mixture was stirred at 35°C for 2 hours and directly concentrated under reduced pressure, and the residue was purified by *prep*-HPLC (acidic conditions, gradient elution condition 2) to obtain compound I-7 (13.3 mg) as a white solid, which was a mixture of two atropisomers. UPLC RT = 4.389 and 4.482 minutes; m/z: [M+H]⁺ 650.2; ¹H NMR (400 MHz, DMSO-*d₆*/D₂O): δ 7.78 (d, *J=* 8.0Hz, 1H), 7.25 (t, *J* = 8.0Hz, 1H), 7.14 (t, *J=* 8.0Hz, 1H), 5.56 (d, *J=* 56.0Hz, 1H), 5.04-4.68 (m, 2H), 4.60-4.44 (m, 3H), 4.43-4.31 (m, 1H), 4.30-4.20 (m, 2H), 3.87-3.75 (m, 3H), 3.45 (d, *J=* 16.0Hz, 1H), 3.32-3.23 (m, 1H), 2.62-2.53 (m, 1H), 2.48-2.44 (m, 1H), 2.31 (dd, *J* = 12.0, 4.0Hz, 1H), 2.23-1.88 (m, 6H), 1.82-1.70 (m, 1H).

### Example 9: Synthesis of 2-amino-4-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)benzo[b]thiophene-3-carbonitrile, atropisomer 1 (compound I-7-1) and 2-amino-4-((SaS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)benzo[b]thiophene-3-carbonitrile, atropisomer 2 (compound I-7-2)

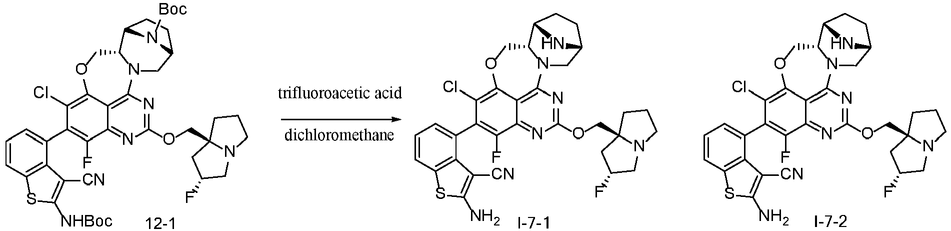

To a solution of compound 12-1 (880 mg, 0.94 mmol) in dichloromethane (2 mL) was added trifluoroacetic acid (1 mL). The reaction mixture was stirred at 35°C for 2 hours and then directly concentrated under reduced pressure. The residue was diluted with ethyl acetate and then added with saturated sodium bicarbonate aqueous solution to adjust the pH to neutrality. The aqueous phase was extracted with ethyl acetate, and the organic phases were combined and concentrated under reduced pressure. The residue was purified by *prep*-HPLC (alkaline conditions, gradient elution condition 5) to obtain compounds I-7-1 (281 mg) and I-7-2 (229 mg), both as off-white solids. I-7-1: HPLC RT = 16.724 minutes, m/z: [M+H]⁺ 650.2; ¹H NMR (400 MHz, DMSO-*d₆*): δ 7.85-7.71 (m, 3H), 7.24-7.18 (m, 1H), 7.14-7.10 (m, 1H), 5.26 (d, *J=* 54.0Hz, 1H), 4.85-4.75 (m, 1H), 4.68-4.58 (m, 1H), 4.20-4.13 (m, 1H), 4.08-4.02 (m, 2H), 3.98-3.92 (m, 1H), 3.57 (s, 1H), 3.48-3.44 (m, 1H), 3.38-3.36 (m, 1H), 3.09-3.02 (m, 3H), 3.01-2.97 (m, 1H), 2.83-2.77 (m, 1H), 2.17-2.08 (m, 1H), 2.05-1.95 (m, 2H), 1.85-1.52 (m, 7H). I-7-2: HPLC RT = 17.162 minutes, m/z: [M+H]⁺ 650.2; ¹H NMR (400 MHz, DMSO-*d₆*): δ 7.82-7.73 (m, 3H), 7.24-7.18 (m, 1H), 7.14-7.10 (m, 1H), 5.26 (d, *J* = 54.0Hz, 1H), 4.88-4.80 (m, 1H), 4.56-4.50 (m, 1H), 4.38-4.28 (m, 1H), 4.08-4.02 (m, 2H), 3.98-3.92 (m, 1H), 3.58 (s, 1H), 3.44 (d, *J=* 8.0Hz, 1H), 3.42-3.38 (m, 1H), 3.12-3.02 (m, 3H), 2.99 (s, 1H), 2.84-2.78 (m, 1H), 2.14-2.12 (m, 1H), 2.05-1.98 (m, 2H), 1.85-1.62 (m, 6H), 1.58-1.48 (m, 1H).

### Example 10: Synthesis of 2-amino-4-((SaS,6S,9R)-3-chloro-1-fluoro-Sa,6,7,8,9,10-hexahydro-5H-6,9-epiminoazepino[2',1':3,4][1,4]oxazepino[5,6,7-de]quinazolin-2-yl)-7-fluorobenzo[b]thiophene-3-carbonitrile trifluoroacetate, atropisomer 1 (compound I-8-1) and 2-amino-4-((5aS,6R,9R)-3-chloro-1-fluoro-5a,6,7,8,9,10-hexahydro-5H-6,9-epiminoazepino[2',1':3,4][1,4]oxazepino[5,6,7-de]quinazolin-2-yl)-7-fluorobenzo[b]thiophene-3-carbonitrile trifluoroacetate, atropisomer 2 (compound I-8-2)

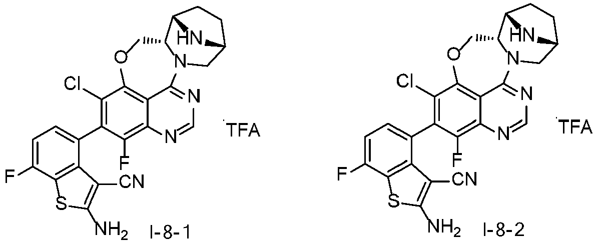

Compounds I-8-1 and I-8-2 were synthesized (*prep*-HPLC, acidic conditions, gradient elution condition 4) with corresponding boronic acid pinacol ester (intermediate 15) using intermediates 2 and 9 as the starting materials using the synthesis method of example 7, both as light yellow solids. I-8-1: UPLC RT = 5.413 minutes; HPLC RT = 16.118 minutes; m/z: [M+H]⁺ 511.0; ¹H NMR (400 MHz, DMSO-*d₆*): δ 8.70 (s, 1H), 8.12 (s, 2H), 7.29-7.14 (m, 2H), 4.98-4.95 (m, 1H), 4.77-4.73 (m, 1H), 4.65-4.52 (m, 2H), 4.42-4.23 (m, 3H), 3.44-3.42 (m, 2H), 2.20-2.08 (m, 1H), 2.00-1.93 (m, 3H). I-8-2: UPLC RT = 5.566 minutes; HPLC RT = 16.489 minutes; m/z: [M+H]⁺ 511.0; ¹H NMR (400 MHz, DMSO-*d₆*): δ 8.71 (s, 1H), 8.14 (s, 2H), 7.29-7.12 (m, 2H), 5.07-5.03 (m, 1H), 4.65-4.63 (m, 2H), 4.42-4.23 (m, 4H), 2.19-2.16 (m, 1H), 2.00-1.88 (m, 4H).

### Example 11: Synthesis of 4-((5aS,6S,9R)-3-chloro-1-fluoro-5a,6,7,8,9,10-hexahydro-5H-6,9-epiminoazepino[2',1':3,4][1,4]oxazepino[5,6,7-de]quinazolin-2-yl)-1H-benzo[d]imidazol-2(3H)-one trifluoroacetate (compound I-9)

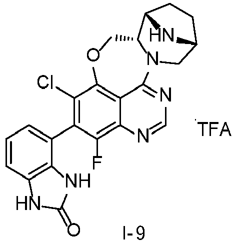

Compound I-9 was synthesized *(prep-HPLC,* acidic conditions, gradient elution condition 4) as a yellow solid with corresponding boronic acid pinacol ester using intermediates 2 and 9 as the starting materials using the synthesis method of steps 1 to 3 in example 7, which was a mixture of two atropisomers. UPLC RT = 3.519 and 3.811 minutes; m/z: [M+H]⁺ 453.2; ¹H NMR (400 MHz, DMSO-*d₆*): δ 10.85 (s, 1H), 10.64 (d, *J* = 6.0Hz, 1H), 9.58 (s, 1H), 9.45 (s, 1H), 8.71 (d, *J* = 4.4Hz, 1H), 7.10-7.03 (m, 2H), 6.93-6.89 (m, 1H), 5.04-4.85 (m, 1H), 4.75-4.59 (m, 2H), 4.41-4.26 (m, 4H), 3.50-3.44 (m, 1H), 2.23-2.15 (m, 1H), 2.01-1.80 (m, 3H).

### Example 12: Synthesis of 2-amino-4-((SaS,6S,9R)-3-chloro-1-fluoro-Sa,6,7,8,9,10-hexahydro-5H-6,9-epiminoazepino[2',1':3,4][1,4]oxazepino[5,6,7-de]quinazolin-2-yl)benzo[b]thiophene-3-carbonitrile trifluoroacetate, atropisomer 1 (compound I-10-1) and 2-amino-4-((5aS,6S,9R)-3-chloro-1-fluoro-5a,6,7,8,9,10-hexahydro-5H-6,9-epiminoazepino[2',1':3,4][1,4]oxazepino[5,6,7-de]quinazolin-2-yl)benzo[b]thiophene-3-carbonitrile trifluoroacetate, atropisomer 2 (compound I-10-2)

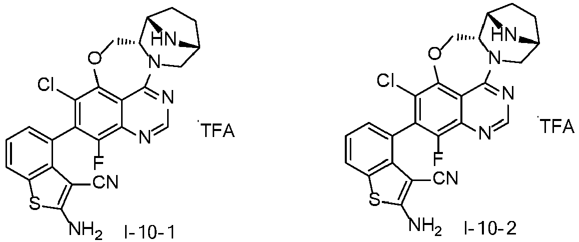

Compounds I-10-1 and I-10-2 were synthesized (*prep*-HPLC, acidic conditions, gradient elution condition 4) with corresponding boronic acid pinacol ester (intermediate 11) using intermediates 2 and 9 as the starting materials using the synthesis method of example 7, both as light yellow solids. I-10-1: UPLC RT = 4.952 minutes; HPLC RT = 15.584 minutes; m/z: [M+H]⁺ 493.0; ¹H NMR (400 MHz, DMSO-*d₆*): δ 8.69 (s, 1H), 7.85 (s, 2H), 7.80 (dd, *J* = 8.0, 1.6Hz, 1H), 7.27-7.18 (m, 2H), 4.98 (dd, *J=* 14.4, 2.8Hz, 1H), 4.77 (dd, *J=* 13.2, 3.2Hz, 1H), 4.56-4.52 (m, 1H), 4.36-4.28 (m, 4H), 3.48 (d, *J* = 14.0 Hz, 1H), 2.17-2.12 (m, 1H), 2.00-1.83 (m, 3H). I-10-2: UPLC RT = 5.153 minutes; HPLC RT = 15.966 minutes; m/z: [M+H]⁺ 493.0; ¹H NMR (400 MHz, DMSO-*d₆*): δ 8.69 (s, 1H), 7.85 (s, 2H), 7.80 (dd, *J*= 8.0, 1.2Hz, 1H), 7.27-7.14 (m, 2H), 4.98 (dd, *J* = 14.4, 2.8Hz, 1H), 4.66 (d, *J* = 5.2Hz, 2H), 4.44-4.41 (m, 1H), 4.30-4.24 (m, 3H), 3.47 (d, *J=* 14.0Hz, 1H), 2.20-2.17 (m, 1H), 2.00-1.88 (m, 3H).

### Example 13: Synthesis of 2-amino-4-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-7-fluorobenzo[b]thiophene-3-carbonitrile, atropisomer 1 (compound I-11-1) and 2-amino-4-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-7-fluorobenzo[b]thiophene-3-carbonitrile, atropisomer 2 (compound I-11-2)

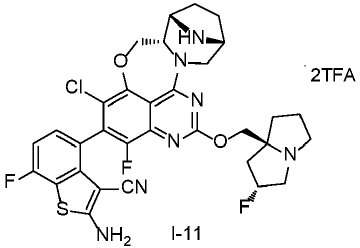

Compound I-11 was synthesized as a light yellow solid with corresponding boronic acid pinacol ester (intermediate 15) using intermediates 2 and 8 as the starting materials using the synthesis method of compound I-7, which was a mixture of two atropisomers. UPLC RT = 4.805 and 4.877 minutes; m/z: [M+H]⁺ 668.2; ¹H NMR (400 MHz, DMSO-*d₆*): δ 8.14 (s, 2H), 7.26-7.15 (m, 2H), 5.65-5.52 (m, 2H), 5.02-4.89 (m, 1H), 4.75-4.51 (m, 4H), 4.43-4.27 (m, 3H), 3.94-3.77 (m, 4H), 2.64-2.58 (m, 2 H), 2.34-2.31 (m, 1H), 2.22-2.07 (m, 3H), 2.04-1.94 (m, 3H), 1.82-1.79 (m, 1H), 1.23 (s, 1H).

Compound I-11 (28 mg) was separated by *prep*-HPLC (alkaline conditions, gradient elution condition 7) to obtain compounds I-11-1 (6.85 mg) and I-11-2 (3.38 mg). I-11-1: UPLC RT = 4.783 minutes, HPLC RT = 17.403 minutes; m/z: [M+H]⁺ 668.0. I-11-2: UPLC RT = 4.820 minutes, HPLC RT = 18.023 minutes; m/z: [M+H]⁺ 668.0.

### Example 14: Synthesis of 4-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)benzo[d]thiazol-2-amine, atropisomer 1 (compound I-12-1) and 4-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)benzo[d]thiazol-2-amine, atropisomer 2 (compound I-12-2)

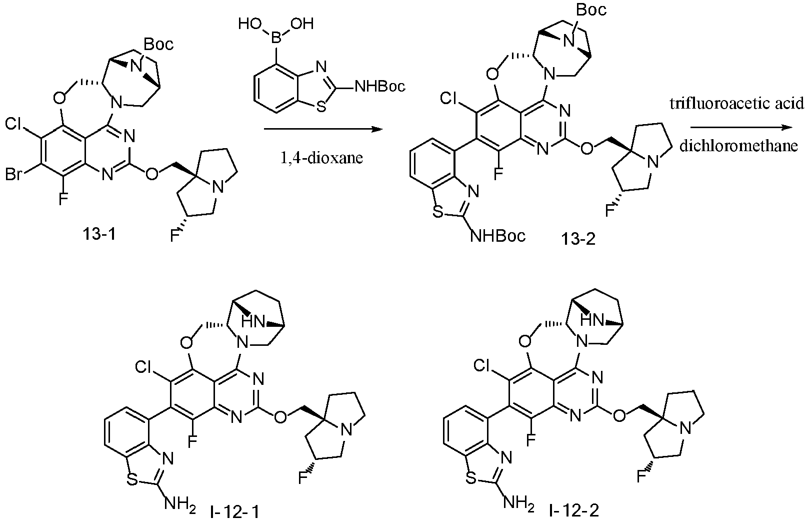

Step 1: A solution of compound 13-1 (synthesized using intermediates 2 and 8 as the starting materials using the synthesis method of steps 1 to 3 in example 1) (50 mg, 0.073 mmol) and (2-((*tert*-butoxycarbonyl)amino)benzo[*d*]thiazol-4-yl)boronic acid (25.8 mg, 0.088 mmol) in 1,4-dioxane (2 mL) was replaced with nitrogen, and then a solution of potassium phosphate (47 mg, 0.22 mmol) in water (0.5 mL) was added dropwise to the above reaction system. The reaction system was replaced with nitrogen again, added with [1,1'-bis(di-*tert-*butylphosphino)ferrocene]dichloropalladium(II) (7.28 mg, 0.011 mmol), and stirred at 100°C for 2 hours. The reaction mixture was cooled to room temperature, concentrated under reduced pressure, and the residue was purified by flash column chromatography (methanol/dichloromethane = 0% to 6%) to obtain compound 13-2 (55 mg) as a yellow solid. m/z: [M+H]⁺ 826.2.

Step 2: To a solution of compound 13-2 (70 mg, 0.08 mmol) in dichloromethane (2 mL) was added trifluoroacetic acid (1 mL). The reaction mixture was stirred at 35°C for 3 hours and directly concentrated under reduced pressure, and the residue was purified by *prep-*HPLC (alkaline conditions, gradient elution condition 6) to obtain compounds 1-12-1 (10. 1 mg) and I-12-2 (3.9 mg), both as white solids. I-12-1: UPLC RT = 4.051 minutes; HPLC RT = 16.558 minutes; m/z: [M+H]⁺ 626.2; ¹H NMR (400 MHz, DMSO-*d₆*): δ 7.82-7.76 (m, 1H), 7.65 (s, 2H), 7.18-7.09 (m, 2H), 5.30 (d, *J=* 56.0Hz, 1H), 4.73 (dd, *J*= 12.0, 4.0Hz, 1H), 4.59 (dd, *J=* 12.0, 4.0Hz, 1H), 4.40 (dd, *J=* 20.0, 8.0Hz, 1H), 4.11 (d, *J*= 12.0Hz, 1H), 4.02-3.96 (m, 2H), 3.62 (s, 1H), 3.58-3.51 (m, 1H), 3.13-3.09 (m, 3H), 3.03 (s, 1H), 2.89-2.81 (m, 1H), 2.58 (s, 1H), 2.15 (d, *J* = 4.0Hz, 1H), 2.08-1.95 (m, 3H), 1.90-1.57 (m, 6H). I-12-2: UPLC RT = 4.164 minutes; HPLC RT = 17.076 minutes; m/z: [M+H]⁺ 626.2; ¹H NMR (400 MHz, DMSO-*d₆*): δ 7.82-7.76 (m, 1H), 7.65 (s, 2H), 7.18-7.09 (m, 2H), 5.30 (d, *J* = 56.0Hz, 1H), 4.79 (dd, *J=* 12.0, 4.0Hz, 1H), 4.58 (dd, *J=* 12.0, 4.0 Hz, 1H), 4.41 (dd, *J=* 12.0, 8.0Hz, 1H), 4.16-3.91 (m, 3H), 3.62 (s, 1H), 3.51 (d, *J* = 8.0Hz, 1H), 3.19-2.96 (m, 3H), 2.87-2.81 (m, 1H), 2.57 (s, 1H), 2.16-2.14 (m, 1H), 2.10-1.96 (m, 3H), 1.91-1.51 (m, 6H).

### Example 15: Synthesis of 2-amino-4-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)pyrazolo[1,5-a]pyridine-3-carbonitrile (compound I-13)

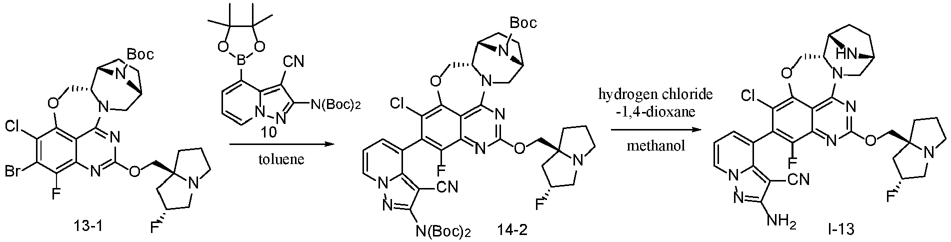

Step 1: To a solution of compound 13-1 (60 mg, 0.09 mmol) and intermediate 10 (44 mg, 0.09 mmol) in toluene (2 mL) were added potassium phosphate (56.4 mg, 0.26 mmol) and dichloro[bis(diphenylphosphinophenyl)ether]palladium(II) (20.0 mg, 0.03 mmol), and the reaction system was replaced with nitrogen and stirred at 105°C for 31 hours. The reaction mixture was cooled to room temperature, concentrated under reduced pressure, and the residue was purified by flash column chromatography (methanol/dichloromethane = 0% to 6%) to obtain compound 14-2 (55 mg) as a yellow solid. m/z: [M+H]⁺ 934.4.

Step 2: To a solution of compound 14-2 (50 mg, 0.05 mmol) in methanol (2 mL) was added a solution of hydrogen chloride in 1,4-dioxane (10 mL), and the reaction mixture was stirred at room temperature for 2 hours and directly concentrated under reduced pressure. The residue was added with acetonitrile (5 mL) and ammonia water (0.1 mL), concentrated under reduced pressure again, and then purified by *prep*-HPLC (alkaline conditions, gradient elution condition 1) to obtain compound I-13 (9.9 mg) as a yellow solid, which was a mixture of two atropisomers. UPLC RT = 2.995 and 3.145 minutes; m/z: [M+H]⁺ 634.2; ¹H NMR (400 MHz, DMSO-*d₆*): δ 8.67 (dd, *J* = 6.8, 1.2Hz, 1H), 7.47 (dd, *J* = 6.8, 1.2Hz, 1H), 7.10 (td, *J* = 6.8, 1.2Hz, 1H), 6.44 (s, 2H), 5.38-5.22 (m, 1H), 4.90-4.82 (m, 1H), 4.69-4.58 (m, 1H), 4.43-4.23 (m, 1H), 4.13-4.06 (m, 2H), 4.01-3.97 (m, 1H), 3.64-3.48 (m, 2H), 3.15-3.02 (m, 5H), 2.20-1.98 (m, 3H), 1.89-1.55 (m, 7H), 1.26 (s, 1H).

### Example 16: Synthesis of 2-amino-4-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-5-fluorobenzo[b]thiophene-3-carbonitrile ditrifluoroacetate (compound I-14)

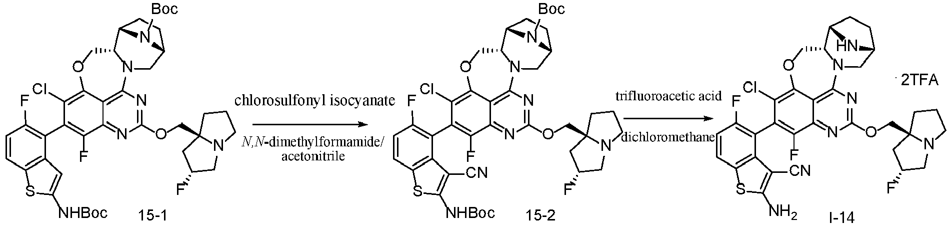

Step 1: To a mixed solution of compound 15-1 (synthesized with corresponding boronic acid pinacol ester (intermediate 13) using intermediates 2 and 8 as the starting materials using the synthesis method of steps 1 to 4 in example 1) (20 mg, 0.024 mmol) in anhydrous acetonitrile (4 mL) and anhydrous *N,N*-dimethylformamide (0.2 mL) was added chlorosulfonyl isocyanate (34 mg, 0.24 mmol) in an ice bath. After the addition was completed, the reaction mixture was stirred for another 10 minutes, then warmed to room temperature, and stirred for 3 hours. The reaction system was added with saturated ammonium chloride aqueous solution to quench, directly concentrated under reduced pressure, and the residue was subjected to flash column chromatography (methanol/dichloromethane = 0 to 10%) to obtain compound 15-2 (15 mg) as a light yellow solid. m/z: [M+H]⁺ 868.2.

Steps 2: Compound I-14 was obtained as a light brown solid by reacting compound 15-2 using the synthesis method of compound I-7, which was a mixture of two atropisomers. UPLC RT = 4.426 and 4.516 minutes; m/z: [M+H]⁺ 668.2; ¹H NMR (400 MHz, CD₃OD): δ 7.73 (dd, *J=* 4.8, 8.8Hz, 1H), 7.07 (t, *J*= 9.2Hz, 1H), 4.80-4.60 (m, 3H), 4.46-4.38 (m, 1H), 4.37-4.28 (m, 2H), 4.19-4.00 (m, 1H), 3.98-3.85 (m, 3H), 3.59-3.45 (m, 3H), 2.75-2.56 (m, 3H), 2.50-2.30 (m, 4H), 2.22-2.08 (m, 4H).

### Example 17: Synthesis of 5-chloro-4-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)benzo[b]thiophen-2-amine ditrifluoroacetate (compound 1-15)

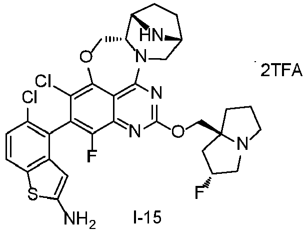

Compound I-15 was synthesized as a light yellow solid with corresponding boronic acid pinacol ester (intermediate 12) using intermediates 2 and 8 as the starting materials using the synthesis method of compound I-7, which was a mixture of two atropisomers. UPLC RT = 4.811 and 4.879 minutes; m/z: [M+H]⁺ 659.2.

### Example 18: Synthesis of 5-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)benzo[b]thiophen-2-amine ditrifluoroacetate (compound I-16)

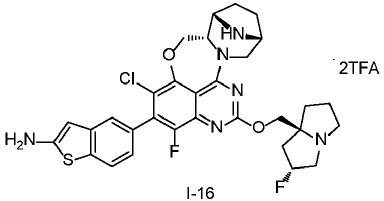

Compound I-16 was synthesized as a yellow solid with corresponding boronic acid pinacol ester using intermediates 2 and 8 as the starting materials using the synthesis method of compound I-7, which was a mixture of two atropisomers. UPLC RT = 4.436 and 4.507 minutes; m/z: [M+H]⁺ 625.2; ¹H NMR (400 MHz, DMSO-*d₆*): 7.68 (dd, *J* = 6.8, 2.0Hz, 1H), 7.22 (s, 1H), 7.10-7.04 (m, 2H), 6.76 (s, 1H), 5.65-5.47 (m, 2H), 5.37-5.31 (m, 1H), 4.94-4.88 (m, 2H), 4.75-4.53 (m, 4H), 4.33-4.27 (m, 3H), 3.84-3.75 (m, 3H), 2.33-2.28 (m, 2H), 2.30-2.14 (m, 4H), 2.04-1.94 (m, 4H), 1.29 (s, 1H).

### Example 19: Synthesis of 2-amino-4-((SaS,6S,9R)-3-chloro-1-fluoro-13-(1-((2R,7aS)-2-fluorohexahydro-1H-pyrrolizin-7a-yl)ethoxy)-5a,6,7,8,9,10-hexahydro-5H-6,9-epiminoazepino[2',1':3,4][1,4]oxazepino[5,6,7-de]quinazolin-2-yl)benzo[b]thiophene-3-carbonitrile ditrifluoroacetate (compound I-17)

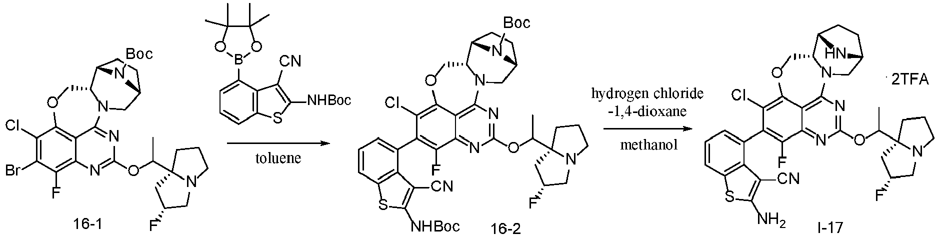

Synthesis of compound 16-1: Using the synthesis method of steps 1 to 3 in example 1, compound 16-1 was synthesized as a yellow solid with intermediate 21 instead of ((2*R*, 7a*S*)-2-fluorohexahydro-1*H*-pyrrolizin-7a-yl)methanol in step 3 using intermediates 2 and 8 as the starting materials. m/z: [M+H]⁺ 670.2.

Synthesis of compound I-17: Compound I-17 was synthesized as a yellow solid with compound 16-1 and corresponding boronic acid pinacol ester using the synthesis method of compound I-13. m/z: [M+H]⁺ 664.2; ¹H NMR (400 MHz, DMSO-*d₆*): δ 7.85 (s, 2H), 7.82 (d, *J=* 8.0 Hz, 1H), 7.25 (t, *J=* 8.0Hz, 1H), 7.14 (t, *J=* 8.0Hz, 1H), 5.56-5.42 (m, 2H), 5.34-5.28 (m, 2H), 4.92-4.88 (m, 1H), 4.73-4.49 (m, 3H), 4.41-4.28 (m, 3H), 3.90-3.66 (m, 4H), 2.22-2.11 (m,4H), 2.03-1.92 (m, 4H), 1.44 (d, *J* = 8.0Hz, 1H), 1.24 (s, 1H).

Compound I-17 (100 mg) was separated by *prep*-HPLC (alkaline conditions, gradient elution condition 7) to obtain compounds I-17-1 (15.1 mg) and I-17-2 (19.7 mg), both as a mixture of stereoisomers. I-17-1: UPLC RT = 4.451 and 4.559 minutes; m/z: [M+H]⁺ 664.0. I-17-2: UPLC RT = 4.548 and 4.593 minutes; m/z: [M+H]⁺ 664.0.

### Example 20: Synthesis of 2-amino-4-((SaS,6S,9R)-3-chloro-1-fluoro-13-(1-((2R,7aS)-2-fluorohexahydro-1H-pyrrolizin-7a-yl)ethoxy)-5a,6,7,8,9,10-hexahydro-5H-6,9-epiminoazepino[2',1':3,4][1,4]oxazepino[5,6,7-de]quinazolin-2-yl)-5-fluorobenzo[b]thiophene-3-carbonitrile ditrifluoroacetate (compound I-18)

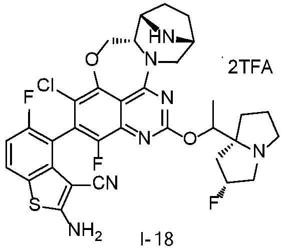

Compound I-18 was obtained as a pink solid by reacting compound 16-1 and intermediate 13 using the synthesis method of compound I-14, which was a mixture of stereoisomers. m/z: [M+H]⁺ 682.2; ¹H NMR (400 MHz, CD₃OD): δ 7.75-7.70 (m, 1H), 7.11-7.04 (m, 1H), 5.50-5.19 (m, 3H), 4.75-4.61 (m, 2H), 4.45-4.28 (m, 3H), 4.04-3.78 (m, 4H), 3.64-3.45 (m, 3H), 2.50-2.29 (m, 4H), 2.26-2.03 (m, 4H), 1.62-1.53 (m, 3H).

### Example 21: Synthesis of 4-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-7-fluorobenzo[d]thiazol-2-amine, atropisomer 1 (compound I-19-1) and 4-((SaS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-7-fluorobenzo[d]thiazol-2-amine, atropisomer 2 (compound I-19-2)

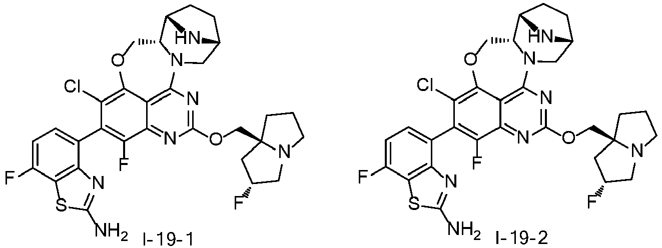

Compounds I-19-1 and I-19-2 were obtained (*prep*-HPLC, alkaline conditions, gradient elution condition 1) by reacting compound 13-1 and intermediate 17 using the synthesis method of compounds I-12-1 and I-12-2, both as white solids. I-19-1: UPLC RT = 4.446 minutes; HPLC RT = 17.152 minutes; m/z: [M+H]⁺ 644.2. I-19-2: UPLC RT = 4.502 minutes; HPLC RT = 17.710 minutes; m/z: [M+H]⁺ 644.2; ¹H NMR (400 MHz, DMSO-*d₆*): δ 7.92 (s, 2H), 7.21 (dd, *J* = 8.0, 4.0Hz, 1H), 7.05 (t, *J* = 8.0Hz, 1H), 5.27 (d, *J* = 52.0Hz, 1H), 4.80-4.72 (m, 1H), 4.65-4.51 (m, 1H), 4.46-4.38 (m, 1H), 4.18-4.08 (m, 1H), 4.02-3.88 (m, 2H), 3.58 (s, 1H), 3.51-3.47 (m, 1H), 3.26 (s, 1H), 3.05 (dd, *J=* 24.0, 12.0Hz, 4H), 2.89-2.75 (m, 1H), 2.18-2.06 (s, 1H), 2.04-1.80 (m, 2H), 1.72-1.42 (m, 6H), 1.30-1.10 (m, 1H).

### Example 22: Synthesis of 2-amino-4-((5aS,6S,9R)-3-chloro-1-fluoro-13-(((2R,7aS)-(5,5-²H₂)-2-fluorohexahydro-1H-pyrrolizin-7a-yl)(methoxy)-5a,6,7,8,9,10-hexahydro-5H-6,9-epiminoazepino[2',1':3,4][1,4]oxazepino[5,6,7-de]quinazolin-2-yl)benzo[b]thiophene-3-carbonitrile, atropisomer 1 (compound I-20-1) and 2-amino-4-((5aS,6S,9R)-3-chloro-1-fluoro-13-(((2R,7aS)-(5,5-²H₂)-2-fluorohexahydro-1H-pyrrolizin-7a-yl)(methoxy)-5a,6,7,8,9,10-hexahydro-5H-6,9-epiminoazepino[2',1':3,4][1,4]oxazepino[5,6,7-de]quinazolin-2-yl)benzo[b]thiophene-3-carbonitrile, atropisomer 2 (compound I-20-2)

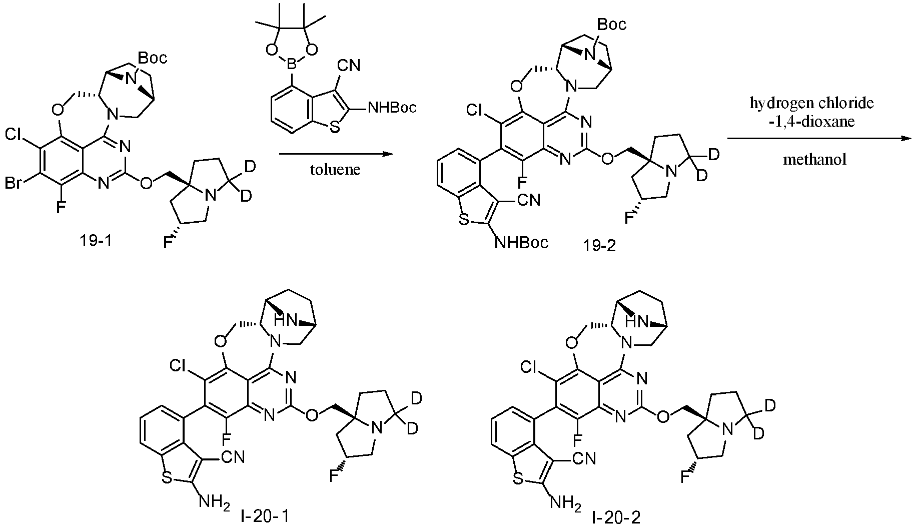

Synthesis of compound 19-1: Using the synthesis method of steps 1 to 3 in example 1, compound 19-1 was synthesized as a yellow solid with intermediate 23 instead of ((2*R*,7a*S*)-2-fluorohexahydro-1*H*-pyrrolizin-7a-yl)methanol in step 3 using intermediates 2 and 8 as the starting materials.

Synthesis of compounds I-20-1 and I-20-2: Compounds I-20-1 and I-20-2 were obtained *(prep-HPLC,* alkaline conditions, gradient elution condition 5) by reacting compound 19-1 and intermediate 11 using the synthesis method of compound I-13, both as white solids. I-20-1: UPLC RT = 4.372 minutes; HPLC RT = 16.793 minutes; m/z: [M+H]⁺ 652.2; ¹H NMR (400 MHz, DMSO-*d₆*): δ 7.80-7.76 (m, 3H), 7.25-7.21 (m, 1H), 7.15-7.13 (m, 1H), 5.34-5.21 (m, 1H), 4.85-4.81 (m, 1H), 4.65-4.61 (m, 1H), 4.21-4.15 (m, 1H), 4.09-4.04 (m, 2H), 3.97-3.94 (m, 1H), 3.60-3.55 (m, 1H), 3.51-3.32 (m, 2H), 3.10-3.00 (m, 3H), 2.15-1.99 (m, 2H), 1.84-1.63 (m, 7H). I-20-2: UPLC RT = 4.415 minutes; HPLC RT = 17.322 minutes; m/z: [M+H]⁺ 652.2; ¹H NMR (400 MHz, DMSO-*d₆*): δ 7.81-7.76 (m, 3H), 7.25-7.21 (m, 1H), 7.15-7.13 (m, 1H), 5.34-5.20 (m, 1H), 4.87-4.84 (m, 1H), 4.56-4.52 (m, 1H), 4.56-4.52 (m, 1H), 4.09-4.06 (m, 2H), 3.98-3.95 (d, *J=* 10.4 Hz; 1H), 3.60-3.56 (m, 1H), 3.48-3.26 (m, 2H), 3.13-3.00 (m, 3H), 2.14-1.99 (m, 2H), 1.85-1.53 (m, 7H).

### Example 23: Synthesis of 2-amino-4-((SaS,6S,9R)-3-chloro-1-fluoro-13-(1-((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-5-methylbenzo[b]thiophene-3-carbonitrile ditrifluoroacetate (compound I-21)

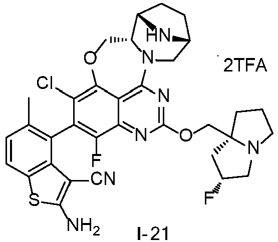

Compound I-21 was obtained as a pink solid by reacting compound 13-1 and intermediate 14 using the synthesis method of compound I-14, which was a mixture of two atropisomers. UPLC RT = 4.565 and 4.631 minutes; m/z: [M+H]⁺ 664.2.

### Example 24: Synthesis of 7-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-1-(difluoromethyl)-1H-benzo[d]imidazol-2-amine ditrifluoroacetate (compound I-22 or I-23) and 4-((5aS,6S,9R)-3-chloro-1-fluoro-13-(((27R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-1-(difluoromethyl)-1H-benzo[d]imidazol-2-amine ditrifluoroacetate (compound I-22 or I-23)

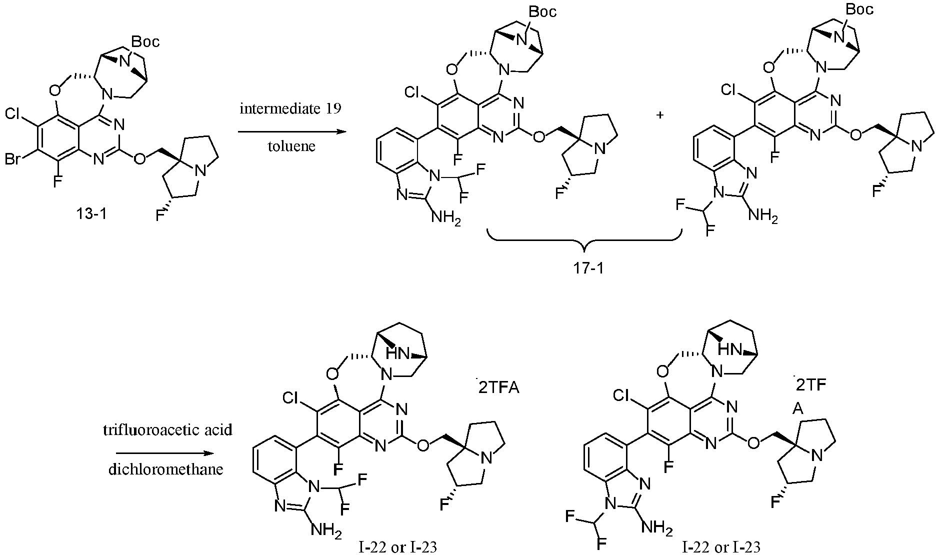

Step 1: To a solution of compound 13-1 (360 mg, 1.16 mmol) and intermediate 19 (955 mg, 1.16 mmol) in 1,4-dioxane (2 mL) were added cesium carbonate (1.15 g, 3.49 mmol) and dichloro[bis(diphenylphosphinophenyl)ether]palladium(II) (170 mg, 0.23 mmol), and the reaction system was replaced with nitrogen and stirred at 110°C for 12 hours. The reaction mixture was cooled to room temperature, concentrated under reduced pressure, and the residue was purified by flash column chromatography (methanol/dichloromethane = 0% to 10%) to obtain compound 17-1 (110 mg) as a yellow solid.

Step 2: To a solution of intermediate 17-1 (110 mg, 0.14 mmol) in dichloromethane (3 mL) was added trifluoroacetic acid (0.5 mL). The reaction mixture was stirred at room temperature for 2 hours and concentrated under reduced pressure, and the residue was purified by *prep*-HPLC (acidic conditions, gradient elution condition 7) to obtain compounds I-22 (3.5 mg) and I-23 (1.7 mg), both as white solids. I-22: HPLC RT = 15.655 minutes; m/z: [M+H]⁺ 659.2. I-23: HPLC RT = 16.017 minutes; m/z: [M+H]⁺ 659.2.

### Example 25: Synthesis of 2-amino-4-((5aS,6S,9R)-3-chloro-1-fluoro-13-(((2R,7aS)-2-fluorohexahydro-1H-pyrrolizin-7a-yl)((²H₂)methoxy))-5a,6,7,8,9,10-hexahydro-5H-6,9-epiminoazepino[2',1':3,4][1,4]oxazepino[5,6,7-de]quinazolin-2-yl)benzo[b]thiophene-3-carbonitrile, atropisomer 1 (compound I-24-1) and 2-amino-4-((5aS,6S,9R)-3-chloro-1-fluoro-13-(((2R,7aS)-2-fluorohexahydro-1H-pyrrolizin-7a-yl)((²H₂)methoxy))-5a,6,7,8,9,10-hexahydro-5H-6,9-epiminoazepino[2',1':3,4][1,4]oxazepino[5,6,7-de]quinazolin-2-yl)benzo[b]thiophene-3-carbonitrile, atropisomer 2 (compound I-24-2)

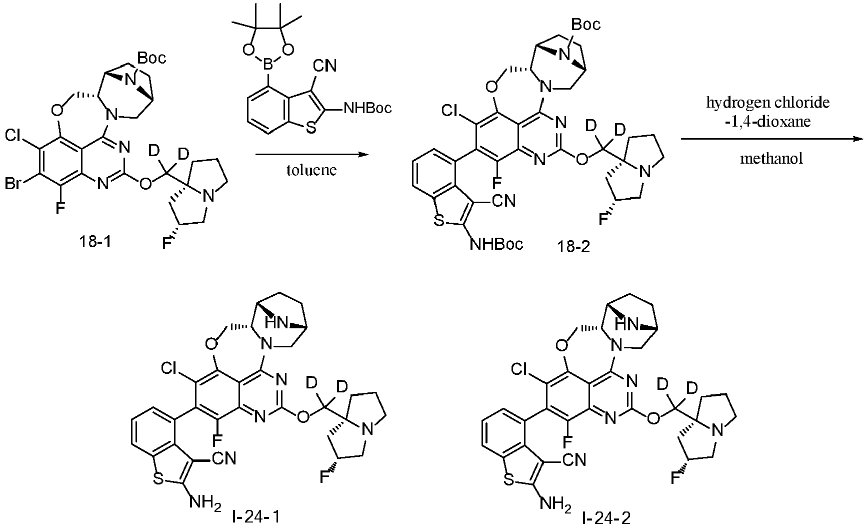

Synthesis of compound 18-1: Using the synthesis method of steps 1 to 3 in example 1, compound 18-1 was synthesized as a yellow solid with intermediate 22 instead of ((2*R*, 7a*S*)-2-fluorohexahydro-1*H*-pyrrolizin-7a-yl)methanol in step 3 using intermediates 2 and 8 as the starting materials.

Synthesis of compounds I-24-1 and I-24-2: Compounds I-24-1 and I-24-2 were obtained *(prep-HPLC,* alkaline conditions, gradient elution condition 5) by reacting compound 18-1 and intermediate 11 using the synthesis method of compound I-13, both as white solids. I-24-1: UPLC RT = 4.341 minutes; HPLC RT = 16.821 minutes; m/z: [M+H]⁺ 652.2; ¹H NMR (400 MHz, DMSO-*d₆*): δ 7.84-7.71 (m, 3H), 7.22 (t, *J=* 8.0Hz, 1H), 7.16-7.11 (m, 1H), 5.27 (d, *J=* 56.0Hz, 1H), 4.82 (dd, *J* = 12.0, 4.0Hz, 1H), 4.63 (dd, *J* = 14.0, 4.0Hz, 1H), 4.22-4.16 (m, 1H), 4.08-4.02 (m, 1H), 3.59 (s, 1H), 3.50-3.46 (m, 1H), 3.28-3.24 (m, 1H), 3.12-2.98 (m, 4H), 2.86-2.78 (m, 1H), 2.19-1.95 (m, 3H), 1.89-1.51 (m, 7H). I-24-2: UPLC RT = 4.426 minutes; HPLC RT = 17.354 minutes; m/z: [M+H]⁺ 652.2; ¹H NMR (400 MHz, DMSO-*d₆*): δ 7.88-7.72 (m, 3H), 7.22 (t, *J=* 8.0Hz, 1H), 7.13 (d, *J=* 8.0Hz, 1H), 5.27 (d, *J=* 54.0Hz, 1H), 4.85 (d, *J=* 12.0Hz, 1H), 4.53 (d, *J=* 12.0Hz, 1H), 4.38-4.26 (m, 1H), 4.10-4.02 (m, 1H), 3.58 (s, 2H), 3.25-3.18 (m, 1H), 3.15-2.95 (m, 4H), 2.85-2.75 (m, 1H), 2.18-1.98 (m, 3H), 1.90-1.44 (m, 7H).

### Example 26: Synthesis of 2-amino-4-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2', 1':3,4][1,4]oxazepino[5,6,7-de]quinazolin-2-yl)-6,7-dihydrobenzo[b]thiophene-3-carbonitrile ditrifluoroacetate (compound I-25)

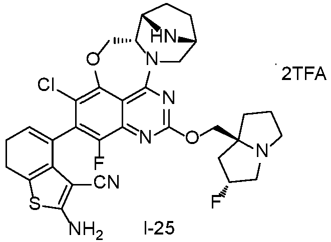

Compound I-25 was synthesized as a light yellow solid with corresponding boronic acid pinacol ester (intermediate 18) using intermediates 2 and 8 as the starting materials using the synthesis method of compound I-7, which was a mixture of two atropisomers. UPLC RT = 4.276 and 4.362 minutes; m/z: [M+H]⁺ 652.2.

### Example 27: Synthesis of 4-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-1H-benzo[4,5]thieno[2,3-c]pyrazol-3-amine, atropisomer 1 (compound I-26-1) and 4-((SaS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-1H-benzo[4,5]thieno[2,3-c]pyrazol-3-amine, atropisomer 2 (compound I-26-2)

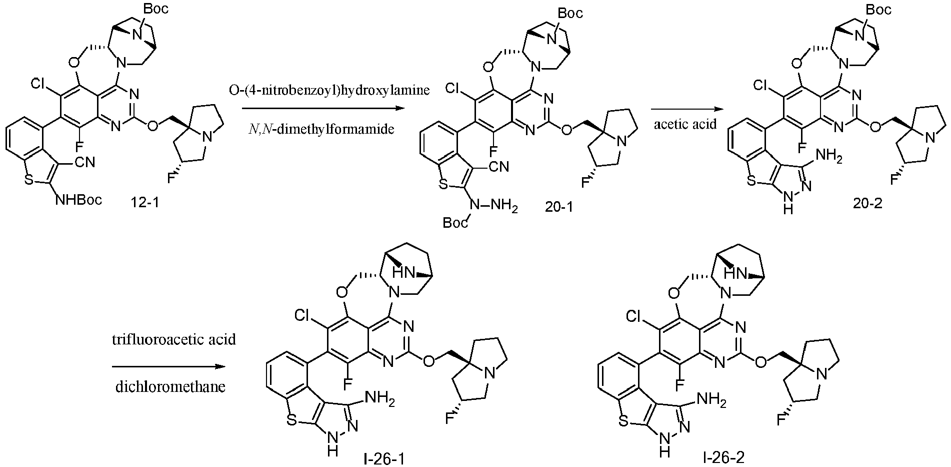

Step 1: A solution of compound 12-1 (100 mg, 0.12 mmol) and cesium carbonate (78 mg, 0.24 mmol) in *N,N*-dimethylformamide (3 mL) was stirred at 50°C for 2 hours, and the reaction system was cooled to 0°C in an ice-water bath and added with O-(4-nitrobenzoyl)hydroxylamine (44 mg, 0.24 mmol). The reaction mixture was stirred at room temperature for another 2 hours, and the reaction system was poured into ice water. The aqueous phase was extracted with ethyl acetate, and the organic phases were combined and concentrated under reduced pressure to obtain compound 20-1 (150 mg, crude) as a brown oil. m/z: [M+H]⁺ 865.3.

Step 2: A solution of compound 20-1 (150 mg) in acetic acid (2 mL) was stirred at 100°C for 2 hours, and then directly concentrated under reduced pressure to obtain compound 20-2 (150 mg, crude) as a brown oil. m/z: [M+H]⁺ 765.2.

Step 3: To a solution of compound 20-2 (150 mg, crude) in dichloromethane (4 mL) was added trifluoroacetic acid (2 mL). The reaction mixture was stirred at room temperature for 1 hour and concentrated under reduced pressure, and the residue was purified by *prep-*HPLC (alkaline conditions, gradient elution condition 1) to obtain compounds I-26-1 (4.4 mg) and I-26-2 (4.9 mg), both as white solids. I-26-1: HPLC RT = 15.452 minutes; m/z: [M+H]⁺ 665.1; ¹H NMR (400 MHz, DMSO-*d₆*): δ 7.94-7.68 (m, 2H), 7.30-7.07 (m, 2H), 6.14 (d, *J* = 5.4Hz, 2H), 5.70-5.45 (m, 2H), 4.94-4.80 (m, 1H), 4.76-4.56 (m, 3H), 4.49-4.30 (m, 2H), 4.21-3.94 (m, 4H), 3.91-3.80 (m, 1H), 3.13-3.04 (m, 1H), 3.00-2.70 (m, 3H), 2.38-2.15 (m, 4H), 1.74-1.49 (m, 4H). I-26-2: HPLC RT = 15.838 minutes; m/z: [M+H]⁺ 665.2; ¹H NMR (400 MHz, DMSO-*d₆*): δ 7.92-7.74 (m, 2H), 7.24 (t, *J=* 8.0Hz, 1H), 7.13 (d, *J* = 7.6Hz, 1H), 6.13 (s, 2H), 5.69-5.49 (m, 2H), 4.92 (d, *J* = 12.4Hz, 1H), 4.78-4.51 (m, 3H), 4.47-4.29 (m, 2H), 4.20-3.94 (m, 3H), 3.90-3.78 (m, 1H), 3.12-2.85 (m, 5H), 2.33-2.12 (m, 4H), 1.78-1.53 (m, 4H).

### Example 28: Synthesis of 2-amino-4-((5aS,6S,9R)-3-chloro-1-fluoro-13-(((2R,7aS)-(5,5-²H₂)-2-fluorohexahydro-1H-pyrrolizin-7a-yl)((²H₂)methoxy))-5a,6,7,8,9,10-hexahydro-5H-6,9-epiminoazepino[2',1':3,4][1,4]oxazepino[5,6,7-de]quinazolin-2-yl)benzo[b]thiophene-3-carbonitrile, atropisomer 1 (compound I-27-1) and 2-amino-4-((5aS,6S,9R)-3-chloro-1-fluoro-13-(((2R,7aS)-(5,5-²H₂)-2-fluorohexahydro-1H-pyrrolizin-7a-yl)((²H₂)methoxy))-5a,6,7,8,9,10-hexahydro-5H-6,9-epiminoazepino[2',1':3,4][1,4]oxazepino[5,6,7-de]quinazolin-2-yl)benzo[b]thiophene-3-carbonitrile, atropisomer 2 (compound I-27-2)

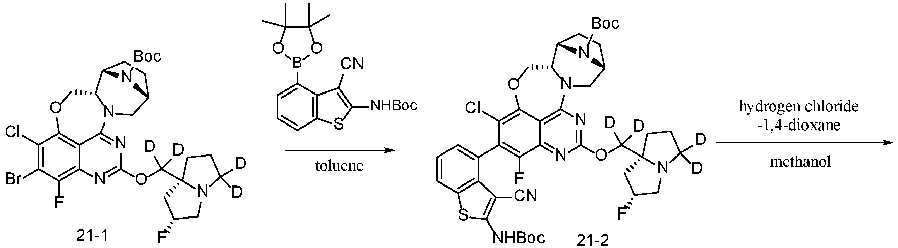

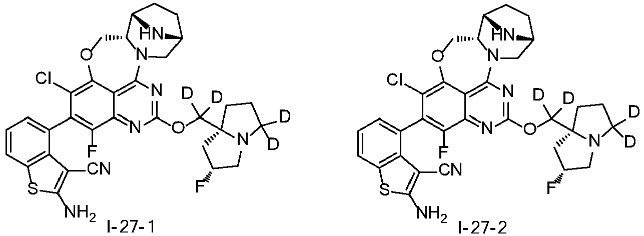

Synthesis of compound 21-1: Using the synthesis method of steps 1 to 3 in example 1, compound 21-1 was synthesized as a light yellow solid with intermediate 24 instead of ((2*R*,7a*S*)-2-fluorohexahydro-1*H*-pyrrolizin-7a-yl)methanol in step 3 using intermediates 2 and 8 as the starting materials. m/z: [M+H]⁺ 662.2.

Synthesis of compounds I-27-1 and I-27-2: Compounds I-27-1 and I-27-2 were obtained *(prep-HPLC,* alkaline conditions, gradient elution condition 1) by reacting compound 21-1 and intermediate 11 using the synthesis method of compound I-13, both as white solids. I-27-1: UPLC RT = 4.347 minutes; HPLC RT = 16.758 minutes; m/z: [M+H]⁺ 654.2; ¹H NMR (400 MHz, DMSO-*d₆*): δ 7.84-7.74 (m, 3H), 7.22 (t, *J* = 8.0Hz, 1H), 7.15-7.13 (m, 1H), 5.37-5.16 (m, 1H), 4.83 (dd, *J=* 12.0, 4.0Hz, 1H), 4.63 (dd, *J=* 12.0Hz, 1H), 4.18 (dd, *J=* 12.0Hz, 1H), 4.05 (dd, *J* = 4.0Hz, 1H), 3.59-3.34 (m, 2H), 3.13-2.95 (m, 3H), 2.05 (d, *J* = 4.0Hz, 3H), 1.86-1.52 (m, 7H). I-27-2: UPLC RT = 4.500 minutes; HPLC RT = 17.293 minutes; m/z: [M+H]⁺ 654.2; ¹H NMR (400 MHz, DMSO-*d₆*): δ 7.85-7.74 (m, 3H), 7.22 (d, *J=* 8.0Hz, 1H), 7.14 (d, *J* = 8.0Hz, 1H), 5.27 (d, *J* = 52.0Hz, 1H), 4.86 (dd, *J* = 12.0Hz, 1H), 4.54 (dd, *J =* 16.0Hz, 1H), 4.35 (dd, *J=* 16.0Hz, 1H), 4.09-4.02 (m, 1H), 3.58 (t, *J=* 4.0Hz, 2H), 3.45 (d, *J* = 8.0Hz, 3H), 3.11-2.97 (m, 1H), 2.19-1.94 (m, 3H), 1.87-1.49 (m, 7H).

### Example 29: Synthesis of 4-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-1H-benzo[4,5]thieno[2,3-c]pyrrole-3-carbonitrile, atropisomer 1 (compound I-28-1) and 4-((SaS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-1H-benzo[4,5]thieno[2,3-c]pyrrole-3-carbonitrile, atropisomer 2 (compound I-28-2)

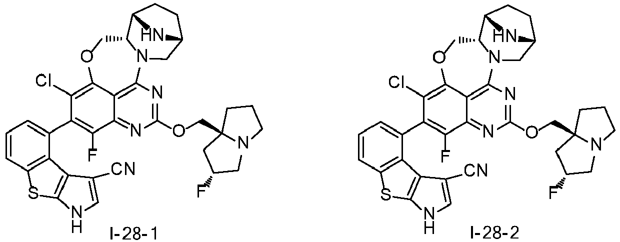

Compounds I-28-1 and I-28-2 were obtained (*prep*-HPLC, alkaline conditions, gradient elution condition 1) by reacting compound 13-1 and intermediate 20 using the synthesis method of compounds I-12-1 and I-12-2, both as white solids. I-28-1: UPLC RT = 4.960 minutes; HPLC RT = 17.687 minutes; m/z: [M+H]⁺ 674.2. I-28-2: UPLC RT = 4.972 minutes; HPLC RT = 18.251 minutes; m/z: [M+H]⁺ 674.2; ¹H NMR (400 MHz, DMSO-*d₆*): δ 8.06 (d, *J* = 8.0Hz, 1H), 7.46 (d, *J* = 7.6Hz, 1H), 7.34 (d, *J* = 7.2Hz, 1H), 6.25 (s, 1H), 5.35 (s, 1H), 5.21 (s, 1H), 4.86 (d, *J* = 12.0Hz, 1H), 4.68 (d, *J* = 11.2Hz, 1H), 4.47-4.37 (m, 1H), 4.18-4.06 (m, 2H), 3.96 (d, *J* = 10.4Hz, 1H), 3.73-3.54 (m, 3H), 3.19-2.97 (m, 3H), 2.88-2.78 (m, 1H), 2.17-1.95 (m, 3H), 1.91-1.67 (m, 4H), 1.64-1.44 (m, 2H).

### Example 30: Synthesis of 2-amino-4-((5aS,6S,9R)-3-chloro-1-fluoro-13-(1-(((R)-1-methylpyrrolidin-2-yl)methoxy)-5a,6,7,8,9,10-hexahydro-5H-6,9-epiminoazepino[2',1':3,4][1,4]oxazepino[5,6,7-de]quinazolin-2-yl)benzo[b]thiophene-3-carbonitrile (compound I-29)

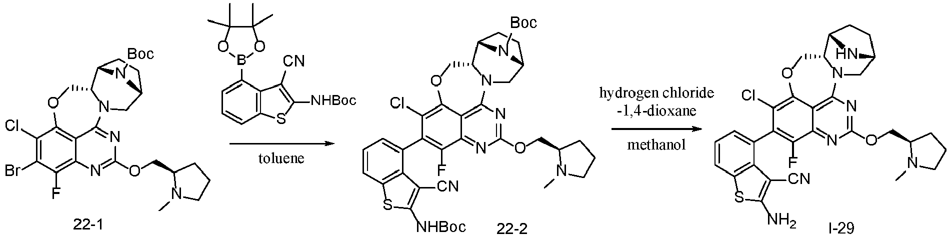

Synthesis of compound 22-1: Using the synthesis method of steps 1 to 3 in example 1, compound 22-1 was synthesized as a yellow oil with (R)-(1-methylpyrrolidin-2-yl)methanol instead of ((2*R*,7a*S*)-2-fluorohexahydro-1*H*-pyrrolizin-7a-yl)methanol in step 3 using intermediates 2 and 8 as the starting materials.

Synthesis of compound I-29: Compound I-29 was synthesized as a yellow solid with compound 22-1 and corresponding boronic acid pinacol ester using the synthesis method of compound I-13, which was a mixture of two atropisomers. m/z: [M+H]⁺ 606.2; UPLC RT = 4.206 and 4.353 minutes; ¹H NMR (400 MHz, DMSO-*d₆*): δ 9.82-9.65 (m, 2H), 7.86-7.80 (m, 3H), 7.26-7.23 (m, 1H), 7.18-7.13 (m, 1H), 4.96-4.88 (m, 1H), 4.76-4.61 (m, 4H), 4.45-4.28 (m, 3H), 3.89-3.79 (m, 1H), 3.67-3.56 (m, 1H), 3.20-3.11 (m, 1H), 2.97 (s, 3H), 2.28-1.78 (m, 8H).

### Example 31: Synthesis of 4-(5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-5-ethynyl-6-fluoronaphthalen-2-ol (compound I-30)

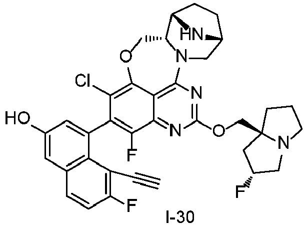

Compound I-30 was obtained as a yellow solid by reacting compound 13-1 and ((2-fluoro-6-(methoxymethoxy)-8-(4,4,5,5-tetramethyl-1,3,2-dioxaborolan-2-yl)naphthalen-1-yl)ethynyl)triisopropylsilane using the synthesis method of steps 4 to 6 in an example. m/z: [M+H]⁺ 662.2; UPLC RT = 5.228 minutes; ¹H NMR (400 MHz, CD₃OD): δ 7.87-7.84 (m, 1H), 7.36-7.18 (m, 3H), 5.51 (s, 1H), 5.21 (s, 1H), 4.80-4.66 (m, 4H), 4.41-4.33 (m, 3H), 3.94-3.89 (m, 3H), 3.58-3.49 (m, 2H), 2.65-2.60 (m, 1H), 2.38-2.36 (m, 3H), 2.22-2.15 (m, 4H), 1.39-1.30 (m, 3H).

### Example 32: Synthesis of 4-((5aS,6S,9R)-3-chloro-1-fluoro-13-(((2R,7aS)-(5,5-²H₂)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-5-ethynyl-6-fluoronaphthalen-2-ol (compound 1-31)

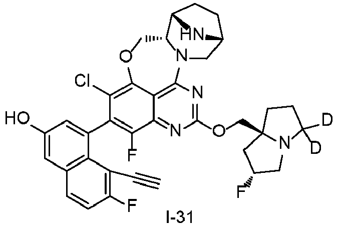

Compound I-31 was obtained as a yellow solid by reacting compound 19-1 and ((2-fluoro-6-(methoxymethoxy)-8-(4,4,5,5-tetramethyl-1,3,2-dioxaborolan-2-yl)naphthalen-1-yl)ethynyl)triisopropylsilane using the synthesis method of steps 4 to 6 in an example. m/z: [M+H]⁺ 664.2; UPLC RT = 5.225 minutes; ¹H NMR (400 MHz, CD₃OD): δ 7.87-7.84 (m, 1H), 7.36-7.18 (m, 3H), 5.51 (s, 1H), 5.24-5.20 (m, 1H), 4.76-4.66 (m, 4H), 4.41-4.33 (m, 3H), 3.94-3.89 (m, 1H), 3.58-3.50 (m, 1H), 2.61-2.60 (m, 2H), 2.40-2.34 (m, 4H), 2.22-2.15 (m, 4H), 1.39-1.30 (m, 2H).

### Example 33: Synthesis of 4-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-1H-benzo[4,5]thieno[2,3-b]pyrrol-3-amine (compound I-32)

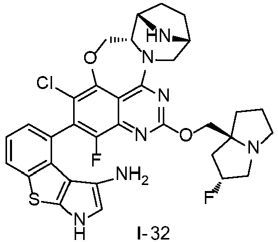

Compound I-32 was obtained as a brown solid by reacting compound 13-1 and intermediate 25 using the synthesis method of compound I-13. m/z: [M+H]⁺ 664.2.

### Example 34: Synthesis of 8-((5aS,6S,9R)-3-chloro-1-fluoro-13-(((2R,7aS)-2-fluorohexahydro-1H-pyrrolizin-7a-yl)methoxy)-5a,6,7,8,9,10-hexahydro-5H-6,9-epiminoazepino[2',1':3,4][1,4]oxazepino[5,6,7-de]quinazolin-2-yl)-1H-imidazo[1,2-a]indole-9-carbonitrile (compound I-33)

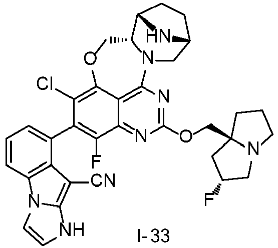

Compound I-33 was obtained as a yellow solid by reacting compound 13-1 and intermediate 26 using the synthesis method of compound I-13, which was a mixture of two atropisomers. m/z: [M+H]⁺ 657.2; HPLC RT = 16.574 and 17.147 minutes.

The structure and identification data of the intermediates of examples 1 to 34 are shown in Table 1 below:

**Table 1**

| Intermediate No. | Structure | Ms |
|---|---|---|
| 22-2 | 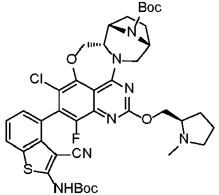 | m/z: [M+H]⁺ 806.2 |
| 21-2 | 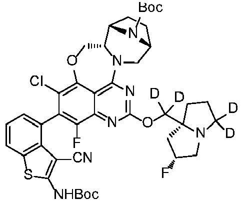 | m/z: [M+H]⁺ 854.2 |
| X-1 | 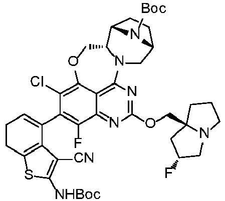 | m/z: [M+H]⁺ 852.3 |
| 18-2 | 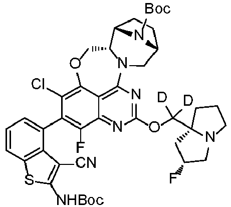 | m/z: [M+H]⁺ 852.2 |
| 19-2 | 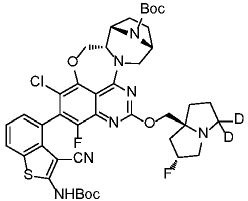 | m/z: [M+H]⁺ 852.3 |
| X-2 | 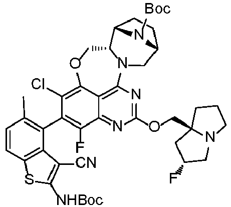 | m/z: [M+H]⁺ 864.2 |
| X-3 | 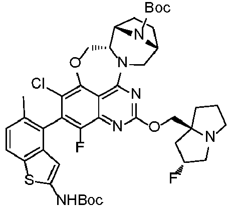 | m/z: [M+H]⁺ 839.2 |
| X-4 | 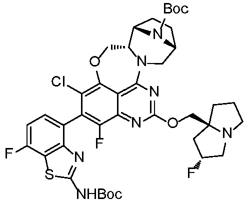 | m/z: [M+H]⁺ 844.2 |
| 16-2 | 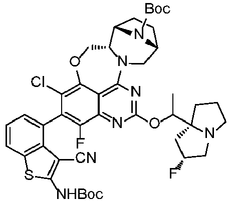 | m/z: [M+H]⁺ 864.2 |
| X-5 | 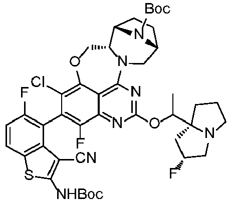 | m/z: [M+H]⁺ 882.3 |
| X-6 | 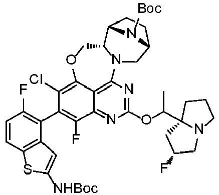 | m/z: [M+H]⁺ 857.2 |
| X-7 | 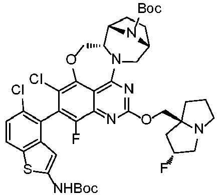 | m/z: [M+H]⁺ 859.2 |
| 15-1 | 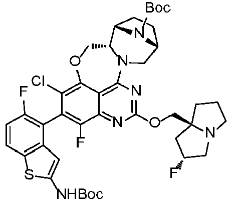 | m/z: [M+H]⁺ 843.2 |
| X-8 | 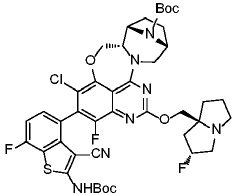 | m/z: [M+H]⁺ 868.2 |
| X-9 | 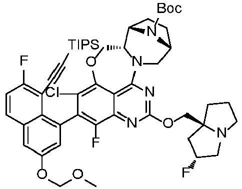 | m/z: [M-156+H]⁺ 806.2 |
| X-10 | 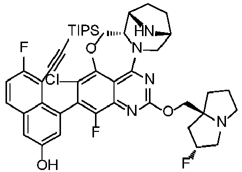 | m/z: [M-156+H]⁺ 662.2 |
| X-11 | 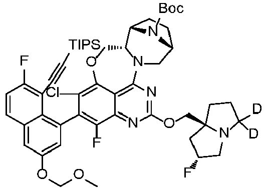 | m/z: [M-156+H]⁺ 808.2 |
| X-12 | 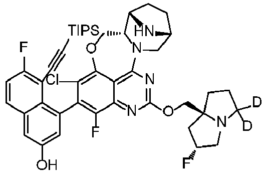 | m/z: [M-156+H]⁺ 664.2 |
| X-13 | 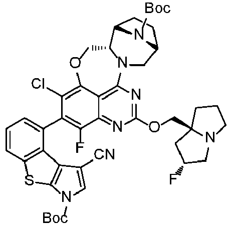 | m/z: [M+H]⁺ 874.2 |
| X-14 | 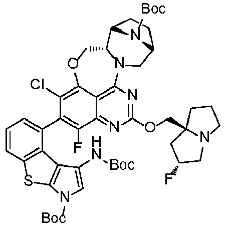 | m/z: [M+H]⁺ 964.2 |
| X-15 | 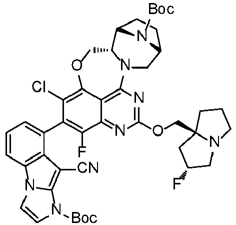 | m/z: [M+H]⁺ 857.2 |

### Biological examples:

### Example 1: Surface plasmon resonance (SPR) binding assay

The binding ability of the compound to KRAS G12D protein was detected using surface plasmon resonance technology, and NeutrAvidin protein (Thermo Scientific^{™}, 31000) was coupled to a CM5 (GE Healthcare, 29-1496-03) chip using an amino coupling kit (GE Healthcare, BR-1006-33). 1-(3-Dimethylaminopropyl)-3-ethylcarbodiimide and *N-*hydroxysuccinimide were mixed in a ratio of 1:1, activated with a flow rate of 10 µL/min for 420 seconds, then injected with 62.5 µg/mL NeutrAvidin protein diluted with acetate buffer (pH = 5.5), activated for 400 seconds, and finally blocked with 1 M ethanolamine for 420 seconds. The amount of NeutrAvidin protein coupled was approximately 10000 RU. Using Buffer A (20 mM HEPES, 100 mM NaCl, 0.02% Tween 20, 1 µM GDP, 5 mM MgClz, 0.5 mM TCEP) as the experimental buffer, KRAS G12D protein was diluted to 5 µg/mL with Buffer A and injected into the Fc2 channel with a flow rate of 10 µL/min for 200 seconds. The capture amount was 3590 to 3890 RU.

The test compound was formulated into a 10 mM concentrated stock solution with DMSO, and 50-fold diluted with Buffer A to obtain a concentrated stock solution with a final DMSO concentration of 2%, then diluted to the highest test concentration with Buffer B (20 mM HEPES, 100 mM NaCl, 0.02% Tween 20, 1 µM GDP, 5 mM MgClz, 0.5 mM TCEP, 2% DMSO), and 3-fold serially diluted to 8 concentrations with Buffer B.

A multi-cycle kinetic test method was set up using Biacore 8K (GE Healthcare) with 7 start-up cycles and 4 zero-concentration cycles, and then the compound was sequentially loaded from low to high concentrations. Each cycle consisted of an association of 90 seconds and a dissociation of 240 seconds. The solvent calibration range was 1.5% to 2.5%, with a total of 6 points. Flow rate: 50 µL/min.

Data were collected at 25°C using Buffer B as the experimental buffer. Data were analyzed using Biacore Insight Evaluation Software. After subtracting the reference channel signal, the binding curve of the sample was obtained using 0 nM as the blank concentration and analyzed by 1: 1 binding affinity and kinetic mode.

### Example 2: KRAS G12D binding assay

The binding ability of the compound to KRAS G12D protein was detected by TR-FRET method, and the inhibitory activity of the compound on KRAS G12D protein was evaluated. Human KRAS G12D His-tagged (Cisbio, 63ADK000CB27PEG) protein was 100-fold diluted with PPI Eu detection buffer (Cisbio, 61DB9RBF), then 5 µL of which was added to a 384-well plate and centrifuged at 2000 rpm for 40 seconds. A 10 mM concentrated stock solution of the test compound was prepared with dimethyl sulfoxide, 3-fold serially diluted, then diluted to make a working solution (0.5% DMSO) with PPI Eu detection buffer, added to a 384-well plate at 5 µL/well, with the control group added with 0.5% dimethyl sulfoxide and the highest concentration of the test compound at 10 µM, and centrifuged at 2000 rpm for 40 seconds. An 80 nM GDP standard working solution (Cisbio, 63ADK000CB27PEG) was prepared with PPI Eu detection buffer and added to a 384-well plate at 5 µL/well, with the final concentration of GDP standard working solution at 20 nM. The plate was centrifuged at 2000 rpm for 40 seconds and reacted at room temperature for 30 minutes. 1x6His Eu cryptate antibody and GTP-Red reagent (Cisbio, 63ADK000CB27PEG) were prepared with PPI Eu detection buffer, mixed in a volume ratio of 1:1, 5 µL of which was then added to a 384-well plate, centrifuged at 2000 rpm for 40 seconds, and incubated at room temperature for 30 minutes. The fluorescence intensity at 620 nm and 665 nm was detected by a microplate reader, and the inhibitory activity of the compound on KRAS G12D protein was obtained by fitting a four-parameter equation. The results are shown in Table 2:

**Table 2**

| Compound No. | IC₅₀ (nM) |
|---|---|
| I-1-1 | 10.97 |
| I-4 | 2.717 |
| I-5 | 25.37 |
| I-6-2 | 53.55 |
| I-7 | 6.588 |
| I-7-1 | 186.4 |
| I-7-2 | 2.444 |
| I-8-2 | 60.43 |
| I-10-2 | 75.80 |
| I-11 | 11.22 |
| I-11-2 | 5.900 |
| I-12-1 | 30.29 |
| I-12-2 | 3.006 |
| I-13 | 32.49 |
| I-14 | 8.523 |
| I-15 | 38.08 |
| I-16 | 24.04 |
| I-17 | 7.770 |
| I-17-1 | 646.0 |
| I-17-2 | 6.900 |
| I-18 | 22.00 |
| I-19-2 | 4.632 |
| I-20-2 | 1.888 |
| I-21 | 9.760 |
| I-23 | 216.9 |
| I-24-1 | 136.0 |
| I-24-2 | 1.745 |
| I-25 | 12.03 |
| I-26-2 | 10.99 |
| I-27-1 | 212.1 |
| I-27-2 | 1.402 |
| I-29 | 10.80 |

### Example 3: pERK cell level assay

In this experiment, the phosphorylation level of ERK1/2 in AGS human gastric cancer cells or ASPC-1 human pancreatic cancer cells was detected using PHOSPHO-ERK1/2 (THR202/TYR 204) (Cisibo, 64ERKPEH) kit, and the inhibitory effect of the compounds on KRAS G12D was investigated based on the phosphorylation level of ERK1/2.

On day 1, the density of AGS cell (ATCC) suspension was adjusted to 2.1 × 10⁵ cells/mL, then the suspension was spread in a 96-well plate at 95 µL/well so that there were 2 × 10⁴ cells per well, and the cell culture plate was incubated in a 37°C, 5% CO₂ incubator for overnight. On day 2, the compound was serially diluted with 100% dimethyl sulfoxide, and then further diluted with a complete culture medium (RPMI-1640 + 10% FBS + 1% PS) to make a working solution of the compound, which was added to a 96-well cell culture plate at 5 µL/well, and incubated in a 37°C, 5% CO₂ incubator for 4 hours. After the incubation was completed, the culture medium was discarded, then 1x lysis buffer was added thereto at 50 µL/well, and the plate was shaken at room temperature for 30 minutes. 16 µL of lysis buffer was added to a white 384-well shallow well plate, then 4 µL of pre-formulated antibody mixture was added thereto, and the plate was sealed with microplate sealer and incubated at room temperature for overnight. On day 3, detection was performed on a TECAN M1000 Pro microplate reader using the HTRF setting, and the IC₅₀ value of the compound was calculated. The results are shown in Table 3:

**Table 3**

| Compound No. | Aspc-1 IC₅₀ (nM) |
|---|---|
| I-7 | 1.588 |
| I-7-2 | 0.544 |
| I-11 | 1.014 |
| I-12-2 | 8.231 |
| I-14 | 1.289 |
| I-17 | 5.577 |

### Example 4: Cell proliferation assay

On day 1, AGS human gastric cancer cells, ASPC-1 human pancreatic cancer cells, or GP2D human colon cancer cells (Nanjing Cobioer Biosciences Co. Ltd.) in the logarithmic growth phase were taken, digested with an appropriate amount of trypsin, formulated into a single cell suspension using RPMI 1640 (Gibco) medium containing 10% fetal bovine serum and 1% penicillin-streptomycin, counted to adjust the cell density, inoculated in a 96-well plate at 95 µL/well so that there were 3000 cells per well, and then the cell culture plate was incubated in a 37°C, 5% CO₂ incubator for overnight. On day 2, a 10 mM concentrated stock solution of KRAS G12D inhibitor was 3-fold serially diluted with dimethyl sulfoxide solution, and then diluted with AGS complete culture medium to make a working solution, which was added to a cell culture plate at 5 µL/well so that the compound had the highest concentration of 10 µM and the lowest concentration of 0.0015 µM, and the control group was 0.5% dimethyl sulfoxide. The cell culture plate was briefly centrifuged and then incubated in a CO₂ incubator for 72 hours. On day 5, the cell culture plate was taken out, added with Cell Titer-Glo (Promega) detection reagent in each well, incubated in the dark for 10 minutes, and then luminescence was detected using a microplate reader (Tecan, Infinite M1000 Pro). Data were analyzed using Graphpad software, and curve fitting was performed with four-parameter equation and the IC₅₀ value of the inhibitor was calculated. The results are shown in Table 4:

**Table 4**

| Compound No. | Aspc-1 IC₅₀ (nM) | GP2D IC₅₀ (nM) |
|---|---|---|
| 1-7 | 5.529 | -- |
| I-7-2 | 1.699 | 0.095 |
| I-11 | 12.57 | 0.336 |
| I-14 | 6.761 | 0.124 |
| I-17 | 31.85 | 0.497 |
| I-20-2 | 6.490 | -- |
| I-21 | 16.86 | -- |
| I-24-2 | 6.662 | -- |
| I-25 | 25.76 | -- |
| I-27-2 | -- | 0.133 |

### Example 5: In vivo pharmacodynamic experiment of subcutaneous xenograft tumor model in mice of GP2D human colon cancer cells

Cell culture: GP2D human colon cancer cells were maintained in monolayer culture in DMEM culture medium containing 10% fetal bovine serum in a constant temperature incubator containing 5% CO₂ at 37°C. Tumor cells were passaged twice a week. Cells in the exponential growth phase were harvested and counted for inoculation.

Experimental animals: BALB/c nude mice, 6 to 8 weeks, 18 to 22 g, purchased from Jiangsu GemPharmatech Co. Ltd.

For Vehicle, Ref.A, and I-7-2, a total of 5 experimental groups are set up as shown in Table 5 below:

**Table 5**

| **Group** | **Number of mice** | **Test compound** | **Dosage** | **Route of administration** | **Administration plan** |
|---|---|---|---|---|---|
| 1 | 6 | Vehicle | -- | i.p. | Twice a day |
| 2 | 6 | Ref A | 10 mg/kg | i.p. | Twice a day |
| 3 | 6 | Ref A | 3 mg/kg | i.p. | Twice a day |
| 4 | 6 | I-7-2 | 10 mg/kg | i.p. | Twice a day |
| 5 | 6 | I-7-2 | 3 mg/kg | i.p. | Twice a day |

| | | | | | |
|---|---|---|---|---|---|
| Note: i.p.: intraperitoneal administration. | | | | | |

Experimental method: GP2D cell line (5.0 × 10^{6+M} cells/mouse) was inoculated subcutaneously on the right back of experimental mice. The inoculation volume of each mouse was 0.1 mL. The growth of the tumor was observed regularly. When the tumor grew to about 150 mm³, the mice were randomly grouped according to the tumor size and body weight, and the drug was administrated according to the administration plan shown in Table 1. The body weight and tumor size were measured twice a week throughout the experiment.

Tumor size calculation formula: tumor volume (mm³) = 0.5 × (tumor long diameter × tumor short diameter²).

The experimental results are shown in Table 6:

**Table 6**

| **Group** | **Tumor volume (11 days, mm³)** | **Tumor volume (21 days, mm³)** | **Tumor regression rate (%)** | **T/C (%)** | **P value** |
|---|---|---|---|---|---|
| 1 | 140.58 ± 15.83 | 453.79 ± 48.78 | N/A | N/A | N/A |
| 2 | 139.90 ± 20.43 | 79.77 ± 13.97 | -43.0 | 17.6 | 0.0000 |
| 3 | 139.25 ± 17.31 | 137.35 ± 18.79 | -1.4 | 30.3 | 0.0001 |
| 4 | 139.69 ± 17.49 | 70.19 ± 11.89 | -49.8 | 15.5 | 0.0000 |
| 5 | 140.32 ± 19.74 | 80.27 ± 11.38 | -39.9 | 18.6 | 0.0000 |

| | | | | | |
|---|---|---|---|---|---|
| Note: Ref.A is MRTX1133 (CAS No.: 2621928-55-8). | | | | | |

## Claims

1. A compound of formula (I), a stereoisomer thereof, a stable deuterated derivative thereof, or a pharmaceutically acceptable salt thereof; 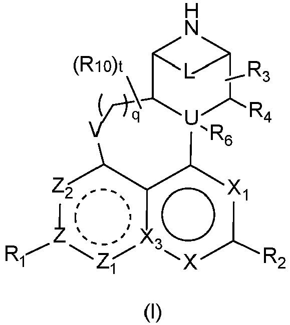 wherein V is -O-;
Z is C; Z₁ and Z₂ are each independently CR₈ or N;
X is N; X₁ is N; X₃ is C;
R₁ is C₆₋₁₂ aryl or 5- to 12-membered heteroaryl; the R₁ is unsubstituted or selectively substituted at any position by one or more than one R₉;
R₂ is hydrogen, -R_{A}, -NR_{A}R_{B}, -OR_{A}, or -SR_{A};
R₃ and R₄ are each independently hydrogen;
U is N and R₆ is absent;
L is -CH₂CH₂-;
each R₈ is independently hydrogen, halogen, cyano, amino, hydroxyl, C₁₋₆ alkyl, C₁₋₆ alkoxy, halo-C₁₋₆ alkyl, or halo-C₁₋₆ alkoxy;
each R₉ is independently hydrogen, hydroxyl, halogen, cyano, amino, C₁₋₆ alkyl, C₂₋₆ alkenyl, C₂₋₆ alkynyl, C₃₋₈ cycloalkyl, 3- to 8-membered heterocycloalkyl, C₆₋₁₀ aryl, 5- to 10-membered heteroaryl, -N(R')₂, -OR', -SR', -NHC(O)R", -C(O)R", -C(O)N(R")₂, -OC(O)R", - OC(O)N(R")₂, or -B(OR")₂; in R₉, the C₁₋₆ alkyl, C₂₋₆ alkenyl, C₂₋₆ alkynyl, or C₃₋₈ cycloalkyl is unsubstituted or selectively substituted at any position by one or more than one substituent selected from halogen, cyano, amino, C₁₋₆ alkyl, C₃₋₈ cycloalkyl, 3- to 8-membered heterocycloalkyl, C₆₋₁₀ aryl, 5- to 10-membered heteroaryl, -N(R')₂, -OR', -SR', -NHC(O)R", - C(O)R", -C(O)N(R")₂, -OC(O)R", -OC(O)N(R")₂, and -B(OR")₂;
each R₁₀ is independently hydrogen, halogen, C₁₋₆ alkyl, or oxo;
R_{A} is hydrogen, C₁₋₆ alkyl, C₂₋₆ alkenyl, C₂₋₆ alkynyl, C₆₋₁₀ aryl, 5- to 10-membered heteroaryl, C₃₋₁₀ cycloalkyl, 3- to 10-membered heterocycloalkyl, C₃₋₁₀ cycloalkyl-C₁₋₆ alkyl, 3- to 10-membered heterocycloalkyl-C₁₋₆ alkyl, C₆₋₁₀ aryl-C₁₋₆ alkyl, or 5- to 10-membered heteroaryl-C₁₋₆ alkyl; the R_{A} is unsubstituted or selectively substituted at any position by one or more than one R_{c};
R_{B} is hydrogen, cyano, hydroxyl, or C₁₋₆ alkyl;
R_{A} and R_{B} are independent substituents, or R_{A} and R_{B} are attached to each other to form a 4- to 8-membered heterocycloalkyl group; the heterocycloalkyl is unsubstituted or selectively substituted at any position by one or more than one substituent selected from hydroxyl, cyano, amino, oxo, halogen, C₁₋₆ alkyl, halo-C₁₋₆ alkyl, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, 5- to 10-membered heteroaryl-C₁₋₄ alkyl, -C(O)R", -(CH₂)ₙOR", and -(CH₂)ₙN(R")₂;
R_{c} is hydroxyl, cyano, amino, oxo, halogen, C₁₋₆ alkyl, C₁₋₆ alkylene, halo-C₁₋₆ alkyl, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, 5- to 10-membered heteroaryl-C₁₋₄ alkyl, -(CH₂)ₙOR', -(CH₂)ₙN(R')₂, -(CH₂)ₙ-N(CN)R', -(CH₂)ₙ-C(O)R', -(CH₂)ₙ-C(O)N(R')₂, -(CH₂)ₙ-S(O)₂N(R')₂, -(CH₂)ₙ-NR"C(O)R', -(CH₂)ₙ-NR"S(O)₂R', -(CH₂)ₙ-N(NH)N(R')₂, -(CH₂)ₙ-NR"C(O)N(R')₂, -(CH₂)ₙ-NR"C(O)OR', -(CH₂)ₙ-OC(O)R', - (CH₂)ₙ-OC(O)N(R')₂, or -(CH₂)ₙ-B(OR")₂; in R_{c}, the C₁₋₆ alkylene, C₃₋₈ cycloalkyl, 3- to 8-membered heterocycloalkyl, C₆₋₁₀ aryl, 5- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, or 5- to 10-membered heteroaryl-C₁₋₄ alkyl is unsubstituted or selectively substituted at any position by one or more than one substituent selected from halogen, hydroxyl, amino, cyano, C₁₋₆ alkoxy, C₁₋₆ alkylamino, and C₁₋₆ alkyl;
each R' is independently hydrogen, C₁₋₄ alkyl, halo-C₁₋₄ alkyl, hydroxy-C₁₋₄ alkyl, cyano-C₁₋₄ alkyl, C₁₋₄ alkoxy-C₁₋₄ alkyl, amino-C₁₋₄ alkyl, C₁₋₆ alkylamino-C₁₋₄ alkyl, C₃₋₈ cycloalkyl, 3- to 8-membered heterocycloalkyl, C₆₋₁₀ aryl, 6- to 10-membered heteroaryl, C₃₋₈ cycloalkyl-C₁₋₄ alkyl, 3- to 8-membered heterocycloalkyl-C₁₋₄ alkyl, C₆₋₁₀ aryl-C₁₋₄ alkyl, or 6- to 10-membered heteroaryl-C₁₋₄ alkyl;
each R" is independently hydrogen or C₁₋₆ alkyl;
n is 0, 1, 2, 3, or 4;
q is 0, 1, 2, or 3;
t is 0, 1, 2, or 3.

2. The compound of formula (I), the stereoisomer thereof, the stable deuterated derivative thereof, or the pharmaceutically acceptable salt thereof according to claim 1, wherein Z is C; Z₁ is CR₈; Z₂ is N; or Z is C; Z₁ is CR₈; Z₂ is CR₈;
and/or, each R₈ is independently hydrogen or halogen.

3. The compound of formula (I), the stereoisomer thereof, the stable deuterated derivative thereof, or the pharmaceutically acceptable salt thereof according to claim 1, wherein R₂ is hydrogen or -OR_{A};
and/or, R_{A} is 3- to 10-membered heterocycloalkyl-C₁₋₆ alkyl; the R_{A} is unsubstituted or selectively substituted at any position by 1 to 5 R_{c};
and/or, R_{c} is deuterium, hydroxyl, cyano, amino, oxo, halogen, C₁₋₆ alkyl, halo-C₁₋₆ alkyl, phenyl, -(CH₂)ₙOR', or -(CH₂)ₙN(R')₂;
and/or, each R' is independently hydrogen, C₁₋₄ alkyl, or halo-C₁₋₄ alkyl.

4. The compound of formula (I), the stereoisomer thereof, the stable deuterated derivative thereof, or the pharmaceutically acceptable salt thereof according to claim 3, wherein R_{A} is 3-to 10-membered heterocycloalkyl-C₁₋₆ alkyl; the R_{A} is unsubstituted or selectively substituted at any position by 1 to 3 R_{c};
and/or, R_{c} is hydroxyl, cyano, amino, oxo, halogen, C₁₋₆ alkyl, halo-C₁₋₆ alkyl, phenyl, - (CH₂)ₙOR', or -(CH₂)ₙN(R')₂.

5. The compound of formula (I), the stereoisomer thereof, the stable deuterated derivative thereof, or the pharmaceutically acceptable salt thereof according to claim 3, wherein R₂ is - OR_{A}; R_{A} is 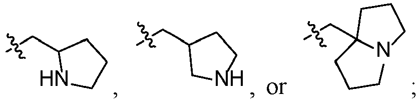 the R_{A} is unsubstituted or selectively substituted at any position by 1 to 5 R_{c}.

6. The compound of formula (I), the stereoisomer thereof, the stable deuterated derivative thereof, or the pharmaceutically acceptable salt thereof according to claim 3, wherein R₂ is - OR_{A}; R_{A} is 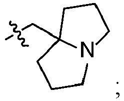 the R_{A} is unsubstituted or selectively substituted at any position by 1 to 5 fluorine, deuterium, and methyl.

7. The compound of formula (I), the stereoisomer thereof, the stable deuterated derivative thereof, or the pharmaceutically acceptable salt thereof according to claim 1, wherein R₁ is phenyl, naphthyl, quinolinyl, isoquinolinyl, benzothienyl, benzothiazolyl, 1*H*-indolyl, 1*H-*indazolyl, pyrazolo[1,5-*a*]pyridyl, or imidazo[1,2-*a*]pyridyl; the R₁ is unsubstituted or selectively substituted at any position by 1 to 3 R₉;
and/or, R₉ is hydrogen, hydroxyl, halogen, cyano, amino, C₁₋₆ alkyl, C₂₋₆ alkenyl, C₂₋₆ alkynyl, C₃₋₆ cycloalkyl, 3- to 6-membered heterocycloalkyl, -N(R')₂, -OR', or -SR'; in R₉, the C₁₋₆ alkyl or C₃₋₆ cycloalkyl is unsubstituted or selectively substituted at any position by 1 to 3 substituents selected from halogen, hydroxyl, cyano, and amino.

8. The compound of formula (I), the stereoisomer thereof, the stable deuterated derivative thereof, or the pharmaceutically acceptable salt thereof according to claim 1, wherein R₁ has any one of the following structures:

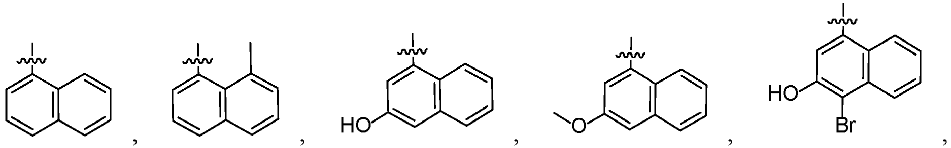

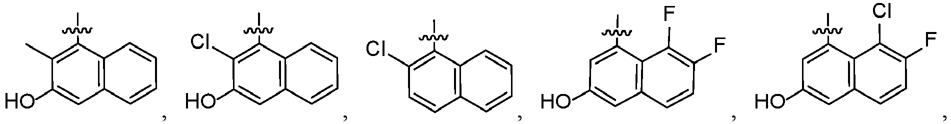

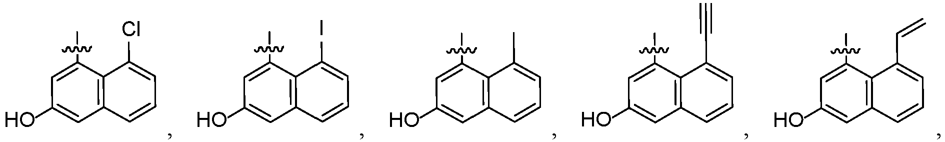

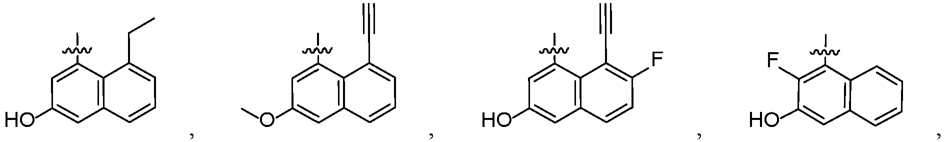

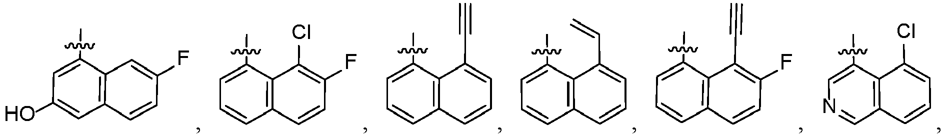

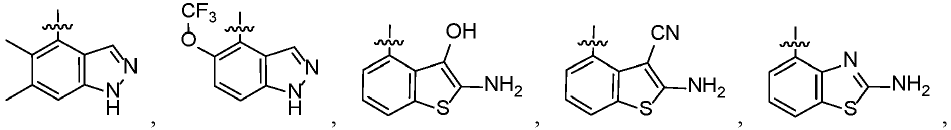

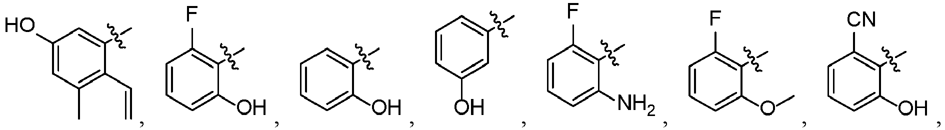

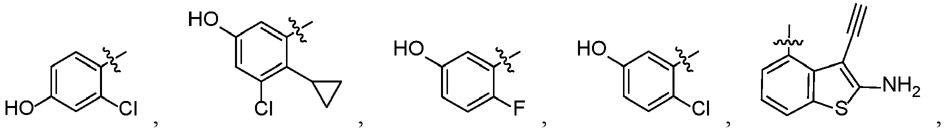

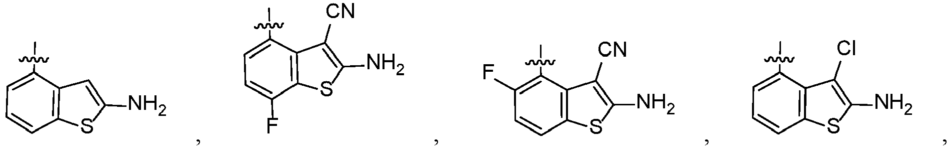

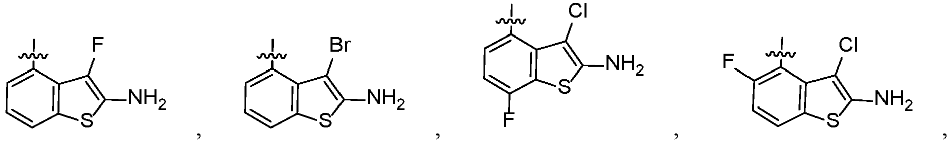

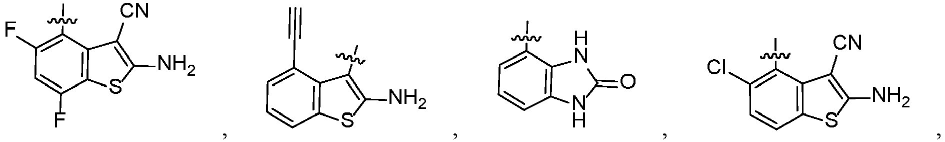

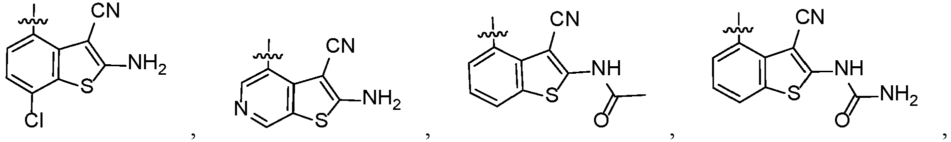

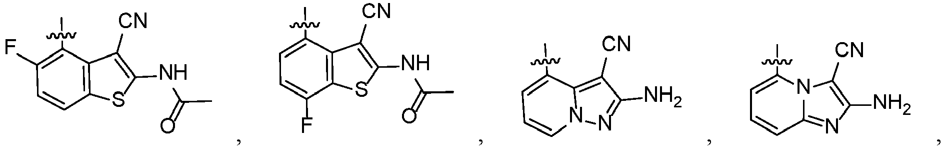

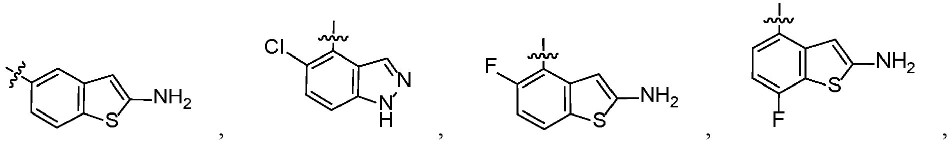

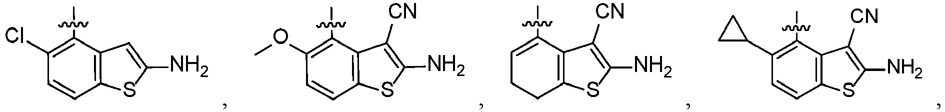

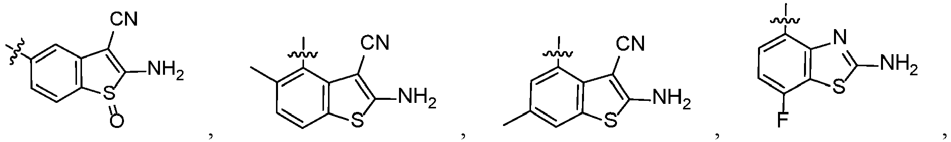

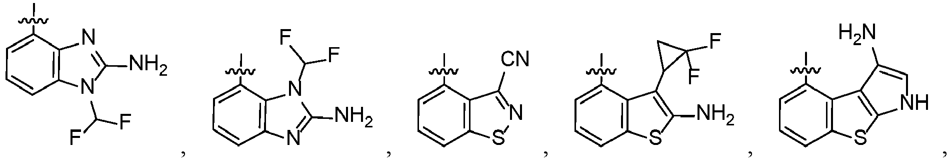

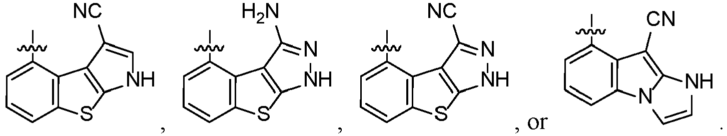

9. The compound of formula (I), the stereoisomer thereof, the stable deuterated derivative thereof, or the pharmaceutically acceptable salt thereof according to claim 1, wherein R₂ has any one of the following structures:

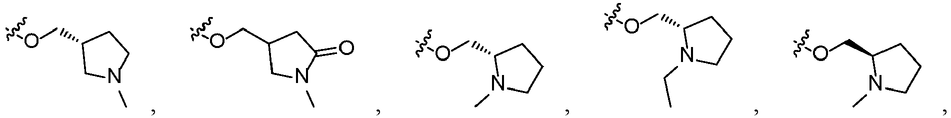

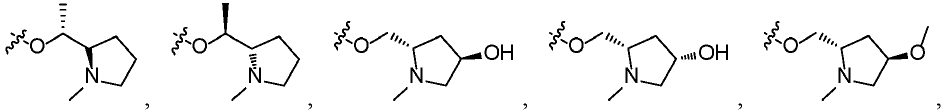

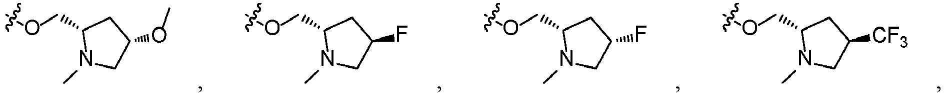

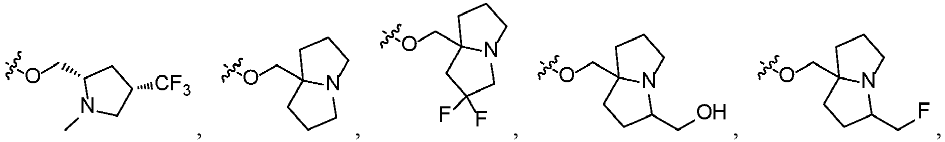

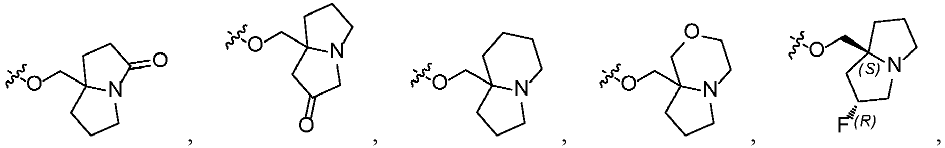

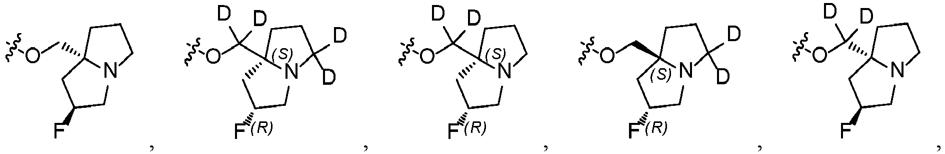

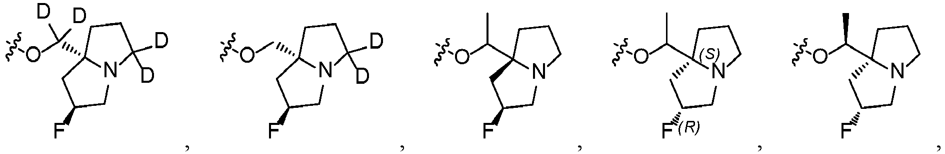

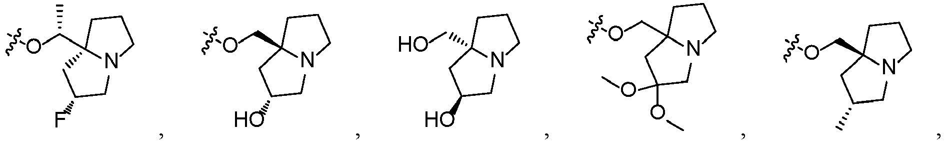

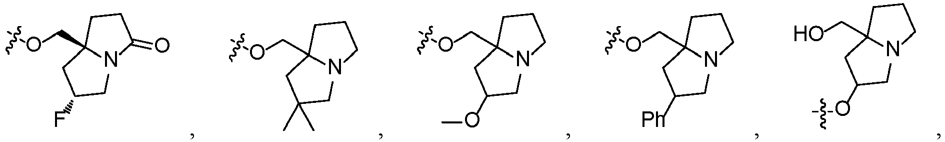

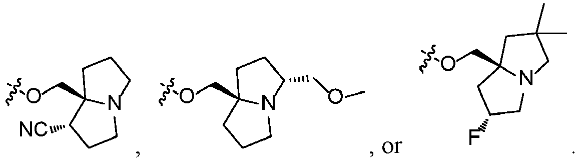

10. The compound of formula (I), the stereoisomer thereof, the stable deuterated derivative thereof, or the pharmaceutically acceptable salt thereof according to any one of claims 1 to 9, which is a compound of formula (II), a stereoisomer thereof, a stable deuterated derivative thereof, or a pharmaceutically acceptable salt thereof; 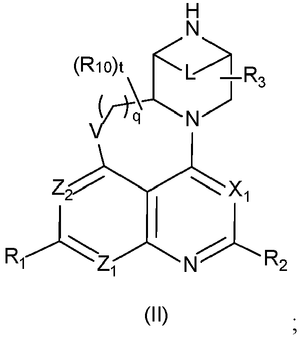 wherein
V is -O-; q is 1; L is -CH₂CH₂-; X₁ is N; t is 0; R₃ is H;
R₁, R₂, Z₁, and Z₂ are as defined in any one of claims 1 to 9.

11. The compound of formula (I), the stereoisomer thereof, the stable deuterated derivative thereof, or the pharmaceutically acceptable salt thereof according to claim 10, wherein Z₁ and Z₂ are each independently CR₈; each R₈ is independently fluorine or chlorine.

12. The compound of formula (I), the stereoisomer thereof, the stable deuterated derivative thereof, or the pharmaceutically acceptable salt thereof according to claim 10, wherein Z₁ is CR₈; Z₂ is N; R₈ is fluorine or chlorine.

13. The compound of formula (I), the stereoisomer thereof, the stable deuterated derivative thereof, or the pharmaceutically acceptable salt thereof according to claim 10, wherein R₂ is

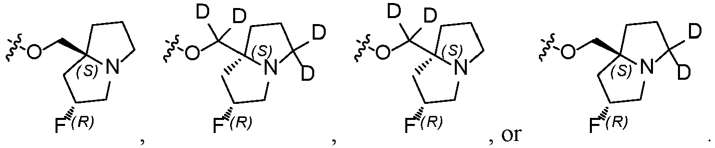

14. The compound of formula (I), the stereoisomer thereof, the stable deuterated derivative thereof, or the pharmaceutically acceptable salt thereof according to claim 1 has any one of the following structures: 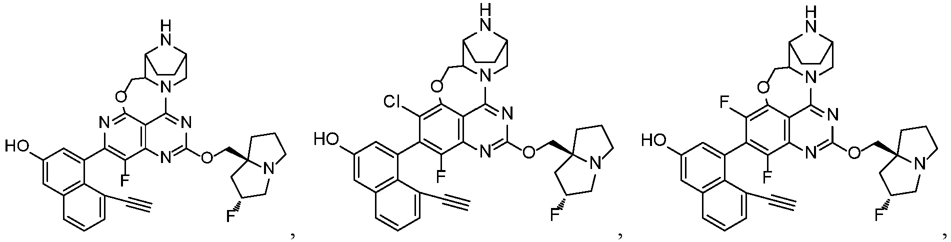 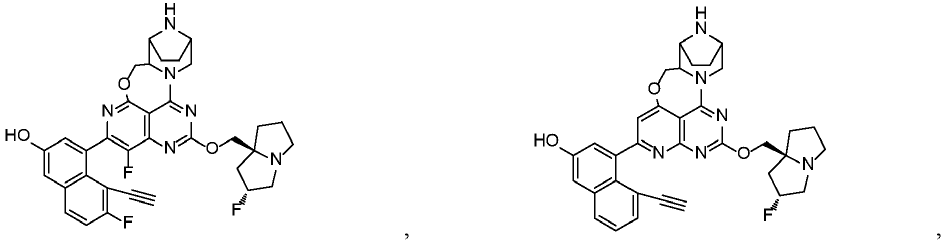 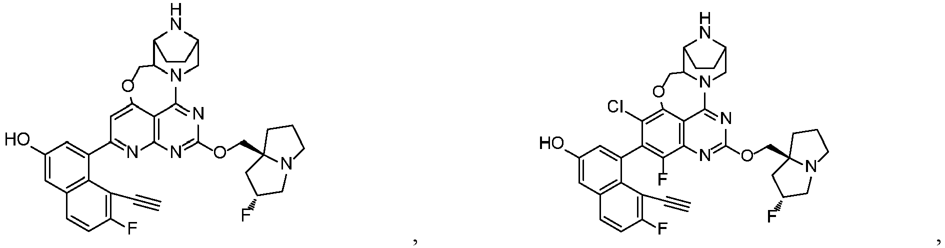 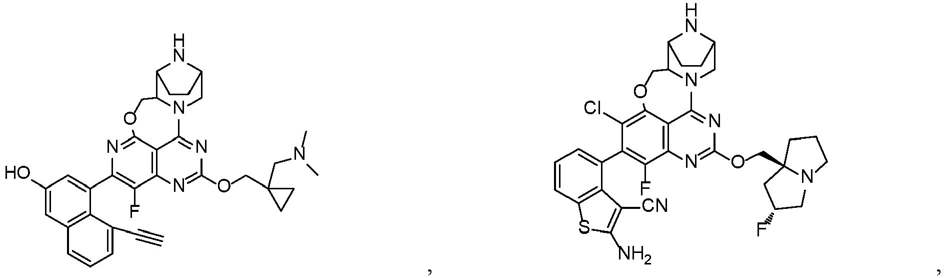 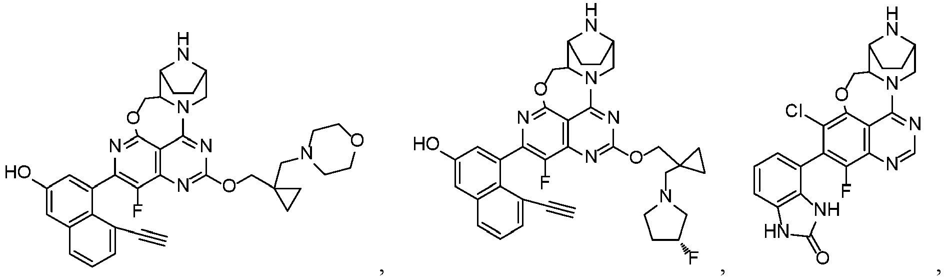 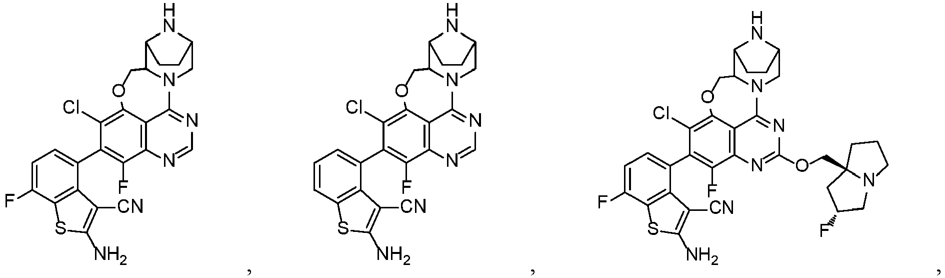 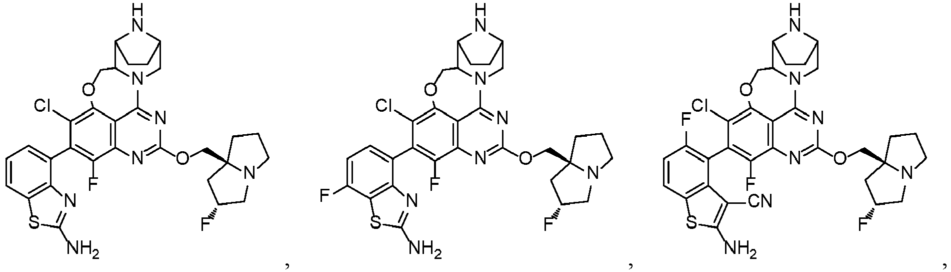 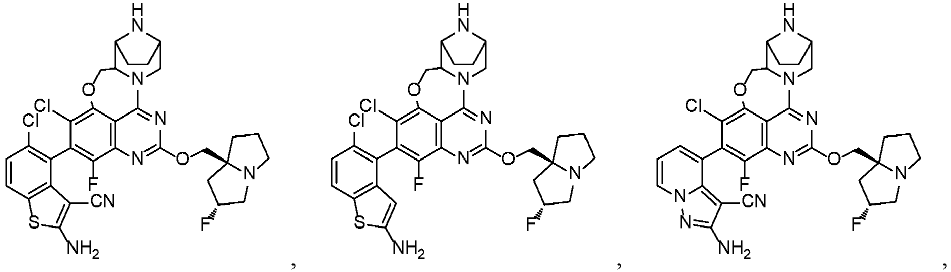 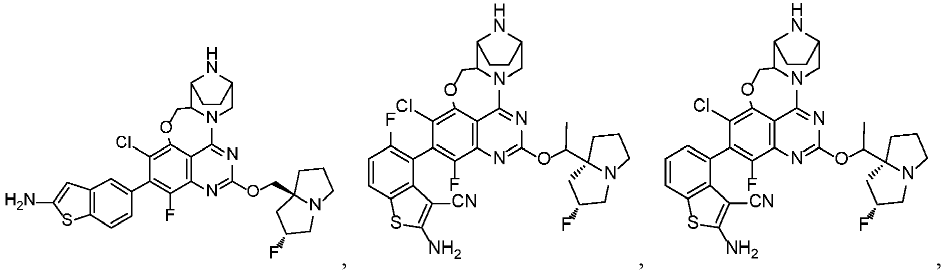 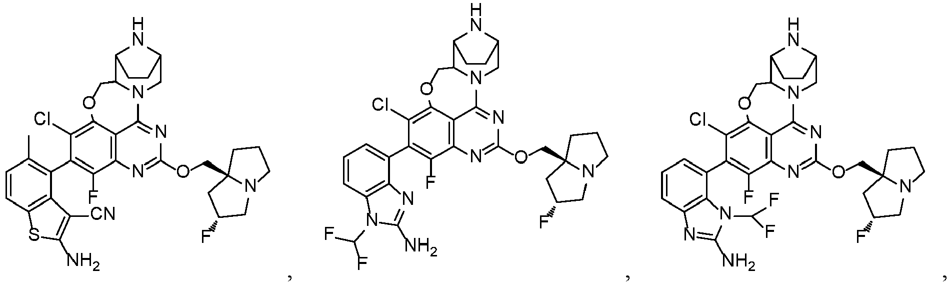 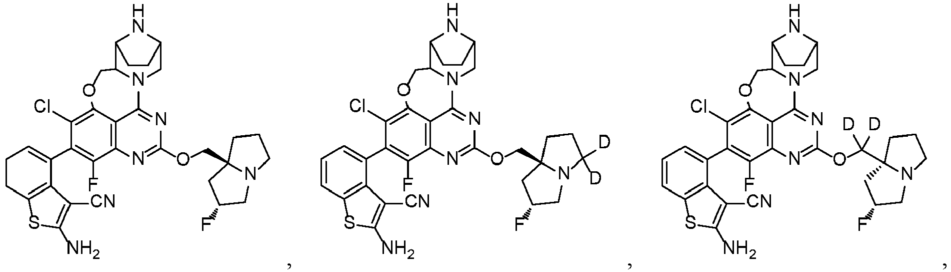 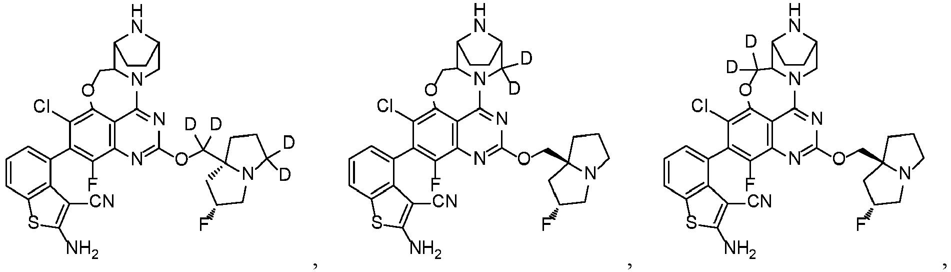 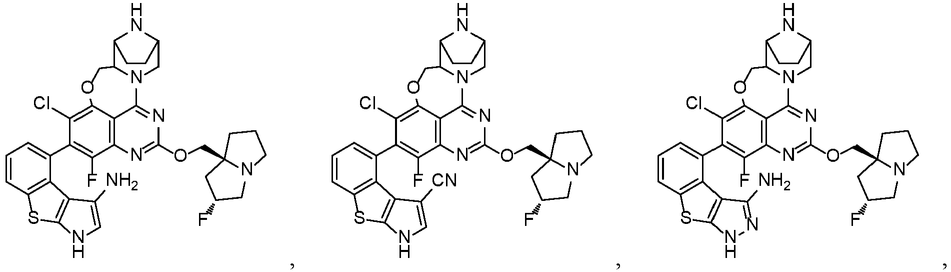 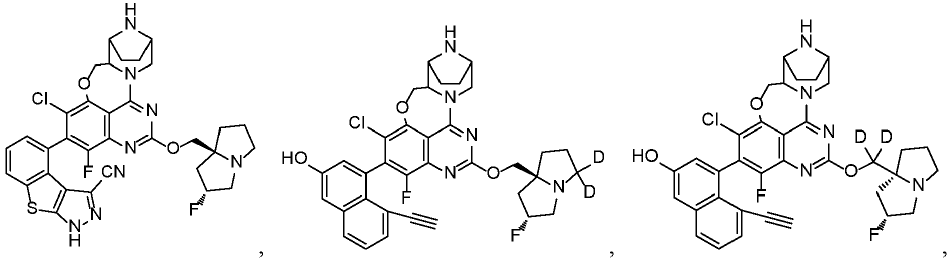 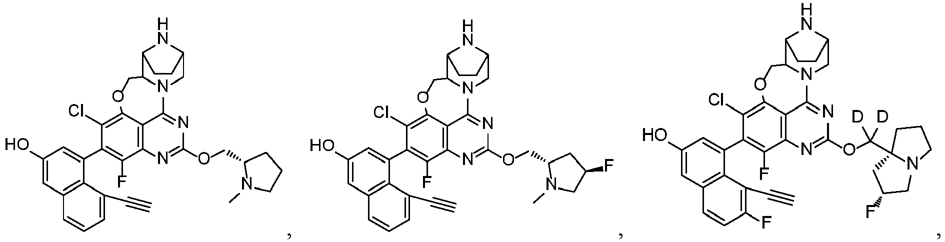 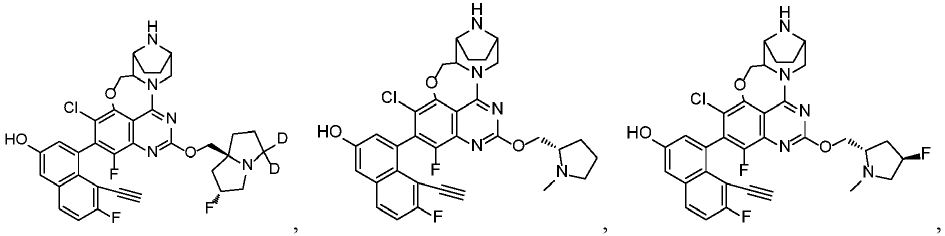 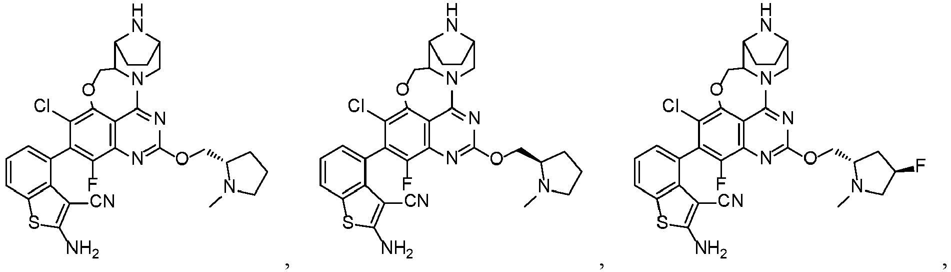 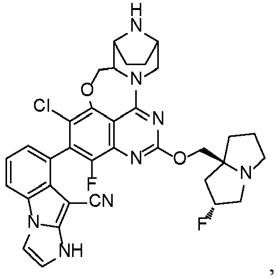 or a pharmaceutically acceptable salt.

15. The compound of formula (I), the stereoisomer thereof, the stable deuterated derivative thereof, or the pharmaceutically acceptable salt thereof according to claim 1 has any one of the following structures: 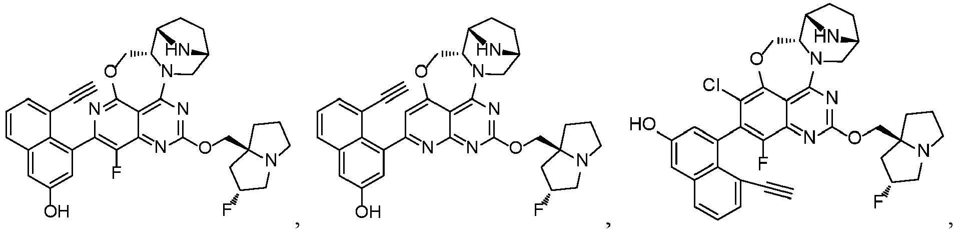 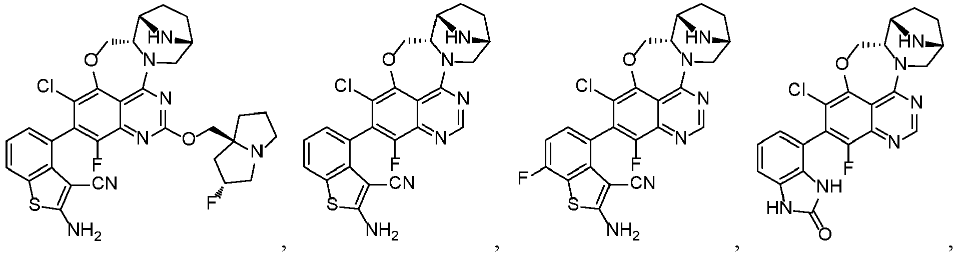 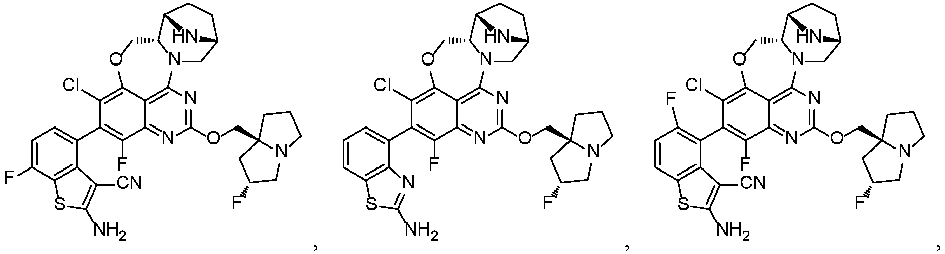 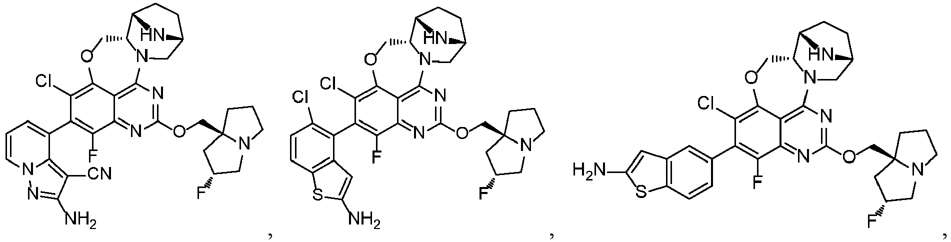 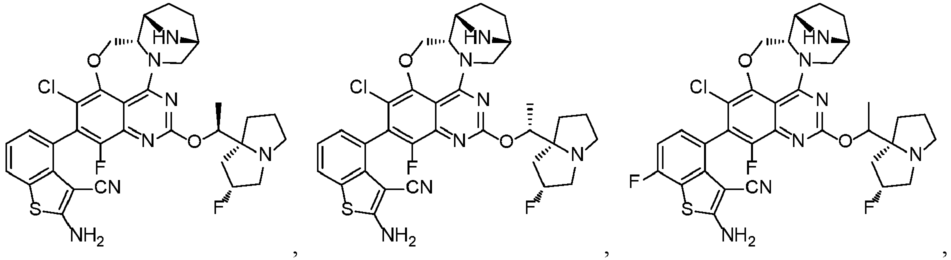 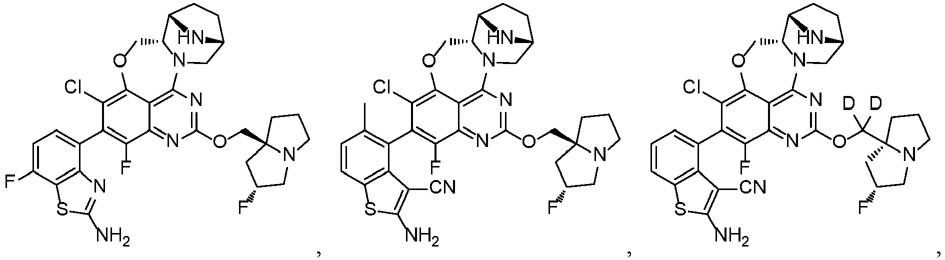 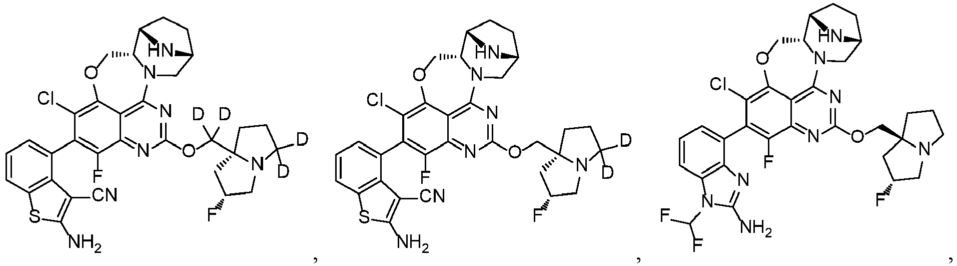 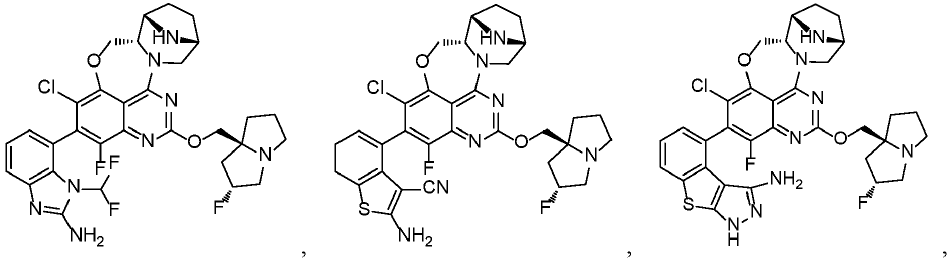 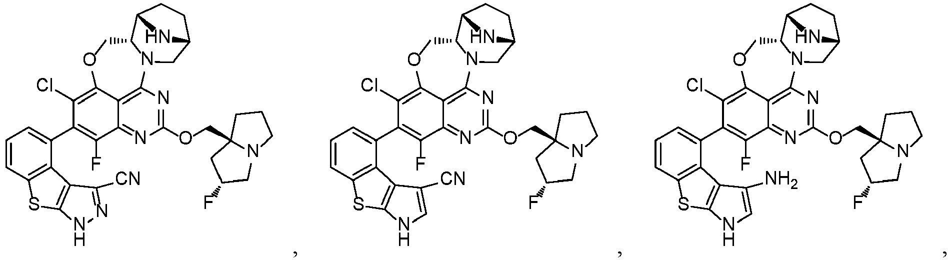 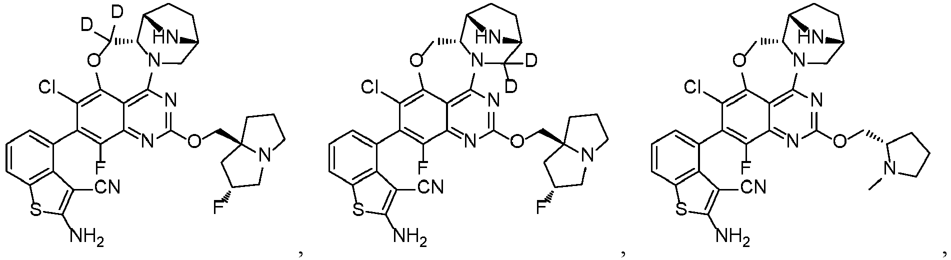 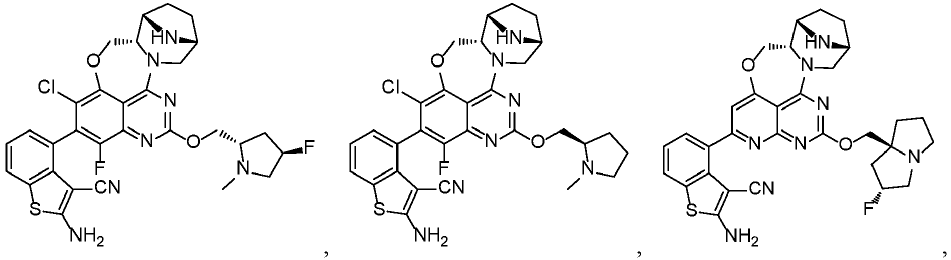 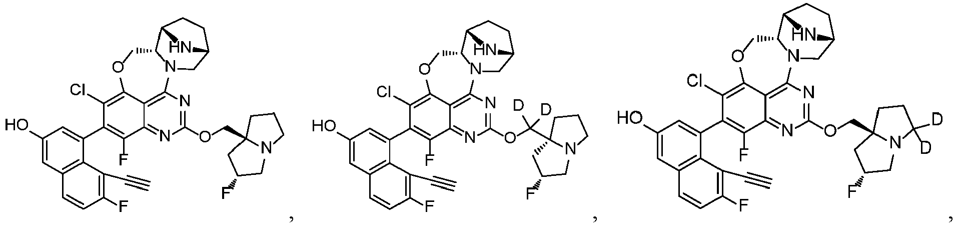 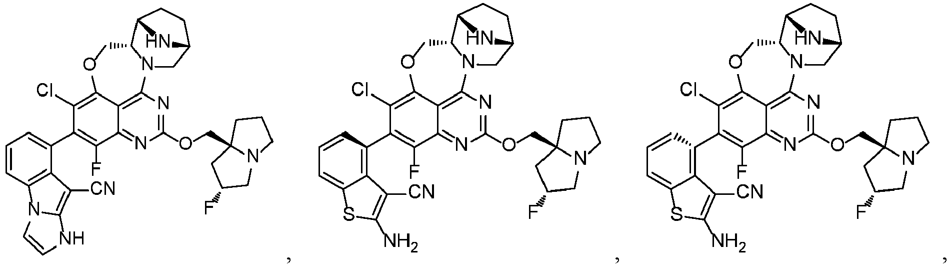 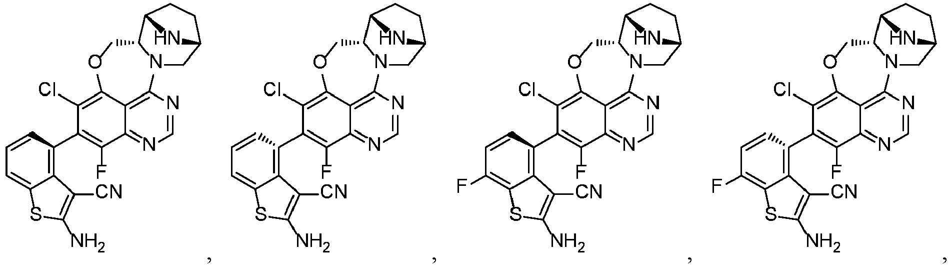 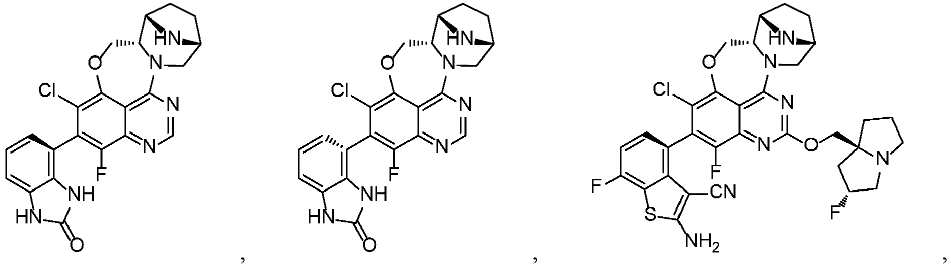 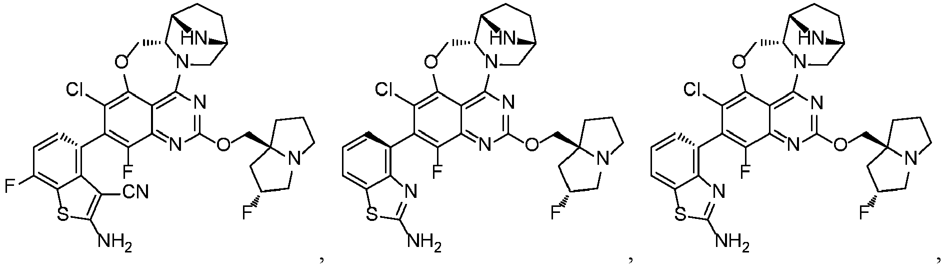 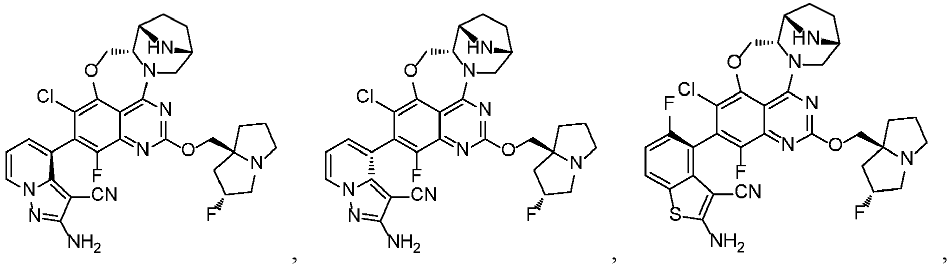 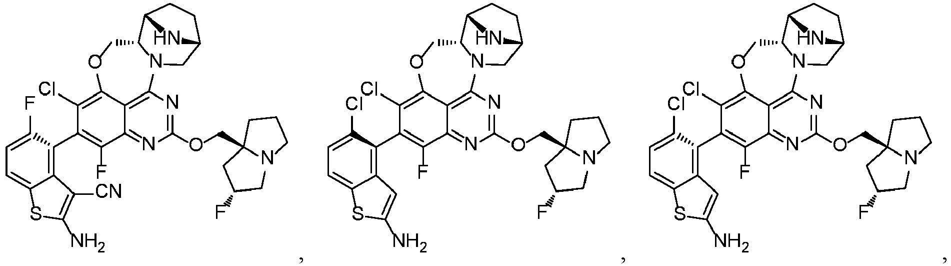 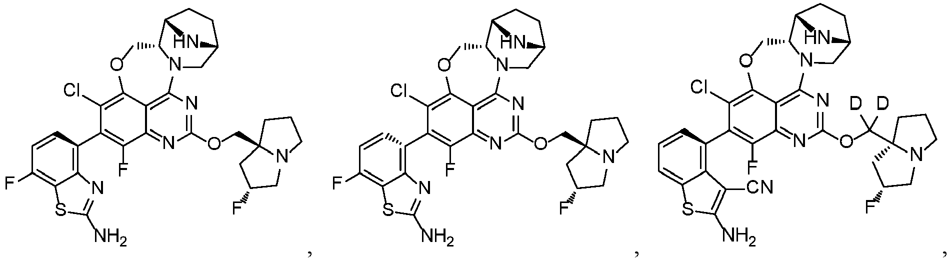 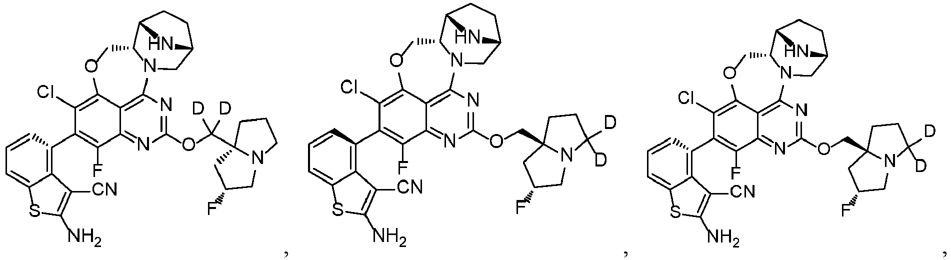 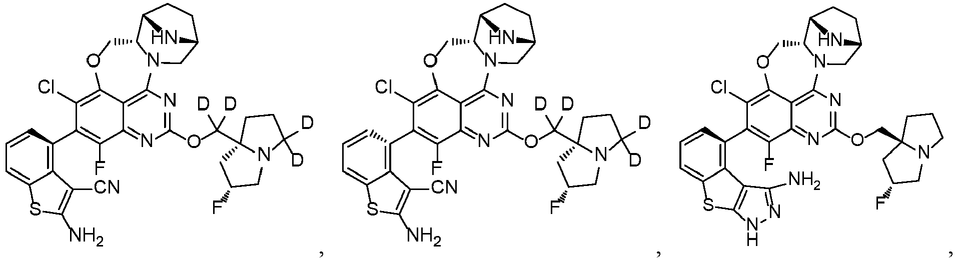 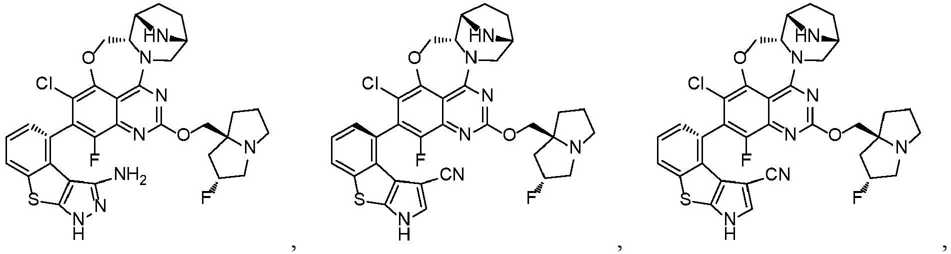 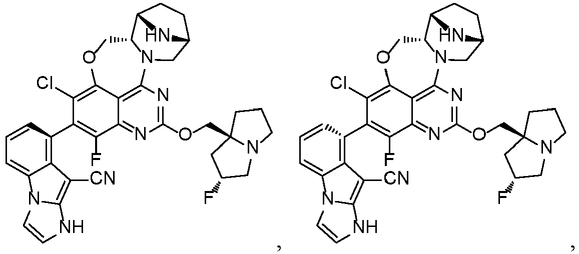 or a pharmaceutically acceptable salt.

16. The compound of formula (I), the stereoisomer thereof, the stable deuterated derivative thereof, or the pharmaceutically acceptable salt thereof according to claim 1 has any one of the following structures: 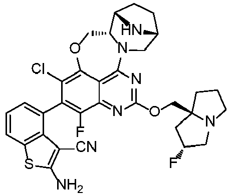 atropisomer 1, HPLC retention time: 16.724 minutes; 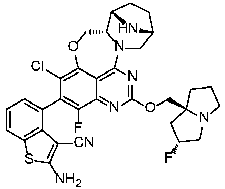 atropisomer 2, HPLC retention time: 17.162 minutes; 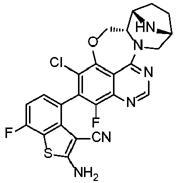 atropisomer 1, HPLC retention time: 16.118 minutes; 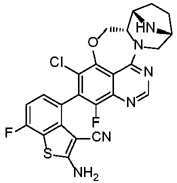 atropisomer 2, HPLC retention time: 16.489 minutes; 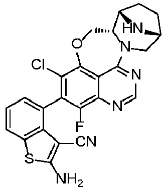 atropisomer 1, HPLC retention time: 15.584 minutes; 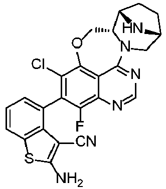 atropisomer 2, HPLC retention time: 15.966 minutes; 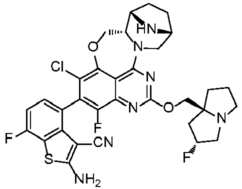 atropisomer 1, HPLC retention time: 17.403 minutes; 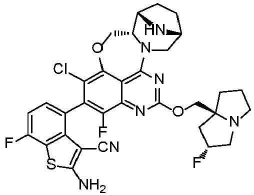 atropisomer 2, HPLC retention time: 18.023 minutes; 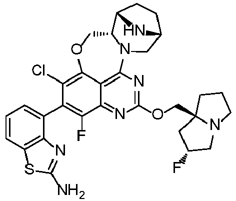 atropisomer 1, HPLC retention time: 16.558 minutes; 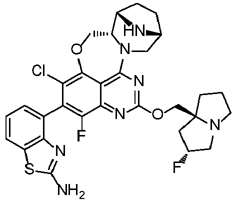 atropisomer 2, HPLC retention time: 17.076 minutes; 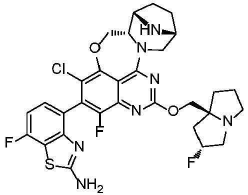 atropisomer 1, HPLC retention time: 17.152 minutes; 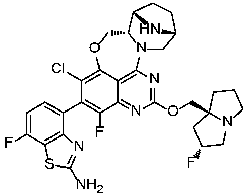 atropisomer 2, HPLC retention time: 17.710 minutes; 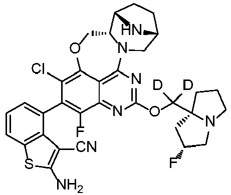 atropisomer 1, HPLC retention time: 16.821 minutes; 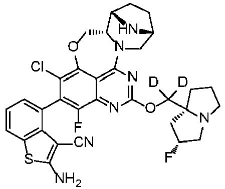 atropisomer 2, HPLC retention time: 17.354 minutes; 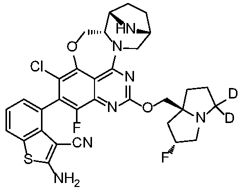 atropisomer 1, HPLC retention time: 16.793 minutes; 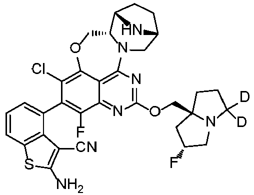 atropisomer 2, HPLC retention time: 17.322 minutes; 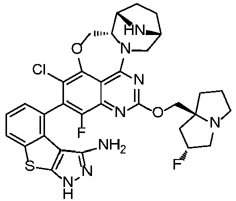 atropisomer 1, HPLC retention time: 15.452 minutes; 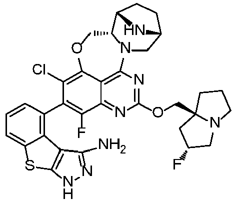 atropisomer 2, HPLC retention time: 15.838 minutes; 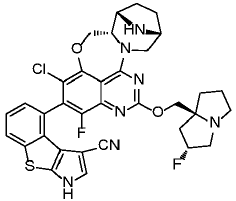 atropisomer 1, HPLC retention time: 17.687 minutes; 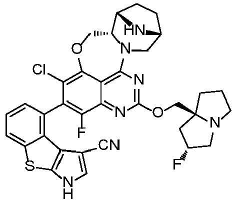 atropisomer 2, HPLC retention time: 18.251 minutes; 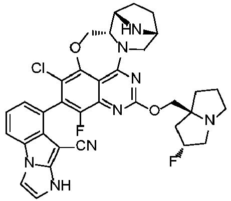 atropisomer 1, HPLC retention time: 16.574 minutes; 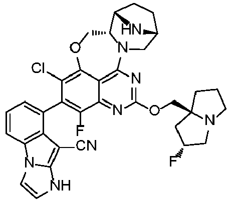 atropisomer 2, HPLC retention time: 17.147 minutes;
wherein the HPLC analysis method is as follows: chromatographic column: Welch Xtimate C18 4.6 mm * 150 mm, 5 µm; mobile phase A: acetonitrile, mobile phase B: 10 mM potassium dihydrogen phosphate buffer, with the pH adjusted to 8.0 with ammonia water; gradient elution: mobile phase B holds at 90% for 5 minutes, B from 90% to 70%, elution time: 5 minutes, B from 70% to 40%, elution time: 4 minutes, B from 40% to 15%, elution time: 12 minutes, B from 15% to 90%, elution time: 2 minutes, B holds at 90% for 2 minutes; detection wavelength: 214 and/or 262 nm; column temperature: 35°C; flow rate: 1 mL/min.

17. A method for preparing the compound of formula (I), the stereoisomer thereof, the stable deuterated derivative thereof, or the pharmaceutically acceptable salt thereof according to claim 1, which is any one of the following methods:
Method 1: 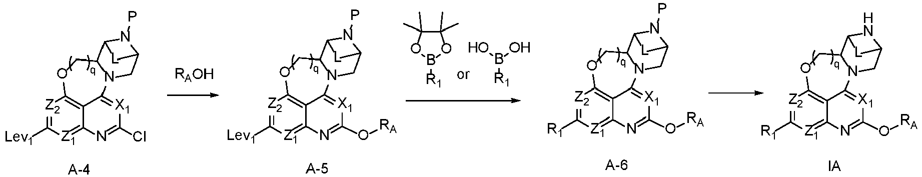
Method 2: 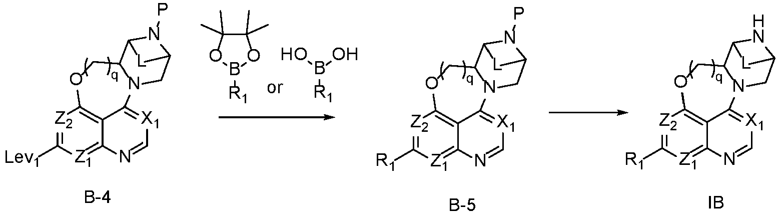 wherein Lev₁ is halogen (preferably chlorine or bromine); P is an amino protecting group; q, Z₁, Z₂, X₁, L, R₁, and R_{A} are as defined in claim 1.

18. Any one of the following compounds: 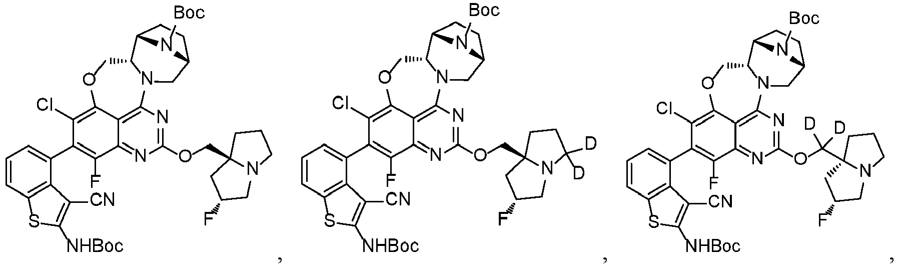 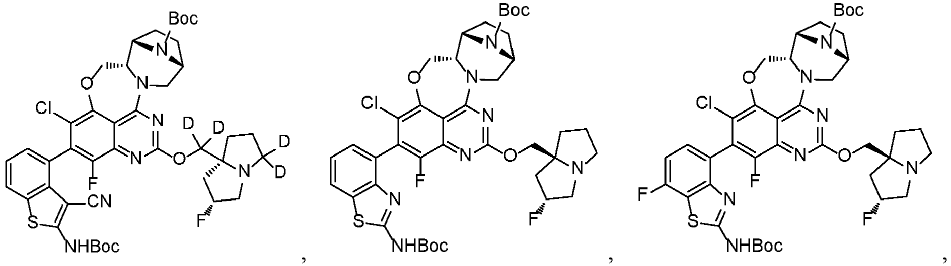 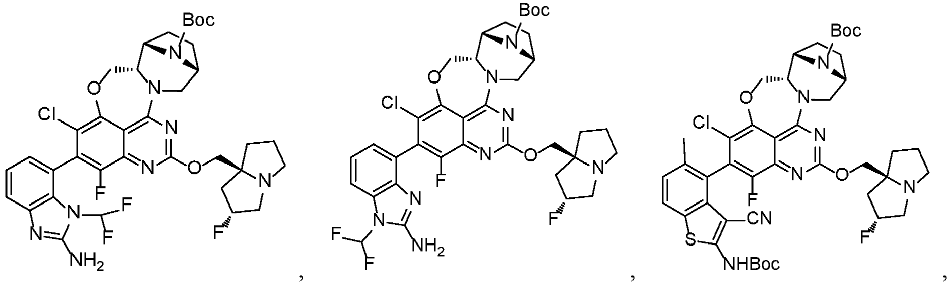 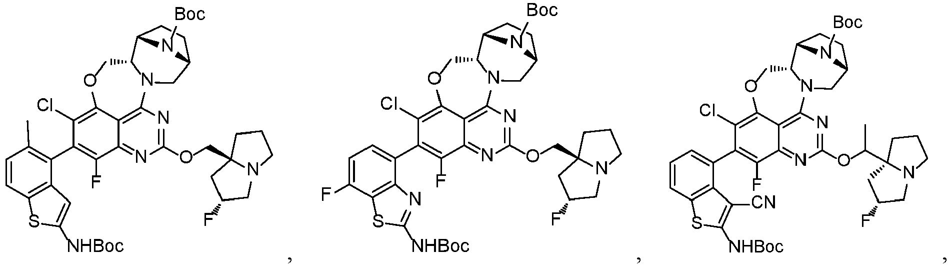 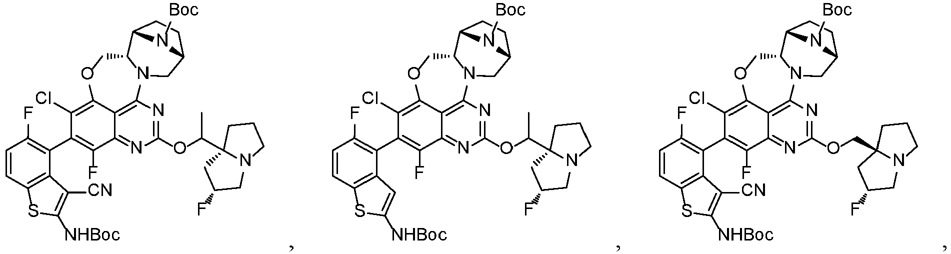 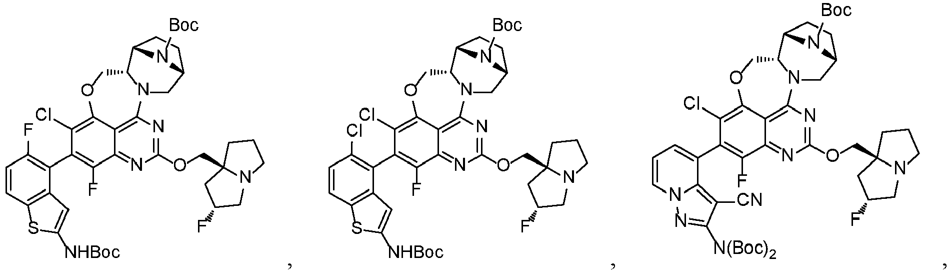 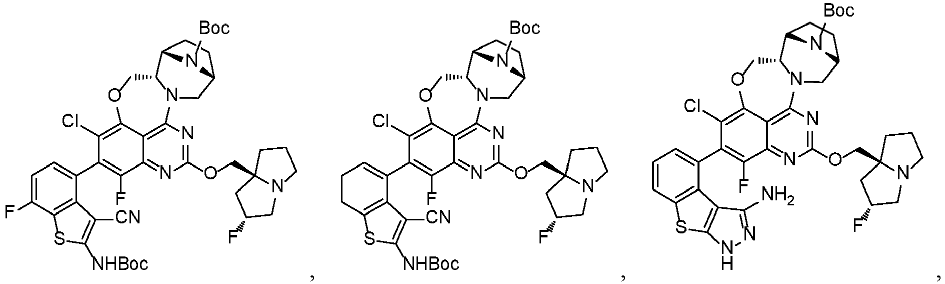 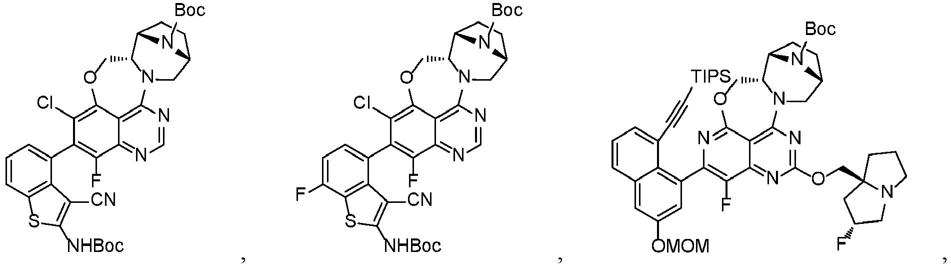 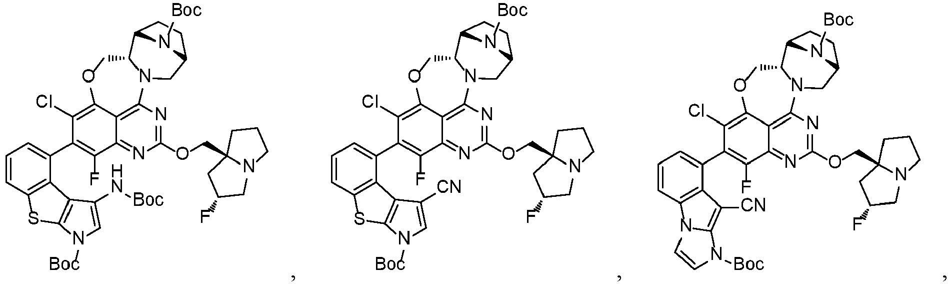 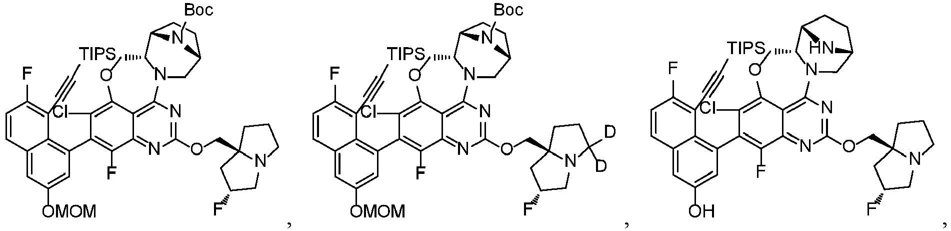 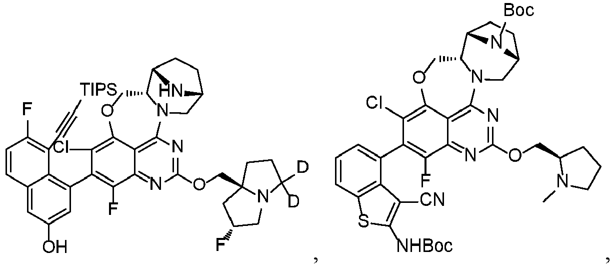 or a pharmaceutically acceptable salt.

19. A pharmaceutical composition, comprising a therapeutically effective amount of an active component and a pharmaceutically acceptable excipient; the active component comprises the compound of formula (I), the stereoisomer thereof, the stable deuterated derivative thereof, or the pharmaceutically acceptable salt thereof according to any one of claims 1 to 16.

20. A use of the compound of formula (I), the stereoisomer thereof, the stable deuterated derivative thereof, or the pharmaceutically acceptable salt thereof according to any one of claims 1 to 16, or the pharmaceutical composition according to claim 19 in the manufacture of a KRAS G12D inhibitor.

21. A use of the compound of formula (I), the stereoisomer thereof, the stable deuterated derivative thereof, or the pharmaceutically acceptable salt thereof according to any one of claims 1 to 16, or the pharmaceutical composition according to claim 19 in the manufacture of a medicament for treating and/or alleviating cancer.

22. The use according to claim 21, wherein the cancer is cancer containing a G12D mutated KRAS gene in tumor cell DNA, and the cancer is preferably one or more than one of pancreatic cancer, endometrial cancer, lung cancer (preferably small cell lung cancer or non-small cell lung cancer), rectal cancer, and colorectal cancer.
